(19) Europäisches Patentamt
European Patent Office
Office européen des brevets

(11) EP 1 845 081 A1

# (12) EUROPEAN PATENT APPLICATION

published in accordance with Art. 158(3) EPC

(43) Date of publication:
**17.10.2007 Bulletin 2007/42**

(21) Application number: **06713085.6**

(22) Date of filing: **31.01.2006**

(51) Int Cl.:
***C07C 235/78*** (2006.01)
***A61K 6/00*** (2006.01)
***A61P 1/00*** (2006.01)
***A61P 3/04*** (2006.01)
***A61P 3/06*** (2006.01)
***A61P 3/10*** (2006.01)
***A61P 9/00*** (2006.01)
***A61P 9/10*** (2006.01)
***A61P 13/12*** (2006.01)
***A61P 15/00*** (2006.01)
***A61P 19/10*** (2006.01)
***A61P 21/00*** (2006.01)
***A61P 25/00*** (2006.01)
***A61P 25/16*** (2006.01)

(86) International application number:
**PCT/JP2006/301942**

(87) International publication number:
**WO 2006/082952 (10.08.2006 Gazette 2006/32)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.02.2005 JP 2005025713**

(71) Applicant: **Takeda Pharmaceutical Company Limited**
**Osaka-shi,**
**Osaka 541-0045 (JP)**

(72) Inventors:
- **OGINO, Masaki,**
  **TAKEDA PHARMACEUTICAL COMPANY LTD.**
  **Osaka-shi, Osaka, 5328686 (JP)**
- **NAKADA, Yoshihisa,**
  **TAKEDA PHARMACEUTICAL COMP. LTD**
  **Osaka-shi, Osaka, 5328686 (JP)**
- **SHIMADA, Mitsuyuki,**
  **TAKEDA PHARMACEUTICAL COMP LTD**
  **Osaka-shi, Osaka, 5328686 (JP)**
- **ASANO, Kouhei,**
  **TAKEDA PHARMACEUTICAL COMPANY LTD**
  **Osaka-shi, Osaka, 5328686 (JP)**
- **TAMURA, Norikazu,**
  **TAKEDA PHARMACEUTICAL COMP. LTD.**
  **Osaka-shi, Osaka, 5328686 (JP)**
- **MASAGO, Minori,**
  **TAKEDA PHARMACEUTICAL COMPANY LTD.**
  **Osaka-shi, Osaka, 5328686 (JP)**

(74) Representative: **Jones, Nicholas Andrew et al**
**Withers & Rogers LLP**
**Goldings House**
**2 Hays Lane**
**London SE1 2HW (GB)**

## (54) AMIDE COMPOUND

(57) The present invention relate to a compound represented by the formula (I) or (II)

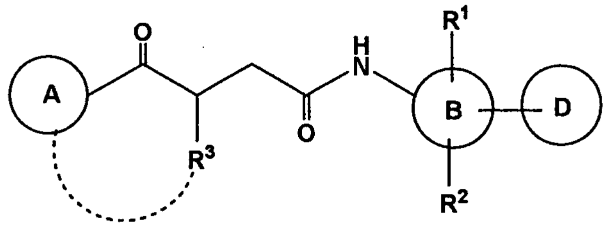

**(I)**

EP 1 845 081 A1

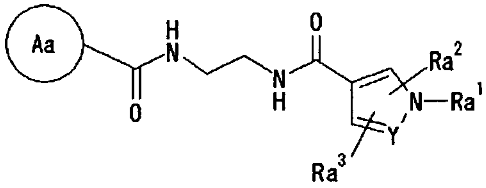 (II)

wherein
ring A is an optionally substituted ring (the ring should not be pyrrolidine, piperidine and piperazine),
ring B is an optionally substituted aromatic ring,
ring D is an optionally substituted ring,
$R^1$ and $R^2$ are each independently a hydrogen atom or a substituent,
$R^3$ is a hydrogen atom or a $C_{1-6}$ alkyl group, or $R^3$ is bonded to ring A to form a non-aromatic ring,
ring Aa is an optionally substituted aromatic hydrocarbon, Y is CH or N,
$Ra^1$ is an optionally substituted hydrocarbon group, and
$Ra^2$ and $Ra^3$ are each independently a hydrogen atom or a substituent,
or a salt thereof.
The present invention provides a compound having a DGAT inhibitory activity, which is useful for the treatment or amelioration of diseases or pathologies caused by high expression or high activation of DGAT.

SEQUENCE LISTING

<110> Takeda Pharmaceutical Company Limited

<120> amide compound

<130> 09870

<150> JP2005-025713

<151> 2005-02-01

<160> 2

<170> PatentIn version 3.1

<210> 1
<211> 66
<212> DNA
<213> Artificial

<220>
<223> primer for cloning human DGAT1-U gene

<400> 1
aattaagaat tcatgggcga ctacaaagac gatgacgacg gcgaccgcgg cagctcccgg 60

cgccgg 66

<210> 2
<211> 45
<212> DNA
<213> Artificial

<220>
<223> primer for cloning human DGAT2-L gene

<400> 2
aattaaacta gttcaggcct ctgccgctgg ggcctcatag ttgag 45

## Description

### Technical Field

The present invention relates to a novel amide compound having a diacylglycerol acyl transferase (hereinafter sometimes to be abbreviated as DGAT in the present specification) inhibitory activity, which is useful for the treatment of obesity, hyperlipidemia, diabetes and the like.

### Background Art

Obesity is a state of excess accumulation of fat, mainly triglyceride, in the body, and is deeply involved in the progression into the pathology such as arteriosclerosis, diabetes, hypertension and the like. Therefore, the development of a drug for the prophylaxis or treatment thereof has been desired. In mammals, two major triglyceride synthesis pathways have been biochemically clarified. One is the glycelophosphoric acid pathway present in all tissues, and the other pathway is a monoglyceride pathway. In any pathway, fatty acid in the cell is converted to acyl coenzyme A by an acyl coenzyme A synthetase and introduced into triglyceride through the both pathways. As the enzyme involved in the final stage of the intracellular or intraorgan triglyceride synthesis process, DGAT has been known. As DGAT, DGAT1 and DGAT2 have been cloned. DGAT1 knockout mice have been created and analyzed. As a result, the mice did not become obese easily with high fat diet and showed promoted energy consumption and insulin sensitivity, as compared to wild-type mice. In a mating test of DGAT1 knockout mice and Ay/a mice, moreover, body weight gain was suppressed with a normal diet and a phenotype of promoted insulin sensitivity and elimination of leptin resistance was shown. Thus, DGAT1 inhibitors are expected to be antiobesity drugs.

DGAT is an enzyme (EC2.3.1.20) also designated as acyl coenzyme A:diacylglycerol acyl transferase. cDNA cloning of DGAT1 is reported in Proc. Natl. Acad. Sci. USA. 95, 13018-13023, 1998, and cDNA cloning of DGAT2 is reported in The Journal of Biochemistry, 276, 42, 38862-38869, 2001 and The Journal of Biochemistry, 276, 42, 38870-38876, 2001. Since the enzyme molecule of DGAT was not clarified for a long time, there is not much finding relating to the DGAT activity. Since the DGAT activity is detected in the endoplasmic reticulum membrane fraction, it was considered to be an endoplasmic reticulum membrane protein. However, ever since cDNA cloning of DGAT was reported, the properties thereof have been rapidly elucidated. For example, it has been reported to be a protein forming a tetramer in Biochem. Journal, 359, 707-714, 2001 etc. A knockout mouse of DGAT1 (DGAT1 defective mouse) was created and its phenotype was reported in Nature Genetics, 25, 87-90, 2000, The Journal of Clinical Investigation, 109, 175-181, 2002 and The Journal of Clinical Investigation, 109, 1049-1055, 2002. From these reports, the DGAT1 inhibitors have been suggested to show an antiobesity action, an anti-insulin resistance action, and an anti-leptin resistance action, and DGAT1 inhibitors are expected to become pharmaceutical products.

In addition, DGAT2 knockout mice were also created and their phenotype is reported in The Journal of Biochemistry, Dec, 10, 1074, 2003 M311000200 as a result, DGAT2 was clarified to be an enzyme that plays a key role in the synthesis of triglyceride in the liver. The Journal of Biochemistry, 274, 35577-35582, 1999 reports that there are a DGAT activity involved in a storage-type triglyceride synthesis in the cytoplasm side of the endoplasmic reticulum membrane and a DGAT activity that supplies triglyceride to be mobilized for lipoprotein secretion in the lumen side, which suggests that DGAT1 and DGAT2 play different roles as triglyceride synthases and further that DGAT2 inhibitors are effective for hypertriglyceridemia.

In addition, since DGAT expression is promoted in various pathologies and diseases such as obesity, diabetes, insulin-resistant diabetes, leptin resistance, arteriosclerosis, hypertriglyceridemia, hypercholesterolemia, arteriosclerosis, hypertension and the like, high expression or hyper activation of DGAT is suggested to be involved in the excess accumulation of triglyceride in the cell, tissue or organ, and closely involved in the onset and aggravation of these diseases.

In, for example, fat organs and adipocytes, expression of DGAT is regulated by hormones such as insulin, leptin and the like, and DGAT is suggested to be deeply involved in the diseases such as insulin resistance, leptin resistance and the like. Therefrom it is considered that a compound having a DGAT inhibitory activity is effective for the treatment of obesity, insulin resistant diabetes, hyperorexia or obesity based on leptin resistance.

As compounds having a DGAT inhibitory action, the following compounds are known.

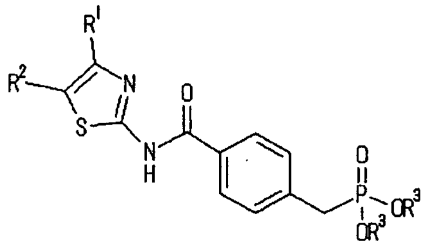

wherein
$R^1$ is phenyl optionally substituted by halogen(s), or the like;
$R^2$ is H, lower alkyl or the like; and $R^3$ is lower alkyl (see, JP-A-2004-67635),

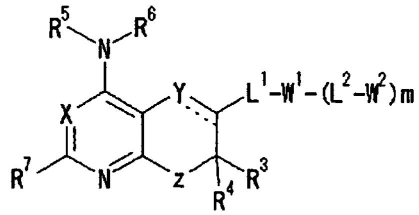

wherein
X is $C(R^1)$ or N; Y is $C(R^1)$, $C(R^2)$ $(R^2)$, N or N $(R^2)$ ; Z is O or S;
$W^1$ and $W^2$ are each optionally substituted cyclo $C_{3-8}$ alkyl, or the like; $L^1$ is a bond, $C_{1-4}$ alkylene, $C_{2-4}$ alkenylene, O or $N(R^a)C(O)$ ;
$L^2$ is a bond, O, $C_{1-4}$ alkylene, $C_{2-4}$ alkenylene, $C_{1-4}$ heteroalkylene or $N(R^a)C(O)$ ; m is 0 or 1; $R^1$, $R^2$, $R^3$ and $R^4$ are each H, $C_{1-8}$ alkyl or the like; $R^5$ and $R^6$ are each H, $C_{1-8}$ alkyl or the like; and $R^7$ is H, $C_{1-8}$ alkyl or the like (see, WO2004/047755),

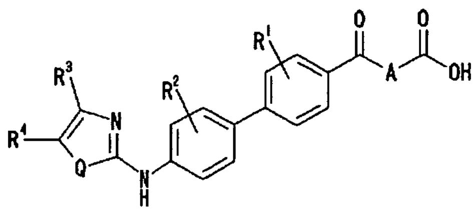

wherein
Q is O, S or $NR^5$; A is

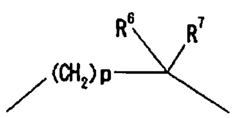
,
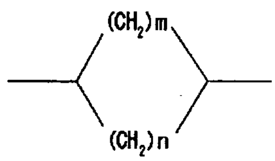

p is 1 or 2; m=0 and n=1 to 4, or m=1 and n=1 to 3; $R^1$ and $R^2$ are each H, halogen or the like; $R^3$ is H, $C_{1-6}$ alkyl optionally substituted by OH, phenyl optionally substituted by $C_{1-6}$ alkyl, or the like; $R^4$ is H, nitro, $C_{1-6}$ alkyl or the like; $R^3$ and $R^4$ in combination optionally form benzene optionally substituted by halogen(s) (see, US 2004/0224997A).

However, none of the above-mentioned prior art reports on the compound of the present invention.

Disclosure of the Invention

There is a demand on the development of a novel compound having a superior DGAT inhibitory activity and

superior in properties (stability, solubility etc.), oral absorbability, migration to target organs and the like.

The present inventors have searched for a compound having a DGAT inhibitory activity, and found that the compounds represented by the below-mentioned formulas (I) and (II) have a superior DGAT inhibitory activity, and are superior in the properties as a pharmaceutical product, such as stability and the like, which resulted in the completion of the present invention.

Accordingly, the present invention relates to

[1] a compound represented by the formula (I):

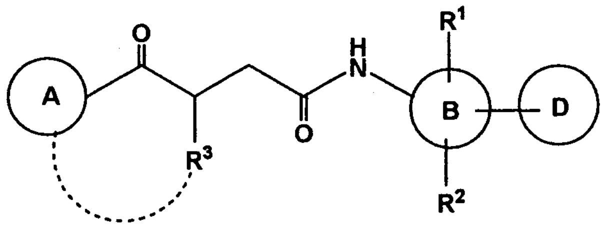

(I)

wherein
ring A is an optionally substituted ring (the ring should not be pyrrolidine, piperidine and piperazine),
ring B is an optionally substituted aromatic ring,
ring D is an optionally substituted ring (ring D should not be a benzene ring substituted by acylalkylcarbonylamino group(s)),
$R^1$ and $R^2$ are each independently a hydrogen atom or a substituent, and
$R^3$ is a hydrogen atom or a $C_{1-6}$ alkyl group, or $R^3$ is bonded to ring A to form a non-aromatic ring,
or a salt thereof,
provided that
N-[4-(4-fluorophenyl)-5-methyl-1,3-thiazol-2-yl]-4-(4-methoxyphenyl)-4-oxobutanamide,
N-[4-(4-methoxyphenyl)-5-methyl-1,3-thiazol-2-yl]-4-oxo-4-phenylbutanamide,
4-(4-tert-butylphenyl)-N-[4-(4-methylphenyl)-5-propyl-1,3-thiazol-2-yl]-4-oxobutanamide,
N-[4-(2,5-dimethoxyphenyl)-5-methyl-1,3-thiazol-2-yl]-4-(6-methyl-2-naphthyl)-4-oxobutanamide,
N-(biphenyl-2-yl)-4-oxo-4-phenylbutanamide,
N-(biphenyl-2-yl)-4-(4-methylphenyl)-4-oxobutanamide,
N-(biphenyl-2-yl)-4-(4-fluorophenyl)-4-oxobutanamide,
N-[4-(4-methylphenyl)-1,3-thiazol-2-yl]-4-oxo-4-.(2-thienyl)butanamide, *
4-(4-chlorophenyl)-N-[4-(4-fluorophenyl)-1,3-thiazol-2-yl]-4-oxobutanamide,
4-(2,5-dimethylphenyl)-N-[4-(4-fluorophenyl)-5-methyl-1,3-thiazol-2-yl]-4-oxobutanamide,
N-[4-(4-fluorophenyl)-5-methyl-1,3-thiazol-2-yl]-4-oxo-4-phenylbutanamide,
4-(4-fluorophenyl)-N-[4-(4-methoxyphenyl)-5-methyl-1,3-thiazol-2-yl]-4-oxobutanamide, and
N-[4-(4-methoxyphenyl)-5-phenyl-1,3-thiazol-2-yl]-4-oxo-4-phenylbutanamide
are excluded (hereinafter to be abbreviated as compound (I));
[2] compound (I) wherein the ring for ring A is a benzene ring, tetrahydronaphthalene or dihydrobenzofuran;
[3] compound (I) wherein the aromatic ring for ring B is a benzene ring, pyridine, pyrimidine, quinoline, isoquinoline, pyrrole, pyrazole, thiophene or thiazole;
[4] compound (I) wherein the ring for ring D is a benzene ring;
[5] compound (I) wherein ring D is a benzene ring optionally substituted by 1 to 3 substituents selected from

(1) a halogen atom;
(2) a $C_{1-6}$ alkyl group optionally substituted by 1 to 3 substituents selected from a hydroxy group, a $C_{1-6}$ alkoxy-carbonyl group, a carboxy group, a cyano group and a non-aromatic heterocyclic group (preferably morpholinyl);
(3) a $C_{2-6}$ alkenyl group optionally substituted by 1 to 3 substituents selected from a $C_{1-6}$ alkoxy-carbonyl group and a carboxy group;
(4) a $C_{1-6}$ alkoxy group optionally substituted by 1 to 3 substituents selected from

(i) a halogen atom,
(ii) a carboxy group,
(iii) a $C_{1-6}$ alkoxy group,

(iv) a $C_{1-6}$ alkoxy-carbonyl group,
(v) an amino group optionally mono- or di-substituted by substituent(s) selected from a $C_{1-6}$ alkyl group and a $C_{1-6}$ alkoxy-carbonyl group, and
(iv) a non-aromatic heterocyclic group (preferably morpholinyl);

(5) a $C_{3-10}$ cycloalkyl group (preferably cyclohexyl);
(6) a $C_{1-3}$ alkylenedioxy group;
(7) a hydroxy group;
(8) a $C_{1-6}$ alkyl-carbonyl group;
(9) a $C_{1-6}$ alkoxy-carbonyl group;
(10) a carboxy group;
(11) a cyano group;
(12) an amino group optionally mono- or di-substituted by $C_{1-6}$ alkyl group(s);
(13) a carbamoyl group; and
(14) a non-aromatic heterocyclic group (preferably morpholinyl);

[6] compound (I) wherein the substituent for $R^1$ or $R^2$ is an optionally substituted hydrocarbon group, an optionally substituted heterocyclic group, an optionally substituted hydroxy group, an acyl group or a halogen atom;
[7] compound (I) which is
N-(5-benzyl-4-phenyl-1,3-thiazol-2-yl)-4-(4-ethoxyphenyl)-4-oxobutanamide;
N-(5-benzyl-4-phenyl-1,3-thiazol-2-yl)-4-oxo-4-phenylbutanamide;
N-(5-benzyl-4-phenyl-1,3-thiazol-2-yl)-4-(3,4-diethoxyphenyl)-4-oxobutanamide;
N-(5-benzyl-4-phenyl-1,3-thiazol-2-yl)-4-(4,5-diethoxy-2-methylphenyl)-4-oxobutanamide;
N-(5-benzyl-4-phenyl-1,3-thiazol-2-yl)-4-(3-ethoxyphenyl)-4-oxobutanamide;
4-(3,4-diethoxyphenyl)-N-(4,6-diphenylpyridin-2-yl)-4-oxobutanamide;
N-(5-benzyl-4-phenyl-1,3-thiazol-2-yl)-4-(3-bromo-4-ethoxyphenyl)-4-oxobutanamide;
N-(5-benzyl-4-phenyl-1,3-thiazol-2-yl)-4-(3-chloro-4-ethoxyphenyl)-4-oxobutanamide;
N-(5-benzyl-4-phenyl-1,3-thiazol-2-yl)-4-(3,4-dimethoxyphenyl)-4-oxobutanamide;
4-(3,4-diethoxyphenyl)-N-[4-(4-hydroxyphenyl)-6-phenylpyridin-2-yl]-4-oxobutanamide;
N-[4-(2-cyanophenyl)-6-phenylpyridin-2-yl]-4-(3,4-diethoxyphenyl)-4-oxobutanamide;
4-(3,4-diethoxyphenyl)-N-[4-(2-hydroxyphenyl)-6-phenylpyridin-2-yl]-4-oxobutanamide;
4-(3,4-diethoxyphenyl)-N-[4-(2-methoxyphenyl)-6-phenylpyridin-2-yl]-4-oxobutanamide; or
4-(3,4-diethoxyphenyl)-N-{4-[2-(hydroxymethyl)phenyl]-6-phenylpyridin-2-yl}-4-oxobutanamide;
[8] a prodrug of compound (I);
[9] a pharmaceutical agent comprising compound (I) or a prodrug thereof;
[10] the pharmaceutical agent of the above-mentioned [9], which is an agent for the prophylaxis or treatment of obesity, hyperlipidemia or diabetes;
[11] a DGAT inhibitor comprising compound (I) or a prodrug thereof;
[12] use of compound (I) or a prodrug thereof for the production of an agent for the prophylaxis or treatment of obesity, hyperlipidemia or diabetes;
[13] use of compound (I) or a prodrug thereof for the production of a DGAT inhibitor;
[14] a method for the prophylaxis or treatment of obesity, hyperlipidemia or diabetes in a mammal, which comprises administering compound (I) or a prodrug thereof to the mammal;
[15] a method of inhibiting DGAT in a mammal, which comprises administering compound (I) or a prodrug thereof to the mammal;
[16] a compound represented by the formula (II):

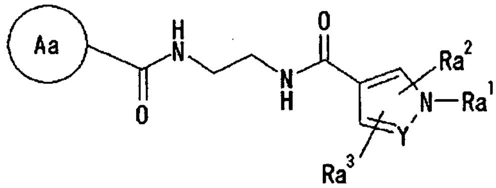
(II)

wherein
ring Aa is an optionally substituted aromatic hydrocarbon,

Y is CH or N,
$Ra^1$ is an optionally substituted hydrocarbon group, and
$Ra^2$ and $Ra^3$ are each independently a hydrogen atom or a substituent,
or a salt thereof (hereinafter to be abbreviated as compound (II)) ;
[17] compound (II) wherein the substituent for $Ra^2$ or $Ra^3$ is an optionally substituted hydrocarbon group;
[18] compound (II) which is
N-{2-[(4-ethoxybenzoyl)amino]ethyl}-1-phenyl-3-(trifluoromethyl)-1H-pyrazole-4-carboxamide;
N-{2-[(4-ethoxybenzoyl)amino]ethyl}-1-(4-methoxyphenyl)-2-methyl-5-phenyl-1H-pyrrole-3-carboxamide; or
N-{2-[(4-ethoxybenzoyl)amino]ethyl}-2-methyl-1-[4-(methylthio)phenyl]-5-phenyl-1H-pyrrole-3-carboxamide;
[19] a prodrug of compound (II);
[20] a pharmaceutical agent comprising compound (II) or a prodrug thereof;
[21] the pharmaceutical agent of the above-mentioned [20], which is an agent for the prophylaxis or treatment of obesity, hyperlipidemia or diabetes;
[22] a DGAT inhibitor comprising compound (II) or a prodrug thereof;
[23] use of compound (II) or a prodrug thereof for the production of an agent for the prophylaxis or treatment of obesity, hyperlipidemia or diabetes;
[24] use of compound (II) or a prodrug thereof for the production of a DGAT inhibitor;
[25] a method for the prophylaxis or treatment of obesity, hyperlipidemia or diabetes in a mammal, which comprises administering compound (II) or a prodrug thereof to the mammal;
[26] a method of inhibiting DGAT in a mammal, which comprises administering compound (II) or a prodrug thereof to the mammal; and the like.

The compound (I) and compound (II) (these are also collectively referred to as the compound of the present invention in this specification) have a DGAT inhibitory activity and are useful for the treatment or amelioration of diseases or pathologies caused by high expression or high activation of DGAT (sometimes to be abbreviated as DGAT-related diseases in this specification).

**Brief Description of the Drawings**

Fig. 1 shows the effect of the compound of Example 4 for the [$^{14}$C]-oleic acid intake by TG in an adipose tissue of KKAy mice (n=3). * p≤0.025, Williams test
Fig. 2 shows the effect of the compound of Example 4 for the [$^{14}$C]-oleic acid intake by TG in a skeletal muscle of KKAy mice (n=3). * p≤0.025, Williams test

**Best Mode for Embodying the Invention**

In the present specification, unless otherwise specified, the "halogen atom" means fluorine atom, chlorine atom, bromine atom or iodine atom.
In the present specification, unless otherwise specified, the "$C_{1-3}$ alkylenedioxy group" means methylenedioxy, ethylenedioxy, trimethylenedioxy or the like.
In the present specification, unless otherwise specified, the "$C_{1-6}$ alkyl group" means methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl or the like.
In the present specification, unless otherwise specified, the "$C_{1-6}$ alkoxy group" means methoxy, ethoxy, propoxy, isopropoxy, butoxy, isobutoxy, sec-butoxy, tert-butoxy or the like.
In the present specification, unless otherwise specified, the "$C_{1-6}$ alkoxy-carbonyl group" means methoxycarbonyl, ethoxycarbonyl, propoxycarbonyl, tert-butoxycarbonyl or the like.
In the present specification, unless otherwise specified, the "$C_{1-6}$ alkyl-carbonyl group" means acetyl, propanoyl, butanoyl, isobutanoyl, pentanoyl, isopentanoyl, hexanoyl or the like.
As the "substituent" for $R^1$ or $R^2$, an "optionally substituted hydrocarbon group", an "optionally substituted heterocyclic group", an "optionally substituted hydroxy group", an "optionally substituted amino group", an "optionally substituted thiol group", a "cyano group", a "nitro group", an "acyl group", a "halogen atom" and the like can be mentioned.
As the "hydrocarbon group" of the aforementioned "optionally substituted hydrocarbon group", for example, a $C_{1-10}$ alkyl group, a $C_{2-10}$ alkenyl group, a $C_{2-10}$ alkynyl group, a $C_{3-10}$ cycloalkyl group, a $C_{3-10}$ cycloalkenyl group, a $C_{4-10}$ cycloalkadienyl group, a $C_{6-14}$ aryl group, a $C_{7-13}$ aralkyl group, a $C_{8-13}$ arylalkenyl group, a $C_{3-10}$ cycloalkyl-$C_{1-6}$ alkyl group and the like can be mentioned.

As used herein, as the $C_{1-10}$ alkyl group, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like can be mentioned.

As the $C_{2-10}$ alkenyl group, for example, ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octeny and the like can be mentioned.

As the $C_{2-10}$ alkynyl group, for example, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like can be mentioned.

As the $C_{3-10}$ cycloalkyl group, for example, cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo [4.2.1]nonyl, bicyclo[4.3.1]decyl, adamantyl and the like can be mentioned.

As the $C_{3-10}$ cycloalkenyl group, for example, 2-cyclopenten-1-yl, 3-cyclopenten-1-yl, 2-cyclohexen-1-yl, 3-cyclohexen-1-yl and the like can be mentioned.

As the $C_{4-10}$ cycloalkadienyl group, for example, 2,4-cyclopentadien-1-yl, 2,4-cyclohexadien-1-yl, 2,5-cyclohexadien-1-yl and the like can be mentioned.

The above-mentioned $C_{3-10}$ cycloalkyl group, $C_{3-10}$ cycloalkenyl group and $C_{4-10}$ cycloalkadienyl group are each optionally condensed with a benzene ring to form a fused cyclic group, and as the fused cyclic group, for example, indanyl, dihydronaphthyl, tetrahydronaphthyl, fluorenyl and the like can be mentioned.

As the $C_{6-14}$ aryl group, for example, phenyl, naphthyl, anthryl, phenanthryl, acenaphthylenyl, biphenylyl and the like can be mentioned. Of these, phenyl, 1-naphthyl, 2-naphthyl and the like are preferable.

As the $C_{7-13}$ aralkyl group, for example, benzyl, phenethyl, naphthylmethyl, biphenylylmethyl and the like can be mentioned.

As the $C_{8-13}$ arylalkenyl group, for example, styryl and the like can be mentioned.

As the $C_{3-10}$ cycloalkyl-$C_{1-6}$ alkyl group, for example, cyclohexylmethyl and the like can be mentioned.

The $C_{1-10}$ alkyl group, $C_{2-10}$ alkenyl group and $C_{2-10}$ alkynyl group exemplified as the aforementioned "hydrocarbon group" optionally have 1 to 3 substituents at substitutable positions.

As such substituents, for example,

(1) a $C_{3-10}$ cycloalkyl group (e.g., cyclopropyl, cyclohexyl);
(2) a $C_{6-14}$ aryl group (e.g., phenyl, naphthyl) optionally substituted by 1 to 3 substituents selected from a $C_{1-6}$ alkyl group optionally substituted by 1 to 3 halogen atoms, a hydroxy group, a $C_{1-6}$ alkoxy group and a halogen atom;
(3) an aromatic heterocyclic group (e.g., thienyl, furyl, pyridyl, oxazolyl, thiazolyl, tetrazolyl, oxadiazolyl, pyrazinyl, quinolyl, indolyl) optionally substituted by 1 to 3 substituents selected from a $C_{1-6}$ alkyl group optionally substituted by 1 to 3 halogen atoms, a hydroxy group, a $C_{1-6}$ alkoxy group and a halogen atom;
(4) a non-aromatic heterocyclic group (e.g., tetrahydrofuryl, morpholinyl, thiomorpholinyl, piperidinyl, pyrrolidinyl, piperazinyl, dioxolyl, dioxolanyl, 1,3-dihydro-2-benzofuranyl, thiazolidinyl) optionally substituted by 1 to 3 substituents selected from a $C_{1-6}$ alkyl group optionally substituted by 1 to 3 halogen atoms, a hydroxy group, a $C_{1-6}$ alkoxy group, an oxo group and a halogen atom;
(5) an amino group optionally mono- or di-substituted by substituent(s) selected from a $C_{1-6}$ alkyl group, a $C_{1-6}$ alkyl-carbonyl group and a $C_{1-6}$ alkoxy-carbonyl group;
(6) a $C_{1-6}$ alkylsulfonylamino group (e.g., methylsulfonylamino);
(7) an amidino group;
(8) a $C_{1-6}$ alkyl-carbonyl group optionally substituted by 1 to 3 halogen atoms;
(9) a $C_{1-6}$ alkoxy-carbonyl group optionally substituted by 1 to 3 halogen atoms;
(10) a $C_{1-6}$ alkylsulfonyl group (e.g., methylsulfonyl) optionally substituted by 1 to 3 halogen atoms;
(11) a carbamoyl group optionally mono or di-substituted by $C_{1-6}$ alkyl group(s) optionally substituted by 1 to 3 halogen atoms;
(12) a thiocarbamoyl group optionally mono or di-substituted by $C_{1-6}$ alkyl group(s) optionally substituted by 1 to 3 halogen atoms;
(13) a sulfamoyl group optionally mono or di-substituted by $C_{1-6}$ alkyl group(s) optionally substituted by 1 to 3 halogen atoms;
(14) a carboxy group;
(15) a hydroxy group;
(16) a $C_{1-6}$ alkoxy group optionally substituted by 1 to 3 substituents selected from

(i) a halogen atom,
(ii) a carboxy group,

(iii) a $C_{1-6}$ alkoxy group,
(iv) a $C_{1-6}$ alkoxy-carbonyl group,
(v) an amino group optionally substituted by 1 to 3 substituents selected from a $C_{1-6}$ alkyl group and a $C_{1-6}$ alkoxy-carbonyl group, and
(vi) a non-aromatic heterocyclic group (e.g., morpholinyl);

(17) a $C_{2-6}$ alkenyloxy group (e.g., ethenyloxy) optionally substituted by 1 to 3 halogen atoms;
(18) a $C_{3-10}$ cycloalkyloxy group (e.g., cyclohexyloxy);
(19) a $C_{7-13}$ aralkyloxy group (e.g., benzyloxy);
(20) a $C_{6-14}$ aryloxy group (e.g., phenyloxy, naphthyloxy);
(21) a $C_{1-6}$ alkyl-carbonyloxy group (e.g., acetyloxy, tert-butylcarbonyloxy);
(22) a thiol group;
(23) a $C_{1-6}$ alkylthio group (e.g., methylthio, ethylthio) optionally substituted by 1 to 3 halogen atoms;
(24) a $C_{7-13}$ aralkylthio group (e.g., benzylthio);
(25) a $C_{6-14}$ arylthio group (e.g., phenylthio, naphthylthio);
(26) a sulfo group;
(27) a cyano group;
(28) an azido group;
(29) a nitro group;
(30) a nitroso group;
(31) a halogen atom;
(32) a $C_{1-6}$ alkylsulfinyl group (e.g., methylsulfinyl);
(33) a $C_{1-3}$ alkylenedioxy group;
and the like can be mentioned.

The $C_{3-10}$ cycloalkyl group, $C_{3-10}$ cycloalkenyl group, $C_{4-10}$ cycloalkadienyl group, $C_{6-14}$ aryl group, $C_{7-13}$ aralkyl group, $C_{8-13}$ arylalkenyl group and $C_{3-10}$ cycloalkyl-$C_{1-6}$ alkyl group exemplified as the aforementioned "hydrocarbon group" optionally have 1 to 3 substituents at substitutable positions.
As such substituents, for example,

(1) those exemplified as the substituents of the aforementioned $C_{1-10}$ alkyl group and the like;
(2) a $C_{1-6}$ alkyl group optionally substituted by 1 to 3 substituents selected from a halogen atom, a carboxy group, a hydroxy group, a $C_{1-6}$ alkoxy-carbonyl group, a $C_{1-6}$ alkyl-carbonyloxy group (e.g., acetyloxy), a carbamoyl group, a cyano group and a non-aromatic heterocyclic group (e.g., morpholinyl);
(3) a $C_{2-6}$ alkenyl group (e.g., ethenyl, 1-propenyl) optionally substituted by 1 to 3 substituents selected from a halogen atom, a carboxy group, a $C_{1-6}$ alkoxy-carbonyl group (e.g., methoxycarbonyl, ethoxycarbonyl) and a carbamoyl group;
(4) a $C_{7-13}$ aralkyl group (e.g., benzyl) optionally substituted by 1 to 3 substituents selected from a $C_{1-6}$ alkyl group optionally substituted by 1 to 3 halogen atoms, a hydroxy group, a $C_{1-6}$ alkoxy group and a halogen atom;
(5) an oxo group; and the like can be mentioned.

As the "heterocyclic group" of the aforementioned "optionally substituted heterocyclic group", an aromatic heterocyclic group and a non-aromatic heterocyclic group can be mentioned.
As used herein, as the aromatic heterocyclic group, for example, a 5- to 7-membered monocyclic aromatic heterocyclic group containing, as a ring-constituting atom besides carbon atoms, 1 to 4 heteroatoms selected from an oxygen atom, a sulfur atom and a nitrogen atom, and a fused aromatic heterocyclic group can be mentioned. As the fused aromatic heterocyclic group, for example, a group wherein these 5- to 7- membered monocyclic aromatic heterocyclic groups and 1 or 2 rings selected from a 5-or 6-membered ring containing 1 or 2 nitrogen atoms, a 5-membered ring containing one sulfur atom and a benzene ring are fused, and the like can be mentioned.
As preferable examples of the aromatic heterocyclic group, monocyclic aromatic heterocyclic groups such as furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl, 6-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), thiazolyl (e.g., 2-thiazolyl, 4-thiazolyl, 5-thiazolyl), isothiazolyl (e.g., 4-isothiazolyl), oxazolyl (e.g., 2-oxazolyl, 4-oxazolyl, 5-oxazolyl), isoxazolyl, oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,3-triazol-1-yl, 1,2,3-triazol-2-yl, 1,2,3-triazol-4-yl), tetrazolyl (e.g., tetrazol-1-yl, tetrazol-5-yl), triazinyl (e.g., 1,2,4-triazol-1-yl, 1,2,4-triazol-3-yl) and the like; fused aromatic heterocyclic groups such as quinolyl (e.g., 2-quinolyl, 3-quinolyl,

4-quinolyl, 6-quinolyl), isoquinolyl (e.g., 3-isoquinolyl), quinazolyl (e.g., 2-quinazolyl, 4-quinazolyl), quinoxalyl (e.g., 2-quinoxalyl, 6-quinoxalyl), benzofuryl (e.g., 2-benzofuryl, 3-benzofuryl), benzothienyl (e.g., 2-benzothienyl, 3-benzothienyl), benzoxazolyl (e.g., 2-benzoxazolyl), benzisoxazolyl (e.g., 7-benzisoxazolyl), benzothiazolyl (e.g., 2-benzothiazolyl), benzimidazolyl (e.g., benzimidazol-1-yl, benzimidazol-2-yl, benzimidazol-5-yl), benzotriazolyl (e.g., 1H-1,2,3-benzotriazol-5-yl), indolyl (e.g., indol-1-yl, indol-2-yl, indol-3-yl, indol-5-yl), indazolyl (e.g., 1H-indazol-3-yl), pyrrolopyrazinyl (e.g., 1H-pyrrolo[2,3-b]pyrazin-2-yl, 1H-pyrrolo[2,3-b]pyrazin-6-yl), imidazopyridinyl (e.g., 1H-imidazo[4,5-b]pyridin-2-yl, 1H-imidazo[4,5-c]pyridin-2-yl, 2H-imidazo[1,2-a]pyridin-3-yl), imidazopyrazinyl (e.g., 1H-imidazo[4,5-b]pyrazin-2-yl), pyrazolopyridinyl (e.g., 1H-pyrazolo[4,3-c]pyridin-3-yl), pyrazolothienyl (e.g., 2H-pyrazolo[3,4-b]thiophen-2-yl), pyrazolotriazinyl (e.g., pyrazolo[5,1-c] [1,2,4] triazin-3-yl) and the like; and the like can be mentioned.

As the non-aromatic heterocyclic group, for example, a 5-to 7-membered monocyclic non-aromatic heterocyclic group containing, as a ring-constituting atom besides carbon atoms, 1 to 4 heteroatoms selected from an oxygen atom, a sulfur atom and a nitrogen atom, and a fused non-aromatic heterocyclic group can be mentioned. As the fused non-aromatic heterocyclic group, for example, a group wherein these 5- to 7- membered monocyclic non-aromatic heterocyclic groups and 1 or 2 rings selected from a 5-or 6-membered ring containing 1 or 2 nitrogen atoms, a 5-membered ring containing one sulfur atom and a benzene ring are fused, and the like can be mentioned.

As preferable examples of the non-aromatic heterocyclic group, pyrrolidinyl (e.g., 1-pyrrolidinyl), piperidinyl (e.g., piperidino), morpholinyl (e.g., morpholino), thiomorpholinyl (e.g., thiomorpholino), piperazinyl (e.g., 1-piperazinyl), hexamethyleniminyl (e.g., hexamethyleniminyl-1-yl), oxazolidinyl (e.g., oxazolidin-3-yl), thiazolidinyl (e.g., thiazolidin-3-yl), imidazolidinyl (e.g., imidazolidin-3-yl), dihydroisoindolyl (e.g., 1,3-dihydro-2H-isoindol-2-yl), dioxolyl (e.g., 1,3-dioxol-4-yl), dioxolanyl (e.g., 1,3-dioxolan-4-yl), dihydrooxadiazolyl (e.g., 4,5-dihydro-1,2,4-oxadiazol-3-yl), 2-thioxo-1,3-oxazolidin-5-yl, tetrahydropyranyl (e.g., 4-tetrahydropyranyl), dihydrobenzofuranyl (e.g., 2,3-dihydro-l-benzofuran-5-yl), dihydrobenzodioxine (e.g., 2,3-dihydro-1,4-benzodioxine), dihydrobenzodioxepine (e.g., 3,4-dihydro-2H-1,5-benzodioxepine), 4,5,6,7-tetrahydro-1-benzofuranyl (e.g., 4,5,6,7-tetrahydro-1-benzofuran-3-yl), indanyl (e.g., indan-5-yl), chromenyl (e.g., 4H-chromen-2-yl, 2H-chromen-3-yl), dihydroisoquinolinyl (e.g., 1,2-dihydroisoquinolin-4-yl), tetrahydroisoquinolinyl (e.g., 1,2,3,4-tetrahydroisoquinolin-4-yl), dihydrophthalazinyl (e.g., 1,4-dihydrophthalazin-4-yl), pyrazolidinyl (e.g., pyrazolidin-1-yl), tetrahydroquinolinyl (e.g., 1, 2, 3, 4-tetrahydroquinolin-4-yl) and the like can be mentioned.

The "heterocyclic group" of the aforementioned "optionally substituted heterocyclic group" optionally has 1 to 3 substituents at substitutable positions. As such substituents, for example, those exemplified as the substituents which the $C_{3-10}$ cycloalkyl group and the like exemplified as the "hydrocarbon group" of the aforementioned "optionally substituted hydrocarbon group" optionally has, can be mentioned.

As the "optionally substituted hydroxy group", for example, a hydroxy group optionally substituted by a substituent selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group, a $C_{1-6}$ alkyl-carbonyl group, a 5 or 6-membered aromatic heterocyclic group, a fused aromatic heterocyclic group and the like, each of which is optionally substituted, can be mentioned.

As used herein, as the $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group and $C_{8-13}$ arylalkenyl group, those exemplified as the "hydrocarbon group" of the aforementioned "optionally substituted hydrocarbon group" can be mentioned.

As the 5 or 6-membered aromatic heterocyclic group, a 5 or 6-membered cyclic group, from among the "aromatic heterocyclic groups" exemplified as the "heterocyclic group" of the aforementioned "optionally substituted heterocyclic group", can be mentioned.

As the fused aromatic heterocyclic group, a fused cyclic group, from among the "aromatic heterocyclic groups" exemplified as the "heterocyclic group" of the aforementioned "optionally substituted heterocyclic group", can be mentioned.

The aforementioned $C_{1-10}$ alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group, $C_{8-13}$ arylalkenyl group, $C_{1-6}$ alkyl-carbonyl group, 5 or 6-membered aromatic heterocyclic group and fused aromatic heterocyclic group optionally have 1 to 3 substituents at substitutable positions.

As the substituents of the $C_{1-10}$ alkyl group, $C_{2-10}$ alkenyl group and $C_{1-6}$ alkyl-carbonyl group, those exemplified as the substituents which the $C_{1-10}$ alkyl and the like exemplified as the "hydrocarbon group" of the aforementioned "optionally substituted hydrocarbon group" optionally has, can be mentioned.

As the substituents of the $C_{3-10}$ cycloalkyl group, $C_{3-10}$ cycloalkenyl group, $C_{6-14}$ aryl group, $C_{7-13}$ aralkyl group, $C_{8-13}$ arylalkenyl group, 5 or 6-membered aromatic heterocyclic group and fused aromatic heterocyclic group, those exemplified as the substituents which the $C_{3-10}$ cycloalkyl and the like exemplified as the "hydrocarbon group" of the aforementioned "optionally substituted hydrocarbon group" optionally has, can be mentioned.

As the aforementioned "optionally substituted thiol group", for example, a thiol group optionally substituted by a substituent selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group, a $C_{8-13}$ arylalkenyl group, a $C_{1-6}$ alkyl-carbonyl group, a 5 or 6-membered aromatic heterocyclic group, a fused aromatic heterocyclic group and the like, each of which is optionally substituted,

can be mentioned.

As the substituents, those exemplified as the substituents of the aforementioned "optionally substituted hydroxy group" can be mentioned.

As the aforementioned "optionally substituted amino group", for example, an amino group optionally substituted by 1 or 2 substituents selected from a $C_{1-10}$ alkyl group, a $C_{2-10}$ alkenyl group, a $C_{3-10}$ cycloalkyl group, a $C_{3-10}$ cycloalkenyl group, a $C_{6-14}$ aryl group, a $C_{7-13}$ aralkyl group and a $C_{8-13}$ arylalkenyl group, each of which is optionally substituted; an acyl group and the like, can be mentioned.

As used herein, as the $C_{1-10}$ alkyl group, $C_{2-10}$ alkenyl group, $C_{3-10}$ cycloalkyl group, $C_{3-10}$ cycloalkenyl group, $C_{6-14}$ aryl group, aralkyl group and $C_{8-13}$ arylalkenyl group, those exemplified as the "hydrocarbon group" of the aforementioned "optionally substituted hydrocarbon group" can be mentioned.

The aforementioned $C_{1-10}$, alkyl group, $C_{2-10}$ alkenyl group, $C_{3-10}$ cycloalkyl group, $C_{3-10}$ cycloalkenyl group, $C_{6-14}$ aryl group, $C_{7-13}$ aralkyl group and $C_{8-13}$ arylalkenyl group optionally have 1 to 3 substituents at substitutable positions.

As the substituents of the $C_{1-10}$ alkyl group and $C_{2-10}$ alkenyl group, those exemplified as the substituents which the $C_{1-10}$ alkyl and the like exemplified as the "hydrocarbon group" of the aforementioned "optionally substituted hydrocarbon group" optionally has, can be mentioned.

As the substituents of the $C_{3-10}$ cycloalkyl group, $C_{3-10}$ cycloalkenyl group, $C_{6-14}$ aryl group, $C_{7-13}$ aralkyl group and $C_{8-13}$ arylalkenyl group, those exemplified as the substituents which the $C_{3-10}$ cycloalkyl and the like exemplified as the "hydrocarbon group" of the aforementioned "optionally substituted hydrocarbon group" optionally has, can be mentioned.

As the "acyl group" exemplified as the substituent of the "optionally substituted amino group", those similar to the "acyl group" below, which is exemplified as the "substituent" for $R^1$ or $R^2$, can be mentioned.

As the "acyl group" which is exemplified as the "substituent" for $R^1$ or $R^2$, for example, a group represented by the formula: $-COR^a$, $-CO-OR^a$, $-SO_3R^a$, $-SO_2R^a$, $-SOR^a$, $-CO-NR^{a'}R^{b'}$ or $-CS-NR^{a'}R^{b'}$ wherein $R^a$ is a hydrogen atom, an optionally substituted hydrocarbon group or an optionally substituted heterocyclic group, and $R^{a'}$ and $R^{b'}$ are the same or different and each is a hydrogen atom, an optionally substituted hydrocarbon group or an optionally substituted heterocyclic group, or $R^{a'}$ and $R^{b'}$ optionally form, together with the adjacent nitrogen atom, an optionally substituted nitrogen-containing heterocycle, and the like can be mentioned.

As the "optionally substituted hydrocarbon group" and "optionally substituted heterocyclic group" for $R^a$, $R^{a'}$ or $R^{b'}$, those similar to the "optionally substituted hydrocarbon group" and "optionally substituted heterocyclic group", which are exemplified as the "substituent" for $R^1$ or $R^2$, can be mentioned.

As the "nitrogen-containing heterocycle" of the "optionally substituted nitrogen-containing heterocycle" formed by $R^{a'}$ and $R^{b'}$ together with the adjacent nitrogen atom, for example, a 5- to 7-membered nitrogen-containing heterocycle containing, as a ring-constituting atom besides carbon atoms, at least one nitrogen atom and optionally further containing one or two heteroatoms selected from an oxygen atom, a sulfur atom and a nitrogen atom can be mentioned. As preferable examples of the nitrogen-containing heterocycle, pyrrolidine, imidazolidine, pyrazolidine, piperidine, piperazine, morpholine, thiomorpholine, oxopiperazine and the like can be mentioned.

The nitrogen-containing heterocycle optionally has 1 to 3 (preferably 1 or 2) substituents at substitutable positions. As such substituents, those exemplified as the substituents which the $C_{3-10}$ cycloalkyl group and the like exemplified as the "hydrocarbon group" of the aforementioned "optionally substituted hydrocarbon group" optionally has, can be mentioned.

As preferable examples of the "acyl group",

(1) a formyl group;
(2) a carboxy group;
(3) a carbamoyl group;
(4) a $C_{1-6}$ alkyl-carbonyl group;
(5) a $C_{1-6}$ alkoxy-carbonyl group optionally substituted by 1 to 3 substituents selected from a carboxy group, a carbamoyl group, a thiocarbamoyl group, a $C_{1-6}$ alkoxy-carbonyl group and a $C_{1-6}$ alkyl-carbonyloxy group (e.g., methoxycarbonyl, ethoxycarbonyl, propoxycarbonyl, tert-butoxycarbonyl; carboxymethoxycarbonyl, carboxyethoxycarbonyl, carboxybutoxycarbonyl; carbamoylmethoxycarbonyl; thiocarbamoylmethoxycarbonyl; ethoxycarbonylmethoxycarbonyl, ethoxycarbonylethoxycarbonyl, methoxycarbonylbutoxycarbonyl, ethoxycarbonylbutoxycarbonyl; tert-butylcarbonyloxymethoxycarbonyl);
(6) a $C_{3-10}$ cycloalkyl-carbonyl group (e.g., cyclopentylcarbonyl, cyclohexylcarbonyl);
(7) a $C_{6-14}$ aryl-carbonyl group (e.g., benzoyl, 1-naphthoyl, 2-naphthoyl) optionally substituted by 1 to 3 substituents selected from a halogen atom, a cyano group, a $C_{1-6}$ alkyl group optionally substituted by 1 to 3 halogen atoms, a $C_{1-6}$ alkoxy group, a carboxy group, a $C_{1-6}$ alkoxy-carbonyl group, an aromatic heterocyclic group (e.g., tetrazolyl, oxadiazolyl), a non-aromatic heterocyclic group (e.g., oxooxadiazolyl) and a carbamoyl group;

(8) a $C_{6-14}$ aryloxy-carbonyl group (e.g., phenyloxycarbonyl, naphthyloxycarbonyl) optionally substituted by 1 to 3 substituents selected from a carboxy group, a $C_{1-6}$ alkoxy-carbonyl group and a carbamoyl group;
(9) a $C_{7-13}$ aralkyloxy-carbonyl group optionally substituted by 1 to 3 substituents selected from a carboxy group, a carbamoyl group, a thiocarbamoyl group, a $C_{1-6}$ alkoxy-carbonyl group, a halogen atom, a cyano group, a nitro group, a $C_{1-6}$ alkoxy group, a $C_{1-6}$ alkylsulfonyl group and a $C_{1-6}$ alkyl group (e.g., benzyloxycarbonyl, phenethyloxycarbonyl; carboxybenzyloxycarbonyl; methoxycarbonylbenzyloxycarbonyl; biphenylylmethoxycarbonyl);
(10) a carbamoyl group mono or di-substituted by $C_{1-6}$ alkyl group(s) optionally substituted by 1 to 3 substituents selected from a halogen atom and a $C_{1-6}$ alkoxy group (e.g., methylcarbamoyl, ethylcarbamoyl, dimethylcarbamoyl, diethylcarbamoyl, ethylmethylcarbamoyl, propylcarbamoyl, isopropylcarbamoyl, butylcarbamoyl, isobutylcarbamoyl, trifluoroethylcarbamoyl, N-methoxyethyl-N-methylcarbamoyl);
(11) a $C_{1-6}$ alkylsulfonyl group optionally substituted by 1 to 3 substituents selected from a carboxy group, a carbamoyl group and a $C_{1-6}$ alkoxy-carbonyl group (e.g., methylsulfonyl, carboxymethylsulfonyl);
(12) a $C_{1-6}$ alkylsulfinyl group (e.g., methylsulfinyl);
(13) a thiocarbamoyl group;
(14) a $C_{7-13}$ aralkyl-carbonyl group (e.g., benzylcarbonyl, phenethylcarbonyl);
(15) an aromatic heterocyclyl (e.g., furyl, thienyl, oxazolyl, thiazolyl, isoxazolyl, isothiazolyl, pyrazolyl, pyridyl, pyrazinyl, benzofuryl, benzothienyl, quinoxalinyl)-carbonyl group (e.g., furylcarbonyl, thienylcarbonyl, thiazolylcarbonyl, pyrazolylcarbonyl, pyridylcarbonyl, pyrazinylcarbonyl, benzofurylcarbonyl, benzothienylcarbonyl, quinoxalinylcarbonyl) optionally substituted by 1 to 3 substituents selected from a $C_{1}$-$_{6}$alkyl group, a $C_{6-14}$ aryl group, a $C_{7-13}$ aralkyl group, a $C_{1-6}$ alkoxy group, a carboxy group, a $C_{1-6}$ alkoxy-carbonyl group and a carbamoyl group;
and the like can be mentioned.

$R^1$ and $R^2$ are each preferably a hydrogen atom, an optionally substituted hydrocarbon group, an optionally substituted heterocyclic group, an optionally substituted hydroxy group, an acyl group or a halogen atom, more preferably an optionally substituted $C_{1-10}$ alkyl group, an optionally substituted $C_{2-10}$ alkenyl group, an optionally substituted $C_{6-14}$ aryl group, an optionally substituted $C_{7-13}$ aralkyl group, an optionally substituted aromatic heterocyclic group, an optionally substituted hydroxy group, an acyl group or a halogen atom. Particularly preferably, one of $R^1$ and $R^2$ is a hydrogen atom or a $C_{1-6}$ alkoxy-carbonyl group, and the other is

(1) a hydrogen atom;
(2) a $C_{1-10}$ alkyl group (preferably a $C_{1-6}$ alkyl group) optionally substituted by 1 to 3 substituents selected from

(i) a $C_{3-10}$ cycloalkyl group,
(ii) a non-aromatic heterocyclic group (preferably morpholinyl),
(iii) a $C_{1-6}$ alkoxy-carbonyl group, and
(iv) a carboxy group;

(3) a $C_{2-10}$ alkenyl group (preferably a $C_{2-6}$ alkenyl group) optionally substituted by 1 to 3 substituents selected from

(i) a $C_{1-6}$ alkoxy-carbonyl group,
(ii) a carboxy group, and
(iii) a cyano group;

(4) a $C_{7-13}$ aralkyl group (preferably benzyl) optionally substituted by 1 to 3 substituents selected from

(i) a halogen atom, and
(ii) a $C_{2-6}$ alkenyl group optionally substituted by $C_{1-6}$ alkoxy-carbonyl group(s);

(5) a $C_{6-14}$ aryl group (preferably phenyl) optionally substituted by 1 to 3 substituents selected from

(i) a halogen atom,
(ii) a $C_{1-10}$ alkyl group optionally substituted by 1 to 3 substituents selected from a hydroxy group, a $C_{1-6}$ alkoxy-carbonyl group, a carboxy group, a cyano group and a non-aromatic heterocyclic group (preferably morpholinyl),
(iii) a $C_{2-10}$ alkenyl group optionally substituted by 1 to 3 substituents selected from a $C_{1-6}$ alkoxy-carbonyl group and a carboxy group,
(iv) a $C_{3-10}$ cycloalkyl group (preferably cyclohexyl),
(v) a $C_{1-6}$ alkoxy group optionally substituted by 1 to 3 halogen atoms,
(vi) a $C_{1-3}$ alkylenedioxy group,

(vii) a hydroxy group,
(viii) a $C_{1-6}$ alkyl-carbonyl group,
(ix) a $C_{1-6}$ alkoxy-carbonyl group,
(x) a carboxy group,
(xi) a cyano group,
(xii) an amino group optionally mono- or di-substituted by $C_{1-6}$ alkyl group (s),
(xiii) a carbamoyl group, and
(xiv) a non-aromatic heterocyclic group (preferably morpholinyl);

(6) a $C_{1-10}$ alkoxy group (preferably a $C_{1-6}$ alkoxy group) optionally substituted by $C_{1-6}$ alkoxy group (s) ;
(7) a $C_{7-13}$ aralkyloxy group (preferably benzyloxy) ;
(8) a $C_{1-6}$ alkoxy-carbonyl group;
(9) a $C_{6-14}$ aryl-carbonyl group (preferably benzoyl) optionally substituted by $C_{1-6}$ alkyl group(s) ;
(10) a halogen atom; or
(11) an aromatic heterocyclic group (preferably pyridyl, furyl, pyrrolyl, pyrazolyl, indolyl) optionally substituted by 1 to 3 substituents selected from

(i) a $C_{1-6}$ alkyl group, and
(ii) a $C_{1-6}$ alkoxy-carbonyl group.

Most preferably, one of $R^1$ and $R^2$ is a hydrogen atom, and the other is a $C_{7-13}$ aralkyl group (preferably benzyl) or a $C_{6-14}$ aryl group (preferably phenyl).
As the "$C_{1-6}$ alkyl group" for $R^3$, for example, methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl and the like can be mentioned.
As the "non-aromatic ring" formed by $R^3$ and ring A, a "non-aromatic cyclic hydrocarbon" and a "non-aromatic heterocycle" can be mentioned.
As the aforementioned "non-aromatic cyclic hydrocarbon", for example, a $C_{3-10}$ cycloalkane, a $C_{3-10}$ cycloalkene, a $C_{4-10}$ cycloalkadine and the like, each of which is optionally condensed with a benzene ring, can be mentioned. As the $C_{3-10}$ cycloalkane, $C_{3-10}$ cycloalkene and $C_{4-10}$ cycloalkadine, rings corresponding to the $C_{3-10}$ cycloalkyl group, $C_{3-10}$ cycloalkenyl group and $C_{4-10}$ cycloalkadienyl group exemplified as the "substituent" for $R^1$ or $R^2$, can be mentioned.
As the aforementioned "non-aromatic heterocycle", a ring corresponding to the non-aromatic heterocyclic group exemplified as the "substituent" for $R^1$ or $R^2$, can be mentioned.
$R^3$ is preferably a hydrogen atom, or bonded to ring A to form a non-aromatic ring, more preferably a hydrogen atom, or bonded to ring A to form a non-aromatic cyclic hydrocarbon (preferably a $C_{3-10}$ cycloalkane), particularly preferably a hydrogen atom.
As the "ring (the ring should not be pyrrolidine, piperidine and piperazine)" of the "optionally substituted ring (the ring should not be pyrrolidine, piperidine and piperazine)" for ring A, for example, an "aromatic hydrocarbon", a "non-aromatic cyclic hydrocarbon", an "aromatic heterocycle", a "non-aromatic heterocycle (excluding pyrrolidine, piperidine and piperazine)" and the like can be mentioned.
As the aforementioned "aromatic hydrocarbon", for example, a $C_{6-14}$ aromatic hydrocarbon can be mentioned. As the $C_{6-14}$ aromatic hydrocarbon, a ring corresponding to the $C_{6-14}$ aryl group exemplified as the "substituent" for $R^1$ or $R^2$, can be mentioned. The aromatic hydrocarbon can be bonded to the carbon atom of the adjacent carbonyl group at any bondable position.
As the aforementioned "non-aromatic cyclic hydrocarbon", for example, a $C_{3-10}$ cycloalkane, a $C_{3-10}$ cycloalkene, a $C_{4-10}$ cycloalkadine and the like, each of which is optionally condensed with a benzene ring, can be mentioned. As the $C_{3-10}$ cycloalkane, $C_{3-10}$ cycloalkene and $C_{4-10}$ cycloalkadine, rings corresponding to the $C_{3-10}$ cycloalkyl group, $C_{3-10}$ cycloalkenyl group and $C_{4-10}$ cycloalkadienyl group exemplified as the "substituent" for $R^1$ or $R^2$, can be mentioned. The non-aromatic cyclic hydrocarbon can be bonded to the carbon atom of the adjacent carbonyl group at any bondable position.
As the aforementioned "aromatic heterocycle", a ring corresponding to the aromatic heterocyclic group exemplified as the "substituent" for $R^1$ or $R^2$, can be mentioned. The aromatic heterocycle can be bonded to the carbon atom of the adjacent carbonyl group at any bondable position.
As the aforementioned "non-aromatic heterocycle (excluding pyrrolidine, piperidine and piperazine)", rings other than pyrrolidine, piperidine and piperazine, from among the rings corresponding to the non-aromatic heterocyclic groups exemplified as the "substituent" for $R^1$ or $R^2$, can be mentioned. The non-aromatic heterocycle can be bonded to the carbon atom of the adjacent carbonyl group at any bondable position.
As the "ring (the ring should not be pyrrolidine, piperidine and piperazine)" of the "optionally substituted ring

(the ring should not be pyrrolidine, piperidine and piperazine)" for ring A, an aromatic hydrocarbon (particularly a benzene ring), a non-aromatic cyclic hydrocarbon (e.g., tetrahydronaphthalene) and a non-aromatic heterocycle (excluding pyrrolidine, piperidine and piperazine, e.g., dihydrobenzofuran) are preferable, a benzene ring, tetrahydronaphthalene and dihydrobenzofuran are more preferable, and a benzene ring is particularly preferable.

The "ring (the ring should not be pyrrolidine, piperidine and piperazine)" of the "optionally substituted ring (the ring should not be pyrrolidine, piperidine and piperazine)" for ring A optionally has 1 to 3 substituents at substitutable positions. As such substituents, for example, those similar to the substituents of the $C_{3-10}$ cycloalkyl group and the like exemplified as the "substituent" for $R^1$ or $R^2$, can be mentioned.

As the substituents of ring A,

(1) a halogen atom;
(2) a hydroxy group;
(3) a $C_{1-6}$ alkyl group optionally substituted by 1 to 3 substituents selected from a carboxy group, a hydroxy group, a $C_{1-6}$ alkoxy-carbonyl group and a $C_{1-6}$ alkyl-carbonyloxy group;
(4) a $C_{2-6}$ alkenyl group optionally substituted by 1 to 3 substituents selected from a carboxy group and a $C_{1-6}$ alkoxy-carbonyl group;
(5) a $C_{1-6}$ alkoxy group optionally substituted by 1 to 3 substituents selected from a halogen atom, a carboxy group, a $C_{1-6}$ alkoxy group and a $C_{1-6}$ alkoxy-carbonyl group; and
(6) a $C_{1-3}$ alkylenedioxy group; are preferable.

Ring A is preferably an aromatic hydrocarbon (particularly preferably a benzene ring), a non-aromatic cyclic hydrocarbon (preferably tetrahydronaphthalene) or a non-aromatic heterocycle (excluding pyrrolidine, piperidine and piperazine, preferably dihydrobenzofuran), each of which is optionally substituted by 1 to 3 substituents selected from the above-mentioned substituents (1) to (6), more preferably, an aromatic hydrocarbon (particularly preferably a benzene ring) optionally substituted by 1 to 3 substituents selected from the above-mentioned substituents (1) to (6).

Ring A is particularly preferably a benzene ring optionally substituted by 1 to 3 substituents selected from

(1) a halogen atom;
(2) a $C_{1-6}$ alkyl group; and
(3) a $C_{1-6}$ alkoxy group.

As the "aromatic ring" of the "optionally substituted aromatic ring" for ring B, an "aromatic hydrocarbon" and an "aromatic heterocycle" can be mentioned.

As the aforementioned "aromatic hydrocarbon", for example, a $C_{6-14}$ aromatic hydrocarbon can be mentioned. As the $C_{6-14}$ aromatic hydrocarbon, a ring corresponding to the $C_{6-14}$ aryl group exemplified as the "substituent" for $R^1$ or $R^2$, can be mentioned. The aromatic hydrocarbon can be bonded to the nitrogen atom of the adjacent amide group and ring D at any bondable position.

As the aforementioned "aromatic heterocycle", a ring corresponding to the aromatic heterocyclic group exemplified as the "substituent" for $R^1$ or $R^2$, can be mentioned. The aromatic heterocycle can be bonded to the nitrogen atom of the adjacent amide group and ring D at any bondable position.

The "aromatic ring" of the "optionally substituted aromatic ring" for ring B optionally has 1 to 3 substituents at substitutable positions. As such substituents, for example, those (excluding an oxo group) similar to the substituents of the $C_{3-10}$ cycloalkyl group and the like exemplified as the "substituent" for $R^1$ or $R^2$, can be mentioned.

Ring B is preferably an aromatic hydrocarbon (particularly a benzene ring), or a 5 or 6-membered aromatic heterocycle or a fused ring thereof with a benzene ring, more preferably a benzene ring, pyridine, pyrimidine, quinoline, isoquinoline, pyrrole, pyrazole, thiophene or thiazole. Ring B is particularly preferably pyridine or thiazole.

As the "ring" of the "optionally substituted ring" for ring D, for example, an "aromatic hydrocarbon", a "non-aromatic cyclic hydrocarbon", an "aromatic heterocycle", a "non-aromatic heterocycle" and the like can be mentioned.

As the aforementioned "aromatic hydrocarbon", for example, a $C_{6-14}$ aromatic hydrocarbon can be mentioned. As the $C_{6-14}$ aromatic hydrocarbon, a ring corresponding to the $C_{6-14}$ aryl group exemplified as the "substituent" for $R^1$ or $R^2$, can be mentioned. The aromatic hydrocarbon can be bonded to adjacent ring B at any bondable position.

As the aforementioned "non-aromatic cyclic hydrocarbon", for example, a $C_{3-10}$ cycloalkane, a $C_{3-10}$ cycloalkene, a $C_{4-10}$ cycloalkadine and the like, each of which is optionally condensed with a benzene ring, can be mentioned. As the $C_{3-10}$ cycloalkane, $C_{3-10}$ cycloalkene and $C_{4-10}$ cycloalkadine, rings corresponding to the $C_{3-10}$ cycloalkyl group, $C_{3-10}$ cycloalkenyl group and $C_{4-10}$ cycloalkadienyl group exemplified as the "substituent" for $R^1$ or $R^2$, can be mentioned. The non-aromatic cyclic hydrocarbon can be bonded to adjacent ring B at any bondable position.

As the aforementioned "aromatic heterocycle", a ring corresponding to the aromatic heterocyclic group exemplified as the "substituent" for $R^1$ or $R^2$, can be mentioned. The aromatic heterocycle can be bonded to adjacent ring B

at any bondable position.

As the aforementioned "non-aromatic heterocycle", a ring corresponding to the non-aromatic heterocyclic group exemplified as the "substituent" for $R^1$ or $R^2$, can be mentioned. The non-aromatic heterocycle can be bonded to adjacent ring B at any bondable position.

As the "ring" of the "optionally substituted ring" for ring D, an aromatic hydrocarbon (particularly a benzene ring), a 5 or 6-membered aromatic heterocycle (e.g., pyridine) and a 5 or 6-membered non-aromatic heterocycle (e.g., piperidine) are preferable, and a benzene ring is particularly preferable.

The "ring" of the "optionally substituted ring" for ring D optionally has 1 to 3 substituents at substitutable positions. As such substituents, for example, those similar to the substituents of the $C_{3-10}$ cycloalkyl group and the like exemplified as the "substituent" for $R^1$ or $R^2$, can be mentioned.

Ring D should not be a benzene ring substituted by acylalkylcarbonylamino group(s).

As the substituents of ring D,

(1) a halogen atom;
(2) a $C_{1-6}$ alkyl group optionally substituted by 1 to 3 substituents selected from a hydroxy group, a $C_{1-6}$ alkoxy-carbonyl group, a carboxy group, a cyano group and a non-aromatic heterocyclic group (preferably morpholinyl);
(3) a $C_{2-6}$ alkenyl group optionally substituted by 1 to 3 substituents selected from a $C_{1-6}$ alkoxy-carbonyl group and a carboxy group;
(4) a $C_{1-6}$ alkoxy group optionally substituted by 1 to 3 substituents selected from

 (i) a halogen atom,
 (ii) a carboxy group,
 (iii) a $C_{1-6}$ alkoxy group,
 (iv) a $C_{1-6}$ alkoxy-carbonyl group,
 (v) an amino group optionally mono- or di-substituted by substituent(s) selected from a $C_{1-6}$ alkyl group and a $C_{1-6}$ alkoxy-carbonyl group, and
 (iv) a non-aromatic heterocyclic group (preferably morpholinyl);

(5) a $C_{3-10}$ cycloalkyl group (preferably cyclohexyl);
(6) a $C_{1-3}$ alkylenedioxy group;
(7) a hydroxy group;
(8) a $C_{1-6}$ alkyl-carbonyl group;
(9) a $C_{1-6}$ alkoxy-carbonyl group;
(10) a carboxy group;
(11) a cyano group;
(12) an amino group optionally mono- or di-substituted by $C_{1-6}$ alkyl group(s);
(13) a carbamoyl group; and
(14) a non-aromatic heterocyclic group (preferably morpholinyl); are preferable.

Ring D is preferably, an aromatic hydrocarbon (particularly preferably a benzene ring), a 5 or 6-membered aromatic heterocycle (preferably pyridine) or a 5 or 6-membered non-aromatic heterocycle (preferably piperidine), each of which is optionally substituted by 1 to 3 substituents selected from the above-mentioned substituents (1) to (14), more preferably an aromatic hydrocarbon (particularly preferably a benzene ring) optionally substituted by 1 to 3 substituents selected from the above-mentioned substituents (1) to (14).

Ring D is particularly preferably a benzene ring optionally substituted by 1 to 3 substituents selected from,

(1) a $C_{1-6}$ alkyl group optionally substituted by hydroxy group(s);
(2) a $C_{1-6}$ alkoxy group;
(3) a hydroxy group; and
(4) a cyano group.

As the "optionally substituted hydrocarbon group" for $Ra^1$, those similar to the "optionally substituted hydrocarbon group" exemplified as the "substituent" for $R^1$ or $R^2$, can be mentioned.

$Ra^1$ is preferably an optionally substituted $C_{6-14}$ aryl group or an optionally substituted $C_{7-13}$ aralkyl group. $Ra^1$ is more preferably

(1) a $C_{6-14}$ aryl group (preferably phenyl) optionally substituted by 1 to 3 substituents selected from

(i) a $C_{1-6}$ alkoxy group optionally substituted by 1 to 3 halogen atoms, and
(ii) a $C_{1-6}$ alkylthio group; or

(2) a $C_{7-13}$ aralkyl group (preferably benzyl) .

As the "substituent" for $Ra^2$ or $Ra^3$, those similar to the "substituent" for $R^1$ or $R^2$, can be mentioned. Of these, an optionally substituted hydrocarbon group is preferable.

$Ra^2$ and $Ra^3$ are each preferably a hydrogen atom or an optionally substituted hydrocarbon group, more preferably, a hydrogen atom, an optionally substituted $C_{1-10}$ alkyl group or an optionally substituted $C_{6-14}$ aryl group (preferably phenyl). $Ra^2$ and $Ra^3$ are each particularly preferably

(1) a hydrogen atom;
(2) a $C_{1-10}$ alkyl group optionally substituted by 1 to 3 halogen atoms; or
(3) a $C_{6-14}$ aryl group (preferably phenyl).

As the "optionally substituted aromatic hydrocarbon" for ring Aa, an aromatic hydrocarbon, form among the "optionally substituted aromatic rings" for ring B, can be mentioned. The "aromatic hydrocarbon" of the "optionally substituted aromatic hydrocarbon" can be bonded to the carbon atom of the adjacent carbonyl group at any bondable position.

Ring Aa is preferably an aromatic hydrocarbon (particularly preferably a benzene ring) optionally substituted by 1 to 3 substituents selected from a $C_{1-6}$ alkoxy group, a hydroxy group, a halogen atom and a $C_{1-6}$ alkyl group, more preferably an aromatic hydrocarbon (particularly preferably a benzene ring) optionally substituted by 1 to 3 $C_{1-6}$ alkoxy groups, particularly preferably a benzene ring.

Compound (I) does not contain N-[4-(4-fluorophenyl)-5-methyl-1,3-thiazol-2-yl]-4-(4-methoxyphenyl)-4-oxobutanamide,
N-[4-(4-methoxyphenyl)-5-methyl-1,3-thiazol-2-yl]-4-oxo-4-phenylbutanamide,
4-(4-tert-butylphenyl)-N-[4-(4-methylphenyl)-5-propyl-1,3-thiazol-2-yl]-4-oxobutanamide,
N-[4-(2,5-dimethoxyphenyl)-5-methyl-1,3-thiazol-2-yl]-4-(6-methyl-2-naphthyl)-4-oxobutanamide,
N-(biphenyl-2-yl)-4-oxo-4-phenylbutanamide,
N-(biphenyl-2-yl)-4-(4-methylphenyl)-4-oxobutanamide,
N-(biphenyl-2-yl)-4-(4-fluorophenyl)-4-oxobutanamide,
N-[4-(4-methylphenyl)-1,3-thiazol-2-yl]-4-oxo-4-(2-thienyl)butanamide,
4-(4-chlorophenyl)-N-[4-(4-fluorophenyl)-1,3-thiazol-2-yl]-4-oxobutanamide,
4-(2,5-dimethylphenyl)-N-[4-(4-fluorophenyl)-5-methyl-1,3-thiazol-2-yl]-4-oxobutanamide,
N-[4-(4-fluorophenyl)-5-methyl-1,3-thiazol-2-yl]-4-oxo-4-phenylbutanamide,
4-(4-fluorophenyl)-N-[4-(4-methoxyphenyl)-5-methyl-1,3-thiazol-2-yl]-4-oxobutanamide, and
N-[4-(4-methoxyphenyl)-5-phenyl-1,3-thiazol-2-yl]-4-oxo-4-phenylbutanamide.

Preferable examples of compound (I) is as follow.
[Compound I-A]
a compound wherein
one of $R^1$ and $R^2$ is a hydrogen atom, and the other is

(1) a hydrogen atom;
(2) a $C_{1-10}$ alkyl group optionally substituted by 1 to 3 substituents selected from

(i) a $C_{3-10}$ cycloalkyl group,
(ii) a non-aromatic heterocyclic group (preferably morpholinyl),
(iii) a $C_{1-6}$ alkoxy-carbonyl group, and
(iv) a carboxy group;

(3) a $C_{7-13}$ aralkyl group optionally substituted by 1 to 3 substituents selected from

(i) a halogen atom, and
(ii) a $C_{2-6}$ alkenyl group optionally substituted by $C_{1-6}$ alkoxy-carbonyl group(s);

(4) a $C_{6-14}$ aryl group optionally substituted by 1 to 3 halogen atoms;
(5) a $C_{1-10}$ alkoxy group;
(6) a $C_{7-13}$ aralkyloxy group;

(7) a $C_{1-6}$ alkoxy-carbonyl group; or
(8) a halogen atom;
$R^3$ is a hydrogen atom, or bonded to ring A to form a non-aromatic cyclic hydrocarbon (preferably a $C_{3-10}$ cycloalkane); ring A is

(1) an aromatic hydrocarbon (particularly preferably a benzene ring) optionally substituted by 1 to 3 substituents selected from

(i) a halogen atom,
(ii) a hydroxy group,
(iii) a $C_{1-6}$ alkyl group optionally substituted by 1 to 3 substituents selected from a carboxy group, a hydroxy group, a $C_{1-6}$ alkoxy-carbonyl group and a $C_{1-6}$ alkyl-carbonyloxy group,
(iv) a $C_{2-6}$ alkenyl group optionally substituted by 1 to 3 substituents selected from a carboxy group and a $C_{1-6}$ alkoxy-carbonyl group, and
(v) a $C_{1-6}$ alkoxy group optionally substituted by 1 to 3 substituents selected from a halogen atom, a carboxy group, a $C_{1-6}$ alkoxy group and a $C_{1-6}$ alkoxy-carbonyl group;

(2) a non-aromatic cyclic hydrocarbon (preferably tetrahydronaphthalene); or
(3) a non-aromatic heterocycle (excluding pyrrolidine, piperidine and piperazine, preferably dihydrobenzofuran); ring B is

(1) an aromatic hydrocarbon (particularly preferably a benzene ring); or
(2) a 5 or 6-membered aromatic heterocycle or a fused ring thereof with a benzene ring (preferably pyridine, pyrimidine, quinoline, pyrrole, pyrazole, thiophene, thiazole); and ring D is a benzene ring optionally substituted by 1 to 3 substituents selected from

(i) a halogen atom,
(ii) a hydroxy group, and
(iii) a $C_{1-6}$ alkoxy group optionally substituted by 1 to 3 substituents selected from a carboxy group, a $C_{1-6}$ alkoxy group and a $C_{1-6}$ alkoxy-carbonyl group.

[Compound I-B]
a compound wherein
one of $R^1$ and $R^2$ is a hydrogen atom or a $C_{1-6}$ alkoxy-carbonyl group, and the other is

(1) a hydrogen atom;
(2) a $C_{1-10}$ alkyl group (preferably a $C_{1-6}$ alkyl group) optionally substituted by 1 to 3 substituents selected from

(i) a $C_{3-10}$ cycloalkyl group,
(ii) a non-aromatic heterocyclic group (preferably morpholinyl),
(iii) a $C_{1-6}$ alkoxy-carbonyl group, and
(iv) a carboxy group;

(3) a $C_{2-10}$ alkenyl group (preferably a $C_{2-6}$ alkenyl group) optionally substituted by 1 to 3 substituents selected from

(i) a $C_{1-6}$ alkoxy-carbonyl group,
(ii) a carboxy group, and
(iii) a cyano group;

(4) a $C_{7-13}$ aralkyl group (preferably benzyl) optionally substituted by 1 to 3 substituents selected from

(i) a halogen atom, and
(ii) a $C_{2-6}$ alkenyl group optionally substituted by $C_{1-6}$ alkoxy-carbonyl group(s);

(5) a $C_{6-14}$ aryl group (preferably phenyl) optionally substituted by 1 to 3 substituents selected from

(i) a halogen atom,
(ii) a $C_{1-10}$ alkyl group optionally substituted by 1 to 3 substituents selected from a hydroxy group, a $C_{1-6}$ alkoxy-

carbonyl group, a carboxy group, a cyano group and a non-aromatic heterocyclic group (preferably morpholinyl),
(iii) a $C_{2-10}$ alkenyl group optionally substituted by 1 to 3 substituents selected from a $C_{1-6}$ alkoxy-carbonyl group and a carboxy group,
(iv) a $C_{3-10}$ cycloalkyl group (preferably cyclohexyl),
(v) a $C_{1-6}$ alkoxy group optionally substituted by 1 to 3 halogen atoms,
(vi) a $C_{1-3}$ alkylenedioxy group,
(vii) a hydroxy group,
(viii) a $C_{1-6}$ alkyl-carbonyl group,
(ix) a $C_{1-6}$ alkoxy-carbonyl group,
(x) a carboxy group,
(xi) a cyano group,
(xii) an amino group optionally mono- or di-substituted by $C_{1-6}$ alkyl group(s),
(xiii) a carbamoyl group, and
(xiv) a non-aromatic heterocyclic group (preferably morpholinyl);

(6) a $C_{1-10}$ alkoxy group (preferably a $C_{1-6}$ alkoxy group) optionally substituted by $C_{1-6}$ alkoxy group(s);
(7) a $C_{7-13}$ aralkyloxy group (preferably benzyloxy);
(8) a $C_{1-6}$ alkoxy-carbonyl group;
(9) a $C_{6-14}$ aryl-carbonyl group (preferably benzoyl) optionally substituted by $C_{1-6}$ alkyl group(s);
(10) a halogen atom; or
(11) an aromatic heterocyclic group (preferably pyridyl, furyl, pyrrolyl, pyrazolyl, indolyl) optionally substituted by 1 to 3 substituents selected from

(i) a $C_{1-6}$ alkyl group, and
(ii) a $C_{1-6}$ alkoxy-carbonyl group;

$R^3$ is a hydrogen atom, or bonded to ring A to form a non-aromatic cyclic hydrocarbon (preferably a $C_{3-10}$ cycloalkane);
ring A is

(1) an aromatic hydrocarbon (particularly preferably a benzene ring);
(2) a non-aromatic cyclic hydrocarbon (preferably tetrahydronaphthalene); or
(3) a non-aromatic heterocycle (excluding pyrrolidine, piperidine and piperazine, preferably dihydrobenzofuran), each of which is optionally substituted by 1 to 3 substituents selected from

(i) a halogen atom;
(ii) a hydroxy group;
(iii) a $C_{1-6}$ alkyl group optionally substituted by 1 to 3 substituents selected from a carboxy group, a hydroxy group, a $C_{1-6}$ alkoxy-carbonyl group and a $C_{1-6}$ alkyl-carbonyloxy group;
(iv) a $C_{2-6}$ alkenyl group optionally substituted by 1 to 3 substituents selected from a carboxy group and a $C_{1-6}$ alkoxy-carbonyl group;
(v) a $C_{1-6}$ alkoxy group optionally substituted by 1 to 3 substituents selected from a halogen atom, a carboxy group, a $C_{1-6}$ alkoxy group and a $C_{1-6}$ alkoxy-carbonyl group; and
(vi) a $C_{1-3}$ alkylenedioxy group;

ring B is

(1) an aromatic hydrocarbon (particularly preferably a benzene ring); or
(2) a 5 or 6-membered aromatic heterocycle or a fused ring thereof with a benzene ring (preferably pyridine, pyrimidine, quinoline, isoquinoline, pyrrole, pyrazole, thiophene, thiazole); and

ring D is

(1) an aromatic hydrocarbon (particularly preferably a benzene ring);
(2) a 5 or 6-membered aromatic heterocycle (preferably pyridine); or
(3) a 5 or 6-membered non-aromatic heterocycle(preferably piperidine),
each of which is optionally substituted by 1 to 3 substituents selected from

(i) a halogen atom;
(ii) a $C_{1-6}$ alkyl group optionally substituted by 1 to 3 substituents selected from a hydroxy group, a $C_{1-6}$ alkoxy-carbonyl group, a carboxy group, a cyano group and a non-aromatic heterocyclic group (preferably morpholinyl);
(iii) a $C_{2-6}$ alkenyl group optionally substituted by 1 to 3 substituents selected from a $C_{1-6}$ alkoxy-carbonyl group and a carboxy group;
(iv) a $C_{1-6}$ alkoxy group optionally substituted by 1 to 3 substituents selected from

(a) a halogen atom,
(b) a carboxy group,
(c) a $C_{1-6}$ alkoxy group,
(d) a $C_{1-6}$ alkoxy-carbonyl group,
(e) an amino group optionally mono- or di-substituted by substituent(s) selected from a $C_{1-6}$ alkyl group and a $C_{1-6}$ alkoxy-carbonyl group, and
(f) a non-aromatic heterocyclic group (preferably morpholinyl);

(v) a $C_{3-10}$ cycloalkyl group (preferably cyclohexyl);
(vi) a $C_{1-3}$ alkylenedioxy group;
(vii) a hydroxy group;
(viii) a $C_{1-6}$ alkyl-carbonyl group;
(ix) a $C_{1-6}$ alkoxy-carbonyl group;
(x) a carboxy group;
(xi) a cyano group;
(xii) an amino group optionally mono- or di-substituted by $C_{1-6}$ alkyl group (s) ;
(xiii) a carbamoyl group; and
(xiv) a non-aromatic heterocyclic group (preferably morpholinyl).

[Compound I-C]
The above-mentioned [compound I-B] wherein one of $R^1$ and $R^2$ is a hydrogen atom, and the other is a $C_{7-13}$ aralkyl group (preferably benzyl) or a $C_{6-14}$ aryl group (preferably phenyl);
$R^3$ is a hydrogen atom;
ring A is a benzene ring optionally substituted by 1 to 3 substituents selected from

(1) a halogen atom;
(2) a $C_{1-6}$ alkyl group; and
(3) a $C_{1-6}$ alkoxy group; ring B is pyridine or thiazole; and

ring D is a benzene ring optionally substituted by 1 to 3 substituents selected from

(1) a $C_{1-6}$ alkyl group optionally substituted by hydroxy group(s);
(2) a $C_{1-6}$ alkoxy group;
(3) a hydroxy group; and
(4) a cyano group.

[Compound I-D]
N-(5-benzyl-4-phenyl-1,3-thiazol-2-yl)-4-(4-ethoxyphenyl)-4-oxobutanamide (Example 1);
N-(5-benzyl-4-phenyl-1,3-thiazol-2-yl)-4-oxo-4-phenylbutanamide (Example 2);
N-(5-benzyl-4-phenyl-1,3-thiazol-2-yl)-4-(3,4-diethoxyphenyl)-4-oxobutanamide (Example 3);
N-(5-benzyl-4-phenyl-1,3-thiazol-2-yl)-4-(4,5-diethoxy-2-methylphenyl)-4-oxobutanamide (Example 4);
N-(5-benzyl-4-phenyl-1,3-thiazol-2-yl)-4-(3-ethoxyphenyl)-4-oxobutanamide (Example 5);
4-(3,4-diethoxyphenyl)-N-(4,6-diphenylpyridin-2-yl)-4-oxobutanamide (Example 11);
N-(5-benzyl-4-phenyl-1,3-thiazol-2-yl)-4-(3-bromo-4-ethoxyphenyl)-4-oxobutanamide (Example 25);
N-(5-benzyl-4-phenyl-1,3-thiazol-2-yl)-4-(3-chloro-4-ethoxyphenyl)-4-oxobutanamide (Example 36);
N-(5-benzyl-4-phenyl-1,3-thiazol-2-yl)-4-(3,4-dimethoxyphenyl)-4-oxobutanamide (Example 71);
4-(3,4-diethoxyphenyl)-N-[4-(4-hydroxyphenyl)-6-phenylpyridin-2-yl]-4-oxobutanamide (Example 123);
N-[4-(2-cyanophenyl)-6-phenylpyridin-2-yl]-4-(3,4-diethoxyphenyl)-4-oxobutanamide (Example 144);
4-(3,4-diethoxyphenyl)-N-[4-(2-hydroxyphenyl)-6-phenylpyridin-2-yl]-4-oxobutanamide (Example 145); 4-(3,4-diethoxyphenyl)-N-[4-(2-methoxyphenyl)-6-phenylpyridin-2-yl]-4-oxobutanamide (Example 146); or
4-(3,4-diethoxyphenyl)-N-{4-[2-(hydroxymethyl)phenyl]-6-phenylpyridin-2-yl}-4-oxobutanamide (Example 147).

Preferable examples of compound (II) is as follows.
[Compound II-A]
a compound wherein
$Ra^1$ is

(1) a $C_{6-14}$ aryl group (preferably phenyl) optionally substituted by 1 to 3 substituents selected from

(i) a $C_{1-6}$ alkoxy group optionally substituted by 1 to 3 halogen atoms, and
(ii) a $C_{1-6}$ alkylthio group; or

(2) a $C_{7-13}$ aralkyl group (preferably benzyl);
$Ra^2$ and $Ra^3$ are each

(1) a hydrogen atom;
(2) a $C_{1-10}$ alkyl group optionally substituted by 1 to 3 halogen atoms; or
(3) a $C_{6-14}$ aryl group (preferably phenyl); ring Aa is an aromatic hydrocarbon (particularly preferably a benzene ring) optionally substituted by 1 to 3 $C_{1-6}$ alkoxy group(s); and
Y is CH or N;

[Compound II-B]
N-{2-[(4-ethoxybenzoyl)amino]ethyl}-1-phenyl-3-(trifluoromethyl)-1H-pyrazole-4-carboxamide (Example 61);
N-{2-[(4-ethoxybenzoyl)amino]ethyl}-1-(4-methoxyphenyl)-2-methyl-5-phenyl-1H-pyrrole-3-carboxamide (Example 64); or
N-{2-[(4-ethoxybenzoyl)amino]ethyl}-2-methyl-1-[4-(methylthio)phenyl]-5-phenyl-1H-pyrrole-3-carboxamide (Example 65).

As a salt of the compound of the present invention, a pharmacologically acceptable salt is preferable. Examples of such a salt include a salt with inorganic base, a salt with organic base, a salt with inorganic acid, a salt with organic acid, a salt with basic or acidic amino acid and the like.

Preferable examples of the salt with inorganic base include alkali metal salts such as sodium salt, potassium salt and the like; alkaline earth metal salts such as calcium salt, magnesium salt and the like; aluminum salt; ammonium salt and the like.

Preferable examples of the salt with organic base include a salt with trimethylamine, triethylamine, pyridine, picoline, ethanolamine, diethanolamine, triethanolamine, tromethamine[tris(hydroxymethyl)methylamine], tert-butylamine, cyclohexylamine, benzylamine, dicyclohexylamine, N,N-dibenzylethylenediamine or the like.

Preferable examples of the salt with inorganic acid include a salt with hydrochloric acid, hydrobromic acid, nitric acid, sulfuric acid, phosphoric acid or the like.

Preferable examples of the salt with organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid or the like.

Preferable examples of the salt with basic amino acid include a salt with arginine, lysine, ornithine or the like.

Preferable examples of the salt with acidic amino acid include a salt with aspartic acid, glutamic acid or the like.

A prodrug of the compound of the present invention is a compound that converts to the compound of the present invention due to the reaction by enzyme, gastric acid and the like under the physiological conditions in the body; that is, a compound that converts to the compound of the present invention by enzymatic oxidation, reduction, hydrolysis and the like, and a compound that converts to the compound of the present invention by hydrolysis and the like by gastric acid and the like. Examples of a prodrug of the compound of the present invention include a compound wherein an amino group of the compound of the present invention is acylated, alkylated or phosphorylated (e.g., a compound where an amino group of the compound of the present invention is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated or tert-butylated, and the like); a compound wherein a hydroxy group of the compound of the present invention is acylated, alkylated, phosphorylated or borated (e.g., a compound where a hydroxy group of the compound of the present invention is acetylated, palmitoylated, propanoylated, pivaloylated, succinylated, fumarylated, alanylated or dimethylaminomethylcarbonylated, and the like); a compound wherein a carboxyl group of the compound of the present invention is esterified or amidated (e.g., a compound where a carboxyl group of the compound of the present invention is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified or methylamidated, and the like) and the like. These compounds can be produced from the compound of the present invention according to a method known *per se.*

A prodrug of the compound of the present invention may be a compound that converts to the compound of the present invention under physiological conditions as described in Development of Pharmaceutical Products, vol. 7, Molecule Design, pp. 163-198, Hirokawa Shoten (1990).

The compound of the present invention may be labeled with an isotope (e.g., $^{3}H$, $^{14}C$, $^{35}S$, $^{125}I$ and the like) and the like.

The compound of the present invention may be an anhydride or a hydrate.

The compound of the present invention and a prodrug thereof (hereinafter sometimes to be simply referred to as the compound of the present invention) show low toxicity and can be used as an agent for the prophylaxis or treatment of various diseases to be mentioned later for mammals (e.g., human, mouse, rat, rabbit, dog, cat, cattle, horse, swine, simian) as they are or by admixing with a pharmacologically acceptable carrier and the like to give a pharmaceutical composition.

Here, various organic or inorganic carriers conventionally used as materials for pharmaceutical preparations are used as a pharmacologically acceptable carrier, which are added as an excipient, a lubricant, a binder, a disintegrant and the like for solid preparations; and a solvent, a dissolution aid, a suspending agent, an isotonicity agent, a buffer, a soothing agent and the like for liquid preparations. Where necessary, an additive for pharmaceutical preparations such as a preservative, an antioxidant, a coloring agent, a sweetening agent and the like can be used.

Preferable examples of the excipient include lactose, sucrose, D-mannitol, D-sorbitol, starch, α-starch, dextrin, crystalline cellulose, low-substituted hydroxypropyl cellulose, sodium carboxymethylcellulose, gum acacia, pullulan, light anhydrous silicic acid, synthetic aluminum silicate, magnesium aluminate metasilicate and the like.

Preferable examples of the lubricant include magnesium stearate, calcium stearate, talc, colloidal silica and the like.

Preferable examples of the binder include α-starch, saccharose, gelatin, gum acacia, methylcellulose, carboxymethylcellulose, sodium carboxymethylcellulose, crystalline cellulose, sucrose, D-mannitol, trehalose, dextrin, pullulan, hydroxypropyl cellulose, hydroxypropyl methylcellulose, polyvinylpyrrolidone and the like.

Preferable examples of the disintegrant include lactose, sucrose, starch, carboxymethylcellulose, calcium carboxymethylcellulose, sodium croscarmellose, sodium carboxymethyl starch, light anhydrous silicic acid, low-substituted hydroxypropyl cellulose and the like.

Preferable examples of the solvent include water for injection, physiological brine, Ringer's solution, alcohol, propylene glycol, polyethylene glycol, sesame oil, corn oil, olive oil, cottonseed oil and the like.

Preferable examples of the dissolution aid include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Preferable examples of the suspending agent include surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropyl cellulose and the like; polysorbates, polyoxyethylene hydrogenated castor oil, and the like.

Preferable examples of the isotonicity agent include sodium chloride, glycerol, D-mannitol, D-sorbitol, glucose and the like.

Preferable examples of the buffer include phosphate buffer, acetate buffer, carbonate buffer, citrate buffer and the like.

Preferable examples of the soothing agent include benzyl alcohol and the like.

Preferable examples of the preservative include p-oxybenzoates, chlorobutanol, benzyl alcohol, phenethyl alcohol, dehydroacetic acid, sorbic acid and the like.

Preferable examples of the antioxidant include sulfite, ascorbate and the like.

Preferable examples of the coloring agent include water-soluble edible tar pigments (e.g., foodcolors such as Food Color Red Nos. 2 and 3, Food Color Yellow Nos. 4 and 5, Food Color Blue Nos. 1 and 2 and the like), water insoluble lake pigments (e.g., aluminum salt of the aforementioned water-soluble edible tar pigment), natural pigments (e.g., beta carotene, chlorophil, red iron oxide) and the like.

Preferable examples of the sweetening agent include saccharin sodium, dipotassium glycyrrhizinate, aspartame, stevia and the like.

The dosage form of the aforementioned pharmaceutical composition is, for example, an oral agent such as tablets (inclusive of sublingual tablets and orally disintegrable tablets), capsules (inclusive of soft capsules and microcapsules), granules, powders, troches, syrups, emulsions, suspensions and the like; or a parenteral agent such as injections (e.g., subcutaneous injections, intravenous injections, intramuscular injections, intraperitoneal injections, drip infusions), external agents (e.g., transdermal preparations, ointments), suppositories (e.g., rectal suppositories, vaginal suppositories), pellets, nasal preparations, pulmonary preparations (inhalations), ophthalmic preparations and the like. These may be administered safely via an oral or parenteral route.

These agents may be controlled-release preparations such as rapid-release preparations and sustained-release preparations (e.g., sustained-release microcapsules).

The pharmaceutical composition can be produced according to a method conventionally used in the field of pharmaceutical preparation, such as the method described in Japan Pharmacopoeia and the like. Concrete production methods of preparations are described in detail in the following.

While the content of the compound of the present invention in the pharmaceutical composition varies depending on the dosage form, dose of the compound of the present invention and the like, it is, for example, about 0.1-100 wt%.

Where necessary, the aforementioned oral agents may be coated with a coating base for the purpose of masking taste, enteric property or sustained release.

Examples of the coating base include a sugar-coating base, a water-soluble film coating base, an enteric film coating base, a sustained-release film coating base and the like.

As the sugar-coating base, sucrose may be used, if necessary, along with one or more species selected from talc, precipitated calcium carbonate, gelatin, gum acacia, pullulan, carnauba wax and the like.

As the water-soluble film coating base, for example, cellulose polymers such as hydroxypropyl cellulose, hydroxypropyl methylcellulose, hydroxyethylcellulose, methylhydroxyethylcellulose and the like; synthetic polymers such as polyvinyl acetal diethylaminoacetate, aminoalkyl methacrylate copolymer E [Eudragit E, trade name, Roehm Pharma], polyvinylpyrrolidone and the like; polysaccharides such as pullulan and the like; and the like are used.

As the enteric film coating base, for example, cellulose polymers such as hydroxypropyl methylcellulose phthalate, hydroxypropyl methylcellulose acetate succinate, carboxymethylethylcellulose, cellulose acetate phthalate and the like; acrylic acid polymers such as methacrylic acid copolymer L [Eudragit L, trade name, Roehm Pharma], methacrylic acid copolymer LD [Eudragit L-30D55, trade name, Roehm Pharma], methacrylic acid copolymer S [Eudragit S, trade name, Roehm Pharma] and the like; natural products such as shellac and the like; and the like are used.

As the sustained-release film coating base, for example, cellulose polymers such as ethylcellulose and the like; acrylic acid polymers such as aminoalkyl methacrylate copolymer RS [Eudragit RS, trade name, Roehm Pharma], ethyl acrylate-methyl methacrylate copolymer suspension [Eudragit NE, trade name, Roehm Pharma] and the like; and the like are used.

Two or more kinds of the above-mentioned coating bases may be mixed in an appropriate ratio for use. In addition, a light shielding agent such as titanium oxide, ferric oxide and the like may be used during coating.

The compound of the present invention shows low toxicity (e.g., acute toxicity, chronic toxicity, genetic toxicity, reproductive toxicity, cardiotoxicity, carcinogenic), causes fewer side effects and can be used as an agent for the prophylaxis, treatment or diagnosis of various diseases for mammals (e.g., human, cattle, horse, dog, cat, simian, mouse, rat, especially human).

The compound of the present invention has a DGAT (DGAT1 or DGAT2 or both) inhibitory action, and is useful for the treatment or amelioration of DGAT-related diseases.

As the DGAT-related diseases, for example, obesity, diabetes (e.g., type 1 diabetes, type 2 diabetes, gestational diabetes), insulin resistance, leptin resistance, arteriosclerosis, hyperlipidemia (e.g., hypertriglyceridemia, hypercholesterolemia, hypo-HDL-cholesterolemia, postprandial hyperlipemia), arteriosclerosis, hypertension, cardiac failure, metabolic syndrome and the like can be mentioned.

For diagnostic criteria of diabetes, Japan Diabetes Society reported new diagnostic criteria in 1999.

According to this report, diabetes is a condition showing any of a fasting blood glucose level (glucose concentration of intravenous plasma) of not less than 126 mg/dl, a 75 g oral glucose tolerance test (75 g OGTT) 2 h level (glucose concentration of intravenous plasma) of not less than 200 mg/dl, and a non-fasting blood glucose level (glucose concentration of intravenous plasma) of not less than 200 mg/dl. A condition not falling under the above-mentioned diabetes and different from "a condition showing a fasting blood glucose level (glucose concentration of intravenous plasma) of less than 110 mg/dl or a 75 g oral glucose tolerance test (75 g OGTT) 2 h level (glucose concentration of intravenous plasma) of less than 140 mg/dl" (normal type) is called a "borderline type".

In addition, ADA (American Diabetes Association) reported new diagnostic criteria of diabetes in 1997 and WHO in 1998.

According to these reports, diabetes is a condition showing a fasting blood glucose level (glucose concentration of intravenous plasma) of not less than 126 mg/dl and a 75 g oral glucose tolerance test 2 h level (glucose concentration of intravenous plasma) of not less than 200 mg/dl.

According to the above-mentioned reports, impaired glucose tolerance is a condition showing a fasting blood glucose level (glucose concentration of intravenous plasma) of less than 126 mg/dl and a 75 g oral glucose tolerance test 2 h level (glucose concentration of intravenous plasma) of not less than 140 mg/dl and less than 200 mg/dl. According to the report of ADA, a condition showing a fasting blood glucose level (glucose concentration of intravenous plasma) of not less than 110 mg/dl and less than 126 mg/dl is called IFG (Impaired Fasting Glucose). According to the report of WHO, among the IFG (Impaired Fasting Glucose), a condition showing a 75 g oral glucose tolerance test 2 h level (glucose concentration of intravenous plasma) of less than 140 mg/dl is called IFG (Impaired Fasting Glycemia).

The compound of the present invention can be also used as an agent for the prophylaxis or treatment of diabetes, borderline type, impaired glucose tolerance, IFG (Impaired Fasting Glucose) and IFG (Impaired Fasting Glycemia), as determined according to the above-mentioned new diagnostic criteria. Moreover, the compound of the present invention can prevent progress of borderline type, impaired glucose tolerance, IFG (Impaired Fasting Glucose) or IFG (Impaired Fasting Glycemia) into diabetes.

The compound of the present invention can be also used as an agent for the prophylaxis or treatment of, for example, diabetic complications [e.g., neuropathy, nephropathy, retinopathy, cataract, macroangiopathy, osteopenia, hyperosmolar diabetic coma, infectious disease (e.g., respiratory infection, urinary tract infection, gastrointestinal infection, dermal soft tissue infection, inferior limb infection), diabetic gangrene, xerostomia, hypacusis, cerebrovascular disorder, peripheral blood circulation disorder], osteoporosis, cachexia (e.g., cancerous cachexia, tuberculous cachexia, diabetic cachexia, blood disease cachexia, endocrine disease cachexia, infectious disease cachexia or cachexia due to acquired immunodeficiency syndrome), fatty liver, polycystic ovary syndrome, kidney disease (e.g., diabetic nephropathy, glomerular nephritis, glomerulosclerosis, nephrotic syndrome, hypertensive nephrosclerosis, end stage kidney disease), muscular dystrophy, myocardial infarction, angina pectoris, cerebrovascular accident (e.g., cerebral infarction, cerebral apoplexy), Alzheimer's disease, Parkinson's syndrome, anxiety, dementia, insulin resistance syndrome, Syndrome X, hyperinsulinemia, hyperinsulinemia-induced sensory disorder, tumor (e.g., leukemia, breast cancer, prostatic cancer, skin cancer), irritable bowel syndrome, acute or chronic diarrhea, inflammatory diseases (e.g., chronic rheumatoid arthritis, spondylitis deformans, osteoarthritis, lumbago, gout, postoperative or traumatic inflammation, swelling, neuralgia, pharyngolaryngitis, cystitis, hepatitis (inclusive of nonalcoholic steatohepatitis), pneumonia, pancreatitis, enteritis, inflammatory bowel diseases (including inflammatory disease of large intestine), ulcerative colitis, gastric mucosal injury (inclusive of gastric mucosal injury caused by aspirin)), small intestine mucous membrane trauma, malabsorption, testis function disorder, visceral obesity syndrome and the like.

The compound of the present invention can also be used for the secondary prophylaxis or suppression of the progression of the above-mentioned various diseases (e.g., cardiovascular events such as cardiac infarction and the like).

While the dose of the compound of the present invention varies depending on the administration subject, administration route, target disease, condition and the like, the compound of the present invention is generally given in a single dose of about 0.01-100 mg/kg body weight, preferably 0.05-30 mg/kg body weight, more preferably 0.1-10 mg/kg body weight, in the case of, for example, oral administration to adult diabetic patients. This dose is desirably given 1 to 3 times a day.

The compound of the present invention can be used in combination with drugs such as a therapeutic agent for diabetes, a therapeutic agent for diabetic complications, an antihyperlipemic agent, an antihypertensive agent, an antiobestic agent, a diuretic, an antithrombotic agent and the like (hereinafter to be referred to as a combination drug), with the aim of enhancing the action of the compound, reducing the dose of the compound and the like. In this case, the timing of administration of the compound of the present invention and a combination drug is not limited. These may be simultaneously administered to an administration subject or administered in a staggered manner. Moreover, the compound of the present invention and a combination drug may be administered as two kinds of preparations each containing an active ingredient, or may be administered as a single preparation containing both active ingredients.

The dose of the combination drug can be determined as appropriate based on the dose clinically employed. The proportion of the compound of the present invention and the combination drug can be appropriately determined depending on the administration subject, administration route, target disease, condition, combination and the like. When, for example, the administration subject is human, the combination drug is used in an amount of 0.01-100 parts by weight per 1 part by weight of the compound of the present invention.

As the therapeutic agent for diabetes, insulin preparations (e.g., animal insulin preparations extracted from the pancreas of bovine or pig; human insulin preparations genetically synthesized using *Escherichia coli* or yeast; zinc insulin; protamine zinc insulin; fragment or derivative of insulin (e.g., INS-1), oral insulin preparation), insulin sensitizers (e.g., pioglitazone or a salt thereof (preferably hydrochloride), rosiglitazone or a salt thereof (preferably maleate), Reglixane (JTT-501), Netoglitazone (MCC-555), DRF-2593, KRP-297, R-119702, Rivoglitazone (CS-011), FK-614, compounds described in WO99/58510 (e.g., (E)-4-[4-(5-methyl-2-phenyl-4-oxazolylmethoxy)benzyloxyimino]-4-phenylbutyric acid), compounds described in WO01/38325, Tesaglitazar (AZ-242), Ragaglitazar (NN-622), Muraglitazar (BMS-298585), ONO-5816, Edaglitazone (BM-13-1258), LM-4156, MBX-102, Naveglitazar (LY-519818), MX-6054, LY-510929, Balaglitazone (NN-2344), T-131 or a salt thereof, THR-0921), PPARγ agonists, PPARγ antagonists, PPARγ/α dual agonists, α-glucosidase inhibitors (e.g., voglibose, acarbose, miglitol, emiglitate), biguanides (e.g., phenformin, metformin, buformin or salts thereof (e.g., hydrochloride, fumarate, succinate)), insulin secretagogues [sulfonylureas (e.g., tolbutamide, glibenclamide, gliclazide, chlorpropamide, tolazamide, acetohexamide, glyclopyramide, glimepiride, glipizide, glybuzole), repaglinide, senaglinide, nateglide, mitiglinide or calcium salt hydrate thereof], GPR40 agonists, GLP-1 receptor agonists [e.g., GLP-1, GLP-1MR, NN-2211, AC-2993 (exendin-4), BIM-51077, Aib(8,35)hGLP-1(7,37) $NH_2$, CJC-1131], amylin agonists (e.g., pramlintide), phosphotyrosine phosphatase inhibitors (e.g., sodium vanadate), dipeptidyl peptidase IV inhibitors (e.g., NVP-DPP-728, PT-100, P32/98, Vidagliptin (LAF-237), P93/01, TS-021, Sitagliptin

(MK-431), Saxagliptin (BMS-477118), T-6666), β3 agonists (e.g., AJ-9677, AZ40140), gluconeogenesis inhibitors (e.g., glycogen phosphorylase inhibitors, glucose-6-phosphatase inhibitors, glucagon antagonists), SGLT (sodium-glucose cotransporter) inhibitors (e.g., T-1095), 11β-hydroxysteroid dehydrogenase inhibitors (e.g., BVT-3498), adiponectin or agonists thereof, IKK inhibitors (e.g., AS-2868), leptin resistance improving drugs, somatostatin receptor agonists (compounds described in WO01/25228, WO03/42204, WO98/44921, WO98/45285 and WO99/22735) and glucokinase activators (e.g., Ro-28-1675) can be mentioned.

Examples of the therapeutic agent for diabetic complications include aldose reductase inhibitors (e.g., Tolrestat, Epalrestat, Zenarestat, Zopolrestat, Minalrestat, Fidarestat, CT-112, Ranirestat), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole)), neuranagenesis stimulators (e.g., Y-128), PKC inhibitors (e.g., ruboxistaurin mesylate; LY-333531), AGE inhibitors (e.g., ALT946, pimagedine, pyratoxanthine, N-phenacylthiazolium bromide (ALT766), ALT-711, EXO-226, Pyridorin, Pyridoxamine), reactive oxygen scavengers (e.g., thioctic acid), cerebral vasodilators (e.g., tiapride, mexiletine), somatostatin receptor agonists (e.g., BIM23190) and apoptosis signal regulating kinase-1 (ASK-1) inhibitors.

Examples of the antihyperlipemic agent include statin compounds (e.g., pravastatin, simvastatin, lovastatin, atorvastatin, fluvastatin, itavastatin, rosuvastatin, pitavastatin and salts thereof (e.g., sodium salt, calcium salt)), squalene synthase inhibitors (e.g., compounds described in WO97/10224, such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid), fibrate compounds (e.g., bezafibrate, clofibrate, simfibrate, clinofibrate), ACAT inhibitors (e.g., Avasimibe, Eflucimibe), anion exchange resins (e.g., colestyramine), probucol, nicotinic acid drugs (e.g., nicomol, niceritrol), ethyl icosapentate and plant sterols (e.g., soysterol, γ-oryzanol).

Examples of the antihypertensive agent include angiotensin converting enzyme inhibitors (e.g., captopril, enalapril, delapril), angiotensin II receptor antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121) and Clonidine.

Examples of the antiobestic agent include antiobestic agents acting on the central nervous system (e.g., Dexfenfluramine, fenfluramine, phentermine, Sibutramine, amfepramone, dexamphetamine, Mazindol, phenylpropanolamine, clobenzorex; MCH receptor antagonists (e.g., SB-568849; SNAP-7941; compounds encompassed in WO01/82925 and WO01/87834); neuropeptide Y antagonists (e.g., CP-422935); cannabinoid receptor antagonists (e.g., SR-141716, SR-147778); ghrelin antagonists; 11β-hydroxysteroid dehydrogenase inhibitors (e.g., BVT-3498)), pancreatic lipase inhibitors (e.g., orlistat, ATL-962), β3 agonists (e.g., AJ-9677, AZ40140), peptidic anorexiants (e.g., leptin, CNTF (Ciliary Neurotropic Factor)), cholecystokinin agonists (e.g., lintitript, FPL-15849) and feeding deterrents (e.g., P-57).

Examples of the diuretic include xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide and furosemide.

Examples of the antithrombotic agent include heparins (e.g., heparin sodium, heparin calcium, dalteparin sodium), warfarins (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.

Hereinafter the production methods of the compound of the present invention are explained.

Compound (I):

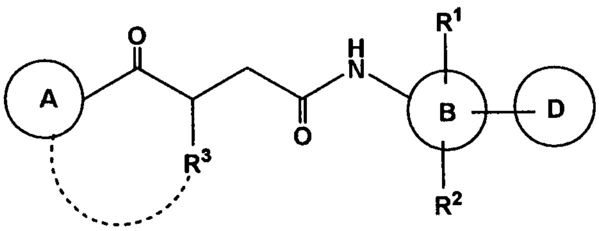

(I)

wherein each symbol is as defined above,
can be produced from, for example, a compound represented by the formula (III):

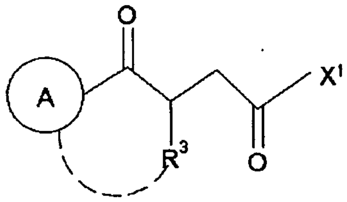

(III)

wherein $X^1$ is a hydroxy group or a halogen atom, and the other each symbol is as defined above,
or a derivative thereof and a compound represented by the formula (IV):

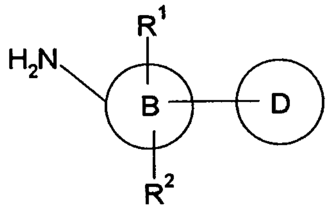

(IV)

wherein each symbol is as defined above.

To be specific, for example, a method of condensing a compound represented by the formula (IIIa):

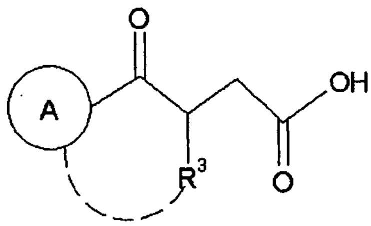

(IIIa)

wherein each symbol is as defined above,
and a compound represented by the formula (IV) with a conventionally known dehydrating condensing agent; a method of activating a carboxylic acid represented by the formula (IIIa) according to a conventionally known activation method, and reacting the resulting compound with a compound represented by the formula (IV); a method of reacting a carboxylic acid derivative of a compound represented by the formula (IIIa) with a compound represented by the formula (IV); or a method of reacting a compound represented by the formula (IIIb):

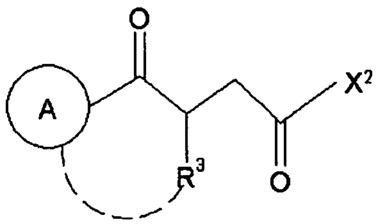

(IIIb)

wherein $X^2$ is a halogen atom, and the other each symbol is as defined above, with a compound represented by the formula (IV), and the like can be mentioned.

As the dehydrating condensing agent, for example, N,N-dicyclohexylamide, 1-ethyl-3-(3-dimethylaminopropyl) carbodiimide (WSC) or a hydrochloride thereof, carbonyldiimidazole, 1H-benzotriazol-1-yloxytris(dimethylamino)phosphonium hexafluorophosphate and the like can be mentioned.

As the method for activating a carboxylic acid, for example, a method of converting an acid anhydride with a

chloroformate, pivaloyl chloride, 2,4,6-trichlorobenzoyl chloride and the like; a method of converting an acid chloride with thionyl chloride, oxalyl chloride and the like; a method of converting an ester with 1-hydroxybenzotriazole, pentafluorophenol and the like together with a dehydrating condensing agent and the like, and the like can be mentioned.

As the carboxylic acid derivative, an $C_{1-6}$ alkyl (e.g., methyl, ethyl, n-propyl, i-propyl, n-butyl, tert-butyl etc.) ester optionally having substituent(s), a phenyl ester optionally having substituent(s), a silyl ester optionally having substituent(s), a mono-$C_{1-6}$ alkylamide optionally having substituent(s), a di-$C_{1-6}$ alkylamide optionally having substituent (s) and the like can be used. As the substituent, a halogen atom, formyl, a $C_{1-6}$ alkyl-carbonyl (e.g., acetyl, ethylcarbonyl, butylcarbonyl etc.), a nitro group, a hydroxy group, a $C_{1-6}$ alkoxy group (e.g., methoxy, ethoxy etc.) and the like can be used. The number of the substituents is 1 to 3.

The compound represented by the formula (IIIa) can be produced, for example, by reacting a compound represented by the formula (V):

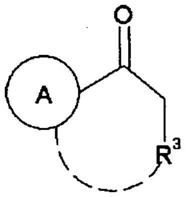

(V)

wherein each symbol is as defined above,
and a compound represented by the formula (VI):

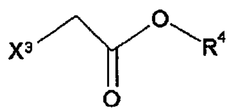

(VI)

wherein $X^3$ is a leaving group, and $R^4$ is a carboxy-protecting, in the presence of a base, and removing of the carboxy-protecting group of the resulting compound.

As the base, for example, lithium bis(trimethylsilyl)amide, lithium diisopropylamide, sodium hydride and the like can be mentioned.

As the leaving group for $X^3$, for example, a halogen atom, an acyloxy group (e.g., acetyloxy etc.), a sulfonyloxy group (e.g., p-toluenesulfonyloxy, methanesulfonyloxy etc.), and the like can be mentioned.

As the carboxy-protecting group for $R^4$, for example, a $C_{1-6}$ alkyl group, a $C_{7-11}$ aralkyl group (e.g., benzyl), a phenyl group, a trityl group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like can be mentioned. These groups are optionally substituted by 1 to 3 substituents selected from a halogen atom, a $C_{1-6}$ alkoxy group and a nitro group.

As the method for removing the protecting group, a method known *per* se or a method analogous thereto can be used. For example, a method of treating with acid, base, reduction, ultraviolet rays, hydrazine, phenylhydrazine, sodium N-methyldithiocarbamate, tetrabutylammonium fluoride, palladium acetate and the like can be used.

The aforementioned compound represented by the formula (V) and compound represented by the formula (VI) are commercially available or can be produced according to a method known *per se.*

Of the compound represented by the formula (IIIa), a compound represented by the formula (IIIc):

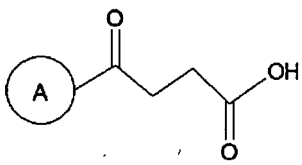

(IIIc)

wherein A is as defined above,
can be produced, for example, according to a method of reacting a compound represented by the formula (VII):

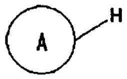

(VII)

wherein A is as defined above,
with succinic anhydride in the presence of a Lewis acid and the like; a method of reacting a compound represented by the formula (VII) with a compound represented by the formula (VIII):

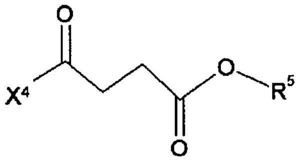

(VIII)

wherein $X^4$ is a leaving group, and $R^5$ is a carboxy-protecting group,
in the presence of a Lewis acid and the like, and removing the carboxy-protecting group of the resulting compound, and the like.

As the Lewis acid, for example, aluminum chloride, titanium chloride and the like can be mentioned.

As the leaving group for $X^4$, for example, those exemplified as the aforementioned $X^3$ can be mentioned.

As the carboxy-protecting group for $R^5$, for example, those exemplified as the aforementioned $R^4$ can be mentioned. The protecting group can be removed in the same manner as in the aforementioned $R^4$.

The aforementioned compound represented by the formula (VII) and compound represented by the formula (VIII) can be produced according to a method known *per se.*

The compound represented by the formula (IIIb) can be produced according to a method of halogenating the compound represented by the formula (IIIa). As the method, for example, a method of reacting the compound represented by the formula (IIIa) with thionyl chloride, oxalyl chloride and the like, and the like can be mentioned.

As the production method of the compound represented by the formula (IV),

1) a method of producing an aromatic amine from an aromatic compound having the other functional group;
2) a method of producing an aromatic amine by a ring B-forming reaction;
3) a method of subjecting a compound represented by the formula (IX):

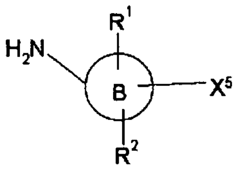

(IX)

wherein $X^5$ is a halogen atom, and the other each symbol is as defined above,
and a compound represented by the formula (X):

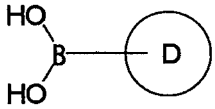

(X)

wherein each symbol is as defined above, to the Suzuki reaction, and the like can be mentioned.

The aforementioned compound represented by formula (IX) and compound represented by the formula (X) are commercially available, or can be produced according to a method known *per* se.

1) As the method of producing an aromatic amine from an aromatic compound having the other functional group, for example, a method of converting a carboxy group of a compound represented by the formula (XI):

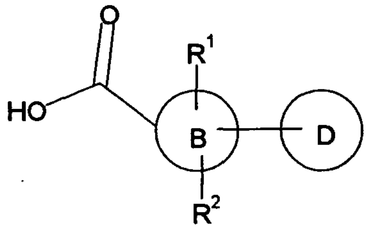

(XI)

wherein each symbol is as defined above, to a protected amino group by Curtius rearrangement and the like, and removing the protecting group of the resulting the compound; a method of reducing a nitro group of a compound represented by the formula (XII) :

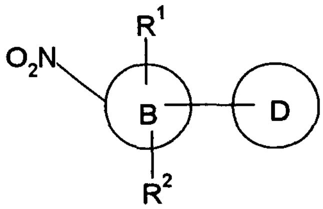

. (XII)

wherein each symbol is as defined above, to an amino group, and the like can be mentioned.

As the method of reducing a nitro group to an amino group, for example, a method of hydrogenating in the presence of a catalyst (e.g., palladium etc.); a method of reducing using a reducing agent (e.g., iron, iron chloride, zinc, zinc chloride etc.), and the like can be mentioned.

The aforementioned compound represented by formula (XI) and compound represented by the formula (XII) are commercially available, or can be produced according to a method known *per se.*

2) As the method of producing an aromatic amine by a ring B-forming reaction, for example, a method of reacting a compound represented by the formula (XIII):

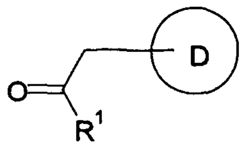

(XIII)

wherein each symbol is as defined above, and a compound represented by the formula (XIV):

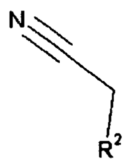

(XIV)

wherein each symbol is as defined above, in the presence of acetate ammonium and acetic acid, and reacting the resulting compound with sulfur; a method of reacting a compound represented by the formula (XV):

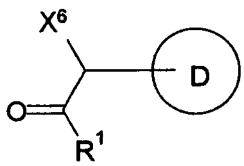

(XV)

wherein $X^6$ is a leaving group, and the other each symbol is as defined above, with thiourea, and the like can be mentioned.

As the leaving group for $X^6$, for example, those exemplified as the aforementioned $X^3$ can be mentioned.

The aforementioned compound represented by formula (XIII), compound represented by the formula (XIV) and compound represented by the formula (XV) are commercially available, or can be produced according to a method known *per se.*

Of the compound represented by the formula (IV), as a production method of a compound represented by the formula (IVa):

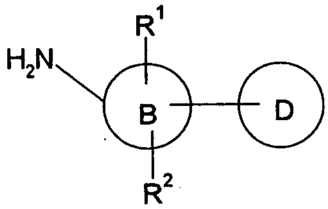

(IVa)

wherein ring B is a quinoline ring, and the other each symbol is as defined above,
for example, a method of reacting a compound represented by the formula (XVI) :

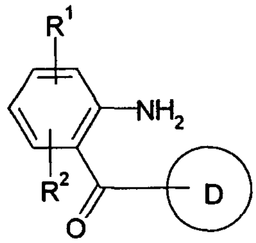

(XVI)

wherein each symbol is as defined above,
and acetonitrile in the presence of a base, and the like can be mentioned.

As the base, for example, lithium bis(trimethylsilyl)amide, lithium diisopropylamide, sodium hydride and the like can be mentioned.

As the production method of the compound represented by the formula (XVI), for example, a method of reacting a compound represented by the formula (XVII):

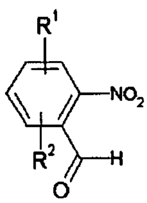

(XVII)

wherein each symbol is as defined above,
with a compound represented by the formula (XVIII):

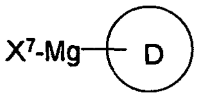

(XVIII)

wherein $X^7$ is a halogen atom, and D is as defined above, and oxidizing the hydroxy group of the resulting compound to ketone with an oxidant and the like, and the like can be mentioned.

As the oxidant, for example, manganese dioxide, sulfur trioxide-pyridine complex and the like can be mentioned.

The aforementioned compound represented by formula (XVII) and compound represented by the formula (XVIII) are commercially available, or can be produced according to a method known *per se.*

3) Of the compound represented by the formula (IV), a production method of a compound represented by the formula (IVb) :

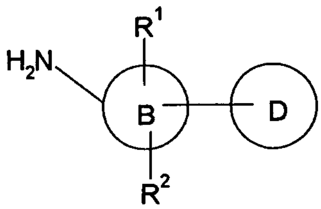

(IVb)

wherein $R^1$ is as defined for ring D, and the other each symbol is as defined above,
for example, a method of subjecting a compound represented by the formula (XIVa):

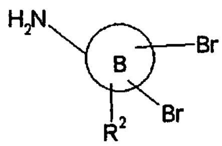

(XIVa)

wherein each symbol is as defined above, and a compound represented by the formula (X) to the Suzuki reaction, and the like can be mentioned.

The aforementioned compound represented by formula (XIVa) is commercially available, or can be produced according to a method known *per se.*

Compound (II):

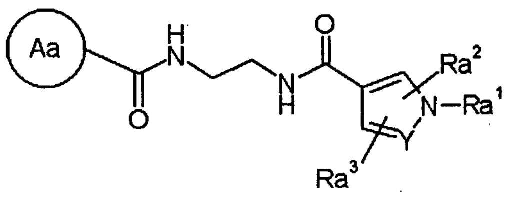

(II)

wherein each symbol is as defined above,
can be produced, for example,

1) from a compound represented by the formula (XIX):

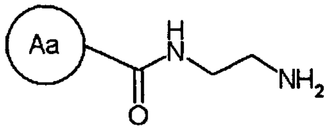

(XIX)

wherein each symbol is as defined above,
and a compound represented by the formula (XX):

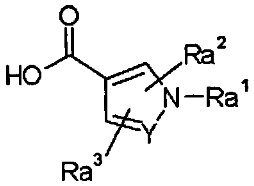

(XX)

wherein each symbol is as defined above,
or a carboxylic acid derivative thereof, or
2) from a compound represented by the formula (XXI):

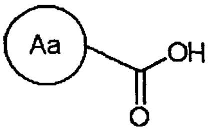

(XXI)

wherein each symbol is as defined above,
or a carboxylic acid derivative thereof and a compound represented by the formula (XXII) :

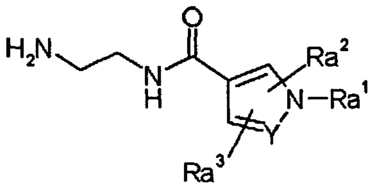

(XXII)

wherein each symbol is as defined above.

1) As the production method of the compound represented by the formula (II) from the compound represented by the formula (XIX) and the compound represented by the formula (XX) or a carboxylic acid derivative thereof, for example, a method of condensing the compound represented by the formula (XIX) and the compound represented by the formula (XX) with a conventionally known dehydrating condensing agent; a method of activating the compound represented by the formula (XX) according to a conventionally known activation method for a carboxylic acid, and reacting the resulting compound with the compound represented by the formula (XIX); a method of reacting the carboxylic acid derivative of the compound represented by the formula (XX) with the compound represented by the formula (XIX), and the like can be mentioned.
As the dehydrating condensing agent, the method for activating a carboxylic acid and the carboxylic acid derivative, those exemplified in the aforementioned reaction of the compound represented by formula (IIIa) or the compound represented by formula (IIIb) and the compound represented by the formula (IV) can be used, respectively.
The aforementioned compound represented by formula (XX) and the compound represented by the formula (XXI) are commercially available, or can be produced according to a method known *per se.*
The compound represented by the formula (XIX) can be produced, for example, by removing an amino-protecting group of a compound represented by the formula (XXIII):

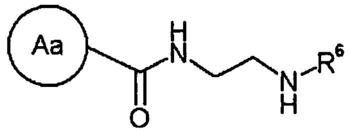

(XXIII)

wherein $R^6$ is an amino-protecting group, and Aa is as defined above.

As the amino-protecting group for $R^6$, for example, a formyl group, a $C_{1-6}$ alkyl-carbonyl group, a phenylcarbonyl group, a $C_{1-6}$ alkoxy-carbonyl group, a phenyloxycarbonyl group, a $C_{7-10}$ aralkyl-carbonyl group (e.g., benzylcarbonyl etc.), a trityl group, a phthaloyl group, a N,N-dimethylaminomethylene group and the like, each of which optionally has substituent(s), can be mentioned. As the substituent, a halogen atom, a $C_{1-6}$ alkyl-carbonyl group, a nitro group and the like can be used. The number of the substituents is 1 to 3.

As the method for removing the protecting group, a method known *per* se or a method analogous thereto can be used. For example, a method of treating with acid, base, reduction, ultraviolet rays, hydrazine, phenylhydrazine, sodium N-methyldithiocarbamate, tetrabutylammonium fluoride, palladium acetate and the like can be used.

The compound represented by the formula (XXIII) can be produced from a compound represented by the formula (XXIV):

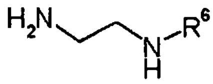

(XXIV)

wherein $R^6$ is as defined above,

and a compound represented by the formula (XXV):

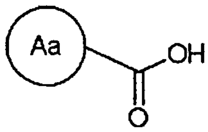

(XXV)

wherein Aa is as defined above, or a carboxylic acid derivative thereof.

For example, a method of condensing a compound represented by the formula (XXIV) and a compound represented by the formula (XXV) with a conventionally known dehydrating condensing agent; a method of activating a compound represented by the formula (XXV) according to a conventionally known activation method for a carboxylic acid, and reacting the resulting compound with a compound represented by the formula (XXIV); a method of reacting a carboxylic acid derivative of a compound represented by the formula (XXV) with a compound represented by the formula (XXIV), and the like can be mentioned.

As the dehydrating condensing agent, the method for activating a carboxylic acid and the carboxylic acid derivative, those exemplified in the aforementioned reaction of the compound represented by formula (IIIa) or the compound represented by the formula (IIIb) and the compound represented by the formula (IV) can be used, respectively.

The aforementioned compound represented by formula (XXIV) and compound represented by the formula (XXV) are commercially available, or can be produced according to a method known *per se*.

2) As the production method of the compound represented by the formula (II) from the compound represented by the formula (XXI) or a carboxylic acid derivative thereof and the compound represented by the formula (XXII), for example, a method of condensing a compound represented by the formula (XXI) and a compound represented by the formula (XXII) with a conventionally known dehydrating condensing agent; a method of activating a compound represented by the formula (XXI) according to a conventionally known activation method for a carboxylic acid, and reacting the resulting compound with a compound represented by the formula (XXII); a method of reacting a carboxylic acid derivative of the compound represented by the formula (XXI) with a compound represented by the formula (XXII), and the like can be mentioned.

As the dehydrating condensing agent, the method for activating a carboxylic acid and the carboxylic acid derivative, those exemplified in the aforementioned reaction of the compound represented by formula (IIIa) or the compound represented by the formula (IIIb) and the compound represented by the formula (IV) can be used, respectively.

The compound represented by the formula (XXII) can be produced, for example, by removing the amino-protecting group of the compound represented by the formula (XXVI):

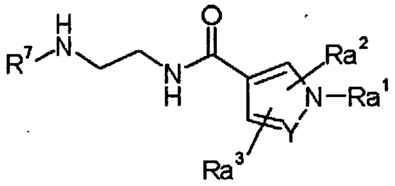

(XXVI)

wherein $R^7$ is an amino-protecting group, and the other each symbol is as defined above.

As the amino-protecting group for $R^7$, for example, those exemplified as the aforementioned $R^6$ can be mentioned. The protecting group can be removed in the same manner as in the aforementioned $R^6$.

The compound represented by the formula (XXVI) can be produced from a compound represented by the formula (XXVII):

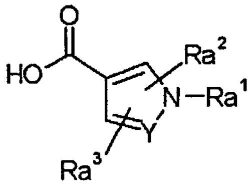

(XXVII)

wherein each symbol is as defined above,
or a carboxylic acid derivative thereof and a compound represented by the formula (XXIVa):

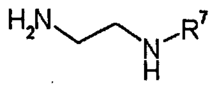

(XXIVa)

wherein $R^7$ is as defined above.

For example, a method of condensing a compound represented by the formula (XXVII) and a compound represented by the formula (XXIVa) with a conventionally known dehydrating condensing agent; a method of activating a compound represented by the formula (XXVII) according to a conventionally known activation method for a carboxylic acid, and reacting the resulting compound with a compound represented by the formula (XXIVa); a method of reacting a carboxylic acid derivative of the compound represented by the formula (XXVII) with a compound represented by the formula (XXIVa), and the like can be mentioned.

As the dehydrating condensing agent, the method for activating a carboxylic acid and the carboxylic acid derivative, those exemplified in the aforementioned reaction of the compound represented by formula (IIIa) or the compound represented by the formula (IIIb) and the compound represented by the formula (IV) can be used, respectively.

The aforementioned compound represented by formula (XXVII) and compound represented by the formula (XXIVa) are commercially available, or can be produced according to a method known *per se.*

Each of the above-mentioned production methods can be carried out in a solvent that does not adversely influence the reaction where necessary.

As such solvent, for example, alcohol solvents, ether solvents, halogenated hydrocarbon solvents, aromatic solvents, hydrocarbon solvents, amide solvents, ketone solvents, sulfoxide solvents, nitrile solvents and the like can be mentioned. These solvents may be used in a mixture at an appropriate ratio.

As the aforementioned "alcohol solvents", for example, methanol, ethanol, isopropanol, tert-butanol and the like can be used.

As the aforementioned "ether solvents", for example, diethyl ether, tetrahydrofuran (THF), 1,4-dioxane, 1,2-dimethoxyethane and the like can be used.

As the aforementioned "halogenated hydrocarbon solvents", for example, dichloromethane, chloroform, 1,2-dichloroethane, carbon tetrachloride and the like can be used.

As the aforementioned "aromatic solvents", for example, benzene, toluene, xylene, pyridine and the like can

be used.

As the aforementioned "hydrocarbon solvents", for example, hexane, pentane, cyclohexane and the like can be used.

As the aforementioned "amide solvents", for example, N,N-dimethylformamide (DMF), N,N-dimethylacetamide, N-methylpyrrolidone and the like can be used.

As the aforementioned "ketone solvents", for example, acetone, methylethylketone and the like can be used.

As the aforementioned "sulfoxide solvents", for example, dimethyl sulfoxide (DMSO) and the like can be used.

As the aforementioned "nitrile solvents", for example, acetonitrile, propionitrile and the like can be used.

The reaction temperature employed in each of the above-mentioned production methods is generally about -20°C to about 100°C, preferably 0°C to 80°C. The reaction time is generally about 0.5 hr to about 24 hr.

The conditions (solvent, reaction temperature, reaction time) for each reaction in each of the above-mentioned production methods can be appropriately chosen depending on the kind of reaction. Specifically, such conditions can be appropriately determined in reference to reference examples and examples

In the thus-obtained compound of the present invention, the functional group in a molecule can also be converted to an objective functional group by combining chemical reactions known *per se.* As such chemical reactions, oxidation reaction, reduction reaction, alkylation reaction, hydrolysis, amination reaction, esterification reaction, aryl coupling reaction, deprotection and the like can be mentioned.

In the above-mentioned production method, when the starting compound has an amino group, a carboxyl group, a hydroxy group or a carbonyl group as a substituent, a protecting group generally used in peptide chemistry and the like may be introduced into these groups. By eliminating the protecting group as necessary after the reaction, the objective compound can be obtained.

As the amino-protecting group, for example, a formyl group, a $C_{1-6}$ alkyl-carbonyl group, a $C_{1-6}$ alkoxy-carbonyl group, a benzoyl group, a $C_{7-10}$ aralkyl-carbonyl group (e.g., benzylcarbonyl), a $C_{7-14}$ aralkyloxy-carbonyl group (e.g., benzyloxycarbonyl, 9-fluorenylmethoxycarbonyl), a trityl group, phthaloyl group, a N,N-dimethylaminomethylene group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like can be mentioned. These groups are optionally substituted by 1 to 3 substituents selected from halogen atom, $C_{1-6}$ alkoxy group and nitro group.

As the carboxyl-protecting group, for example, those exemplified as $R^4$ can be mentioned.

As the hydroxy-protecting group, for example, a $C_{1-6}$ alkyl group, a phenyl group, a trityl group, a $C_{7-10}$ aralkyl group (e.g., benzyl), a formyl group, a $C_{1-6}$ alkyl-carbonyl group, a benzoyl group, a $C_{7-10}$ aralkyl-carbonyl group (e.g., benzylcarbonyl), a 2-tetrahydropyranyl group, a 2-tetrahydrofuranyl group, a substituted silyl group (e.g., trimethylsilyl, triethylsilyl, dimethylphenylsilyl, tert-butyldimethylsilyl, tert-butyldiethylsilyl), a $C_{2-6}$ alkenyl group (e.g., 1-allyl) and the like can be mentioned. These groups are optionally substituted by 1 to 3 substituents selected from a halogen atom, a $C_{1-6}$ alkyl group, a $C_{1-6}$ alkoxy group and a nitro group.

As the carbonyl-protecting group, for example, a cyclic acetal (e.g., 1,3-dioxane), a non-cyclic acetal (e.g., di-$C_{1-6}$ alkylacetal) and the like can be mentioned.

For elimination of the above-mentioned protecting group, a method known *per se*, for example, a method described in Protective Groups in Organic Synthesis, John Wiley and Sons (1980) and the like can be mentioned. For example, employed is a method using acid, base, UV light, hydrazine, phenyl hydrazine, sodium N-methyldithiocarbamate, tetrabutylammonium fluoride, palladium acetate, trialkylsilyl halide (e.g., trimethylsilyl iodide, trimethylsilyl bromide and the like) and the like, reduction and the like.

In the above-mentioned production methods, the starting compound may be in the form of a salt. As such salt, those similar to the salts of the aforementioned compound of the present invention can be mentioned.

The compound of the present invention obtained according to the above-mentioned production method can be isolated and purified by a known means, such as solvent extraction, liquid conversion, phase transfer, crystallization, recrystallization, chromatography and the like.

When the compound of the present invention contains an optical isomer, a stereoisomer, a positional isomer or a rotational isomer, these are encompassed in the compound of the present invention, and obtained as a single product according to a synthetic method and separation method known *per se*. For example, an optical isomer and an optical isomer resolved from this compound are also encompassed in the compound of the present invention.

The optical isomer can be produced by a method known *per se.*

The compound of the present invention may be in the form of a crystal.

The crystal of the compound of the present invention (hereinafter sometimes to be abbreviated as the crystal of the present invention) can be produced by crystallization of the compound of the present invention according to a crystallization method known *per se.*

In the present specification, the melting point refers to that measured using, for example, micromelting point measuring apparatus (Yanako, MP-500D or Buchi, B-545) or DSC (differential scanning calorimetry) device (SEIKO, EXSTAR6000) and the like.

In general, melting points vary depending on measurement apparatuses, measurement conditions and the like. The crystal in the present specification may show a different melting point described in the present specification, as long as it is within general error range.

The crystal of the present invention is superior in physicochemical properties (e.g., melting point, solubility, stability and the like) and biological properties (e.g., pharmacokinetics (absorption, distribution, metabolism, excretion), efficacy expression and the like), and is extremely useful as a pharmaceutical agent.

**Example**

The present invention is explained in more detail by way of the following Examples, Formulation Examples and Experimental Examples, which do not limit the present invention.

The $^1$H-NMR spectrum was measured by BRUKER AVANCE300 (300. MHz) or Varian Gemini 300 (300 MHz) using tetramethylsilane as the internal standard, and all δ values are shown in ppm. The mass spectrometry (MASS) was measured by Waters Micromass ZQ. The elemental analysis (Anal.) was measured by Vario EL (EL-4). The melting point (mp.) was measured by Buchi melting Point B-545. Unless otherwise specified, the numerical value shown for mixed solvent is a volume mixing ratio of each solvent. Unless otherwise specified, % means wt%. Unless otherwise specified, the ratio of elution solvents used for silica gel chromatography is a volume ratio. In the Examples, room temperature (ambient temperature) means a temperature of from about 20°C to about 30°C.

Abbreviations in the Examples have the following meanings: Me: methyl, Et: ethyl, THF: tetrahydrofuran, DMSO: dimethyl sulfoxide, DMF: N,N-dimethylformamide, HOBt: 1-hydroxybenztriazole, WSC: 1-(3-dimethylaminopropyl)-3-ethylcarbodiimide, DPPA: diphenylphosphoryl azide, s: singlet, d: doublet, t: triplet, q: quartet, m: multiplet, br: broad, M: molecular weight peak.

Example 1

N-(5-benzyl-4-phenyl-1,3-thiazol-2-yl)-4-(4-ethoxyphenyl)-4-oxobutanamide

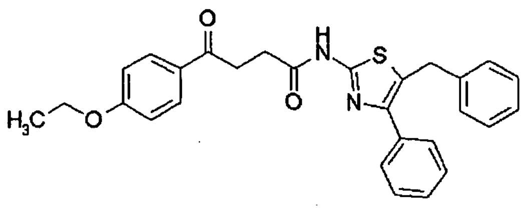

(1) 4-(4-ethoxyphenyl)-4-oxobutanoic acid

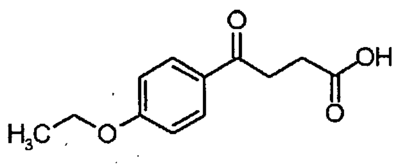

To a solution of phenetole (7.33 g, 0.06 mol) and succinic anhydride (7.21 g, 0.072 mol) in dichloromethane (70 mL) was gradually added aluminum chloride (17.6 g, 0.132 mol) under ice-cooling, and the mixture was stirred for 35 min under ice-cooling. The reaction mixture was poured into ice, and concentrated hydrochloric acid (100 mL) was added. The mixture was stirred for 30 min, and extracted with ethyl acetate. The organic layer was washed with saturated brine, dried over anhydrous magnesium sulfate, and concentrated under reduced pressure. The residue was collected by filtration, and washed with diisopropyl ether to give 4-(4-ethoxyphenyl)-4-oxobutanoic acid (5.89 g).
MASS m/z: 223.1 ($MH^+$)
(2) 5-benzyl-4-phenyl-1,3-thiazol-2-amine

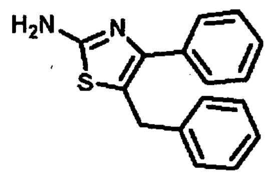

To a solution of 2-bromo-1,3-diphenylpropan-l-one (14 g) in ethanol (100 mL) was added thiourea (5.2 g), and the mixture was heated under reflux for 3 hr. The reaction mixture was concentrated, and the obtained residue was alkalified with 1N aqueous sodium hydroxide solution, and extracted with a mixed solvent of ethyl acetate-THF. The obtained organic layer was washed with water, dried over anhydrous magnesium sulfate, and concentrated under reduced pressure to give 5-benzyl-4-phenyl-1,3-thiazol-2-amine (13.7 g) as crude crystals.

(3) N-(5-benzyl-4-phenyl-1,3-thiazol-2-yl)-4-(4-ethoxyphenyl)-4-oxobutanamide

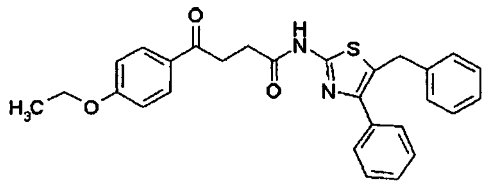

A solution of 4-(4-ethoxyphenyl)-4-oxobutanoic acid obtained in Example 1-(1) (0.334 g, 1.5 mmol), 5-benzyl-4-phenyl-1,3-thiazol-2-amine obtained in Example 1-(2) (0.20 g, 0.751 mmol), WSC (0.237 mL, 1.35 mmol) and HOBt (0.21 g, 1.35 mmol) in acetonitrile was stirred at 50°C for 15 hr. The reaction mixture was extracted with ethyl acetate, and the organic layer was washed with saturated aqueous sodium hydrogencarbonate solution and saturated brine, dried over anhydrous sodium sulfate, and concentrated under reduced pressure. The obtained crystals were recrystallized from ethyl acetate to give N-(5-benzyl-4-phenyl-1,3-thiazol-2-yl)-4-(4-ethoxyphenyl)-4-oxobutanamide (22 mg).

$^1$H-NMR (DMSO-$d_6$) δ: 1.35(3H, t, J=7.0Hz), 2.76(2H, t, J=6.4Hz), 3.27-3.34(2H, m), 4.12(2H, q, J=7.0Hz), 4.22(2H, s), 7.03(2H, d, J=8.7Hz), 7.18-7.25(2H, m), 7.34 (3H, ddd, J=16.2Hz, 7.3Hz, 7.1Hz), 7.45 (2H, t, J=7.4Hz), 7.62 (2H, d, J=7.2Hz), 7.94 (2H, d, J=8.9Hz), 12.22 (1H, s)

MASS m/z: 471.1 ($MH^+$)

Example 2

N-(5-benzyl-4-phenyl-1,3-thiazol-2-yl)-4-oxo-4-phenylbutanamide

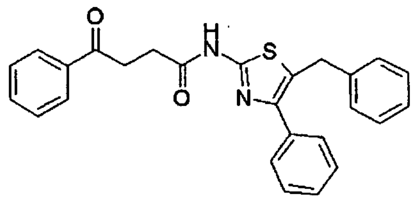

(1) potassium 4,4-dimethoxy-4-phenylbutanoate

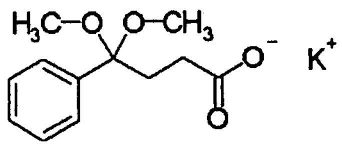

To a solution of 4-oxo-4-phenylbutanoic acid (1.78 g, 10 mmol) in a mixed solvent of trimethyl orthoformate (15

mL)-methanol (15 mL) was added two drops of concentrated sulfuric acid with a pipette, and the mixture was heated under reflux overnight. The reaction mixture was concentrated under reduced pressure, and the residue was extracted with ethyl acetate. The organic layer was washed with saturated aqueous sodium hydrogencarbonate solution and saturated brine, and dried over anhydrous magnesium sulfate. The extract was concentrated under reduced pressure, and the residue was dissolved in methanol (15 mL). Potassium hydroxide (0.66 g, 10 mmol) was added, and the mixture was stirred for 24 hr, and concentrated. The residue was washed with diisopropyl ether and a small amount of acetonitrile to give potassium 4,4-dimethoxy-4-phenylbutanoate (1.49 g).

$^1$H-NMR (DMSO-$d_6$) δ: 1.48 (2H, ddd, J=8.6Hz, 4.5Hz, 4.4 Hz), 2.01 (2H, ddd, J=8.5Hz, 4.5Hz, 4.3 Hz), 3.04(6H, s), 7.27-7.38 (5H, m)

(2) N-(5-benzyl-4-phenyl-1,3-thiazol-2-yl)-4-oxo-4-phenylbutanamide

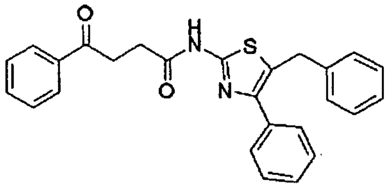

A solution of 5-benzyl-4-phenyl-1,3-thiazol-2-amine obtained in Example 1-(2) (0.18 g, 0.667 mmol), potassium 4,4-dimethoxy-4-phenylbutanoate obtained in Example 2-(1) (0.262 g, 1 mmol), 1-ethyl-3-(3-dimethylaminopropyl)carbodiimide hydrochloride (0.256 g, 1.33 mmol) and HOBt (0.20 g, 1.33 mmol) in N,N-dimethylformamide (2 mL) was stirred at 50°C for 12 hr, and then at room temperature for 2 days. The reaction mixture was extracted with ethyl acetate, and the organic layer was washed with aqueous sodium hydrogencarbonate solution and saturated brine, dried over anhydrous magnesium sulfate, and concentrated under reduced pressure. The residue was dissolved in a mixed solvent of acetone (4 mL)-water (2 mL), trifluoroacetic acid (1 mL) was added, and the mixture was stirred for 10 min. The resulting crystals were collected by filtration, and washed with water and acetone to give N-(5-benzyl-4-phenyl-1,3-thiazol-2-yl)-4-oxo-4-phenylbutanamide (45 mg) .

$^1$H-NMR(DMSO-$d_6$) δ: 2.80(2H, t, J=6.1Hz), 3.31-3.42(2H, m), 4.22(2H, s), 7.22(3H, t, J=7.3Hz), 7.28-7.40(3H, m), 7.42-7.57(4H, m), 7.63(3H, d, J=7.7Hz), 7.99(2H, d, J=7.5Hz)

Example 3

N-(5-benzyl-4-phenyl-1,3-thiazol-2-yl)-4-(3,4-diethoxyphenyl)-4-oxobutanamide

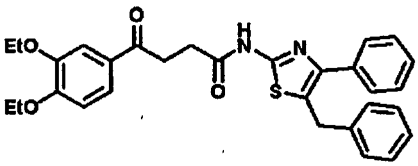

(1) 4-(3,4-diethoxyphenyl)-4-oxobutanoic acid

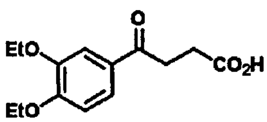

To a suspension of 1,2-diethoxybenzene (15.3 g) and succinic anhydride (11.0 g) in dichloromethane (70 mL) was gradually added aluminum chloride (30.6 g) under ice-cooling, and the mixture was stirred for 0.5 hr. The reaction mixture was poured into concentrated hydrochloric acid (100 mL)-ice, and the mixture was stirred for 1 hr, and extracted with ethyl acetate. The obtained organic layer was washed with water, dried over anhydrous magnesium sulfate, and concentrated under reduced pressure to give 4-(3,4-diethoxyphenyl)-4-oxobutanoic acid (15.3 g) as crude crystals.

$^1$H-NMR ($CDCl_3$) δ: 1.47 (3H, t, J=6. 9Hz) , 1.49 (3H, t, J=6. 9Hz) , 2.79(2H, t, J=6.6Hz), 3.28(2H, t, J=6.6Hz), 4.11-4.20(4H, m), 6.88(2H, d, J=8.4Hz), 7.57(2H, m)

(2) N-(5-benzyl-4-phenyl-1,3-thiazol-2-yl)-4-(3,4-diethoxyphenyl)-4-oxobutanamide

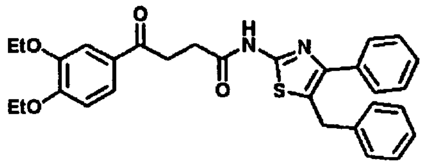

To a suspension of 5-benzyl-4-phenyl-1,3-thiazol-2-amine obtained in Example 1-(2) (266 mg), 4-(3,4-diethoxyphenyl)-4-oxobutanoic acid obtained in Example 3-(1) (480 mg) and HOBt (306 mg) in acetonitrile (20 mL) was added WSC (0.35 mL), and the mixture was stirred at 50°C for 2 hr. The reaction solution was cooled under ice bath, and the precipitated solid was collected by filtration to give N-(5-benzyl-4-phenyl-1,3-thiazol-2-yl)-4-(3,4-diethoxyphenyl)-4-oxobutanamide (233 mg).

$^1$H-NMR ($CDCl_3$) δ: 1.46 (3H, t, J=7.2Hz), 1.49 (3H, t, J=7.2Hz), 2.40(2H, t, J=6.6Hz), 3.15(2H, t, J=6.6Hz), 4.10-4.17 (4H, m), 4.20(2H, s), 6.86(1H, d, J=8.1Hz), 7.19-7.31(6H, m), 7.38(2H, m), 7.48-7.60(4H, m), 10.72(1H, br s)

MASS m/z: 515.1 ($MH^+$)

Example 4

N-(5-benzyl-4-phenyl-1,3-thiazol-2-yl)-4-(4,5-diethoxy-2-methylphenyl)-4-oxobutanamide

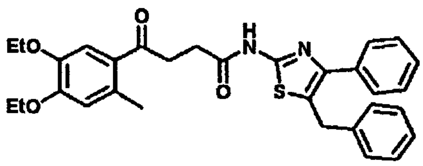

(1) 4-(4,5-diethoxy-2-methylphenyl)-4-oxobutanoic acid

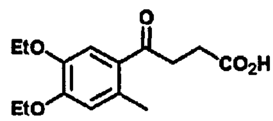

To a suspension of 3,4-diethoxytoluene (11.9 g) and succinic anhydride (7.9 g) in dichloromethane (40 mL) was gradually added aluminum chloride (22.0 g) under ice-cooling, and the mixture was stirred for 0.5 hr. The reaction mixture was poured into concentrated hydrochloric acid (100 mL)-ice, and the mixture was stirred for 1 hr, and extracted with ethyl acetate. The obtained organic layer was washed with water, dried over anhydrous magnesium sulfate, and concentrated under reduced pressure to give 4-(4,5-diethoxy-2-methylphenyl)-4-oxobutanoic acid (7.6 g) as crude crystals.

$^1$H-NMR ($CDCl_3$) δ: 1.45 (3H, t, J=6.9Hz), 1.47 (3H, t, J=6.9Hz), 2.49 (3H, s), 2.77 (2H, t, J=6.6Hz), 3.21 (2H, t, J=6.6Hz), 4.07-4.17(4H, m), 6.69 (1H, s), 7.32 (1H, s)

(2) N-(5-benzyl-4-phenyl-1,3-thiazol-2-yl)-4-(4,5-diethoxy-2-methylphenyl)-4-oxobutanamide

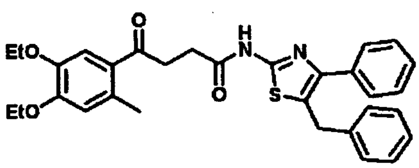

To a suspension of 5-benzyl-4-phenyl-1,3-thiazol-2-amine obtained in Example 1-(2) (15 g), 4-(4,5-diethoxy-2-methylphenyl)-4-oxobutanoic acid obtained in Example 4-(1) (27.4 g) and HOBt (17.4 g) in acetonitrile (950 mL) was added WSC (19.8 mL), and the mixture was stirred at 50°C for 2 hr. The reaction solution was cooled under ice bath, and the precipitated solid was collected by filtration, and recrystallized from ethyl acetate to give N-(5-benzyl-4-phenyl-1,3-thiazol-2-yl)-4-(4,5-diethoxy-2-methylphenyl)-4-oxobutanamide (10.7 g).
$^1$H-NMR ($CDCl_3$) δ: 1.44(3H, t, J=7.2Hz), 1.47(3H, t, J=7.2Hz), 2.48(3H, s), 2.50(2H, t, J=6.3Hz), 3.15(2H, t, J=7.2Hz), 4.80(2H, q, J=7.2Hz), 4.16(2H, q, J=7.2Hz), 4.20(2H, s), 6.69(1H, s), 7.20-7.32(7H, m), 7.41(2H, m), 7.57(2H, d, J=6.9Hz), 10.45(1H, br s)
Anal. Calcd for $C_{31}H_{32}N_2O_4S$: C, 70.43; H, 6.10; N, 5.30.
Found: C, 69.93; H, 6.17; N, 5.55.
MASS m/z: 529.1 ($MH^+$)

Example 5

N-(5-benzyl-4-phenyl-1,3-thiazol-2-yl)-4-(3-ethoxyphenyl)-4-oxobutanamide

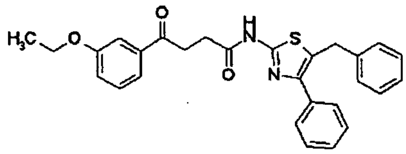

(1) ethyl 4-(3-ethoxyphenyl)-4-oxobutanoate

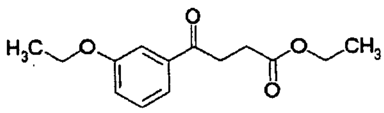

To a solution of 1-(3-ethoxyphenyl)ethanone (13.1 g, 0.0798 mol) in THF (100 mL) was added dropwise 1.1M lithium bis(trimethylsilyl)amide THF solution (79.8 mL, 0.08776 mol) at -78°C under nitrogen stream, and the mixture was stirred at the same temperature for 1 hr. Ethyl bromoacetate was added, and the mixture was stirred for 2 hr without cool-bath. Saturated aqueous ammonium chloride solution was added to the reaction mixture, and the mixture was extracted with ethyl acetate. The organic layer was washed with water and saturated brine, dried over anhydrous sodium sulfate, and concentrated under reduced pressure. The residue was crudely purified by silica gel column chromatography to give ethyl 4-(3-ethoxyphenyl)-4-oxobutanoate (6.1 g).
(2) 4-(3-ethoxyphenyl)-4-oxobutanoic acid

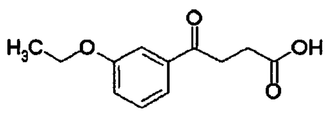

To a solution of ethyl 4-(3-ethoxyphenyl)-4-oxobutanoate obtained in Example 5-(1) (6.1 g) in ethanol (60 mL) was added 2N aqueous sodium hydroxide solution (24 mL, 0.0487 mol), and the mixture was stirred for 12 hr. The reaction mixture was extracted with water, and the aqueous layer was washed with diethyl ether. The extract was acidified with 1N hydrochloric acid, and extracted with ethyl acetate. The organic layer was washed with saturated brine, and dried over anhydrous magnesium sulfate. The residue was purified by silica gel column chromatography, and the obtained crystals were recrystallized from diisopropyl ether to give 4-(3-ethoxyphenyl)-4-oxobutanoic acid (2.162 g) as crystals.
$^1$H-NMR ($CDCl_3$) δ: 1.43 (3H, t, J=7.0Hz), 2.80 (2H, t, J=6.6Hz), 3.30 (2H, t, J=6.5Hz), 4.08 (2H, q, J=7.0Hz), 7.11 (1H, ddd, J=8.2Hz, 2.6Hz, 0.8Hz), 7.37 (1H, t, J=7.9Hz), 7.47 - 7.57 (2H, m)
MASS m/z: 223.1 ($MH^+$)
(3) N-(5-benzyl-4-phenyl-1,3-thiazol-2-yl)-4-(3-ethoxyphenyl)-4-oxobutanamide

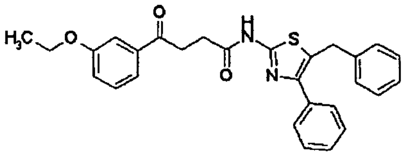

A solution of 5-benzyl-4-phenyl-1,3-thiazol-2-amine obtained in Example 1-(2) (0.20 g, 0.751 mmol), 4-(3-ethoxyphenyl)-4-oxobutanoic acid obtained in Example 5-(2) (0.334 g, 1.502 mmol), WSC (0.263 mL, 1.502 mmol) and HOBt (0.23 g, 1.502 mmol) in acetonitrile was stirred at 50°C for 3 hr, and then heated under reflux for 1 hr. The reaction mixture was extracted with ethyl acetate. The organic layer was washed with water and saturated brine, dried over anhydrous sodium sulfate, and concentrated under reduced pressure. The residue was purified by silica gel column chromatography, and the obtained crystals were recrystallized from ethyl acetate-diisopropyl ether to give N-(5-benzyl-4-phenyl-1,3-thiazol-2-yl)-4-(3-ethoxyphenyl)-4-oxobutanamide (52 mg).
$^1$H-NMR (DMSO-$d_6$) δ: 1.34 (3H, t, J=6.9Hz), 2.78 (2H, t, J=6.1Hz), 3.33 - 3.38 (2H, m), 4.09 (2H, q, J=7.0Hz), 4.22 (2H, s), 7.18 - 7.25 (4H, m), 7.28 - 7.34 (2H, m), 7.36 - 7.48 (5H, m), 7.54 - 7.65 (3H, m), 12.24 (1H, s)
MASS m/z: 471.1 ($MH^+$)

Example 6

N-(5-benzyl-4-phenyl-1,3-thiazol-2-yl)-2-(5,6-dimethoxy-1-oxo-2,3-dihydro-1H-inden-2-yl)acetamide

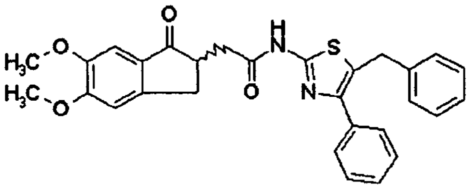

Using 5,6-dimethoxyindan-1-one (2.6 g, 0.0135 mol) as a starting material and in the same manner as in Example 5, N-(5-benzyl-4-phenyl-1,3-thiazol-2-yl)-2-(5,6-dimethoxy-1-oxo-2,3-dihydro-1H-inden-2-yl)acetamide (86 mg) was synthesized.
$^1$H-NMR (DMSO-$d_6$) δ: 2.65 - 2.80 (2H, m), 2.89 - 3.02 (2H, m), 3.25 (1H, dd, J=16 . 9Hz, 7.6 Hz), 3.80 (3H, s), 3.86 (3H, s), 4 . 22 (2H, s), 7.09 (2H, d, J=7.7 Hz), 7.18 - 7.26 (3H, m), 7.28 - 7.39 (3H, m), 7.45 (2H, t, J=7.4 Hz), 7.62 (2H, d, J=7.3 Hz), 12.23 (1H, s)
MASS m/z: 499.0 ($MH^+$)

Example 7

N-(5-benzyl-4-phenyl-1,3-thiazol-2-yl)-4-oxo-4-(5,6,7,8-tetrahydronaphthalen-2-yl)butanamide

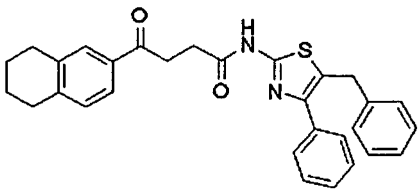

(1) 4-oxo-4-(5,6,7,8-tetrahydronaphthalen-2-yl)butanoic acid

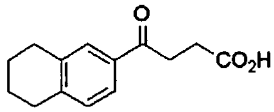

1, 2, 3, 4-Tetrahydronaphthalene (6.6 g) and succinic anhydride (5.0 g) were dissolved in dichloromethane (100 mL), and aluminum chloride (7.33 g) was added at 0°C. The mixture was stirred at room temperature for 3 hr, poured into water, and extracted with ethyl acetate. The organic layer was washed with 2N hydrochloric acid and saturated brine, dried over anhydrous magnesium sulfate, and dried under vacuum. The obtained crystals were recrystallized from ethyl acetate to give 4-oxo-4-(5,6,7,8-tetrahydronaphthalen-2-yl)butanoic acid (6.02 g) as pale-yellow crystals.
$^1$H-NMR(DMSO-$d_6$)δ: 1.75-1.88(4H, m), 2.73-2.89(6H, m), 3.28(2H, t, J=6.69Hz), 7.09-7.19 (1H, m), 7.69 (2H, dd, J=4.24Hz, 2.35Hz)

(2) N- (5-benzyl-4-phenyl-1, 3-thiazol-2-yl) -4-oxo-4- (5, 6, 7, 8-tetrahydronaphthalen-2-yl)butanamide

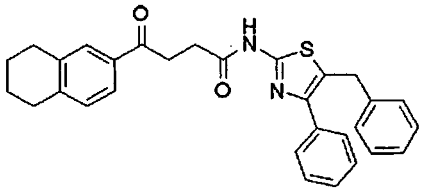

A solution of 4-oxo-4-(5,6,7,8-tetrahydronaphthalen-2-yl)butanoic acid obtained in Example 7-(1) (465 mg), 5-benzyl-4-phenyl-1,3-thiazol-2-amine obtained in Example 1-(2) (266 mg), HOBt (270 mg) and WSC (354 μL) in acetonitrile (30 mL) was stirred at 50°C for 3 days. The precipitated crystals were collected by filtration, and dried to give N-(5-benzyl-4-phenyl-1,3-thiazol-2-yl)-4-oxo-4-(5,6,7,8-tetrahydronaphthalen-2-yl)butanamide (250 mg) as crystals.
$^1$H-NMR (DMSO-$d_6$) δ: 1.68-1.82(4H, m), 2.70-2.84(6H, m), 3.27-3.36(2H, m), 4.22(2H, s), 7.16-7.50(9H, m), 7.59-7.71 (4H, m), 12.23(1H, s)

Example 8

4-(3,4-diethoxyphenyl)-4-oxo-N-(1,1':3',1"-terphenyl-5'-yl)butanamide

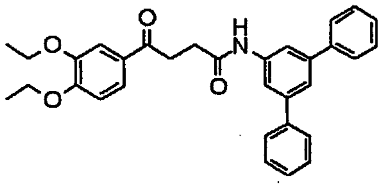

(1) methyl 1,1':3',1"-terphenyl-5'-carboxylate

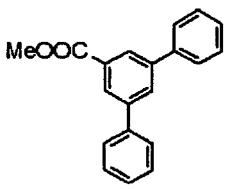

Methyl 3,5-dibromobenzoate (1.9 g), phenylboronic acid (1.9 g), bis(triphenylphosphine)dichloropalladium (93 mg) and sodium carbonate (1.8 g) were dissolved in a mixed solvent of 1,2-dimethoxyethane(20 mL)-water (4 mL), and the mixture was stirred at 70°C for 4 hr under nitrogen atmosphere. The reaction solution was extracted with ethyl acetate, and the organic layer was washed with 1N hydrochloric acid, saturated aqueous sodium hydrogencarbonate

solution and saturated brine, dried over anhydrous magnesium sulfate, and concentrated under reduced pressure. The residue was purified by silica gel column chromatography [developing solvent: n-hexane-ethyl acetate (4:1)], and the obtained crystals were recrystallized from ethyl acetate-n-hexane (3:1) to give methyl 1,1':3',1"-terphenyl-5'-carboxylate (0.8 g) as white crystals.

$^1$H-NMR ($CDCl_3$) δ : 3.98 (3H, s), 7.37-7.52 (6H, m), 7.68 (4H, d, J=7.3Hz), 8.00(1H, s), 8.26(2H, d, J=1.7Hz)

MASS m/z: 289.04 ($MH^+$)

(2) 1,1':3',1"-terphenyl-5'-carboxylic acid

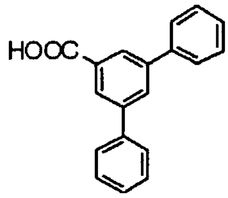

Methyl 1,1':3',1"-terphenyl-5'-carboxylate obtained in Example 8-(1) (2.1 g) was dissolved in a mixed solvent of methanol (20 mL)-THF (20 mL), and 2N aqueous sodium hydroxide solution (7 mL) was added. The mixture was stirred at room temperature for 65 hr, and concentrated under reduced pressure. The residue was adjusted to pH 5 with 1N hydrochloric acid, and extracted with ethyl acetate. The organic layer was washed with water and saturated brine, dried over anhydrous magnesium sulfate, and concentrated under reduced pressure. The obtained crystals were recrystallized from ethyl acetate-n-hexane (3:1) to give 1,1':3',1" -terphenyl-5'-carboxylic acid (1.7 g) as white crystals.

$^1$H-NMR ($CDCl_3$) δ: 7.41-7.48(2H, m), 7.49-7.56(4H, m), 7.78-7.85(4H, m), 8.13-8.19(3H, m)

MASS m/z: 275.03($MH^+$)

(3) tert-butyl (1,1':3',1"-terphenyl-5'-yl)carbamate

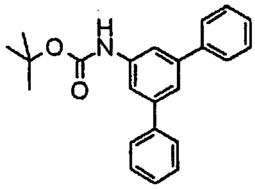

To a solution of 1,1':3',1" -terphenyl-5'-carboxylic acid obtained in Example 8-(2) (1.8 g) and triethylamine (1.2 mL) in toluene (50 mL) was added DPPA (1.7 mL), and the mixture was stirred at room temperature for 2 hr. Then, tert-butanol (2.5 mL) was added to reaction solution, and the mixture was stirred at 60°C for 15 hr. The solvent was evaporated under reduced pressure, and the residue was purified by silica gel column chromatography [developing solvent: n-hexane-ethyl acetate (4:1)], and the obtained crystals were recrystallized from ethyl acetate-n-hexane (3:1) to give tert-butyl (1,1':3',1"-terphenyl-5'-yl)carbamate (0.7 g) as white crystals.

$^1$H-NMR($CDCl_3$)δ : 1.51-1.58 (9H, m), 6.62(1H, s), 7.33-7.49(7H, m), 7.57-7.66(6H, m)

(4) 1,1':3',1''-terphenyl-5'-amine

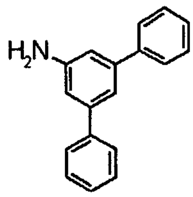

To a solution of tert-butyl (1,1':3',1"-terphenyl-5'-yl)carbamate obtained in Example 8-(3) (0.7 g) in dichloromethane (50 mL) was added trifluoroacetic acid (1.5 mL), and the mixture was stirred at room temperature for 7 hr. The solvent was evaporated under reduced pressure, and the residue was dissolved in ethyl acetate. The mixture was washed with saturated aqueous sodium hydrogencarbonate solution and water. The organic layer was dried over

anhydrous magnesium sulfate, and concentrated under reduced pressure, and the obtained crystals were recrystallized from ethyl acetate-n-hexane (3:1) to give 1,1':3',1" -terphenyl-5'-amine (0.3 g) as white crystals. $^1$H-NMR ($CDCl_3$) δ: 2.72(2H, s), 6.90(2H, d, J=1.5Hz), 7.21(1H, t, J=1.5Hz), 7.31-7.39(2H, m), 7.40-7.47(4H, m), 7.58-7.65 (4H, m) MASS m/z: 246.03($MH^+$)

(5) ethyl 4-(3,4-diethoxyphenyl)-4-oxobutanoate

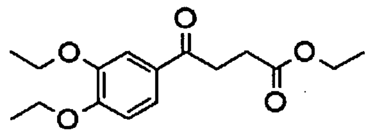

To a solution of 1,2-diethoxybenzene (20.0 g) and ethylsuccinyl chloride (18.0 mL) in dichloromethane (200 mL) was slowly added aluminum chloride (33.6 g) under ice-cooling, and the mixture was stirred for 45 min. The reaction mixture was poured into ice water, and concentrated hydrochloric acid (100 mL) was added. The mixture was extracted with chloroform. The organic layer was washed with saturated brine, dried over anhydrous magnesium sulfate, and concentrated under reduced pressure. The residue was purified by silica gel column chromatography [developing solvent: n-hexane-ethyl acetate (4:1)], and the obtained crystals were recrystallized from ethyl acetate-n-hexane (1:3) to give ethyl 4-(3,4-diethoxyphenyl)-4-oxobutanoate (24.2 g) as white crystals.

$^1$H-NMR ($CDCl_3$) δ: 1.27(3H, t, J=7.2Hz), 1.47(3H, t, J=7.1Hz), 1.49(3H, t, J=7.2Hz), 2.74(2H, t, J=6.7Hz), 3.27(2H, t, J=6.7Hz), 4.11-4.21(6H, m), 6.88(1H, d, J=8.5Hz), 7.53(1H, d, J=2.1Hz), 7.59(1H, dd, J=8.4Hz, 2.0Hz)

MASS m/z: 316.99($MNa^+$)

(6) methyl 4-(3,4-diethoxyphenyl)-4,4-dimethoxybutanoate

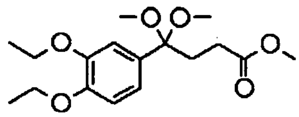

To a solution of ethyl 4-(3,4-diethoxyphenyl)-4-oxobutanoate obtained in Example 8-(5) (24.2 g) in methanol (250 mL) were added methyl orthoformate (45 mL) and concentrated sulfuric acid (0.1 mL), and the mixture was stirred overnight at 50°C. Sodium hydrogencarbonate (5.0 g) was added to the reaction mixture, and the solvent was evaporated under reduced pressure. The residue was dissolved in ethyl acetate, and the mixture was washed with saturated aqueous sodium hydrogencarbonate solution and saturated brine, and dried over anhydrous magnesium sulfate. After filtration, the solvent was evaporated to give methyl 4-(3,4-diethoxyphenyl)-4,4-dimethoxybutanoate (27.6 g).

$^1$H-NMR($CDCl_3$)δ: 1.45(6H, t, J=7.0Hz), 1.99-2.10(2H, m), 2.13-2.24(2H, m), 3.15(6H, s), 3.57(3H, s), 4.05-4.16 (4H, m), 6.80-6.90(1H, m), 6.92-6.99(2H, m)

(7) potassium 4-(3,4-diethoxyphenyl)-4,4-dimethoxybutanoate

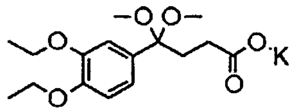

To a solution of methyl 4-(3,4-diethoxyphenyl)-4,4-dimethoxybutanoate obtained in Example 8-(6) (27.0 g) in methanol (150 mL) was added potassium hydroxide (4.6 g), and the mixture was stirred at 30°C for 18 hr, and concentrated under reduced pressure. The residue was poured into diethyl ether (300 mL), and the mixture was stirred at room temperature for 30 min. The obtained white crystals were collected by filtration to give potassium 4-(3,4-diethoxyphenyl)-4,4-dimethoxybutanoate (10.6 g).

$^1$H-NMR($DMSO_6$) δ: 1.31(3H, t, J=7.0Hz), 1.32(3H, t, J=6.8Hz), 1.35-1.43(2H, m), 1.87-1.97(2H, m), 2.97-3.05(6H, m), 3.94-4.05(4H, m), 6.82-6.92(3H, m)

(8) 4-(3,4-diethoxyphenyl)-4-oxo-N-(1,1':3',1"-terphenyl-5'-yl)butanamide

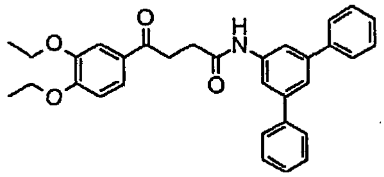

A solution of potassium 4-(3,4-diethoxyphenyl)-4,4-dimethoxybutanoate obtained in Example 8-(7) (144 mg), 1,1':3',1"-terphenyl-5'-amine obtained in Example 8-(4) (100 mg), 1-ethyl-3-(3-dimethylaminopropyl)carbodiimide hydrochloride (118 mg), HOBt (94 mg) and triethylamine (144 μL) in dimethylformamide (10 mL) was stirred at 60°C for 5 hr. The solvent was evaporated under reduced pressure, and the residue was dissolved in ethyl acetate. The mixture was washed with 6N hydrochloric acid, saturated aqueous sodium hydrogencarbonate solution and saturated brine, dried over anhydrous magnesium sulfate, and concentrated under reduced pressure. The residue was purified by silica gel column chromatography [developing solvent: n-hexane-ethyl acetate (9:1)], and the obtained crystals were recrystallized from ethyl acetate-n-hexane (3:1) to give 4-(3,4-diethoxyphenyl)-4-oxo-N-(1,1':3',1"-terphenyl-5'-yl)butanamide (116 mg) as white crystals.
$^1$H-NMR ($CDCl_3$) δ: 1.47 (6H, dt, J=8.1Hz, 7.0Hz), 2.85(2H, t, J=6.3 Hz), 3.45(2H, t, J=6.3 Hz), 4.09-4.20(4H, m), 6.88 (1H, d, J=8.5Hz), 7.33-7.47(6H, m), 7.51-7.58(2H, m), 7.60-7.66(5H, m), 7.77(2H, d, J=1.3Hz), 7.98(1H, s)
MASS m/z: 494.08 ($MH^+$)

Example 9

ethyl 3-(5-{[4-(3,4-diethoxyphenyl)-4-oxobutanoyl]amino}biphenyl-2-yl)propanoate

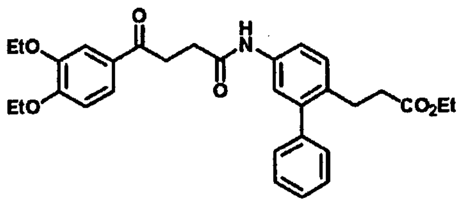

(1) ethyl (2E)-3-(5-nitrobiphenyl-2-yl)acrylate

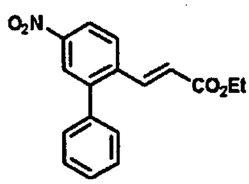

To a solution of ethyl diethylphosphonoacetate (1.41 g) in DMF (20 mL) was added sodium hydride (60%, in oil, 0.28 g) under ice-cooling, and the mixture was stirred for 0.5 hr. 4-Nitro-2-phenylbenzaldehyde (1.3 g) was added, and the mixture was stirred for 0.5 hr under ice-cooling. Water was added to the reaction mixture, and the mixture was extracted with ethyl acetate. The obtained organic layer was washed with water, dried over magnesium sulfate, and concentrated to give ethyl (2E)-3-(5-nitrobiphenyl-2-yl)acrylate (2.0 g) as crude crystals. The crystals were used for the next step without purification.
$^1$H-NMR ($CDCl_3$) δ: 1.30(3H, t, J=7.2Hz), 4.23(2H, q, J=7.2Hz), 6.50(1H, d, J=15.9Hz), 7.33(2H, m), 7.47(3H, m), 7.68(1H, d, J=15.9Hz), 8.82(1H, d, J=8.4 Hz), 8.22 (2H, m)
(2) ethyl 3-(5-aminobiphenyl-2-yl)propanoate

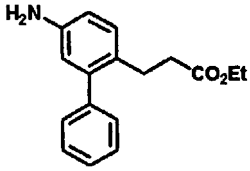

To a solution of ethyl (2E)-3-(5-nitrobiphenyl-2-yl)acrylate obtained in Example 9-(1) (2.0 g) in a mixed solvent of ethanol (50 mL)-THF (30 mL) was added 10% palladium-carbon (0.3 g), and the mixture was stirred overnight under hydrogen atmosphere. The reaction mixture was filtrated through celite, and the filtrate was concentrated. The obtained residue was purified by silica gel column chromatography [developing solvent: hexane-ethyl acetate (2: 1)] to give ethyl 3-(5-aminobiphenyl-2-yl)propanoate (1.4 g) as an oil.

$^1$H-NMR ($CDCl_3$) δ: 1.18 (3H, t, J=7.0Hz), 2.34(2H, t, J=8.6Hz), 2. 81 (2H, t, J=8.6Hz), 3. 61 (2H, br s), 4.04(2H, q, J=7.0 Hz), 6.55(1H, d, J=2.6 Hz), 6.64(1H, dd, J=8.0 Hz, J=2.6 Hz), 7.07(1H, d, J=8.0 Hz), 7.26-7.40(5H, m)

(3) ethyl 3-(5-{[4-(3,4-diethoxyphenyl)-4-oxobutanoyl]amino}biphenyl-2-yl)propanoate

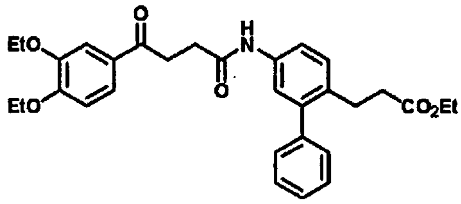

To a suspension of ethyl 3-(5-aminobiphenyl-2-yl)propanoate obtained in Example 9-(2) (500 mg), 4-(3,4-diethoxyphenyl)-4-oxobutanoic acid (643 mg, synthesized from 1,2-diethoxybenzene as a starting material in the same manner as in Example 1-(1)) and HOBt (426 mg) in acetonitrile (5 mL) was added WSC (0.49 mL), and the mixture was stirred at 50°C for 2 hr. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure to give ethyl 3-(5-{[4-(3,4-diethoxyphenyl)-4-oxobutanoyl]amino}biphenyl-2-yl)propanoate (730 mg) as crystals.

$^1$H-NMR ($CDCl_3$) δ: 1.18(3H, t, J=7.4Hz), 1.48(6H, m), 2.36(2H, t, J=8.0Hz), 2.77(2H, t, J=6.2Hz), 2.88(2H, t, J=8.2Hz), 3.40(2H, t, J=6.2Hz), 4.04(2H, q, J=7.4Hz), 4.15(4H, m), 6.87(1H, d, J=6.4Hz), 7.20-7.40(8H, m), 7.50-7.79 (2H, m), 7.79(1H, br s)

Example 10

3-(5-{[4-(3,4-diethoxyphenyl)-4-oxobutanoyl]amino}biphenyl-2-yl)propanoic acid

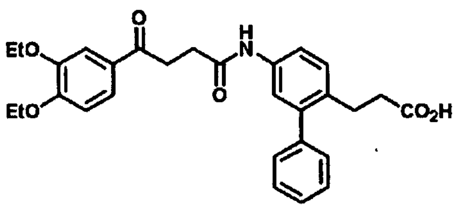

To a solution of ethyl 3-(5-{[4-(3,4-diethoxyphenyl)-4-oxobutanoyl]amino}biphenyl-2-yl)propanoate obtained in Example 9-(3) (0.66 g) in a mixed solvent of THF (30 mL)-methanol (10 mL) was added 1N aqueous sodium hydroxide solution (10 mL), and the mixture was stirred overnight. The solvent was evaporated under reduced pressure, and the residue was acidified with diluted hydrochloric acid, and extracted with ethyl acetate. The organic layer was washed with water, dried over anhydrous magnesium sulfate, and concentrated under reduced pressure to give 3-(5-{[4-(3,4-diethoxyphenyl)-4-oxobutanoyl]amino}biphenyl-2-yl)propanoic acid (0.55 g) as crude crystals.

$^1$H-NMR ($CDCl_3$) δ: 1.47(6H, m), 2.39(2H, t, J=7.2Hz), 2.78(2H, t, J=6.3Hz), 2.88(2H, t, J=7.2Hz), 3.40(2H, t, J=6.3Hz), 4.13(4H, m), 6.87(1H, d, J=8.7Hz), 7.20-7.40(7H, m), 7.52(2H, m), 7.60(1H, m), 7.97(1H, br s)

Example 11

4-(3,4-diethoxyphenyl)-N-(4,6-diphenylpyridin-2-yl)-4-oxobutanamide

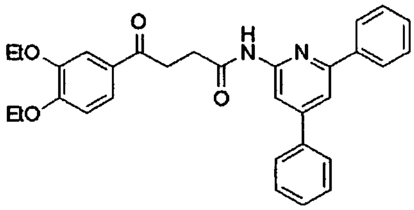

Potassium 4-(3,4-diethoxyphenyl)-4,4-dimethoxybutanoate obtained in Example 8-(7) (0.35 g), 2,4,6-trichlorobenzoyl chloride (0.27 g) and triethylamine (0.11 g) were dissolved in THF (5 mL), and the mixture was stirred for 4 hr. 4,6-Diphenylpyridin-2-amine (0.25 g, synthesized according to a method described in Chem Ber, 1961, 94, 698-704) was added to the reaction mixture, and the mixture was stirred overnight at 50°C. 2N Hydrochloric acid was added to the reaction mixture, and the mixture was extracted with ethyl acetate. The organic layer was washed with saturated brine, dried over anhydrous magnesium sulfate, and vacuum-dried. The obtained crystals were recrystallized from acetate-diethyl ether to give 4-(3,4-diethoxyphenyl)-N-(4,6-diphenylpyridin-2-yl)-4-oxobutanamide (0.33 g) as colorless crystals.
$^1$H-NMR (DMSO-$d_6$) δ: 1.30-1.41(6H, m), 2.85(2H, t, J=6.12 Hz), 3.28-3.37(2H, m), 4.03-4.17(4H, m), 7.08 (1H, d, J=8.48Hz), 7.43-7.58(7H, m), 7.67(1H, dd, J=8.48Hz, 2.07Hz), 7.79-7.85(2H, m), 7.93(1H, d, J=1.32Hz), 8.18-8.24(2H, m), 8.34(1H, s), 10.71(1H, s)

Example 12

4-(3,4-diethoxyphenyl)-N-(2,6-diphenylpyridin-4-yl)-4-oxobutanamide

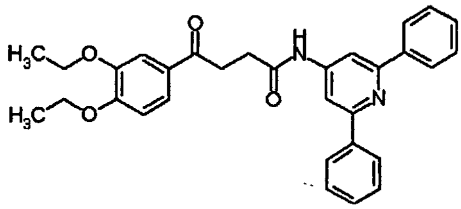

(1) 2-(trimethylsilyl)ethyl (2,6-diphenylpyridin-4-yl)carbamate

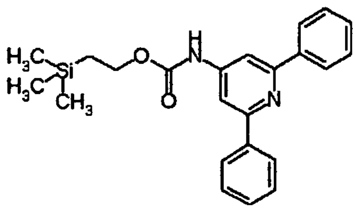

To a solution of 2,6-diphenylisonicotinic acid (400 mg) in THF (10.0 mL) were added triethylamine (0.52 mL) and DPPA (0.804 mL), and the mixture was stirred at room temperature for 1 hr. The reaction mixture was heated under

reflux for 1 hr, and 2-(trimethylsilyl)ethanol (0.535 mL) was added, and the mixture was heated under reflux for 2 hr. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated under reduced pressure. The residue was purified by silica gel column chromatography [developing solvent: n-hexane-ethyl acetate (4:1→1:9)] to give 2-(trimethylsilyl) ethyl (2,6-diphenylpyridin-4-yl)carbamate (115.0 mg) as a colorless liquid.
$^1$H-NMR($CDCl_3$)δ: 0.09 (9H, s), 1.10(2H, t, J=8.7Hz), 4.33(2H, t, J=8.7Hz), 7.28-7.51(10H, m), 7.77(1H, s), 8.12 (1H, s), 8.15(1H, s)
MASS m/z: 391.0 ($MH^+$)
(2) 2,6-diphenylpyridin-4-amine

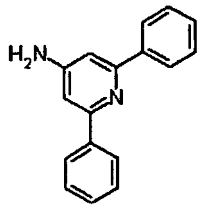

To a solution of 2-(trimethylsilyl)ethyl (2,6-diphenylpyridin-4-yl)carbamate obtained in Example 12-(1) (71.1 mg) in THF (5.0 mL)-water (0.18 mL) was added 1M tetrabutylammonium fluoride-THF solution (0.364 mL), and the mixture was stirred at room temperature stirred for 3 hr. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated under reduced pressure. The residue was purified by silica gel column chromatography [developing solvent: n-hexane-ethyl acetate (9:1→1:1)] to give 2,6-diphenylpyridin-4-amine (30.3 mg) as a colorless solid.
$^1$H-NMR ($CDCl_3$) δ: 4.22(2H, br s), 6.97 (2H, s), 7.37-7.49 (6H, m), 8.06-8.10(4H, m)
MASS m/z: 247.0($MH^+$)
(3) 4-(3,4-diethoxyphenyl)-N-(2,6-diphenylpyridin-4-yl)-4-oxobutanamide

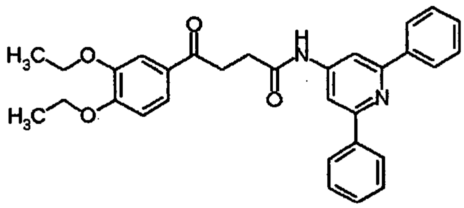

To a suspension of potassium 4-(3,4-diethoxyphenyl)-4,4-dimethoxybutanoate obtained in Example 8-(7) (41.1 mg) in THF (5.0 mL) were added triethylamine (36 μL) and 2,4,6-trichlorobenzoyl chloride (38.4 μL), and the mixture was stirred at room temperature stirred for 4 hr. 2,6-Diphenylpyridin-4-amine obtained in Example 12-(2) (30.3 mg) in THF (5.0 mL) was added to the reaction mixture, and the mixture was heated under reflux for 3 days. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated under reduced pressure. The residue was purified by silica gel column chromatography [developing solvent: n-hexane-ethyl acetate (9:1→1:4)] to give white crystals. The crystals were dissolved in dichloromethane (5 mL), and trifluoroacetic acid (1 mL) was added. The mixture was stood still at room temperature for 5 min, and concentrated under reduced pressure. The residue was purified by silica gel column chromatography [developing solvent: n-hexane-ethyl acetate (4:1-1:4)], and the obtained crystals were recrystallized from ethyl acetate-n-hexane (1:2) to give 4-(3,4-diethoxyphenyl)-N-(2,6-diphenylpyridin-4-yl)-4-oxobutanamide (10.3 mg) as white crystals.
$^1$H-NMR($CDCl_3$)δ: 1.49 (6H, q, J=6.8Hz), 2.87 (2H, t, J=5.4Hz), 3.47 (2H, t, J=5.4Hz), 4.16 (6H, t, J=6.8Hz), 6.90 (1H, d, J=7.2Hz), 7.42-7.65(8H, m), 7.92(2H, s), 8.15(4H, d, J=7.2Hz), 8.18(1H, br s)
MASS m/z: 495.1 ($MH^+$)

Example 13

4-(3,4-diethoxyphenyl)-N-(3,5-diphenylpyridin-2-yl)-4-oxobutanamide

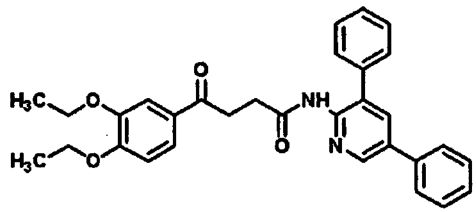

(1) 3,5-diphenylpyridin-2-amine

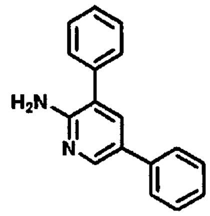

To a solution of 2-amino-3,5-dibromopyridine (2.0 g) and phenylboronic acid (2.0 g) in methanol (15 mL) were added aqueous sodium carbonate solution (15 g, in water 30 mL), toluene (70 mL) and bis(triphenylphosphine)dichloropalladium (0.28 g), and the mixture was heated under reflux for 3 days under nitrogen atmosphere. The organic layer was washed with water, dried over anhydrous magnesium sulfate, and concentrated under reduced pressure. The obtained residue was purified by silica gel column chromatography [developing solvent: hexane-ethyl acetate (2:1)] to give 3,5-diphenylpyridin-2-amine (1.4 g) as crystals.
$^1$H-NMR ($CDCl_3$) δ: 4.64 (2H, br s), 7.30-7.58 (10H, m), 7.62 (1H, d, J=2.4Hz), 8.33 (1H, d, J=2.4Hz)
(2) 4-(3,4-diethoxyphenyl)-N-(3,5-diphenylpyridin-2-yl)-4-oxobutanamide

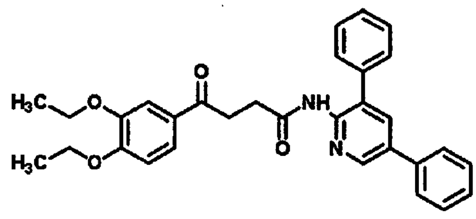

To a suspension of potassium 4-(3,4-diethoxyphenyl)-4,4-dimethoxybutanoate obtained in Example 8-(7) (350 mg) and 2,4,6-trichlorobenzoyl chloride (0.17 mL) in THF (5 mL) was added triethylamine (0.28 mL), and the mixture was stirred at room temperature for 4 hr. 3,5-Diphenylpyridin-2-amine obtained in Example 13-(1) (240 mg) was added to the reaction mixture, and the mixture was heated at 50°C for 2 days. Diluted hydrochloric acid was added to the reaction mixture, and the mixture was stirred for 1 hr, and extracted with ethyl acetate. The organic layer was washed with water, dried over anhydrous magnesium sulfate, and concentrated under reduced pressure. The obtained residue was purified by silica gel column chromatography [developing solvent: hexane-ethyl acetate (1:1)] to give 4-(3,4-diethoxyphenyl)-N-(3,5-diphenylpyridin-2-yl)-4-oxobutanamide (44 mg) as crystals.
$^1$H-NMR ($CDCl_3$) δ: 1.48(6H, m), 2.99 (2H, m), 3.41(2H, m), 4.14 (4H, m), 6.88 (1H, d, J=8.7Hz), 7.40-7.60(13H, m), 7.81 (1H, m), 8.65 (1H, br s)

Example 14

4-(3,4-diethoxyphenyl)-N-(2,6-diphenylpyrimidin-4-yl)-4-oxobutanamide

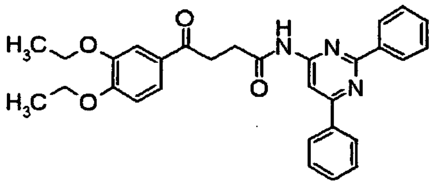

(1) methyl 2,6-diphenylpyrimidine-4-carboxylate

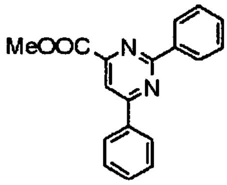

To a solution of methyl 2,6-dibromopyrimidine-4-carboxylate (2.0 g), phenylboronic acid (5.9 g) and [1,1'-bis(diphenylphosphino)ferrocene]dichloropalladium (400 mg) in 1,2-dimethoxyethane (20 mL) was added 2M aqueous potassium carbonate solution (24 mL), and the mixture was stirred at 80°C for 4 hr under nitrogen atmosphere. The reaction solution was extracted with ethyl acetate, and the organic layer was washed with 1N hydrochloric acid, saturated aqueous sodium hydrogencarbonate solution and saturated brine, dried over anhydrous magnesium sulfate, and concentrated under reduced pressure. The residue was purified by silica gel column chromatography [developing solvent: n-hexane-ethyl acetate (4:1)], and the obtained crystals were recrystallized from ethyl acetate-n-hexane (3:1) to give methyl 2,6-diphenylpyrimidine-4-carboxylate (0.5 g) as white crystals.
$^1$H-NMR ($CDCl_3$) δ: 4.09(3H, s), 7.51-7.60 (6H, m), 8.27-8.35(3H, m), 8.65(2H, dd, J=6.7Hz, 2.9Hz)
MASS m/z: 291.01($MH^+$)
(2) 2,6-diphenylpyrimidine-4-carboxylic acid

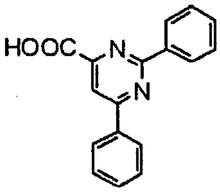

Methyl 2,6-diphenylpyrimidine-4-carboxylate obtained in Example 14-(1) (0.5 g) was dissolved in a mixed solvent of methanol (5 mL)-THF (5 mL), 2N aqueous sodium hydroxide solution (9 mL) was added, and the mixture was stirred at room temperature for 2 hr, and concentrated under reduced pressure. The residue was adjusted to pH 5 with 1N hydrochloric acid, and extracted with ethyl acetate. The organic layer was washed with saturated brine, dried over anhydrous magnesium sulfate, and concentrated under reduced pressure. The obtained crystals were recrystallized from ethyl acetate-n-hexane (3:1) to give 2,6-diphenylpyrimidine-4-carboxylic acid (0.4 g) as white crystals. $^1$H-NMR ($CDCl_3$) δ: 7.53-7.63(6H, m), 8.29-8.37(2H, m), 8.44 (1H, s), 8.54-8.62(2H, m)
MASS m/z: 277.01 ($MH^+$)
(3) tert-butyl (2,6-diphenylpyrimidin-4-yl)carbamate

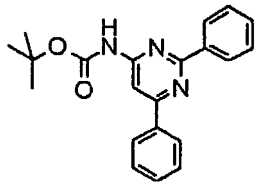

To a solution of 2,6-diphenylpyrimidine-4-carboxylic acid obtained in Example 14-(2) (0.4 g) and triethylamine (0.3 mL) in toluene (10 mL) was added DPPA (0.4 mL), and the mixture was stirred at room temperature for 2 hr. Then, tert-butanol (1.0 mL) was added to the reaction solution, and the mixture was stirred at 50°C for 18 hr. The solvent was evaporated under reduced pressure, and the residue was purified by silica gel column chromatography [developing solvent: n-hexane-ethyl acetate (4:1)], and the obtained crystals were recrystallized from ethyl acetate-n-hexane (3:1) to give tert-butyl (2,6-diphenylpyrimidin-4-yl)carbamate (0.5 g) as white crystals.

$^1$H-NMR ($CDCl_3$) δ: 1.53-1.60(9H, m), 7.43-7.59(6H, m), 8.23-8.34(3H, m), 8.51(2H, dd, J=6.8Hz, 3.0Hz)

MASS m/z: 348.05($MH^+$)

(4) 2,6-diphenylpyrimidin-4-amine

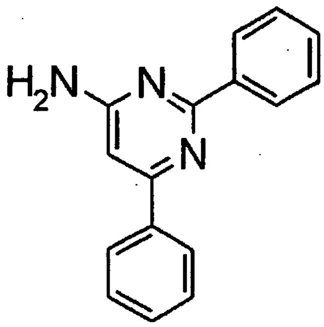

To a solution of tert-butyl (2,6-diphenylpyrimidin-4-yl)carbamate obtained in Example 14-(3) (0.5 g) in dichloromethane (50 mL) was added trifluoroacetic acid (3.1 mL), and the mixture was stirred at room temperature for 25 hr. The solvent was evaporated under reduced pressure, and the residue was dissolved in ethyl acetate. The mixture was washed with saturated aqueous sodium hydrogencarbonate solution and water, dried over anhydrous magnesium sulfate, and concentrated under reduced pressure. The obtained crystals were recrystallized from ethyl acetate-n-hexane (4:1) to give 2,6-diphenylpyrimidin-4-amine (0.3 g) as white crystals.

$^1$H-NMR ($CDCl_3$) δ: 7.01(1H, s), 7.52-7.64(6H, m), 8.15(2H, dd, J=7.9Hz, 1.7Hz), 8.41-8.47(2H, m)

MASS m/z: 248.04 ($MH^+$)

(5) 4-(3,4-diethoxyphenyl)-N-(2,6-diphenylpyrimidin-4-yl)-4-oxobutanamide

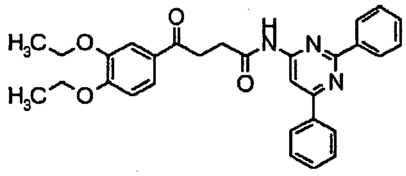

To a solution of potassium 4-(3,4-diethoxyphenyl)-4,4-dimethoxybutanoate obtained in Example 8-(7) (343 mg) and triethylamine (150 μL) in THF (2 mL) was added 2,4,6-trichlorobenzoyl chloride (150 μL), and the mixture was stirred at room temperature for 96 hr. 2,6-Diphenylpyrimidin-4-amine obtained in Example 14-(4) (120 mg) was added to the reaction mixture, and the mixture was stirred at 70°C for 18 hr. The solvent was evaporated under reduced pressure, and the residue was dissolved in ethyl acetate. The mixture was washed with 6N hydrochloric acid, saturated aqueous sodium hydrogencarbonate solution and saturated brine, dried over anhydrous magnesium sulfate, and concentrated under reduced pressure. The residue was purified by silica gel column chromatography [developing solvent: n-hexane-ethyl acetate (9:1)], and the obtained crystals were recrystallized from ethyl acetate-n-hexane (3:1) to give 4-(3,4-diethoxyphenyl)-N-(2,6-diphenylpyrimidin-4-yl)-4-oxobutanamide (12 mg) as white crystals.

$^1H$-NMR ($CDCl_3$) δ : 1.47(3H, t, J=7.0Hz), 1.50(3H, t, J=7.0Hz), 2.93 (2 H, t, J=6.2 Hz), 3.45 (2 H, t, J=6.2 Hz), 4.15(2H, q, J=7.0Hz), 4.18(2H, q, J=7.0Hz), 6.90 (1H, d, J=8.5Hz), 7.47-7.54(6H, m), 7.57(1H, d, J=2.1Hz), 7.64(1H, dd, J= 8.4Hz, 2.0Hz), 8.21-8.28(2H, m), 8.39(1H, s), 8.50-8.57(3H, m)
MASS m/z: 496.05($MH^+$)

Example 15

4-(3,4-diethoxyphenyl)-N-{4-[4-(methoxymethoxy)phenyl]quinolin-2-yl}-4-oxobutanamide

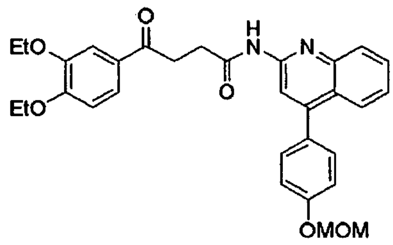

(1) [4-(methoxymethoxy)phenyl](2-nitrophenyl)methanol

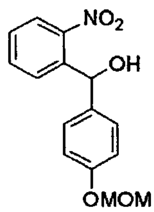

1-Bromo-4-(methoxymethoxy)benzene (11 g) was dissolved in THF (60 mL), and magnesium (1.6 g) was added. The reaction mixture was cooled to -78°C, 2-nitrobenzaldehyde (6.4 g) was added, and the mixture was stirred for 30 min. Water was added to the reaction mixture, and the mixture was extracted with ethyl acetate. The organic layer was washed with saturated brine, dried over anhydrous magnesium sulfate, and vacuum-dried to give [4-(methoxymethoxy)phenyl](2-nitrophenyl)methanol (4.9 g) as yellow crystals. This compound was used for the next step without purification.
(2) [4-(methoxymethoxy)phenyl](2-nitrophenyl)methanone

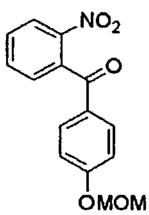

[4-(Methoxymethoxy)phenyl](2-nitrophenyl)methanol obtained in Example 15-(1) (4.9 g) was dissolved in dichloromethane (400 mL), manganese dioxide (17.7 g) was added, and the mixture was stirred overnight. The manganese dioxide was removed by filtration, and the filtrate was vacuum-dried. The obtained crystals were recrystallized from ethyl acetate-diisopropyl ether to give [4-(methoxymethoxy)phenyl](2-nitrophenyl)methanone (3.66 g).
$^1H$-NMR ($CDCl_3$) δ: 3.48(3H s), 5.23(2H, s), 7.01-7.11(2H, m), 7.47 (1H, dd, J=7.44Hz, 1.41Hz), 7.61-7.82(4H, m), 8.23 (1H, dd, J=8.19Hz, 1.04Hz)
(3) (2-aminophenyl)[4-(methoxymethoxy)phenyl]methanone

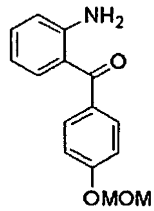

[4-(Methoxymethoxy)phenyl](2-nitrophenyl)methanone obtained in Example 15-(2) (3.66 g) was dissolved in ethyl acetate (30 mL), palladium-carbon (1.0 g) was added, and the mixture was stirred overnight under hydrogen atmosphere. The palladium-carbon was removed by filtration, and the filtrate was vacuum-dried. The residue was purified by silica gel column chromatography [developing solvent: n-hexane-ethyl acetate (4:1)] to give (2-aminophenyl)[4-(methoxymethoxy)phenyl]methanone (2.8 g) as a yellow oil.
$^1$H-IVMR ($CDCl_3$) δ: 3.51 (3H, s), 5.25 (2H, s), 5.89 (2H, s), 6.58-6.66 (1H, m), 6.73 (1H, dd, J=8.29Hz, 0.75Hz), 7.04-7.13 (2H, m), 7.23-7.33 (1H, m) 7.47 (1H, dd, J=8.10Hz, 1.51Hz), 7.61-7.71 (2H, m)
(4) 4-[4-(methoxymethoxy)phenyl]quinolin-2-amine

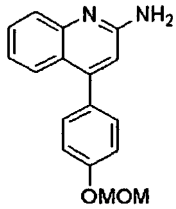

(2-Aminophenyl)[4-(methoxymethoxy)phenyl]methanone obtained in Example 15-(3) (2.8 g), acetonitrile (1.34 g) and sodium hydride (0.65 g) were dissolved in pyridine (20 mL), and the mixture was refluxed overnight. Water was added to the reaction mixture, and the mixture was extracted with ethyl acetate. The organic layer was washed with saturated brine, dried over anhydrous magnesium sulfate, and vacuum-dried. The residue was purified by silica gel column chromatography [developing solvent: ethyl acetate] to give 4-[4-(methoxymethoxy)phenyl]quinolin-2-amine (1.84 g) as brown crystals.
$^1$H-NMR ($CDCl_3$) δ: 3.54(3H, s), 4.75(2H, s), 5.26 (2H, s), 6. 65 (1H, s), 7.14 - 7.25(3H, m), 7.38-7.45(2H, m), 7.52-7.60(1H, m), 7.68-7.76(2H, m)
(5) 4-(3,4-diethoxyphenyl)-N-{4-[4-(methoxymethoxy)phenyl]quinolin-2-yl}-4-oxobutanamide

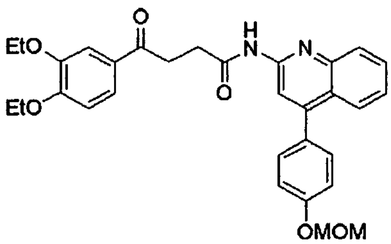

4-[4-(Methoxymethoxy)phenyl]quinolin-2-amine obtained in Example 15-(4) (1.84 g), 4-(3,4-diethoxyphenyl)-4-oxobutanoic acid (3.5 g), WSC (2.99 g) and HOBt (3.01 g) were dissolved in acetonitrile (35 mL), and the mixture was stirred overnight at 50°C. The reaction mixture was concentrated under reduced pressure, and the residue was purified by silica gel column chromatography, and the obtained crystals were recrystallized from diethyl ether-diisopropyl ether to give 4-(3,4-diethoxyphenyl)-N-{4-[4-(methoxymethoxy)phenyl]quinolin-2-yl}-4-oxobutanamide (2.8 g) as pale-brown crystals.
$^1$H-NMR (DMSO-$d_6$) δ: 1.34 (6H, m), 2.84 (2H, t, J=6.22Hz), 3.27-3.34(2H, m), 3.42(3H, s), 4.01-4.18(4H, m), 5.29(2H, s), 7.06(1H, d, J=8.67Hz), 7.16 - 7.26 (2H, m), 7.42 - 7.52 (4H, m), 7.61 - 7.92 (4H, m), 8.22 (1H, s), 10.98 (1H, s)

Example 16

4-(3,4-diethoxyphenyl)-N-(2,4-diphenyl-1,3-thiazol-5-yl)-4-oxobutanamide

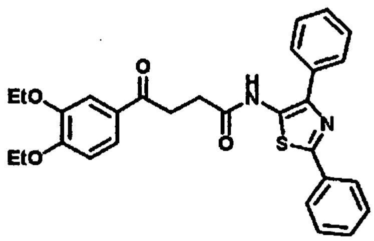

(1) 2,4-diphenyl-1,3-thiazol-5-amine

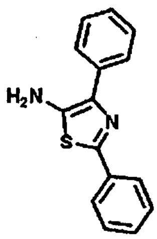

To a solution of 2,4-diphenyl-1,3-thiazole-5-carboxylic acid synthesized according to a method described in Tetrahedron, 58, 2002, 8581-8590 (2.0 g) in toluene (40 mL) were added triethylamine (1.3 mL) and DPPA (1.8 mL), and the mixture was stirred at room temperature for 1 hr. tert-Butanol (3.0 mL) was added, and the mixture was heated overnight at 90°C. The reaction mixture was concentrated. The obtained residue was purified by silica gel column chromatography [developing solvent: hexane-ethyl acetate (4:1)] to give tert-butyl (2,4-diphenyl-1,3-thiazol-5-yl) carbamate (1.7 g) as crystals. Trifluoroacetic acid (5 mL) was added thereto, and the mixture was stirred at room temperature for 2 hr, and concentrated. The residue was alkalified with aqueous sodium hydrogencarbonate solution, and extracted with ethyl acetate. The organic layer was washed with water, dried over anhydrous magnesium sulfate, and concentrated under reduced pressure. The obtained crystals were removed by filtration, and the filtrate was concentrated to give 2,4-diphenyl-1,3-thiazol-5-amine (0.56 g) as a oil. This was used for the next step without purification.
$^{1}$H-NMR ($CDCl_3$) δ: 7.20-7.49(6H, m), 7.79(2H, d, J=7.2Hz), 7.87(2H, d, J=7.2Hz)
MASS m/z: 253.0
(2) 4-(3,4-diethoxyphenyl)-N-(2,4-diphenyl-1,3-thiazol-5-yl)-4-oxobutanamide

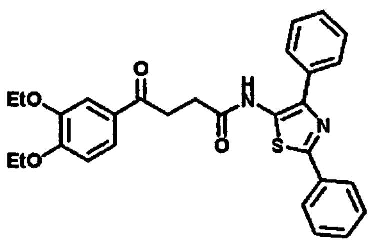

To a suspension of potassium 4-(3,4-diethoxyphenyl)-4,4-dimethoxybutanoate obtained in Example 8-(7) (350 mg) and 2,4,6-trichlorobenzoyl chloride (0.17 mL) in THF (5 mL) was added triethylamine (0.28 mL), and the mixture was stirred at room temperature for 4 hr. 2,4-Diphenyl-1,3-thiazol-5-amine obtained in Example 16-(1) (240 mg) was added to the reaction mixture, and the mixture was heated at 50°C for 2 days. Diluted hydrochloric acid was added to the reaction mixture, and the mixture was stirred for 1 hr, and extracted with ethyl acetate. The organic layer was washed

with water, dried over anhydrous magnesium sulfate, and concentrated under reduced pressure to give 4-(3,4-diethoxyphenyl)-N-(2,4-diphenyl-1,3-thiazol-5-yl)-4-oxobutanamide (125 mg) as crystals.
$^1$H-NMR ($CDCl_3$) δ: 1. 46 (6H, m), 2. 83 (2H, t, J=6. 3Hz), 3.43 (2H, t, J=6.3 Hz), 4.18(4H, m), 6.89 (1H, d, J=8.2Hz), 7.44(4H, m), 7.56(4H, m), 7.75(2H, m), 7.96(2H, m), 8.68 (1H, br s) Anal. Calcd for $C_{29}H_{28}N_2O_4S \cdot O.2H_2O$: C, 69 . 08; H, 5 . 68; N, 5.56.Found: C, 68.82; H, 5.70; N, 5.36.
mp. 139-140°C

Example 17

N-(5-benzyl-4-phenyl-2-thienyl)-4-(3,4-diethoxyphenyl)-4-oxobutanamide

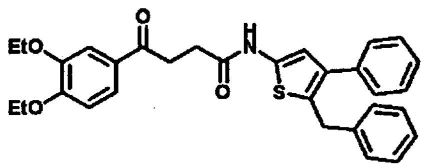

(1) ethyl 2-amino-5-benzyl-4-phenylthiophene-3-carboxylate

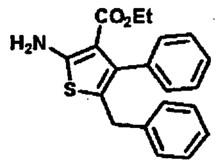

To a solution of 1,3-diphenylacetone (5.77 g) and ethyl cyanoacetate (2.9 mL) in toluene (6 mL) were added ammonium acetate (0.42 g) and acetic acid (1.26 mL), and the mixture was heated under reflux overnight while evaporating water using Dean-Stark. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure. The obtained residue was dissolved in ethanol (100 mL), sulfur (0.9 g) and diisopropyl ether (4 mL) were added, and the mixture was heated overnight at 70°C. The reaction mixture was concentrated, and the obtained residue was purified by silica gel column chromatography [developing solvent: hexane-acetate ethyl (7:1)] to give ethyl 2-amino-5-benzyl-4-phenylthiophene-3-carboxylate (6.9 g) as crystals.
$^1$H-NMR ($CDCl_3$) δ: 0.79 (3H, t, J=6.9Hz), 3.73 (2H, s), 3. 91 (2H, q, J=6.9Hz), 5.98(2H, br s), 7.12(2H, d, J=6.6Hz), 7.14-7.34(9H, m)
(2) 5-benzyl-4-phenylthiophen-2-amine

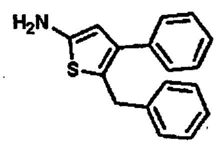

To a solution of ethyl 2-amino-5-benzyl-4-phenylthiophene-3-carboxylate obtained in Example 17-(1) (25.5 g) in ethanol (100 mL) was added aqueous potassium hydroxide solution (potassium hydroxide 60 g, in water 300 mL), and the mixture was heated under reflux for 4 days. The reaction mixture was extracted with ethyl acetate, and the organic layer was washed with saturated brine, and dried over anhydrous magnesium sulfate, and the solvent was evaporated under reduced pressure. The obtained residue was purified by silica gel column chromatography [developing solvent: hexane-ethyl acetate (7:1)] to give 5-benzyl-4-phenylthiophen-2-amine (11.8 g) as crystals. $^1$H-NMR ($CDCl_3$) δ: 4.18 (2H, s), 6.22(2H, br s), 7.07-7.31(11H, m)
(3) N-(5-benzyl-4-phenyl-2-thienyl)-4-(3,4-diethoxyphenyl)-4-oxobutanamide

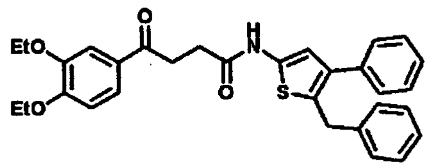

To a suspension of 5-benzyl-4-phenylthiophen-2-amine obtained in Example 17-(2) (265 mg), 4-(3,4-diethoxyphenyl)-4-oxobutanoic acid obtained in Example 3-(1) (530 mg) and HOBt (306 mg) in acetonitrile (20 mL) was added WSC (0.35 mL), and the mixture was stirred at 50°C for 2 hr. The reaction solution was cooled under ice bath, and the precipitated solid was collected by filtration to give N-(5-benzyl-4-phenyl-2-thienyl)-4-(3,4-diethoxyphenyl)-4-oxobutanamide (221 mg).
$^1$H-NMR ($CDCl_3$) δ: 1.47(6H, m), 2.80(2H, t, J=6.6Hz), 3.41(2H, d, J=6.6Hz), 4.10(2H, m), 4.16(4H, m), 6.64(1H, s), 6.87(1H, d, J=8.4Hz), 7.16-7.39(10H, m), 7.51(1H, d, J = 1.8 Hz), 7.59(1H, dd, J=8.4 Hz, 1.8Hz), 8.63(1H, br s)
Anal. Calcd for $C_{31}H_{31}NO_4$: C, 72.49; H, 6.08; N, 2.73.Found: C, 72.31; H, 6.05; N, 2.54.

Example 18

4-(3,4-diethoxyphenyl)-N-(2,5-diphenyl-3-thienyl)-4-oxobutanamide

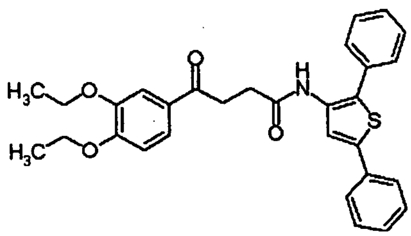

(1) methyl 2,5-diphenylthiophene-3-carboxylate

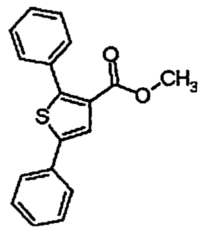

To a solution of methyl 2,5-dichlorothiophene-3-carboxylate (2.11 g) in 1,2-dimethoxyethane (15 mL) were added phenylboronic acid (2.43 g), tetrakis(triphenylphosphine)palladium(0) (324 mg) and 2N aqueous sodium carbonate solution (25 mL), and the mixture was heated under reflux for 16 hr under nitrogen atmosphere. The reaction mixture was extracted with ethyl acetate, and the organic layer was washed with saturated brine, dried over anhydrous magnesium sulfate, and filtered, and the solvent was evaporated under reduced pressure. The residue was purified by silica gel column chromatography [developing solvent: n-hexane-ethyl acetate (10:1)] to give methyl 2,5-diphenylthiophene-3-carboxylate (2.26 g).
$^1$H-NMR($CDCl_3$)δ: 3.76(3H,s), 7.29-7.47(6H, m), 7.50-7.66(4H,m), 7.71 (1H, s)
(2) 2,5-diphenylthiophene-3-carboxylic acid

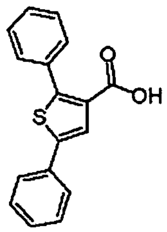

To a solution of methyl 2,5-diphenylthiophene-3-carboxylate obtained in Example 18-(1) (2.26 g) in methanol (40 mL)-THF (40 mL) was added 1N aqueous sodium hydroxide solution (9.22 mL), and the mixture was heated under reflux for 16 hr. 1N Hydrochloric acid (9.22 mL) was added to the reaction mixture, and the mixture was concentrated under reduced pressure. Water was added to the residue, and the precipitate was collected by filtration, and washed with water and n-hexane to give 2,5-diphenylthiophene-3-carboxylic acid (2.052 g).
$^1$H-NMR($CDCl_3$)δ: 7.32-7.50(6H, m), 7.51-7.59(2H, m), 7.68-7.74(2H, m), 7.79(1H, s), 12.77(1H, br s)
(3) (2-trimethylsilyl)ethyl (2,5-diphenyl-3-thienyl)carbamate

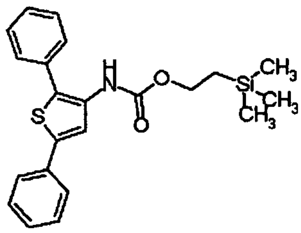

To a solution of 2,5-diphenylthiophene-3-carboxylic acid obtained in Example 18-(2) (692 mg) in THF (10 mL) were added triethylamine (0.516 mL) and DPPA (0.585 mL), and the mixture was stirred at room temperature stirred for 1 hr. 2-(Trimethylsilyl)ethanol (1.061 mL) was added to the reaction mixture, and the mixture was heated under reflux overnight. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated under reduced pressure. The residue was purified by silica gel column chromatography [developing solvent: n-hexane-ethyl acetate (19:1→7:3)] to give (2-trimethylsilyl)ethyl (2,5-diphenyl-3-thienyl)carbamate (342.4 mg) as a colorless solid.
$^1$H-NMR($CDCl_3$)δ: 0.06(9H, s), 1.01-1.09(2H, m), 4.22-4.31(2H, m), 6.72(1H, s), 7.28-7.42(4H, m), 7.44-7.52(4H, m), 7.59-7.69(2H, m), 7.91(1H, s)
(4) (2,5-diphenyl-3-thienyl)amine

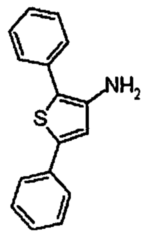

To a solution of (2-trimethylsilyl)ethyl (2,5-diphenyl-3-thienyl)carbamate obtained in Example 18-(3) (771.1 mg) in THF (30.0 mL)-water (3.0 mL) was added 1M tetrabutylammonium fluoride-THF solution (3.6 mL), and the mixture was stirred overnight at 60°C. 1M Tetrabutylammonium fluoride-THF solution (1.8 mL) was added again, and the mixture was heated under reflux for 3 days. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated under reduced pressure. The residue was purified by silica gel column chromatography [developing solvent: n-hexane-ethyl acetate (19:1→1:1)], and the obtained crystals were recrystallized from ethyl acetate-n-hexane (1:3)

to give (2,5-diphenyl-3-thienyl)amine (452.7 mg) as yellow crystals.
$^1$H-NMR($CDCl_3$)δ: 3.74(2H, br s), 6.91(1H, s), 7.26-7.31(2H, m), 7.34-7.46(4H,m), 7.53-7.61(4H, m)
MASS m/z: 252.04 ($MH^+$)
(5) 4-(3,4-diethoxyphenyl)-N-(2,5-diphenyl-3-thienyl)-4-oxobutanamide

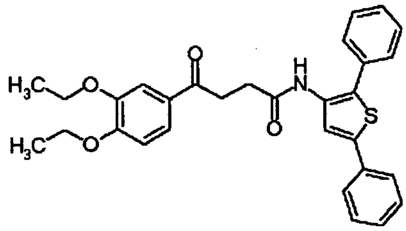

Using potassium 4-(3,4-diethoxyphenyl)-4,4-dimethoxybutanoate obtained in Example 8-(7) (403.0 mg), triethylamine (166 μL), 2,4,6-trichlorobenzoyl chloride (180 μL) and (2,5-diphenyl-2-thienyl)amine obtained in Example 18-(4) (145 mg) as starting materials and in the same manner as in Example 12-(3), 4-(3,4-diethoxyphenyl)-N-(2,5-diphenyl-3-thienyl)-4-oxobutanamide (209.9 mg) was obtained as white crystals.
$^1$H-NMR ($CDCl_3$) δ: 1.47(3H, t, J=7.0Hz), 1.49(3H, t, J=7.0Hz), 2.75(2H, t, J=6.4Hz), 3.41(2H, t, J=6.4Hz), 4.15(2H, q, J=7.0Hz), 4.17(2H, q, J=7.0Hz), 6.89(1H, d, J=8.5Hz), 7.27-7.40(4H, m), 7.45-7.56(5H, m), 7.59-7.65(3H, m), 7.76(1H, s), 8.04(1H, s)
MASS m/z: 500.13($MH^+$)
mp/ 144.6-145.3°C

Example 19

N-(1-benzyl-2-phenyl-1H-pyrrol-3-yl)-4-(3,4-diethoxyphenyl)-4-oxobutanamide

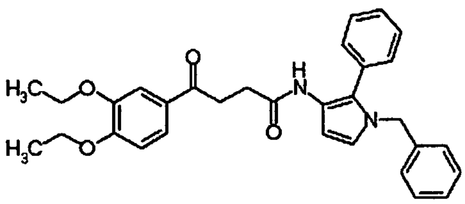

(1) 1-benzyl-2-phenyl-1H-pyrrole-3-carboxylic acid

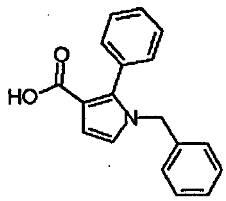

To a solution of methyl 1-benzyl-2-phenyl-1H-pyrrole-3-carboxylate synthesized according to a method described in J. Org. Chem. 1984, 49, 3314-3322 (1.264 g) in THF (20 mL)-ethanol (20 mL) were added 1N aqueous sodium hydroxide solution (17.4 mL) and 1N aqueous lithium hydroxide solution (4.34 mL), and the mixture was heated under reflux overnight. The solvent was concentrated under reduced pressure, and the residue was dissolved in water. The mixture was acidified with 1N hydrochloric acid, and extracted with ethyl acetate. The organic layer was washed with saturated brine, dried over anhydrous sodium sulfate, and filtered, and the solvent was evaporated under reduced pressure. The residue was purified by silica gel column chromatography [developing solvent: n-hexane-ethyl acetate (4:1→1:4)], and the obtained crystals were recrystallized from ethyl acetate-n-hexane (1:3) to give 1-benzyl-2-phenyl-1H-pyrrole-3-carboxylic acid (1.033 g) as white crystals.
$^1$H-NNR ($CDCl_3$) δ: 4.90 (2H, s), 6.67 (1H, d, J=3.Hz), 6.76 (1H, d, J=3.0Hz), 6.93(2H, dd, J=7.5Hz, 2.1Hz), 7.19-7.39 (8H, m)
MASS m/z: 278.1 ($MH^+$)
(2) 2-(trimethylsilyl)ethyl (1-benzyl-2-phenyl-1H-pyrrol-3-yl)carbamate

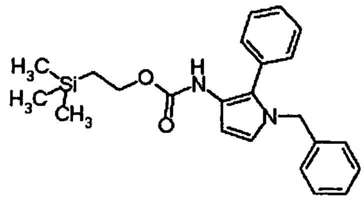

To a solution of 1-benzyl-2-phenyl-1H-pyrrole-3-carboxylic acid obtained in Example 19-(1) (401 mg) in THF (10 mL) were added triethylamine (0.302 mL) and DPPA (0.343 mL), and the mixture was heated under reflux for 2 hr, and cooled to room temperature. 2-(Trimethylsilyl)ethanol (0.622 mL) was added to the reaction mixture, and the mixture was heated under reflux for 2 hr. The reaction mixture was poured into 10% aqueous sodium hydrogencarbonate solution, and the mixture was extracted with ethyl acetate. The organic layer was washed with saturated brine, dried over anhydrous sodium sulfate, and filtered, and the solvent was evaporated under reduced pressure. The residue was purified by silica gel column chromatography [developing solvent: n-hexane-ethyl acetate (19:1→7:3)] to give 2-(trimethylsilyl)ethyl (1-benzyl-2-phenyl-1H-pyrrol-3-yl)carbamate (325.4 mg) as a colorless liquid.
$^1$H-NMR ($CDCl_3$) δ: 0.02 (9H, s), 0.92-1.06(2H,m), 4.14-4.24(2H,m), 4.97(2H, s), 6.63(1H, s), 6.93(1H, s), 6.95(1H, d, J=1.6Hz), 7.23-7.42(10H, m)
MASS m/z: 393.2 ($MH^+$)
(3) 1-benzyl-2-phenyl-1H-pyrrol-3-amine

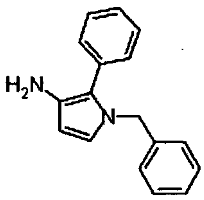

To a solution of 2-(trimethylsilyl)ethyl (1-benzyl-2-phenyl-1H-pyrrol-3-yl)carbamate obtained in Example 19-(2). (325.4 mg) in THF (10.0 mL)-water (0.015 mL) was added 1M tetrabutylammonium fluoride-THF solution (0.829 mL), and the mixture was stirred at room temperature for 2 hr. 1M Tetrabutylammonium fluoride-THF solution (0.829 mL) was added again, and the mixture was stirred at 60°C for 2 hr. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated under reduced pressure. The residue was purified by silica gel column chromatography [developing solvent: n-hexane-ethyl acetate (9:1→2:3)], and the obtained crystals were recrystallized from ethyl acetate-n-hexane (1:3) to give 1-benzyl-2-phenyl-1H-pyrrol-3-amine (152.8 mg) as a yellow liquid.
$^1$H-NMR ($CDCl_3$) δ: 4.97(2H,s), 5.92(1H, d, J=2.8Hz), 6.54(1H, d, J=2.8Hz), 6.94(1H, s), 6.96(2H, d, J=1.3Hz), 7.22-7.43(9H, m) MASS m/z: 249.1($MH^+$)
(4) N-(1-benzyl-2-phenyl-1H-pyrrol-3-yl)-4-(3,4-diethoxyphenyl)-4-oxobutanamide

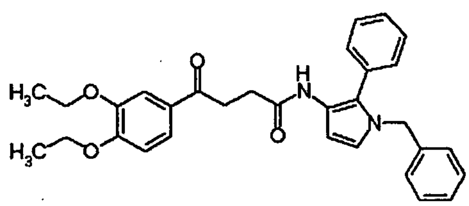

To a suspension of potassium 4-(3,4-diethoxyphenyl)-4,4-dimethoxybutanoate obtained in Example 8-(7) (431 mg) in THF (10 mL) were added triethylamine (0.18 mL) and 2,4,6-trichlorobenzoyl chloride (0.192 mL), and the mixture was stirred at room temperature stirred for 4 hr. A solution of 1-benzyl-2-phenyl-1H-pyrrol-3-amine obtained in Example 19-(3) (152.8 mg) in THF (15 mL) was added to the reaction mixture, and the mixture was heated under reflux overnight. The reaction mixture was poured into 10% aqueous sodium hydrogencarbonate solution, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (10:1→1:1)] to give a brown liquid. This was dissolved in acetonitrile (5 mL)-water (0.05 mL), and trifluoroacetic acid (1.0 mL) was added. The mixture was stood still at room temperature for 20 min, and concentrated under reduced pressure. The residue was purified by silica gel column chromatography [developing solvent: n-hexane-ethyl acetate (9:1-.1:1)] to give N-(1-benzyl-2-phenyl-1H-pyrrol-3-yl)-4-(3,4-diethoxyphenyl)-4-oxobutanamide (59.5 mg) as a colorless viscous liquid.
$^1$H-NMR($CDCl_3$)δ: 1.47(3H, t, J=7.0Hz), 1.49(3H, t, J=7.0Hz), 2.65(2H, t, J=6.7Hz), 3.35 (2H, t, J=6. 7Hz), 4 .14 (2H, q, J=7.0Hz), 4.17(2H, q, J=7.0Hz), 4.98(2H, s), 6.65(1H, d, J=3.0Hz), 6.78 (1H, d, J=3.0Hz), 6.84-6.98(3H, m), 7.15-7.41 (9H, m), 7.49(1H, d, J=2.1Hz), 7.59(1H, m)
MASS m/z: 497.2 ($MH^+$)

<u>Example 20</u>

N-(1-benzyl-3-phenyl-1H-pyrazol-4-yl)-4-(3,4-diethoxyphenyl)-4-oxobutanamide

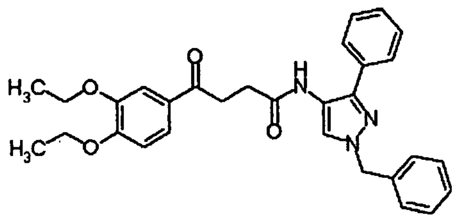

(1) ethyl 1-benzyl-3-phenyl-1H-pyrazole-4-carboxylate and ethyl 1-benzyl-5-phenyl-1H-pyrazole-4-carboxylate

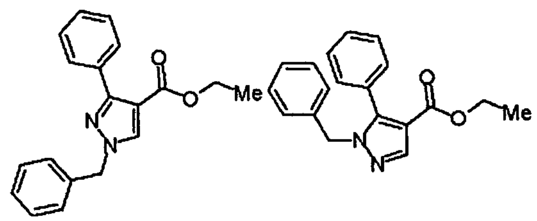

A solution of ethyl benzoylacetate (3.00 g) and N,N-dimethylacetamide dimethylacetal (2.49 mL) in toluene (50 mL) was heated under reflux overnight. The reaction mixture was concentrated under reduced pressure, and the residue was dissolved in ethanol (50 mL). Benzylhydrazine hydrochloride (2.72 g) and trimethylamine (2.39 mL) were added, and the mixture was heated under reflux for 3 hr. The reaction mixture was poured into water, and the mixture was extracted with ethyl acetate. The organic layer was washed with saturated brine, dried over anhydrous sodium sulfate, and filtered, and the solvent was evaporated. The residue was purified by silica gel column chromatography [developing solvent: n-hexane-ethyl acetate (19:1→7:3)] to give ethyl 1-benzyl-3-phenyl-1H-pyrazole-4-carboxylate (815 mg) as a colorless liquid, and ethyl 1-benzyl-5-phenyl-1H-pyrazole-4-carboxylate (2.25 g) as a colorless solid.
ethyl 1-benzyl-3-phenyl-1H-pyrazole-4-carboxylate

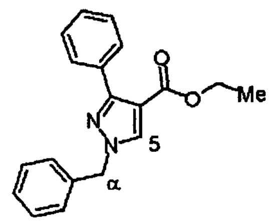

$^1$H-NMR ($CDCl_3$) δ: 1.25(3H, t, J=7.0Hz), 4.21(2H, q, J=7.0Hz), 5.34(2H, s), 7.29-7.44(8H, m), 7.78(2H, dd, J=7.7Hz, 1.7Hz), 7.91 (1H, s)
NOE was observed for H at the 5-position of pyrazole and H at the α-position of benzyl group.
$^{13}$C-NMR ($CDCl_3$) : 14.24, 56.50, 60.11, 112.26, 127.82, 128.11, 128.41, 128.52, 129.02, 129.34, 132.43, 134.86 (the 5-position of pyrazole), 135.29, 153.17, 163.06
MASS m/z: 307.05($MH^+$)
ethyl benzyl-5-phenyl-1H-pyrazole-4-carboxylate

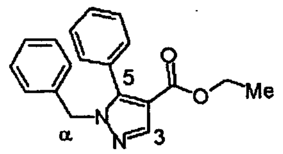

$^1$H-NMR ($CDCl_3$)δ: 1.15(3H, t, J=7.1Hz), 4.14(2H, q, J=7.0Hz), 5.18(2H, s), 7.00(2H, dd, J=7.0Hz, 2.5Hz), 7.25-7.29 (5H, m), 7.38-7.49(3H, m), 8.06(1H, s)
$^{13}$C-NMR($CDCl_3$):14.09, 53.38(α-position of benzyl group), 59.85, 113.19, 127.22, 127.83, 128.23, 128.65, 129.06, 129.38, 130.00, 136.46, 141.60(the 3-position of pyrazole), 146.3(the 5-position of pyrazole), 162.99
The long range coupling was observed for C at the 5-position of pyrazole and H at α-position of benzyl group.
MASS m/z: 307.07 ($MH^+$)
(2) 1-benzyl-3-phenyl-1H-pyrazole-4-carboxylic acid

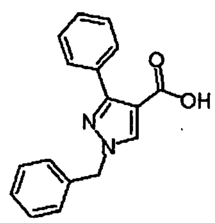

To a solution of ethyl 1-benzyl-3-phenyl-1H-pyrazole-4-carboxylate obtained in Example 20-(1) (815 mg) in THF: EtOH=1:1 (60 mL) were added 1N aqueous sodium hydroxide solution (20 mL) and 1N aqueous lithium hydroxide solution (2.0 mL), and the mixture was heated under reflux for 8 hr. The solvent was concentrated under reduced pressure, and the residue was dissolved in water. The mixture was washed with ether. The aqueous layer was adjusted to pH 2 with 1N hydrochloric acid, and extracted with ethyl acetate. The organic layer was washed with saturated brine, dried over anhydrous sodium sulfate, and filtered, and the solvent was evaporated under reduced pressure. The residue was recrystallized from ethyl acetate-n-hexane (1:3) to give 1-benzyl-3-phenyl-1H-pyrazole-4-carboxylic acid (667 mg) as white crystals.
$^1$H-NMR($CDCl_3$)δ: 5.34(2H, s), 7.30-7.44 (8H,m), 7.75-7.80 (2H,m), 7.95 (1H, s)
MASS m/z: 279.06($MH^+$)
(3) 2-(trimethylsilyl)ethyl (1-benzyl-3-phenyl-1H-pyrazol-4-yl)carbamate

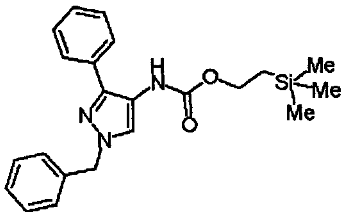

To a solution of 1-benzyl-3-phenyl-1H-pyrazole-4-carboxylic acid obtained in Example 20-(2) (300.2 mg) and triethylamine (0.226 mL) in THF (10 mL) was added DPPA (0.268 mL), and the mixture was stirred at room temperature stirred for 1 hr. Then, 2-(trimethylsilyl)ethanol (0.464 mL) was added to the reaction mixture, and the mixture was heated under reflux for 6 hr. The reaction mixture was poured into 5% aqueous sodium hydrogencarbonate solution, and the mixture was extracted with ethyl acetate. The organic layer was washed with saturated aqueous sodium hydrogencarbonate solution and saturated brine, dried over anhydrous sodium sulfate, and filtered, and the solvent was evaporated under reduced pressure. The residue was purified by silica gel column chromatography [developing solvent: n-hexane-ethyl acetate (9:1→1:1)] to give 2-(trimethylsilyl)ethyl (1-benzyl-3-phenyl-1H-pyrazol-4-yl)carbamate (425 mg) as a colorless liquid.

$^1$H-NMR ($CDCl_3$) δ: 0.04 (9H, s), 0.87-1.00 (2H, m), 4.18-4.25(2H, m), 5.30(2H, s), 6.41(2H, br s), 7.27-7.39(6H, m), 7.45(2H, t, J=7.4Hz), 7.56-7.69 (2H, m), 7.83 (1H, s)

MASS m/z: 394.06($MH^+$)

(4) 1-benzyl-3-phenyl-1H-pyrazol-4-amine

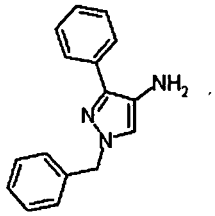

To a solution of 2-(trimethylsilyl)ethyl (1-benzyl-3-phenyl-1H-pyrazol-4-yl)carbamate obtained in Example 20-(3) (423.7 mg) in THF (10 mL)-water (1 mL) was added 1M tetra-n-butylammonium fluoride-THF solution (5 mL), and the mixture was heated under reflux for 6 hr. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated under reduced pressure. The residue was purified by silica gel column chromatography [developing solvent: n-hexane-ethyl acetate (4:1)→ethyl acetate], and the obtained crystals were recrystallized from ethyl acetate-n-hexane (1:3) to give 1-benzyl-3-phenyl-1H-pyrazol-4-amine (215.4 mg).

$^1$H-NMR ($CDCl_3$) δ: 5.25 (2H, s), 7.01(1H, s), 7.24-7.25(2H, m), 7.27-7.38(6H, m), 7.40-7.46(2H, m), 7.73-7.78(2H, m)

MASS m/z: 250.08 ($MH^+$)

(5) N-(1-benzyl-3-phenyl-1H-pyrazol-4-yl)-4-(3,4-diethoxyphenyl)butanamide

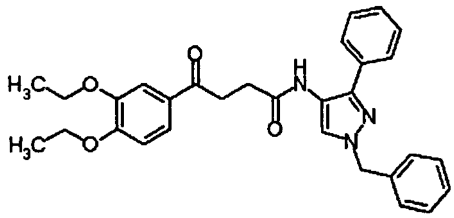

To a suspension of potassium 4-(3,4-diethoxyphenyl)-4,4-dimethoxybutanoate obtained in Example 8-(7) (116.0 mg) in THF (5.0 mL) were added triethylamine (0.070 mL) and 2,4,6-trichlorobenzoyl chloride (0.074 mL), and the mixture was stirred at room temperature 5 hr. 1-Benzyl-3-phenyl-1H-pyrazol-4-amine obtained in Example 20-(4) (59.2 mg) was added to the reaction mixture, and the mixture was stirred at 60°C for 3 days. The reaction mixture was poured into water, and the mixture was extracted with ethyl acetate. The organic layer was washed with saturated

aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (4:1→2:3)] to give a red liquid (98.7 mg). This was dissolved in acetonitrile (10 mL), and trifluoroacetic acid (1.0 mL) was added. The mixture was stood still at room temperature for 5 min, and concentrated under reduced pressure. The residue was purified by silica gel column chromatography [developing solvent: n-hexane-ethyl acetate (4:1-.1:4)] to give N-(1-benzyl-3-phenyl-1H-pyrazol-4-yl)-4-(3,4-diethoxyphenyl)butanamide (75.5 mg) as a colorless viscous liquid.
$^1$H-NMR ($CDCl_3$) δ: 1.47(3H, t, J=7.0Hz), 1.49(3H, t, J=7.0Hz), 2.74(2H, t, J=6.5Hz), 3.37(2H, t, J=6.5Hz), 4.13(2H, q, J=7.0Hz), 4.15(2H, q, J=7.0Hz), 5.29(2H, s), 6.88(1H, d, J=8.5Hz), 7.28-7.41(6H, m), 7.45-7.54(3H,m), 7.57-7.69(4H, m), 8.08(1H, s)
MASS m/z: 498.12($MH^+$)

Example 21

N-(1-benzyl-5-phenyl-1H-pyrazol-4-yl)-4-(3,4-diethoxyphenyl)-4-oxobutanamide

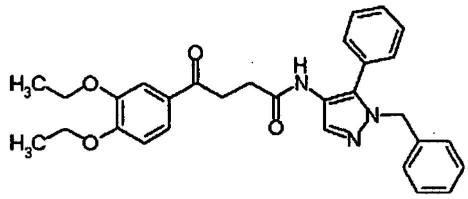

(1) 1-benzyl-5-phenyl-1H-pyrazole-4-carboxylic acid

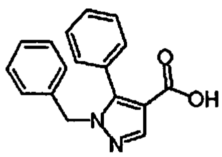

To a solution of ethyl 1-benzyl-5-phenyl-1H-pyrazole-4-carboxylate obtained in Example 20-(1) (2.115 g) in THF (30 mL)-ethanol (30 mL) were added 1N aqueous sodium hydroxide solution (20 mL) and 1N aqueous lithium hydroxide solution (6 mL), and the mixture was heated under reflux for 4 hr. The reaction mixture was concentrated under reduced pressure, and the residue was dissolved in water. The mixture was washed with diethyl ether. The aqueous layer was acidified with 1N hydrochloric acid, and extracted with ethyl acetate. The organic layer was washed with saturated brine, dried over anhydrous sodium sulfate, and filtered, and the solvent was evaporated under reduced pressure. The residue was recrystallized from ethyl acetate-n-hexane (1:3) to give 1-benzyl-5-phenyl-1H-pyrazole-4-carboxylic acid (1.8667 g) as white crystals.
$^1$H-NMR($CDCl_3$)δ: 5.16 (2H, s), 6.97-7.03(2H, m), 7.25-7. 31 (5H, m), 7.38-7.49(3H, m), 8.09(1H, s)
MASS m/z: 279.00($MH^+$)
(2) 2-(trimethylsilyl)ethyl (1-benzyl-5-phenyl-1H-pyrazol-4-yl)carbamate

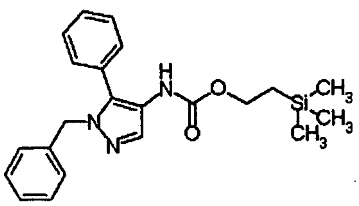

To a solution of 1-benzyl-5-phenyl-1H-pyrazole-4-carboxylic acid obtained in Example 21-(1) (400.5 mg) in THF (10 mL) were added triethylamine (0.301 mL) and DPPA (0.357 mL), and the mixture was stirred at room temperature

for 30 min. 2-(Trimethylsilyl)ethanol (0.619 mL) was added to the reaction mixture, and the mixture was heated under reflux for 24 hr. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated under reduced pressure. The residue was purified by silica gel column chromatography [developing solvent: n-hexane-ethyl acetate (10:1→1:1)], and the obtained crystals were recrystallized from n-hexane to give 2-(trimethylsilyl)ethyl (1-benzyl-5-phenyl-1H-pyrazol-4-yl)carbamate (494.8 mg) as white crystals.
$^1$H-NMR ($CDCl_3$) δ : 0.03(9H, s), 0.95-1.05(2H, m), 4.17-4.28(2H, m), 5.21(1H, s), 6.02(1H, br s), 6.97-7.03(2H, m), 7.20-7.25(6H, m), 7.40-7.48 (3H, m)
MASS m/z: 394.05 ($MH^+$)
(3) 1-benzyl-5-phenyl-1H-pyrazol-4-amine

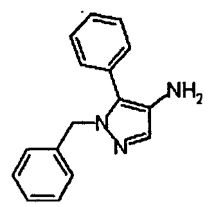

To a solution of 2-(trimethylsilyl)ethyl (1-benzyl-5-phenyl-1H-pyrazol-4-yl)carbamate obtained in Example 21-(2) (472.3 mg) in THF (20 mL)-water (1 mL) was added 1M tetra-n-butylammonium fluoride-THF solution (2.4 mL), and the mixture was heated under reflux for 24 hr. 1M Tetra-n-butylammonium fluoride-THF solution (1.2 mL) was added again, and the mixture was heated under reflux for 2 days. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated under reduced pressure. The residue was purified by silica gel column chromatography [developing solvent: n-hexane-ethyl acetate (4:1)→ethyl acetate], and the obtained crystals were recrystallized from ethyl acetate-n-hexane (1:3) to give 1-benzyl-5-phenyl-1H-pyrazol-4-amine (255.8 mg).
$^1$H-NMR($CDCl_3$) δ:5.20(2H, s), 6.93-7.05(2H, m), 7.18-7.46(11H, m) MASS m/z: 250.08($MH^+$)
(4) N-(1-benzyl-5-phenyl-1H-pyrazol-4-yl)-4-(3,4-diethoxyphenyl)-4-oxobutanamide

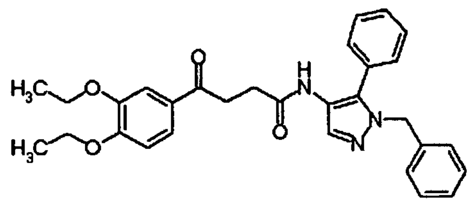

To a suspension of potassium 4-(3,4-diethoxyphenyl)-4,4-dimethoxybutanoate obtained in Example 8 -(7) (213 mg) in THF (10 mL) were added triethylamine (88.9 μL) and 2,4,6-trichlorobenzoyl chloride (95 μL), and the mixture was stirred at room temperature 5 hr. 1-Benzyl-5-phenyl-1H-pyrazol-4-amine obtained in Example 21-(3) (75.8 mg) was added to the reaction mixture, and the mixture was stirred overnight at 60°C. The reaction mixture was poured into water, and the mixture was extracted with ethyl acetate. The organic layer was washed with 1N hydrochloric acid, water, saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (7:3)→ethyl acetate] to give N-(1-benzyl-5-phenyl-1H-pyrazol-4-yl)-4-(3,4-diethoxyphenyl)-4-oxobutanamide (102.6 mg) as colorless viscous liquid.
$^1$H-NMR ($CDCl_3$) δ: 1.47 (3H, t, J=7.0Hz), 1.49(3H, t, J=7.0Hz), 2.67 (2H, t, J=6.5Hz), 3.35 (2H, t, J=6. 5Hz) , 4 .14 (2H, q, J=7.0Hz), 4.17 (2H, q, J=7.0Hz), 5.22 (2H, s), 6.87 (1H, d, J=8.3Hz), 6. 96-7.01(2H, m), 7.16-7.25(6H,m), 7.42-7.48 (4H,m), 7.58(1H, dd, J=8.5Hz, 2.0Hz), 8.15 (1H, s)
MASS m/z: 498.11 ($MH^+$)

Example 22

4-(3,4-diethoxyphenyl)-N-[4-(4-hydroxyphenyl)quinolin-2-yl]-4-oxobutanamide

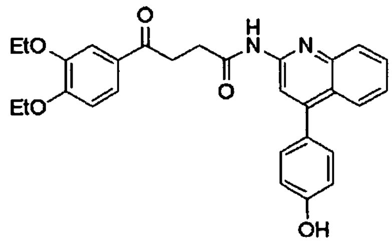

4-(3,4-Diethoxyphenyl)-N-{4-[4-(methoxymethoxy)phenyl]quinolin-2-yl}-4-oxobutanamide obtained in Example 15-(5) (2.8 g) was dissolved in THF (50 mL), concentrated hydrochloric acid (2 mL) was added, and the mixture was stirred overnight. The reaction mixture was concentrated under reduced pressure to give 4-(3,4-diethoxyphenyl)-N-[4-(4-hydroxyphenyl)quinolin-2-yl]-4-oxobutanamide (2.16 g).
$^1$H-NMR(DMSO-$d_6$)δ: 1.34 (6H, m), 2.78-2.89(2H, m), 3.26-3.36(2H, m), 4.01-4.18 (4H, m), 6.95 (2H, d, J=8.48Hz), 7.06 (1H, d, J=8.48Hz), 7.35(2H, d, J=8.48 Hz), 7.42-7.50(2H, m), 7.62-7.76 (2H, m), 7.85 (2H, t, J=8.95Hz), 8.19 (1H, s), 9.80 (1H, s), 10.95(1H, s)

Example 23

[4-(2-{[4-(3,4-diethoxyphenyl)-4-oxobutanoyl]amino}quinolin-4-yl)phenoxy]acetic acid

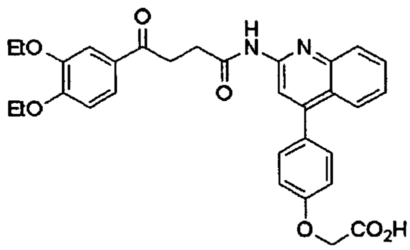

(1) tert-butyl [4-(2-{[4-(3,4-diethoxyphenyl)-4-oxobutanoyl]amino}quinolin-4-yl)phenoxy]acetate

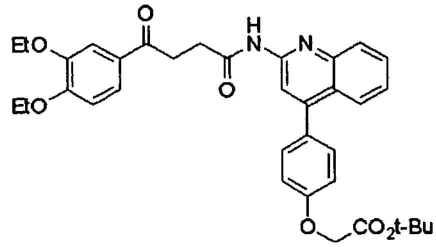

4-(3,4-Diethoxyphenyl)-N-[4-(4-hydroxyphenyl)quinolin-2-yl]-4-oxobutanamide obtained in Example 22 (0.100 g), tert-butyl bromoacetate (0.060 g), potassium carbonate (0.057 g) and potassium iodide (0.051 g) were dissolved in DMF (3 mL), and the mixture was stirred for 3 hr. Water was added to the reaction mixture, and the mixture was extracted with ethyl acetate. The organic layer was washed with saturated brine, dried over anhydrous magnesium

sulfate, and vacuum-dried. The obtained crystals were recrystallized from diethyl ether to give tert-butyl [4-(2-{[4-(3,4-diethoxyphenyl)-4-oxobutanoyl]amino}quinolin-4-yl)phenoxy]acetate (0.081 g) as colorless crystals.
(2) [4-(2-{[4-(3,4-diethoxyphenyl)-4-oxobutanoyl]amino}quinolin-4-yl)phenoxy]acetic acid

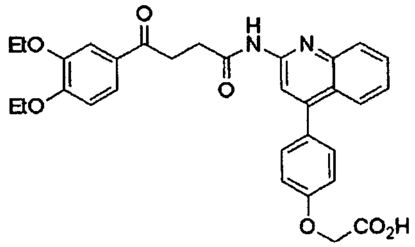

tert-Butyl [4-(2-{[4-(3,4-diethoxyphenyl)-4-oxobutanoyl]amino}quinolin-4-yl)phenoxy]acetate obtained in Example 23-(1) (0.080 g) was dissolved in trifluoroacetic acid (0.8 mL), and the mixture was stirred for 30 min. Trifluoroacetic acid was evaporated under reduced pressure, and the obtained crystals were recrystallized from diethyl ether to give [4-(2-{[4-(3,4-diethoxyphenyl)-4-oxobutanoyl]amino}quinolin-4-yl)phenoxy]acetic acid (0.069 g) as pale-yellow crystals.
$^1$H-NMR(DMSO-$d_6$) δ: 1.34 (6H, q, J=6.78 Hz), 2.84(2H, t, J=6.12Hz), 3.31(2H, t, J=6.22 Hz) 3.98-4.18(4H, m), 4.78(2H, s), 7.03-7.15(3H, m), 7.43-7.52(4H, m), 7.66(1H, dd, J=8.48Hz, 2.07Hz), 7.70-7.78(1H, m), 7.81 (1H, d, J=8.29 Hz), 7.91 (1H, d, J=8.29 Hz), 8.19(1H, s), 11.04 (1H, s)

Example 24

N-(5-benzyl-4-phenyl-1,3-thiazol-2-yl)-4-(4-ethoxy-3-propylphenyl)-4-oxobutanamide

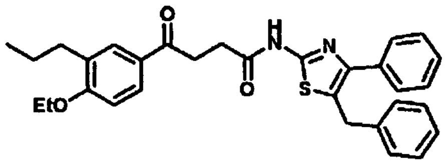

(1) 4-(4-ethoxy-3-propylphenyl)-4-oxobutanoic acid

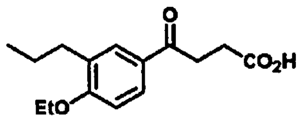

To a suspension of ethoxy-2-propylbenzene (12.0 g) and succinic anhydride (3.66 g) in dichloromethane (30 mL) was gradually added aluminum chloride (10.1 g) under ice-cooling, and the mixture was stirred for 0.5 hr. The reaction mixture was poured into concentrated hydrochloric acid (100 mL)-ice, and the mixture was stirred for 1 hr, and extracted with ethyl acetate. The obtained organic layer was washed with water, dried over anhydrous magnesium sulfate, and concentrated under reduced pressure to give 4-(4-ethoxy-3-propylphenyl)-4-oxobutanoic acid (3.8 g) as crude crystals.
$^1$H-NMR ($CDCl_3$) δ: 0.94 (3H, t, J=7.5Hz), 1.45(3H, t, J=7.2 Hz), 1.62(2H, m), 2.62(2H, t, J=7.8Hz), 2.80(2H, t, J=6.6Hz), 3.28(2H, t, J=6.6Hz), 4.10(2H, q, J=7.2Hz), 6.84(1H, d, J=8.4Hz), 7.82(2H, m)
(2) N-(5-benzyl-4-phenyl-1,3-thiazol-2-yl)-4-(4-ethoxy-3-propylphenyl)-4-oxobutanamide

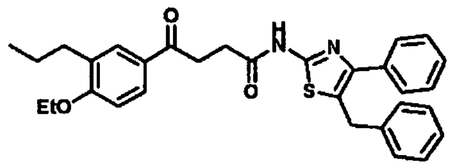

To a suspension of 5-benzyl-4-phenyl-1,3-thiazol-2-amine obtained in Example 1-(2) (266 mg), 4-(4-ethoxy-3-propylphenyl)-4-oxobutanoic acid obtained in Example 24-(1) (476 mg) and HOBt (306 mg) in acetonitrile (20 mL) was added WSC (0.35 mL), and the mixture was stirred at 50°C for 2 hr. The reaction solution was cooled under ice bath, and the precipitated solid was collected by filtration to give N-(5-benzyl-4-phenyl-1,3-thiazol-2-yl)-4-(4-ethoxy-3-propylphenyl)-4-oxobutanamide (213 mg).
$^1$H-NMR ($CDCl_3$) δ: 0.94(3H, t, J=6.9Hz), 1.44(3H, t, J=6.9Hz), 1.60(2H, m), 2.43(2H, t, J=6.4Hz), 2.60(2H, t, J=7.5Hz), 3.17(2H, t, J=6.4Hz), 4.10(2H, q, J=6.9Hz), 4.20(2H, s), 6.82(1H, d, J=8.4Hz), 7.19-7.29(6H, m), 7.38(2H, m), 7.59(2H, d, J=8.4Hz), 7.76(2H, m), 10.75(1H, br s)
Anal. Calcd for $C_{31}H_{32}N_2O_3S$: C, 72.63; H, 6.29; N, 5.46.Found: C, 72.42; H, 6.33; N, 5.46.

Example 25

N-(5-benzyl-4-phenyl-1,3-thiazol-2-yl)-4-(3-bromo-4-ethoxyphenyl)-4-oxobutanamide

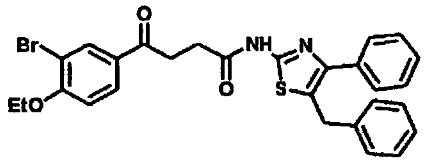

(1) 4-(3-bromo-4-ethoxyphenyl)-4-oxobutanoic acid

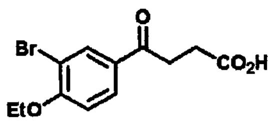

To a suspension of bromo-2-ethoxybenzene (12.0 g) and succinic anhydride (6.9 g) in dichloromethane (40 mL) was gradually added aluminum chloride (19.0 g) under ice-cooling, and the mixture was heated with stirring at 50°C for 0.5 hr. The reaction mixture was cooled, and poured into concentrated hydrochloric acid (100 mL)-ice. The mixture was stirred for 1 hr, and extracted with ethyl acetate. The obtained organic layer was washed with water, dried over anhydrous magnesium sulfate, and concentrated under reduced pressure to give 4-(3-bromo-4-ethoxyphenyl)-4-oxobutanoic acid (6.5 g) as crude crystals.
$^1$H-NMR ($CDCl_3$)δ: 1.51(3H, t, J=7.2Hz), 2.74(2H, t, J=6.6Hz), 3.34(2H, t, J=6.6Hz), 4.19(2H, q, J=7.2 Hz), 6.92 (1H, d, J=8.7Hz), 7.92(1H, dd, J=8.7Hz, 2.4Hz), 8.19(1H, d, J=2.4Hz)
(2) N-(5-benzyl-4-phenyl-1,3-thiazol-2-yl)-4-(3-bromo-4-ethoxyphenyl)-4-oxobutanamide

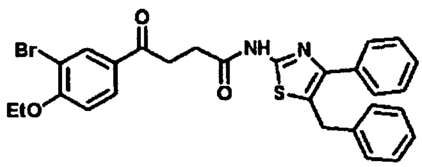

To a suspension of 5-benzyl-4-phenyl-1,3-thiazol-2-amine obtained in Example 1-(2) (266 mg), 4-(3-bromo-4-ethoxyphenyl)-4-oxobutanoic acid obtained in Example 25-(1) (542 mg) and HOBt (306 mg) in acetonitrile (20 mL) was added WSC (0.35 mL), and the mixture was stirred at 50°C for 2 hr. The reaction solution was cooled under ice bath,

and the precipitated solid was collected by filtration to give N-(5-benzyl-4-phenyl-1,3-thiazol-2-yl)-4-(3-bromo-4-ethoxyphenyl)-4-oxobutanamide (160 mg).
$^1$H-NMR ($CDCl_3$) δ: 1.51 (3H, t, J=6.9Hz), 2.36 (2H, t, J=6.6Hz), 3.09 (2H, d, J=6.6Hz), 4.15(2H, q, J=6.9Hz), 4.16 (2H, s), 6.89(1H, d, J=9.0Hz), 7.19-7.35(6H, m), 7.36(2H, t, J=7.2Hz), 7.59(2H, d, J=6.9Hz), 7.85(1H, m), 8.13(1H, d, J=1.8Hz), 10.86(1H, br s) Anal. Calcd for $C_{28}H_{25}BrN_2O_3S$: C, 61.20; H, 4.59; N, 5.10. Found: C, 60.92; H, 4.45; N, 5.16.

Example 26

N-(5-benzyl-4-phenyl-1,3-thiazol-2-yl)-4-oxo-4-(4-propylphenyl)butanamide

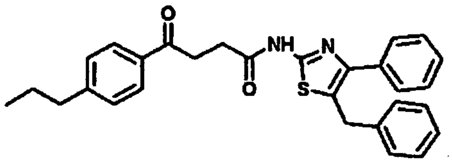

To a suspension of 5-benzyl-4-phenyl-1,3-thiazol-2-amine obtained in Example 1-(2) (266 mg), 4-oxo-4-(4-propylphenyl)butanoic acid (400 mg) and HOBt (306 mg) in acetonitrile (20 mL) was added WSC (0.35 mL), and the mixture was stirred at 50°C for 2 hr. The reaction solution was cooled under ice bath, and the precipitated solid was collected by filtration to give N-(5-benzyl-4-phenyl-1,3-thiazol-2-yl)-4-oxo-4-(4-propylphenyl)butanamide (178 mg).
$^1$H-NMR ($CDCl_3$) δ: 0.94(3H, t, J=7.5Hz) , 1.65 (2H, m), 2.42(2H, t, J=6.6Hz), 2.64(2H, t, J=7.5Hz), 3.18(2H, t, J=6.6Hz), 4.20(2H, s), 7.19-7.30(8H, m), 7.38(2H, t, J=7.2Hz), 7.59(2H, d, J=7.2Hz), 7.85(2H, d, J=7.8Hz), 10.68(1H, br s)
Anal. Calcd for $C_{29}H_{28}N_2O_2S \cdot 0.1H_2O$: C, 74.04; H, 6.04; N, 5.95.Found: C, 73.85; H, 5.99; N, 6.01.

Example 27

N-(5-benzyl-4-phenyl-1,3-thiazol-2-yl)-4-(2,3-dihydro-1-benzofuran-5-yl)-4-oxobutanamide

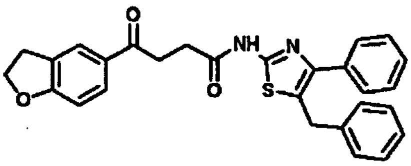

(1) 4-(2,3-dihydro-1-benzofuran-5-yl)-4-oxobutanoic acid

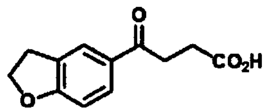

To a suspension of 2,3-dihydro-1-benzofuran (5.2 g) and succinic anhydride (5.2 g) in dichloromethane (30 mL) was gradually added aluminum chloride (14.4 g) under ice-cooling, and the mixture was heated with stirring at 50°C for 0.5 hr. The reaction mixture was cooled, and poured into concentrated hydrochloric acid (100 mL)-ice. The mixture was stirred for 1 hr, and extracted with ethyl acetate. The obtained organic layer was washed with water, dried over anhydrous magnesium sulfate, and concentrated under reduced pressure, and the obtained residue was recrystallized from ethyl acetate to give 4-(2,3-dihydro-1-benzofuran-5-yl)-4-oxobutanoic acid as crystals (3.8 g).
$^1$H-NMR ($CDCl_3$) δ: 2.80 (2H, t, J=6.0 Hz), 3.26(4H, m), 4.67(2H, t, J=9.0 Hz), 6. 82 (1H, d, J=8.4 Hz), 7.85(2H, m)
(2) N-(5-benzyl-4-phenyl-1,3-thiazol-2-yl)-4-(2,3-dihydro-1-benzofuran-5-yl)-4-oxobutanamide

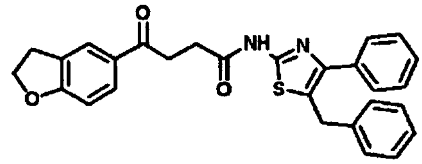

To a suspension of 5-benzyl-4-phenyl-1,3-thiazol-2-amine obtained in Example 1-(2) (266 mg), 4-(2,3-dihydro-1-benzofuran-5-yl)-4-oxobutanoic acid obtained in Example 27-(1) (400 mg) and HOBt (306 mg) in acetonitrile (20 mL) was added WSC (0.35 mL), and the mixture was stirred at 50°C for 2 hr. The reaction solution was cooled under ice bath, and the precipitated solid was collected by filtration to give N-(5-benzyl-4-phenyl-1,3-thiazol-2-yl)-4-(2,3-dihydro-1-benzofuran-5-yl)-4-oxobutanamide (105 mg).

$^1$H-NMR ($CDCl_3$) δ: 2.39 (2H, t, J=6.3Hz), 3.12 (2H, t, J=6.3Hz), 3.22(2H, t, J=9.0Hz), 4.19(2H,s), 4.65 (2H,t, J=9.0Hz), 6.78(1H, d, J=8.1Hz), 7.18-7.29(6H,m), 7.36 (2H, t, J=7.2Hz), 7.57(2H, d, J=7.8Hz), 7.78(2H, m), 10.75 (1H, br s)

Anal. Calcd for $C_{28}H_{24}N_2O_3S$: C, 71.77; H, 5.16; N, 5. 98. Found: C, 71.26; H, 5.19; N, 5.94.

Example 28

N-(5-benzyl-4-phenyl-1,3-thiazol-2-yl)-4-oxo-4-[4-(trifluoromethoxy)phenyl]butanamide

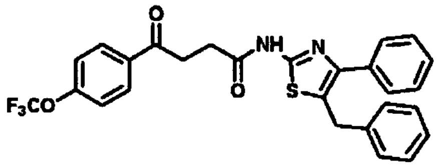

(1) 4-oxo-4-[4-(trifluoromethoxy)phenyl]butanoic acid

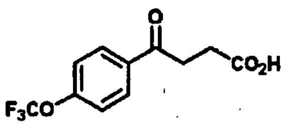

Under ice-cooling, to a solution of succinic anhydride (1.2 g) in THF (50 mL) was added dropwise a solution of 4-(trifluoromethoxy) phenylmagnesium bromide (prepared from magnesium (0.34 g) and 4-bromo-(trifluoromethoxy) benzene (2.84 g)) in THF (15 mL), and the mixture was stirred for 2 hr. Diluted hydrochloric acid was added to the reaction mixture, and the mixture was extracted with ethyl acetate. The obtained organic layer was washed with water, dried over anhydrous magnesium sulfate, and concentrated under reduced pressure, and the obtained crystals were removed by filtration. 1N Aqueous sodium hydroxide solution was added to the obtained filtrate, and the mixture was washed with diethyl ether. The aqueous layer was acidified with diluted hydrochloric acid, and extracted with ethyl acetate. The obtained organic layer was washed with water, dried over anhydrous magnesium sulfate, and concentrated under reduced pressure to give 4-oxo-4-[4-(trifluoromethoxy)phenyl]butanoic acid as crystals (0.7 g).

$^1$H-NMR ($CDCl_3$) δ: 2.82 (2H, t, J=6.3Hz), 3.30 (2H, t, J=6.3Hz), 7.30 (2H, d, J=7.8Hz), 8.04 (2H, d, J=7.8Hz)

(2) N-(5-benzyl-4-phenyl-1,3-thiazol-2-yl)-4-oxo-4-[4-(trifluoromethoxy)phenyl]butanamide

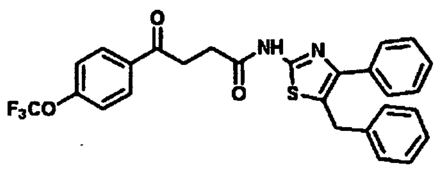

To a suspension of 5-benzyl-4-phenyl-1,3-thiazol-2-amine obtained in Example 1-(2) (266 mg), 4-oxo-4-[4-(trifluoromethoxy)phenyl]butanoic acid obtained in Example 28-(1) (480 mg) and HOBt (306 mg) in acetonitrile (20 mL) was added WSC (0.35 mL), and the mixture was stirred at 50°C for 2 hr. Water was added to the reaction mixture, and the mixture was extracted with ethyl acetate. The obtained organic layer was washed with water, dried over anhydrous magnesium sulfate, and concentrated under reduced pressure. The obtained residue was purified by silica gel column chromatography [developing solvent: hexane-ethyl acetate (3:1)] to give N-(5-benzyl-4-phenyl-1,3-thiazol-2-yl)-4-oxo-4-[4-(trifluoromethoxy)phenyl]butanamide (42 mg) as crystals.
$^1$H-NMR ($CDCl_3$) δ: 2.47(2H, t, J=6.0Hz), 3.20(2H, t, J=6.0Hz), 4.20(2H, s), 7.19-7.32(8H, m), 7.39(2H, m), 7.59(2H, d, J=6.9Hz), 8.00 (2H, d, J=8.7Hz), 10.45 (1H, br s)
Anal. Calcd for $C_{27}H_{21}F_3N_2O_3S$: C, 63.52; H, 4.15; N, 5.49.Found: C, 63.46; H, 4.37; N, 5.30.

Example 29

N-(5-benzyl-4-phenyl-1,3-thiazol-2-yl)-4-(3-methoxy-5,6,7,8-tetrahydronaphthalen-2-yl)-4-oxobutanamide

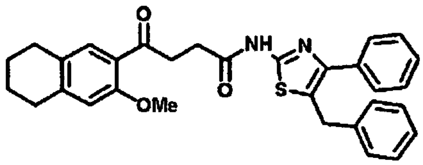

(1) 4-(3-hydroxy-5,6,7,8-tetrahydronaphthalen-2-yl)-4-oxobutanoic acid

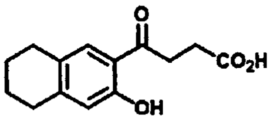

To a suspension of 3-methoxy-5,6,7,8-tetrahydronaphthalene (5.0 g) and succinic anhydride (3.7 g) in dichloromethane (30 mL) was gradually added aluminum chloride (10.3 g) under ice-cooling, and the mixture was stirred at room temperature for 3 hr. The reaction mixture was cooled, and poured into concentrated hydrochloric acid (100 mL)-ice. The mixture was stirred for 1 hr, and extracted with ethyl acetate. The obtained organic layer was washed with water, dried over anhydrous magnesium sulfate, and concentrated under reduced pressure, and the obtained residue was recrystallized from ethyl acetate to give 4-(3-hydroxy-5,6,7,8-tetrahydronaphthalen-2-yl)-4-oxobutanoic acid (4.9 g) as crystals.
$^1$H-NMR ($CDCl_3$) δ: 1.78(4H, m), 2.77(6H, m), 3.30(2H, m), 6.69(1H, s), 7.46(1H, s), 11.74(1H, s)
(2) 4-(3-methoxy-5,6,7,8-tetrahydronaphthalen-2-yl)-4-oxobutanoic acid

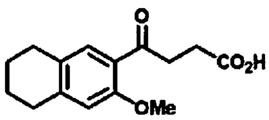

To a solution of 4-(3-hydroxy-5,6,7,8-tetrahydronaphthalen-2-yl)-4-oxobutanoic acid obtained in Example 29-(1) (2.0 g) in DMF (10 mL) was added sodium hydride (60%, in oil, 0.71 g) under ice-cooling, and the mixture was stirred for 1 hr. Methyl iodide (1.3 mL) was added at room temperature, and the mixture was stirred overnight. Water was added to the reaction mixture, and the mixture was extracted with ethyl acetate. The obtained organic layer was washed with water, dried over anhydrous magnesium sulfate, and concentrated under reduced pressure. THF (20 mL) and methanol (50 mL) were added to the obtained residue, 1N aqueous sodium hydroxide solution (40 mL) was added, and the mixture was stirred overnight at room temperature. The reaction mixture was concentrated, the obtained residue was acidified with diluted hydrochloric acid, and the mixture was extracted with ethyl acetate. The obtained organic layer was washed with water, dried over anhydrous magnesium sulfate, and concentrated under reduced pressure to give 4-(3-methoxy-5,6,7,8-tetrahydronaphthalen-2-yl)-4-oxobutanoic acid (1.42 g) as crystals.
$^1$H-NMR ($CDCl_3$) δ: 1.77(4H, m), 2.70(4H, m), 2.77(2H, m), 3.31(2H, t, J=6.9Hz), 3.70(3H, s), 6.64(1H, s), 7.50(1H, s)

(3) N-(5-benzyl-4-phenyl-1,3-thiazol-2-yl)-4-(3-methoxy-5,6,7,8-tetrahydronaphthalen-2-yl)-4-oxobutanamide

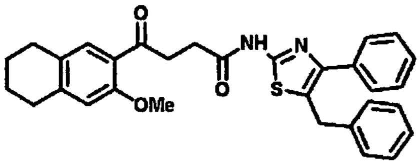

To a suspension of 5-benzyl-4-phenyl-1,3-thiazol-2-amine obtained in Example 1-(2) (266 mg), 4-(3-methoxy-5,6,7,8-tetrahydronaphthalen-2-yl)-4-oxobutanoic acid obtained in Example 29-(2) (418 mg) and HOBt (306 mg) in acetonitrile (20 mL) was added WSC (0.35 mL), and the mixture was stirred at 50°C. for 2 hr. Water was added to the reaction mixture, and the mixture was extracted with ethyl acetate. The obtained organic layer was washed with water, dried over anhydrous magnesium sulfate, and concentrated under reduced pressure to give N-(5-benzyl-4-phenyl-1,3-thiazol-2-yl)-4-(3-inethoxy-5,6,7,8-tetrahydronaphthalen-2-yl)-4-oxobutanamide (135 mg).
$^1$H-NMR ($CDCl_3$) δ: 1.77 (4H, m), 2.63 (2H, t, J=6.3Hz), 2. 69 (2H, m), 2.77(2H, m), 3.35(2H, t, J=6.3Hz), 3.85(3H, s), 4.20(2H, s), 6.62(1H, s), 7.19-7.33(7H, m), 7.39(2H, t, J=6.6Hz), 7.51(1H, s), 7.57(2H, d, J=6.6Hz), 9.72(1H, br s)
Anal. Calcd for $C_{31}H_{30}N_2O_2S{\cdot}0.1H_2O$: C, 72.66; H, 5.94; N, 5.47.Found: C, 72.39; H, 5.85; N, 5.46.

Example 30

ethyl (2E)-3- (5-{4-[(5-benzyl-4-phenyl-1,3-thiazol-2-yl)amino]-4-oxobutanoyl}-2-ethoxyphenyl)acrylate

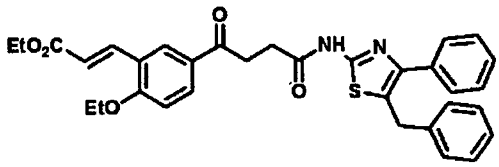

To a solution of N-(5-benzyl-4-phenyl-1,3-thiazol-2-yl)-4-(3-bromo-4-ethoxyphenyl)-4-oxobutanamide obtained in Example 25 (570 mg) in DMF (2 mL) were added palladium acetate (220 mg), triphenylphosphine (520 mg), triethylamine (0.42 mL) and ethyl acrylate (0.34 mL), and the mixture was heated overnight at 100°C under nitrogen atmosphere. Water was added to the reaction mixture, and the mixture was extracted with ethyl acetate. The obtained organic layer was washed with water, dried over anhydrous magnesium sulfate, and concentrated under reduced pressure. The obtained residue was purified by silica gel column chromatography [developing solvent: hexane-ethyl acetate (2:1)] to give ethyl (2E)-3-(5-14-[(5-benzyl-4-phenyl-1,3-thiazol-2-yl)amino]-4-oxobutanoyl}-2-ethoxyphenyl)acrylate (240 mg) as crystals.
$^1$H-NMR ($CDCl_3$) δ: 1.35 (3H, t, J=6.9Hz), 1.52 (3H, t, J=6.9Hz), 2.49(2H, t, J=6.3Hz), 3.20(2H, t, J=6.3Hz), 4.15-4.31 (6H, m), 6.59(1H, d, J=15.9Hz), 6.93(1H, d, J=9.0Hz), 7.18-7.36(8H, m), 7.59(2H, d, J=8.1 Hz), 7.94(2H, m), 8.11(1H, s), 10.43(1H, br s)

Example 31

(2E)-3-(5-{4-[(5-benzyl-4-phenyl-1,3-thiazol-2-yl)amino]-4-oxobutanoyl}-2-ethoxyphenyl)acrylic acid

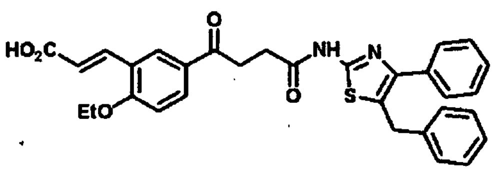

To a solution of ethyl (2E)-3-(5-{4-[(5-benzyl-4-phenyl-1,3-thiazol-2-yl)amino]-4-oxobutanoyl}-2-ethoxyphenyl)acrylate obtained in Example 30 (200 mg) in a mixed solvent of methanol (4 mL)-THF (50 mL) was added 1N aqueous sodium hydroxide solution (40 mL), and the mixture was stirred overnight at room temperature. The reaction mixture was concentrated, the obtained residue was acidified with diluted hydrochloric acid, and extracted with ethyl acetate. The obtained organic layer was washed with water, dried over anhydrous magnesium sulfate, and concentrated under reduced pressure to give (2E)-3-(5-{4-[(5-benzyl-4-phenyl-1,3-thiazol-2-yl)amino]-4-oxobutanoyl}-2-ethoxyphenyl)acrylic acid (110 mg) as crystals.
$^1$H-NMR ($CDCl_3$) δ: 1.50(3H, t, J=6.9Hz), 2.87(2H, t, J=6.3Hz), 3.52(2H, t, J=6.3Hz), 4.15(2H, m), 4.19(2H, s), 6.57(1H, d, J=15.9Hz), 6.94(1H, d, J=9.0Hz), 7.16-7.41(8H, m), 7.57(2H, d, J=8.1Hz), 7.96(2H, m), 8.15(1H, d, J=1.8Hz), 11.60 (1H, br s)

Example 32

N-(5-benzyl-4-phenyl-1,3-thiazol-2-yl)-4-oxo-4-[4-(methoxymethoxy)phenyl]butanamide

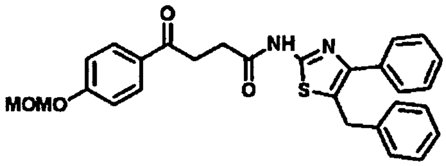

(1) 4-[4-(methoxymethoxy)phenyl]-4-oxobutanoic acid

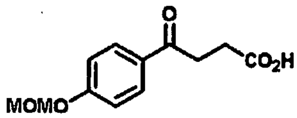

Under ice-cooling, to a solution of succinic anhydride (4.5 g) in THF (100 mL) was added dropwise a solution of 4-(methoxymethoxy)phenylmagnesium bromide (prepared from magnesium (1.23 g) and 4-bromo-(methoxymethoxy) benzene (10 g)) in THF (50 mL), and the mixture was stirred for 2 hr. Diluted hydrochloric acid was added to the reaction mixture, and the mixture was extracted with ethyl acetate. The obtained organic layer was washed with water, dried over anhydrous magnesium sulfate, and concentrated under reduced pressure. 1N Aqueous sodium hydroxide solution was added to the obtained residue, and the mixture was washed with ether. The aqueous layer was acidified with diluted hydrochloric acid, and the mixture was extracted with ethyl acetate. The obtained organic layer was washed with water, dried over anhydrous magnesium sulfate, and concentrated under reduced pressure to give 4-[4-(methoxymethoxy)phenyl]-4-oxobutanoic acid (1.2 g) as crystals.
$^1$H-NMR ($CDCl_3$) δ: 2.74(2H, t, J=6.6Hz), 3.26(2H, t, J=6.6Hz), 3.49(3H, s), 5.24(2H, s), 7.07(2H, d, J=8.7Hz), 7.96 (2H, d, J=8.7Hz)
(2) N-(5-benzyl-4-phenyl-1,3-thiazol-2-yl)-4-oxo-4-[4-(methoxymethoxy)phenyl]butanamide

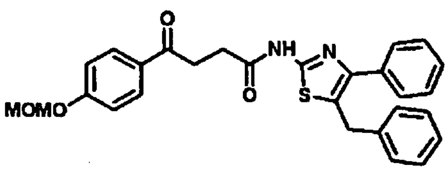

To a suspension of 5-benzyl-4-phenyl-1,3-thiazol-2-amine obtained in Example 1-(2) (740 mg), 4-[4-(methoxymethoxy)phenyl]-4-oxobutanoic acid obtained in Example 32-(1) (1.2 g) and HOBt (850 mg) in acetonitrile (50 mL) was added WSC (0.97 mL), and the mixture was stirred at 50°C for 2 hr. Water was added to the reaction mixture, and the mixture was extracted with ethyl acetate. The obtained organic layer was washed with water, dried over anhydrous magnesium sulfate, and concentrated under reduced pressure to give N-(5-benzyl-4-phenyl-1,3-thiazol-2-yl)-4-oxo-4-[4-(methoxymethoxy)phenyl]butanamide (660 mg).
$^1$H-NMR ($CDCl_3$) δ: 2.40 (2H, t, J=6.6Hz), 3.15 (2H, t, J=6.6Hz), 3.49(3H, s), 4.20(2H, s), 5.23(2H, s), 7.06(2H, d,

J=8.1Hz), 7.20-7.30(6H, m), 7.38(2H, t, J=7.8Hz), 7.58(2H, d, J=7.2Hz), 7.90 (2H, d, J=8.4Hz), 10.72 (1H, br s) Anal. Calcd for $C_{28}H_{26}N_2O_4S$: C, 69.11; H, 5.39; N, 5.76.Found: C, 68.749; H, 5.35; N, 5.84.

Example 33

N-(5-benzyl-4-phenyl-1,3-thiazol-2-yl)-4-(4-hydroxyphenyl)-4-oxobutanamide

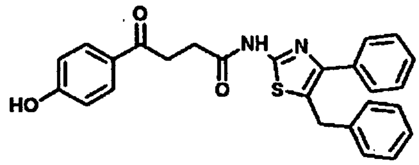

To a solution of N-(5-benzyl-4-phenyl-1,3-thiazol-2-yl)-4-oxo-4-[4-(methoxymethoxy)phenyl]butanamide obtained in Example 32 (0.5 g) in THF (50 mL) was added concentrated hydrochloric acid (2 mL), and the mixture was stirred overnight at room temperature. The reaction mixture was concentrated, water was added to the obtained residue, and the mixture was extracted with ethyl acetate. The obtained organic layer was washed with water, dried over anhydrous magnesium sulfate, and concentrated under reduced pressure to give N-(5-benzyl-4-phenyl-1,3-thiazol-2-yl)-4-(4-hydroxyphenyl)-4-oxobutanamide (0.43 g).
$^1$H-NMR ($CDCl_3$) δ: 2.84(2H, t, J=6.9Hz), 3.32(2H, t, J=6.9Hz), 4.20(2H, s), 6.87(2H, d, J=7.8Hz), 7.20-7.42(8H, m), 7.58(2H, d, J=6.9Hz), 7.86(2H, d, J=9.0Hz), 9.62(1H, br s), 11.38(1H, br s) Anal. Calcd for $C_{31}H_{30}N_2O_2S \cdot 0.2H_2O$: C, 70.00; H, 5.06; N, 6.28.Found: C, 69.88; H, 5.15; N, 6.11.

Example 34

ethyl 4-{4-[(5-benzyl-4-phenyl-1,3-thiazol-2-yl)amino]-4-oxobutanoyl}phenyl)acetate

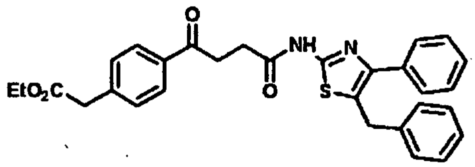

(1) 4-[4-(2-ethoxy-2-oxoethyl)phenyl]-4-oxobutanoic acid

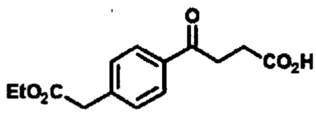

To a suspension of succinic anhydride (7.3 g) in dichloromethane (20 mL) was gradually added aluminum chloride (20.3 g) under ice-cooling, and the mixture was stirred for 0.5 hr. Ethyl phenylacetate (10.0 g) was added dropwise, and the mixture was heated at 50°C for 1 hr. The reaction mixture was cooled, and poured into concentrated hydrochloric acid (100 mL)-ice, and the mixture was stirred for 1 hr, and extracted with ethyl acetate. The organic layer was washed with water, dried over anhydrous magnesium sulfate, and concentrated under reduced pressure. 1N Aqueous sodium hydroxide solution was added to the obtained residue, and the mixture was washed with ether. The aqueous layer was acidified with diluted hydrochloric acid, and extracted with ethyl acetate. The obtained organic layer was washed with water, dried over anhydrous magnesium sulfate, and concentrated under reduced pressure to give 4-[4-(2-ethoxy-2-oxoethyl)phenyl]-4-oxobutanoic acid as an oil. This was used for the next step without purification.
$^1$H-NMR ($CDCl_3$) δ: 1.26(3H, t, J=6.6Hz), 2.81(2H, t, J=6.3Hz), 3.31(2H, t, J=6.3Hz), 3.68(2H, s), 4.16(2H, q, J=6.6Hz), 7.39 (2H, d, J=8.4Hz), 7.95(2H, d, J=8.4Hz)

(2) ethyl 4-{4-[(5-benzyl-4-phenyl-1,3-thiazol-2-yl)amino]-4-oxobutanoyl}phenyl)acetate

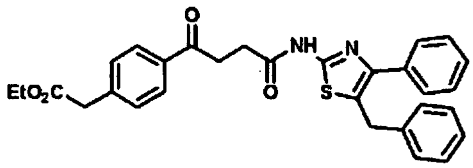

To a suspension of 5-benzyl-4-phenyl-1,3-thiazol-2-amine obtained in Example 1-(2) (530 mg), 4-[4-(2-ethoxy-2-oxoethyl)phenyl]-4-oxobutanoic acid obtained in Example 34-(1) (950 mg) and HOBt (610 mg) in acetonitrile (20 mL) was added WSC (0.70 mL), and the mixture was stirred at 50°C for 2 hr. Water was added to the reaction mixture, and the mixture was extracted with ethyl acetate. The obtained organic layer was washed with water, dried over anhydrous magnesium sulfate, and concentrated under reduced pressure. The obtained residue was purified by silica gel column chromatography [developing solvent: hexane-ethyl acetate (2:1)] to give ethyl 4-{4-[(5-benzyl-4-phenyl-1,3-thiazol-2-yl) amino]-4-oxobutanoyl}phenyl)acetate (170 mg) as crystals.
$^1$H-NMR ($CDCl_3$) δ: 1.25(3H, t, J=6.6Hz), 2.38(2H, t, J=6.3Hz), 3.16(2H, t, J=6.3Hz), 3.67(2H, s), 4.16(2H, q, J=6.6Hz), 4.20(2H, s), 7.19-7.53(10H, m), 7.59(2H, d, J=7.5Hz), 7.87(2H, m), 10.78(1H, br s)

Example 35

4-{4-[(5-benzyl-4-phenyl-1,3-thiazol-2-yl) amino]-4-oxobutanoyl}phenyl)acetic acid

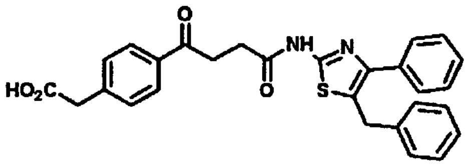

To a solution of ethyl 4-{4-[(5-benzyl-4-phenyl-1,3-thiazol-2-yl)amino]-4-oxobutanoyl}phenyl)acetate obtained in Example 34-(2) (0.15 g) in a mixed solvent of methanol (5 mL)-THF (5 mL) was added 1N aqueous sodium hydroxide solution (2 mL), and the mixture was stirred overnight at room temperature. The reaction mixture was concentrated, and the obtained residue was acidified with diluted hydrochloric acid, and extracted with ethyl acetate. The obtained organic layer was washed with water, dried over anhydrous magnesium sulfate, and concentrated under reduced pressure to give 4-{4-[(5-benzyl-4-phenyl-1,3-thiazol-2-yl)amino]-4-oxobutanoyl}phenyl)acetic acid (93 mg) as crystals.
$^1$H-NMR ($CDCl_3$) δ: 2.88(2H, m), 3.37(2H, m), 3.65(2H, s), 4.20 (2H, s), 7.19-7.50 (10H, m), 7.57 (2H, d, J=7.5Hz), 7.917 (2H, m), 11.80(1H, br s)
Anal. Calcd for $C_{28}H_{24}N_2O_4S$: C, 69.40; H, 4.99; N, 5.78.Found: C, 68.99; H, 4.99; N, 5.72.

Example 36

N-(5-benzyl-4-phenyl-1,3-thiazol-2-yl)-4-(3-chloro-4-ethoxyphenyl)-4-oxobutanamide

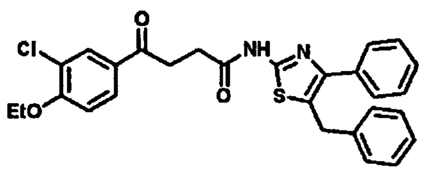

(1) 4-(3-chloro-4-ethoxyphenyl)-4-oxobutanoic acid

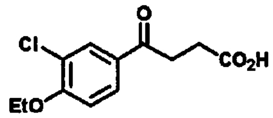

To a suspension of chloro-2-ethoxybenzene (5.0 g) and succinic anhydride (3.8 g) in dichloromethane (50 mL) was gradually added aluminum chloride (10.6 g) under ice-cooling, and the mixture was heated with stirring at 50°C for 0.5 hr. The reaction mixture was cooled, and poured into concentrated hydrochloric acid (100 mL)-ice, and the mixture was stirred for 1 hr, and extracted with ethyl acetate. The obtained organic layer was washed with water, dried over anhydrous magnesium sulfate, and concentrated under reduced pressure to give 4-(3-chloro-4-ethoxyphenyl)-4-oxobutanoic acid (1.6 g) as crude crystals.
$^1$H-NMR ($CDCl_3$) δ: 1.51(3H, t, J=7.2Hz), 2.80(2H, t, J=6.6Hz), 3.24(2H, t, J=6.6Hz), 4.18(2H, q, J=7.2Hz), 6.94 (1H, d, J=8.7Hz), 7.86(1H, dd, J=8.7 Hz, 2.4Hz), 8.00(1H, d, J=2.4Hz)
(2) N-(5-benzyl-4-phenyl-1,3-thiazol-2-yl)-4-(3-chloro-4-ethoxyphenyl)-4-oxobutanamide

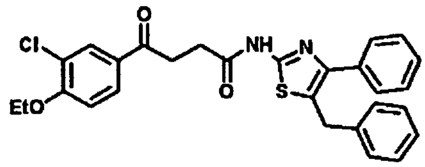

To a suspension of 5-benzyl-4-phenyl-1,3-thiazol-2-amine obtained in Example 1-(2) (266 mg), 4-(3-chloro-4-ethoxyphenyl)-4-oxobutanoic acid obtained in Example 36-(1) (460 mg) and HOBt (306 mg) in acetonitrile (20 mL) was added WSC (0.35 mL), and the mixture was stirred at 50°C for 2 hr. The reaction solution was cooled under ice bath, and the precipitated solid was collected by filtration to give N-(5-benzyl-4-phenyl-1,3-thiazol-2-yl)-4-(3-chloro-4-ethoxyphenyl)-4-oxobutanamide (171 mg).
$^1$H-NMR ($CDCl_3$) δ: 1.51 (3H, t, J=7.2Hz), 2.37 (2H, t, J=6.6Hz), 3.10 (2H, d, J=6.6Hz), 4.18 (2H, q, J=7.2Hz), 4.19 (2H, s), 6.91 (1H, d, J=9.0Hz), 7.19-7.35 (6H, m), 7.37 (2H, t, J=7.5Hz), 7.57 (2H, d, J=7.2Hz), 7.80 (1H, dd, J=9.0Hz, 1.8Hz), 7.94 ( 1H, d, J=1.8Hz), 10.76 (1H, br s)
Anal. Calcd for $C_{28}H_{25}ClN_2O_3S$: C, 66.59; H, 4.99; N, 5.55. Found: C, 66.45; H, 4.93; N, 5.48.

Example 37

ethyl 4-{4-[(5-benzyl-4-phenyl-1,3-thiazol-2-yl)amino]-4-oxobutanoyl}phenoxy)acetate

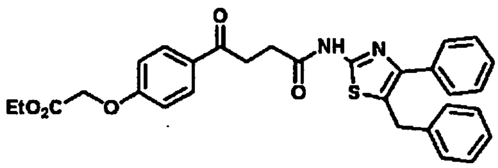

To a solution of N-(5-benzyl-4-phenyl-1,3-thiazol-2-yl)-4-(4-hydroxyphenyl)-4-oxobutanamide obtained in Example 33 (0.39 g) in DMF (5 mL) were added potassium carbonate (0.15 g) and ethyl bromoacetate (0.11 mL), and the mixture was stirred at room temperature for 2 hr. Water was added to the reaction mixture, and the mixture was extracted with ethyl acetate. The obtained organic layer was washed with water, dried over anhydrous magnesium sulfate, and concentrated under reduced pressure to give ethyl 4-{4-[(5-benzyl-4-phenyl-1,3-thiazol-2-yl)amino]-4-oxobutanoyl}phenoxy)acetate (0.35 g) as crystals.
$^1$H-NMR ($CDCl_3$) δ: 1.30(3H, t, J=6.9Hz), 2.39(2H, m), 3.13(2H, m), 4.19(2H, s), 4.28(2H, q, J=6.9Hz), 4.68(2H, s), 6.93 (2H, d, J=9.0 Hz), 7.19-7.30(6H, m), 7.38(2H, t, J=7.5Hz), 7.58(2H, d, J=7.2Hz), 7.91(2H, d, J=9.0Hz), 10.74(1H, br s)
Anal. Calcd for $C_{30}H_{28}N_2O_5S$: C, 68.16; H, 5.34; N, 5.30.Found: C, 67.81; H, 5.33; N, 5.21.

Example 38

4-{4-[(5-benzyl-4-phenyl-1,3-thiazol-2-yl)amino]-4-oxobutanoyl}phenoxy)acetic acid

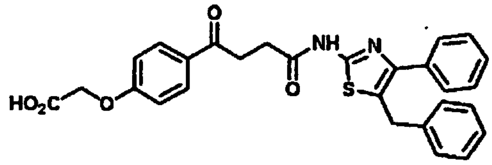

To a solution of ethyl 4-{4-[(5-benzyl-4-phenyl-1,3-thiazol-2-yl)amino]-4-oxobutanoyl}phenoxy)acetate obtained in Example 37 (0.3 g) in a mixed solvent of methanol (10 mL)-THF (10 mL) was added 1N aqueous sodium hydroxide solution (3 mL), and the mixture was stirred overnight at room temperature. The reaction mixture was concentrated, and the obtained residue was acidified with diluted hydrochloric acid, and extracted with ethyl acetate. The obtained organic layer was washed with water, dried over anhydrous magnesium sulfate, and concentrated under reduced pressure to give 4-14-[(5-benzyl-4-phenyl-1,3-thiazol-2-yl)amino]-4-oxobutanoyl}phenoxy)acetic acid (229 mg) as crystals. $^1$H-NMR ($CDCl_3$) δ: 2.80 (2H, t, J=6.9Hz), 3. 31 (2H, t, J=6.9Hz), 4.19(2H, s), 4.66(2H, s), 6.94(2H, d, J=8.7Hz), 7.19-7.41(8H, m), 7.57(2H, d, J=7.2Hz), 7.93(2H, d, J=9.0Hz)
Anal. Calcd for $C_{28}H_{24}N_2O_5S \cdot 0.5H_2O$: C, 66.00; H, 4.94; N, 5.50. Found : C, 66.05; H, 4.98; N, 5.63.

Example 39

4-(3,4-diethoxyphenyl)-N-[6-(morpholin-4-ylmethyl)biphenyl-3-yl]-4-oxobutanamide

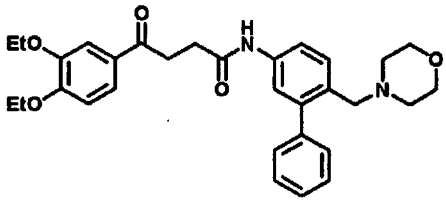

(1) 4-[(5-nitrobiphenyl-2-yl)methyl]morpholine

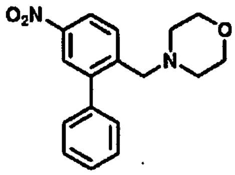

To a solution of 2-(bromomethyl)-5-nitrobiphenyl (0.5 g) in THF (10 mL) was added morpholine (0.5 mL), and the mixture was heated at 50°C for 1 hr. Aqueous sodium hydrogencarbonate solution was added to the reaction mixture, and the mixture was extracted with ethyl acetate. The obtained organic layer was washed with water, and dried over anhydrous magnesium sulfate, and the solvent was evaporated under reduced pressure to give 4-[(5-nitrobiphenyl-2-yl)methyl]morpholine (0.42 g) as crystals. $^1$H-NMR ($CDCl_3$) δ: 2 .36 (4H, m), 3.47(2H, s), 3. 66 (4H, m), 7.32-7.36 (2H, m), 7.42-7.49(3H, m), 7.79 (1H,d,J=8.7Hz),8.13(1H, d, J=2.4Hz), 8.18(1H, dd, J=8.7Hz, 2.4Hz)
(2) 6-(morpholin-4-ylmethyl)biphenyl-3-amine

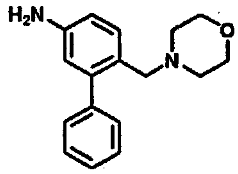

To a solution of 4-[(5-nitrobiphenyl-2-yl)methyl]morpholine obtained in Example 39-(1) (0.42 g) in a mixed solvent of ethanol (30 mL)-water (5.2 mL) were added iron (0.4 g) and calcium chloride (80 mg), and the mixture was heated under reflux for 5 hr. After the reaction mixture was filtrated through celite, the filtrate was concentrated, water was added to the obtained residue, and the mixture was extracted with diethyl ether. The organic layer was washed with water, and dried over anhydrous magnesium sulfate, and the solvent was evaporated under reduced pressure to give 6-(morpholin-4-ylmethyl)biphenyl-3-amine (0.25 g) as an oil.

$^1$H-NMR ($CDCl_3$) δ: 2.31 (4H, m), 3.27 (2H, s), 3.62(4H, m), 3.66(2H, br s), 6.60 (1H, d, J=2.7 Hz), 6.67 (1H, dd, J=7.8Hz, 2.7Hz), 7.23-7.42(6H, m)

(3) 4-(3,4-diethoxyphenyl)-N-[6-(morpholin-4-ylmethyl)biphenyl-3-yl]-4-oxobutanamide

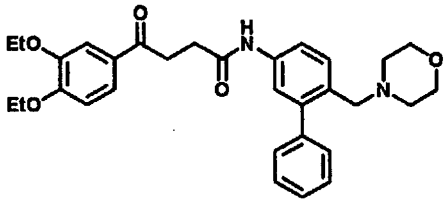

To a suspension of 6-(morpholin-4-ylmethyl)biphenyl-3-amine obtained in Example 39-(2) (250 mg), 4-(3,4-diethoxyphenyl)-4-oxobutanoic acid obtained in Example 3-(1) (323 mg) and HOBt (214 mg) in acetonitrile (5 mL) was added WSC (0.25 mL), and the mixture was stirred at 50°C for 2 hr. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure. The obtained residue was purified by silica gel column chromatography [developing solvent: hexane-ethyl acetate (1:1)] to give 4-(3,4-diethoxyphenyl)-N-[6-(morpholin-4-ylmethyl)biphenyl-3-yl]-4-oxobutanamide (266 mg) as an oil.

$^1$H-NMR ($CDCl_3$) δ: 1.46(3H, t, J=6.9Hz), 1.49(3H, t, J =6.9Hz), 2.32(4H, m), 2.79(2H, t, J=6.0Hz), 3.34(2H, s), 3.40(2H, t, J=6.0Hz), 3.62 (4H, m), 4.11 (4H, m), 6.88 (1H, d, J=8.7Hz), 7.29-7.62(10H, m), 7.83(1H, br s)

<u>Example 40</u>

4-(3,4-diethoxyphenyl)-N-(4-ethoxy-6-phenylpyridin-2-yl)-4-oxobutanamide

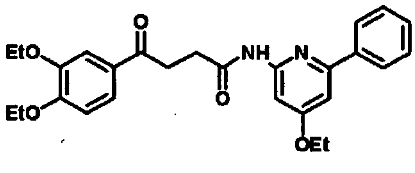

(1) tert-butyl (4-ethoxy-6-phenylpyridin-2-yl)carbamate

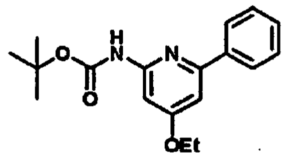

To a solution of 4-ethoxy-6-phenylpyridine-2-carboxylic acid (1.3 g) in toluene (20 mL) were added triethylamine (1.0 mL) and DPPA (1.38 mL), and the mixture was stirred at room temperature for 1 hr. tert-Butanol (3 mL) was added, and the mixture was heated overnight at 90°C. The reaction mixture was concentrated. The obtained residue was purified by silica gel column chromatography [developing solvent: hexane-ethyl acetate (4:1)] to give tert-butyl (4-ethoxy-6-phenylpyridin-2-yl)carbamate (1.26 g) as crystals.

$^1$H-NMR ($CDCl_3$) δ: 1.45 (3H, t, J=6.9Hz), 1.53 (9H, s), 4.17 (2H, q, J=6.9Hz), 6.93(1H, d, J=1.8 Hz), 7.27(1H, br s), 7.36-7.47(4H, m), 7.89 (2H, m)

(2) 4-ethoxy-6-phenylpyridin-2-amine

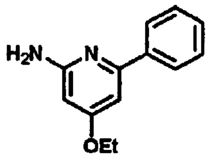

To tert-butyl (4-ethoxy-6-phenylpyridin-2-yl)carbamate obtained in Example 40-(1) (1.26 g) was added trifluoroacetic acid (5 mL), and the mixture was stirred at room temperature for 3 hr. The reaction mixture was concentrated, and the residue was alkalified with aqueous sodium hydrogencarbonate solution, and extracted with ethyl acetate. The organic layer was washed with water, dried over anhydrous magnesium sulfate, and concentrated under reduced pressure to give 4-ethoxy-6-phenylpyridin-2-amine as crystals (0.8 g).

$^1$H-NMR ($CDCl_3$) δ: 1.43 (3H, t, J=7.2Hz), 4.09 (2H, q, J=7.2Hz), 4.48 (2H, br), 5.95 (1H, d, J=2.1Hz), 6. 68 (1H, d, J=2.1Hz), 7.38(3H, m), 7.88 (2H, d, J=8.1Hz)

(3) 4-(3,4-diethoxyphenyl)-N-(4-ethoxy-6-phenylpyridin-2-yl)-4-oxobutanamide

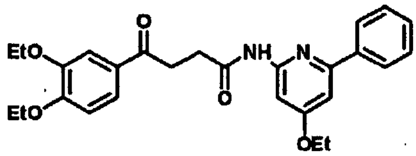

To a suspension of potassium 4-(3,4-diethoxyphenyl)-4,4-dimethoxybutanoate obtained in Example 8-(7) (350 mg) and 2,4,6-trichlorobenzoyl chloride (0.17 mL) in THF (5 mL) was added triethylamine (0.28 mL), and the mixture was stirred at room temperature for 4 hr. 4-Ethoxy-6-phenylpyridin-2-amine obtained in Example 40-(2) (300 mg) was added to the reaction mixture, and the mixture was heated overnight at 50°C. Diluted hydrochloric acid was added to the reaction mixture, and the mixture was stirred for 1 hr, and extracted with ethyl acetate. The organic layer was washed with water, dried over anhydrous magnesium sulfate, and concentrated under reduced pressure to give 4-(3,4-diethoxyphenyl)-N-(4-ethoxy-6-phenylpyridin-2-yl)-4-oxobutanamide (268 mg) as crystals.

$^1$H-NMR ($CDCl_3$) δ: 1.40-1.51 (9H, m), 2.81 (2H, t, J=6.3Hz), 3.37 (2H, t, J=6.3Hz), 4.10-4.20 (6H, m), 6.87(1H, d, J=8.4Hz), 6.98 (1H, d, J=2.1Hz), 7.36-7.54(3H, m), 7.53 (1H, d, J=1.8Hz), 7.61 (1H, dd, J=8.4Hz, 1.8Hz), 7.75(1H, s), 7.89 (2H, m), 8.34 (1H, br s) mp. 172-173°C

Example 41

4-(3,4-diethoxyphenyl)-N-(4-(3-methylbutoxy)-6-phenylpyridin-2-yl)-4-oxobutanamide

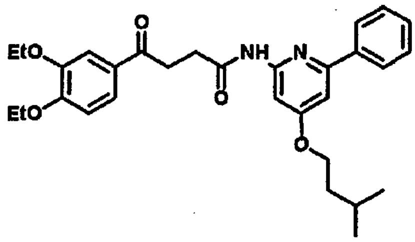

(1) 4-(3-methylbutoxy)-6-phenylpyridin-2-amine

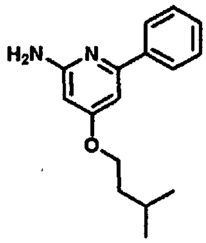

To a solution of 4-oxo-6-phenyl-1,4-dihydropyridine-2-carboxylic acid synthesized according to a method described in J. Med. Chem. 26, 1499-1504 (1983) (1.9 g) in DMF (15 mL) were added potassium carbonate (3.9 g) and 1-bromo-3-methylbutane (4.5 mL), and the mixture was heated at 80°C 2 hr. Water was added to the reaction mixture, and the mixture was extracted with ethyl acetate. The organic layer was washed with water, and dried over anhydrous magnesium sulfate, and the solvent was evaporated under reduced pressure. The obtained residue was purified by silica gel column chromatography [developing solvent: hexane-ethyl acetate (3:1)] to give 3-methylbutyl 4-(3-methylbutoxy)-6-phenyl-pyridine-2-carboxylate as an oil. This was dissolved in methanol (20 mL), 2N aqueous sodium hydroxide solution (10 mL) was added, and the mixture was stirred at room temperature for 1 hr. The reaction mixture was concentrated, and the obtained residue was acidified with 1N hydrochloric acid, and extracted with ethyl acetate. The organic layer was washed with water, and dried over anhydrous magnesium sulfate, and the solvent was evaporated under reduced pressure to give 4-(3-methylbutoxy)-6-phenyl-pyridine-2-carboxylic acid as an oil (0.84 g). To a solution of this compound in toluene (20 mL) were added triethylamine (1.34 mL) and DPPA (1.9 mL), and the mixture was stirred at room temperature for 1 hr. tert-Butanol (5 mL) was added, and the mixture was heated overnight at 90°C. The reaction mixture was concentrated. The obtained residue was purified by silica gel column chromatography [developing solvent: hexane-ethyl acetate (4:1)] to give tert-butyl [4-(3-methylbutoxy)-6-phenylpyridin-2-yl]carbamate as crystals (1.5 g). Trifluoroacetic acid (5 mL) was added thereto, and the mixture was stirred at room temperature for 3 hr. The reaction mixture was concentrated, and the residue was alkalified with aqueous sodium hydrogencarbonate solution, and extracted with ethyl acetate. The organic layer was washed with water, dried over anhydrous magnesium sulfate, and concentrated under reduced pressure to give.4-(3-methylbutoxy)-6-phenylpyridin-2-amine (1.2 g) as crystals. The crystals were used for the next step without purification.

(2) 4-(3,4-diethoxyphenyl)-N-(4-(3-methylbutoxy)-6-phenylpyridin-2-yl)-4-oxobutanamide

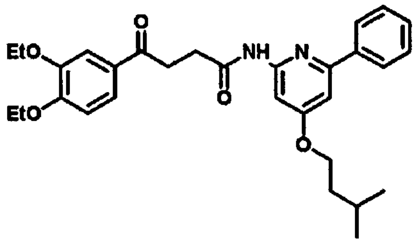

To a suspension of potassium 4-(3,4-diethoxyphenyl)-4,4-dimethoxybutanoate obtained in Example 8-(7) (350 mg) and 2,4,6-trichlorobenzoyl chloride (0.17 mL) in THF (5 mL) was added triethylamine (0.28 mL), and the mixture was stirred at room temperature for 4 hr. 4-(3-Methylbutoxy)-6-phenylpyridin-2-amine obtained in Example 41-(1) (250 mg) was added to the reaction mixture, and the mixture was heated overnight at 50°C. Diluted hydrochloric acid was

added to the reaction mixture, and the mixture was stirred for 1 hr, and extracted with ethyl acetate. The organic layer was washed with water, dried over anhydrous magnesium sulfate, and concentrated under reduced pressure. The obtained residue was purified by silica gel column chromatography [developing solvent: hexane-ethyl acetate (3:1)] to give 4-(3,4-diethoxyphenyl)-N-(4-(3-methylbutoxy)-6-phenylpyridin-2-yl)-4-oxobutanamide (192 mg) as crystals.
$^1$H-NMR ($CDCl_3$) δ: 0.96(6H, d, J=6.6Hz), 1.46-1.51(6H, m), 1.69(2H, m), 1.83(1H, m), 2.83(2H, t, J=6.6Hz), 3.89(2H, t, J=6.6Hz), 4.07-4.20(6H, m), 6.87(1H, d, J=8.4Hz), 6.98(1H, d, J = 2.4 Hz), 7.36-7.47(3H, m), 7.54(1H, d, J=2.4Hz), 7.61(1H, dd, J = 8.1Hz, 2.4Hz), 7.76(1H, s), 7.91 (2H, m), 8.25(1H, br s) mp. 122-123°C

Example 42

4-(3,4-diethoxyphenyl)-N-(4-(benzyloxy)-6-phenylpyridin-2-yl)-4-oxobutanamide

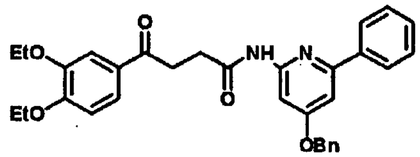

(1) 4-(benzyloxy)-6-phenylpyridin-2-amine

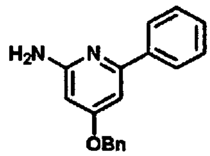

To a solution of 4-oxo-6-phenyl-1,4-dihydropyridine-2-carboxylic acid synthesized according to a method described in J. Med. Chem. 26, 1499-1504 (1983) (1.6 g) in DMF (15 mL) were added potassium carbonate (3.1 g) and benzyl bromide (3.5 mL), and the mixture was heated at 80°C for 2 hr. Water was added to the reaction mixture, and the mixture was extracted with ethyl acetate. The organic layer was washed with water, and dried over anhydrous magnesium sulfate, and the solvent was evaporated under reduced pressure. The obtained residue was purified by silica gel column chromatography [developing solvent: hexane-ethyl acetate (4:1)] to give benzyl 4-(benzyloxy)-6-phenylpyridine-2-carboxylate as crystals (2.3 g). This compound was dissolved in a mixed solvent of methanol (20 mL)-THF (10 mL), 2N aqueous sodium hydroxide solution (10 mL) was added, and the mixture was stirred at room temperature for 1 hr. The reaction mixture was concentrated, and the obtained residue was acidified with 1N hydrochloric acid, and extracted with ethyl acetate. The organic layer was washed with water, and dried over anhydrous magnesium sulfate, and the solvent was evaporated under reduced pressure to give 4-(benzyloxy)-6-phenylpyridine-2-carboxylic acid as an oil (2.15 g). To a solution of this compound in toluene (30 mL) were added triethylamine (1.3 mL) and DPPA (1.8 mL), and the mixture was stirred at room temperature for 1 hr. tert-Butanol (5 mL) was added, and the mixture was heated overnight at 90°C. The reaction mixture was concentrated. The obtained residue was purified by silica gel column chromatography [developing solvent: hexane-ethyl acetate (4:1)] to give tert-butyl [4-(benzyloxy)-6-phenylpyridin-2-yl]carbamate as an oil (2.4 g). Trifluoroacetic acid (5 mL) was added thereto, and the mixture was stirred at room temperature for 3 hr. The reaction mixture was concentrated, and the residue was alkalified with aqueous sodium hydrogencarbonate solution, and extracted with ethyl acetate. The organic layer was washed with water, dried over anhydrous magnesium sulfate, and concentrated under reduced pressure to give 4-(benzyloxy)-6-phenylpyridin-2-amine as an oil. This was used for the next step without purification.
(2) 4-(3,4-diethoxyphenyl)-N-(4-(benzyloxy)-6-phenylpyridin-2-yl)-4-oxobutanamide

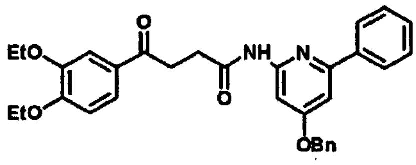

To a suspension of potassium 4-(3,4-diethoxyphenyl)-4,4-dimethoxybutanoate obtained in Example 8-(7) (350 mg) and 2,4,6-trichlorobenzoyl chloride (0.17 mL) in THF (5 mL) was added triethylamine (0.28 mL), and the mixture was stirred at room temperature for 4 hr. 4-(Benzyloxy)-6-phenylpyridin-2-amine obtained in Example 42-(1) (300 mg) was added to the reaction mixture, and the mixture was heated overnight at 50°C. Diluted hydrochloric acid was added to the reaction mixture, and the mixture was stirred for 1 hr, and extracted with ethyl acetate. The organic layer was washed with water, dried over anhydrous magnesium sulfate, and concentrated under reduced pressure. The obtained residue was purified by silica gel column chromatography [developing solvent: hexane-ethyl acetate (3:1)] to give 4-(3,4-diethoxyphenyl)-N-(4-(benzyloxy)-6-phenylpyridin-2-yl)-4-oxobutanamide (168 mg) as crystals.
$^1$H-NMR ($CDCl_3$) δ: 1.46(3H, t, J=6.9Hz), 1.49(3H, t, J=6.9Hz), 2.84 (2H, t, J=6.6Hz), 3.40 (2H, t, J=6.6Hz), 4.15 (2H, q, J=6.9Hz), 4.17 (2H, q, J=6.9Hz), 5.16 (2H, s), 6.89 (1H, d, J=8.7Hz), 7.08 (1H, d, J=1.8Hz), 7.32-7.56(8H, m), 7.55 (1H, d, J=2.1Hz), 7.61(1H, dd, J=8.4Hz, 2.1Hz), 7.91 (3H, m), 8.31(1H, br s)
mp. 156-157°C

Example 43

4-(3,4-diethoxyphenyl)-N-(4-(4-fluorophenyl)-6-phenylpyridin-2-yl)-4-oxobutanamide

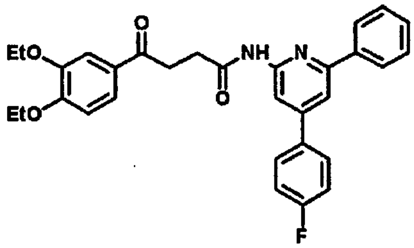

(1) tert-butyl 4-chloro-6-phenylpyridin-2-yl)carbamate

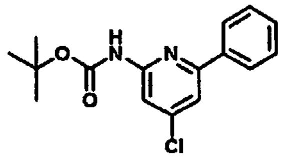

To a solution of 4-chloro-6-phenylpyridine-2-carboxylic acid (5.27 g) in toluene (100 mL) were added triethylamine (4.1 mL) and DPPA (5.8 mL), and the mixture was stirred at room temperature for 1 hr. tert-Butanol (10 mL) was added, and the mixture was heated overnight at 90°C. The reaction mixture was concentrated. The obtained residue was purified by silica gel column chromatography [developing solvent: hexane-ethyl acetate (5:1)] to give tert-butyl (4-chloro-6-phenylpyridin-2-yl)carbamate as an oil (5.46 g).
$^1$H-NMR ($CDCl_3$) δ: 1.54 (9H, s), 7.34 (1H, brs), 7.40-7.49(4H, m), 7.88-7.94(3H, m)
(2) 4-chloro-6-phenylpyridin-2-amine

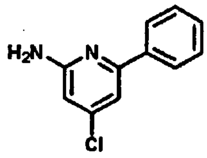

To tert-butyl (4-chloro-6-phenylpyridin-2-yl)carbamate obtained in Example 43-(1) (1.26 g) was added trifluoroacetic acid (15 mL), and the mixture was stirred at room temperature for 3 hr. The reaction mixture was concentrated, and alkalified with aqueous sodium hydrogencarbonate solution, and the mixture was extracted with ethyl acetate. The organic layer was washed with water, dried over anhydrous magnesium sulfate, and concentrated under reduced pressure to give 4-chloro-6-phenylpyridin-2-amine (3.5 g) as crystals. The crystals were used for the next step without purification.

(3) N-(4-chloro-6-phenylpyridin-2-yl)-4-(3,4-diethoxyphenyl)-4-oxobutanamide

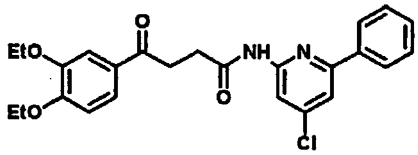

To a suspension of potassium 4-(3,4-diethoxyphenyl)-4,4-dimethoxybutanoate obtained in Example 8-(7) (1.5 g) and 2,4,6-trichlorobenzoyl chloride (0.74 mL) in THF (20 mL) was added triethylamine (1.2 mL), and the mixture was stirred at room temperature for 4 hr. 4-Chloro-6-phenylpyridin-2-amine (0.8 g) obtained in Example 43-(2) was added to the reaction mixture, and the mixture was heated overnight at 50°C. Diluted hydrochloric acid was added to the reaction mixture, and the mixture was stirred for 1 hr, and extracted with ethyl acetate. The organic layer was washed with water, dried over anhydrous magnesium sulfate, and concentrated under reduced pressure to give N-(4-chloro-6-phenylpyridin-2-yl)-4-(3,4-diethoxyphenyl)-4-oxobutanamide (1.26 g) as crystals.

$^1$H-NMR ($CDCl_3$) δ: 1.47(3H, t, J=6.9Hz), 1.49(3H, t, J=6.9Hz), 2.86(2H, t, J=6.6Hz), 3.41(2H, t, J=6.6Hz), 4.15(4H, m), 6.89(1H, d, J=8.1Hz), 7.41-7.65(6H, m), 7.93 (2H, m), 8.21(1H, d, J=1.2 Hz), 8.31(1H, br s)

mp. >138°C decomp.

(4) 4-(3,4-diethoxyphenyl)-N-(4-(4-fluorophenyl)-6-phenylpyridin-2-yl)-4-oxobutanamide

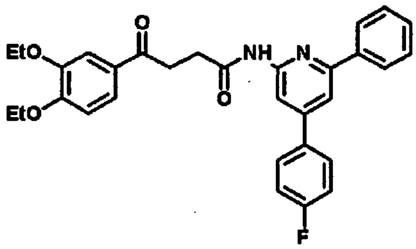

To a solution of N-(4-chloro-6-phenylpyridin-2-yl)-4-(3,4-diethoxyphenyl)-4-oxobutanamide obtained in Example 43-(3) (200 mg) in DME (1 mL) were added palladium acetate (5 mg), tri-o-tolylphosphine (27 mg), 2N aqueous potassium carbonate solution (0.6 mL) and 4-fluorophenylboronic acid (75 mg), and the mixture was heated overnight at 100°C under nitrogen atmosphere. Water was added to the reaction mixture, and the mixture was extracted with ethyl acetate. The obtained organic layer was washed with water, dried over anhydrous magnesium sulfate, and concentrated under reduced pressure. The obtained residue was purified by silica gel column chromatography [developing solvent: hexane-ethyl acetate (3:1)] to give 4-(3,4-diethoxyphenyl)-N-(4-(4-fluorophenyl)-6-phenylpyridin-2-yl)-4-oxobutanamide (69 mg) as crystals.

$^1$H-NMR ($CDCl_3$) δ: 1.46 (3H, t, J=7.2 Hz), 1 .49 (3H, t, J=7.2Hz), 2.89 (2H, t, J=6.3Hz), 3.43 (2H, t, J=6.3Hz), 4.14 (2H, q, J=7.2Hz), 4.17 (2H, q, J=7.2Hz), 6.89 (1H, d, J=8.2Hz), 7.15 (2H, t, J=8.7Hz), 7.44-7.72(8H, m), 8.00(2H, d, J=8.4Hz), 8.32(1H, s), 8.38(1H, br s)

Example 44

N-(5-benzyl-4-phenyl-2-thienyl)-4-(4-ethoxyphenyl)-4-oxobutanamide

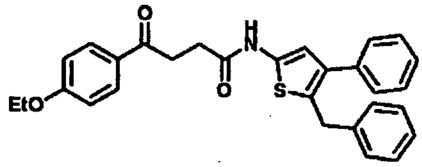

To a suspension of 5-benzyl-4-phenylthiophen-2-amine obtained in Example 17-(2) (200 mg), 4-(4-ethoxyphenyl)-4-oxobutanoic acid obtained in Example 1-(1) (300 mg) and HOBt (230 mg) in acetonitrile (10 mL) was added WSC (0.27 mL), and the mixture was stirred at 50°C for 2 hr. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure to give N-(5-benzyl-4-phenyl-2-thienyl)-4-(4-ethoxyphenyl)-4-oxobutanamide (15 mg).

Example 45

N-(5-benzyl-4-phenyl-2-thienyl)-4-(3-ethoxyphenyl)-4-oxobutanamide

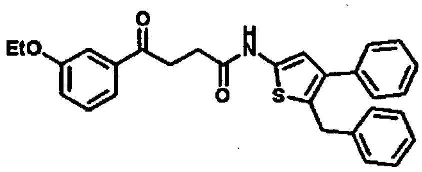

To a suspension of 5-benzyl-4-phenylthiophen-2-amine obtained in Example 17-(2) (200 mg), 4-(3-ethoxyphenyl)-4-oxobutanoic acid obtained in Example 5-(2) (300 mg) and HOBt (230 mg) in acetonitrile (10 mL) was added WSC (0.27 mL), and the mixture was stirred at 50°C for 2 hr. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure to give N-(5-benzyl-4-phenyl-2-thienyl)-4-(3-ethoxyphenyl)-4-oxobutanamide (30 mg).

Example 46

N-(5-(cyclohexylmethyl)-4-phenyl-2-thienyl)-4-(3,4-diethoxyphenyl)-4-oxobutanamide

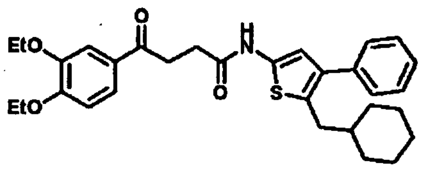

(1) ethyl 2-amino-5-(cyclohexylmethyl)-4-phenylthiophene-3-carboxylate

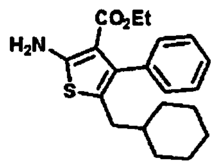

To a solution of 3-cyclohexyl-1-phenylpropan-1-one (13.2 g) and ethyl cyanoacetate (6.9 g) in toluene (14 mL) were added ammonium acetate (0.94 g) and acetic acid (2.8 mL), and the mixture was heated under reflux overnight using Dean-Stark while evaporating water. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure. The obtained residue was dissolved in ethanol (50 mL), and sulfur (2.0 g) and diisopropyl ether (8.5 mL) were added. The mixture was heated at 70°C for 2 hr, and concentrated. The obtained residue was purified by silica gel column chromatography [developing solvent: hexane-ethyl acetate (7:1)] to give ethyl 2-amino-5-(cyclohexylmethyl)-4-phenylthiophene-3-carboxylate (10.0 g) as crystals.

$^1$H-NMR ($CDCl_3$) δ: 0.74 (2H, m), 0.78 (3H, t, J=6.9Hz), 1.03-1.22(4H, m), 1.36 (1H, m), 1.63 (4H, m), 2.27 (2H, d, J=7.2Hz), 3.89(2H, q, J=6.9Hz), 5.96(2H, br s), 7.12(2H, m), 7.29 (3H, m)

(2) 5-(cyclohexylmethyl)-4-phenylthiophen-2-amine

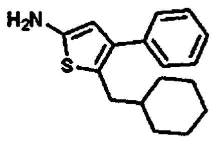

To a solution of ethyl 2-amino-5-(cyclohexylmethyl)-4-phenylthiophene-3-carboxylate obtained in Example 46-(1) (3.0 g) in ethanol (30 mL) was added aqueous potassium hydroxide solution (potassium hydroxide 6 g, in water 30 mL), and the mixture was heated under reflux for 4 days. The reaction mixture was extracted with ethyl acetate. The organic layer was washed with saturated brine, and dried over anhydrous magnesium sulfate, and the solvent was evaporated under reduced pressure to give 5-(cyclohexylmethyl)-4-phenylthiophen-2-amine (1.5 g) as crystals. $^1$H-NMR ($CDCl_3$) δ: 0.83(2H, m), 1.15(2H, m), 1.38-1.76(7H, m), 2.57(2H, d, J=7.0Hz), 3.63(2H, br s), 6.12(1H, s), 7.23-7.40(5H, m)

(3) N-(5-(cyclohexylmethyl)-4-phenyl-2-thienyl)-4-(3,4-diethoxyphenyl)-4-oxobutanamide

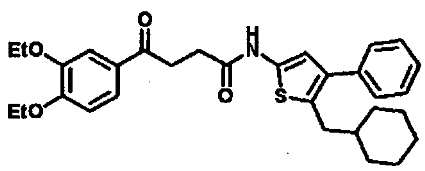

To a suspension of 5-(cyclohexylmethyl)-4-phenyl-2-thiophen-2-amine obtained in Example 46-(2) (270 mg), 4-(3,4-diethoxyphenyl)-4-oxobutanoic acid obtained in Example 3-(1) (530 mg) and HOBt (306 mg) in acetonitrile (10 mL) was added WSC (0.35 mL), and the mixture was stirred at 50°C for 2 hr. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure. The obtained residue was purified by silica gel column chromatography [developing solvent: hexane-ethyl acetate (3:1)] to give N-(5-(cyclohexylmethyl)-4-phenyl-2-thienyl)-4-(3,4-diethoxyphenyl)-4-oxobutanamide (280 mg).

$^1$H-NMR ($CDCl_3$) δ: 0.83(2H, m), 1.11(2H, m), 1.48(4H, m), 1.50-1.73(7H, m), 2.64(2H, d, J=6.9Hz), 2.81(2H, t, J=6.0Hz), 3.43(2H, t, J=6.0Hz), 4.16(4H, m), 6.56(1H, s), 6.878(1H, d, J=8 .1Hz), 7.28-7.40(5H, m), 7.53(1H, d, J=2.1Hz), 7.61 (1H, dd, J=8.4Hz, 2.1Hz), 8.52(1H, br s)

Anal. Calcd for $C_{31}H_{37}NO_4S$: C, 71.64; H, 7.18; N, 2.70. Found: C, 71.15; H, 7.23; N, 2.61.

Example 47

N-[5-(4-bromobenzyl)-4-phenyl-2-thienyl]-4-(3,4-diethoxyphenyl)-4-oxobutanamide

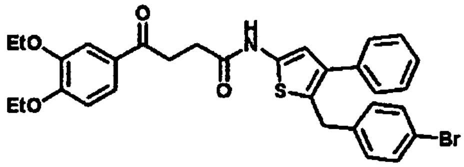

(1) ethyl 2-amino-5-(4-bromobenzyl)-4-phenylthiophene-3-carboxylate

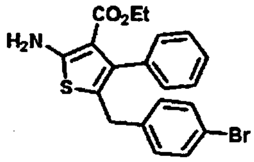

To a solution of 3-(4-bromophenyl)-1-phenylpropan-1-one (12 g) and ethyl cyanoacetate (4.9 g) in toluene (10 mL) were added ammonium acetate (0.67 g) and acetic acid (2.0 mL), and the mixture was heated under reflux overnight using Dean-Stark while evaporating water. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure. The obtained residue was dissolved in ethanol (40 mL), and sulfur (1.4 g) and diisopropyl ether (6.1 mL) were added. The mixture was heated overnight at 70°C, and concentrated. The obtained residue was purified by silica gel column chromatography [developing solvent: hexane-ethyl acetate (7:1)] to give ethyl 2-amino-5-(4-bromobenzyl)-4-phenylthiophene-3-carboxylate as an oil. This was used for the next step without purification.
$^1$H-NMR ($CDCl_3$) δ: 0.78 (3H, t, J=6.9Hz), 3.68 (2H, s), 3.91 (2H, q, J=6.9Hz), 6.01(2H, br s), 6.95 (2H, d, J=6.6Hz) , 7.15 (2H, m), 7.28-7.38(5H, m)
(2) 5-(4-bromobenzyl)-4-phenylthiophen-2-amine

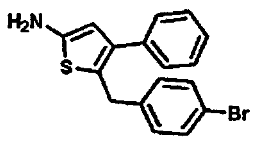

To a solution of ethyl 2-amino-5-(4-bromobenzyl)-4-phenylthiophene-3-carboxylate obtained in Example 47-(1) in ethanol (100 mL) was added aqueous potassium hydroxide solution (potassium hydroxide 20 g, in water 100 mL), and the mixture was heated under reflux for 4 days. The reaction mixture was extracted with ethyl acetate. The organic layer was washed with saturated brine, and dried over anhydrous magnesium sulfate, and the solvent was evaporated under reduced pressure. The obtained residue was purified by silica gel column chromatography [developing solvent: hexane-ethyl acetate (7:1)] to give 5-(4-bromobenzyl)-4-phenylthiophen-2-amine (4.4 g) as crystals. $^1$H-NMR ($CDCl_3$) δ: 4.00(2H, s), 6.17(1H, s), 7.04(2H, m), 7.27-7.39(7H, m)
(3) N-[5-(4-bromobenzyl)-4-phenyl-2-thienyl]-4-(3,4-diethoxyphenyl)-4-oxobutanamide

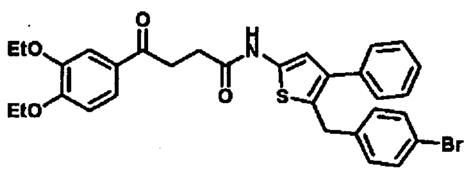

To a suspension of 5-(4-bromobenzyl)-4-phenylthiophen-2-amine obtained in Example 47-(2) (1.5 g), 4-(3,4-diethoxyphenyl)-4-oxobutanoic acid obtained in Example 3-(1) (1.5 g) and HOBt (1.0 g) in acetonitrile (20 mL) was added WSC (1.15 mL), and the mixture was stirred at 50°C for 2 hr. The reaction solution was cooled under ice bath, and the precipitated solid was collected by filtration to give N-[5-(4-bromobenzyl)-4-phenyl-2-thienyl]-4-(3,4-diethoxyphenyl)-4-oxobutanamide (2.0 g).
$^1$H-NMR ($CDCl_3$) δ: 1.47 (6H, m), 2.80 (2H, t, J=6.6Hz), 3.41 (2H, d, J=6.6Hz), 4.10 (2H, s), 4.16 (4H, m), 6.64 (1H, s), 6.87 (1H, d, J=8.4Hz), 7.16-7.39 (9H, m), 7.51 (1H, d, J=1.8Hz), 7.58 (1H, dd, J=8.4Hz, 1.8Hz), 8.63 (1H, br s)
Anal. Calcd for $C_{31}H_{30}BrNO_4S$: C, 62.84; H, 5.10; N, 2.36.Found: C, 62.78; H, 5.02; N, 2.23.

Example 48

N-(5-benzyl-4-phenyl-2-thienyl)-4-(3,4-dimethoxyphenyl)-4-oxobutanamide

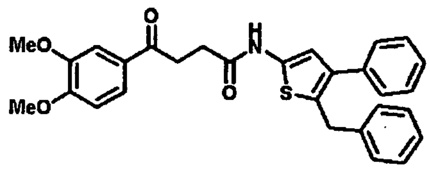

To a suspension of 5-benzyl-4-phenylthiophen-2-amine obtained in Example 17-(2) (200 mg), 4-(3,4-dimethoxyphenyl)-4-oxobutanoic acid (233 mg) and HOBt (173 mg) in acetonitrile (5 mL) was added WSC (0.20 mL), and the mixture was stirred at 50°C for 2 hr. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure. The obtained residue was purified by silica gel column chromatography [developing solvent: hexane-ethyl acetate (2:1)] to give N-(5-benzyl-4-phenyl-2-thienyl)-4-(3,4-dimethoxyphenyl)-4-oxobutanamide (72 mg) as crystals.
$^1$H-NMR ($CDCl_3$) δ: 2 . 80 (2H, t, J=6.0Hz), 3.42 (2H, t, J=6.0Hz), 3.91(3H, s), 3.94(3H, s), 4.10(2H, s), 6.63(1H, s), 6.88 (1H, d, J=8.4Hz), 7.15-7.50(10H, m), 7.50(1H, d, J=2.1Hz), 7.61(1H, dd, J=8.4Hz, 2.1Hz), 8.55(1H, br s)
Anal. Calcd for $C_{29}H_{27}NO_4S{\cdot}O.1H_2O$: C, 71.46; H, 5.62; N, 2.87.Found: C, 71.19; H, 5.65; N, 2.79.

Example 49

N-(5-benzyl-4-phenyl-2-thienyl)-4-(2,3-dihydro-1-benzofuran-5-yl)-4-oxobutanamide

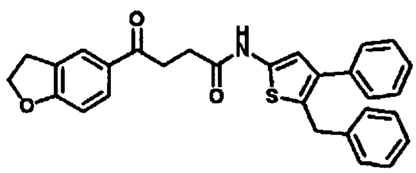

To a suspension of 5-benzyl-4-phenylthiophen-2-amine obtained in Example 17-(2) (200 mg), 4-(2,3-dihydro-1-benzofuran-5-yl)-4-oxobutanoic acid obtained in Example 27-(1) (215 mg) and HOBt (173 mg) in acetonitrile (5 mL) was added WSC (0.20 mL), and the mixture was stirred at 50°C for 1 hr. The reaction solution was cooled under ice bath, and the precipitated solid was collected by filtration to give N-(5-benzyl-4-phenyl-2-thienyl)-4-(2,3-dihydro-1-benzofuran-5-yl)-4-oxobutanamide (136 mg).
$^1$H-NMR ($CDCl_3$) δ: 2.81 (2H, t, J=6.3Hz), 3.22 (2H, t, J=8.7Hz), 3.38 (2H, t, J=6.3Hz), 4 .10 (2H, s), 4.65(2H, t, J=8.7Hz), 6. 63 (1H, s), 6.80(1H, d, J=8.7Hz), 7.16-7.38(10H, m), 7.84(2H, m), 8.75(1H, br s)
Anal. Calcd for $C_{29}H_{25}NO_3S$: C, 74.49; H, 5.39; N, 3.00.Found: C, 74.22; H, 5.34; N, 2.94.

Example 50

N-(5-benzyl-4-phenyl-2-thienyl)-4-(4,5-diethoxy-2-methylphenyl)-4-oxobutanamide

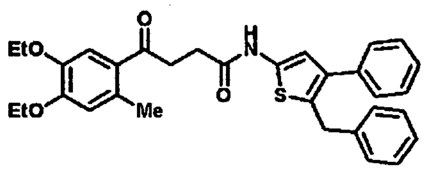

To a suspension of 5-benzyl-4-phenylthiophen-2-amine obtained in Example 17-(2) (270 mg), 4-(4,5-diethoxy-2-methylphenyl)-4-oxobutanoic acid obtained in Example 4-(1) (370 mg) and HOBt (234 mg) in acetonitrile (5 mL) was added WSC (0.27 mL), and the mixture was stirred at 50°C for 2 hr. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure. The obtained residue was purified by silica gel column chromatography [developing solvent: hexane-ethyl acetate (3:1)] to give N-(5-benzyl-4-phenyl-2-thienyl)-4-(4,5-diethoxy-2-methylphenyl)-4-oxobutanamide (188 mg) as crystals.
$^1$H-NMR ($CDCl_3$) δ: 1.45 (3H, t, J=7.2Hz), 1.47 (3H, t, J=7.2Hz), 2.50 (3H, s), 2.77 (2H, t, J=6.6Hz), 3.45 (2H, d, J=6.6Hz), 4.10 (4H, m), 4.16(2H, s), 6.63 (1H, s), 6.69 (1H, s), 7.16-7.39 (11H, m), 8.52 (1H, br s)

Example 51

ethyl (2E)-3-{4-[(5-{[4-(3,4-diethoxyphenyl)-4-oxobutanoyl]amino}-3-phenyl-2-thienyl)methyl]phenyl}acrylate

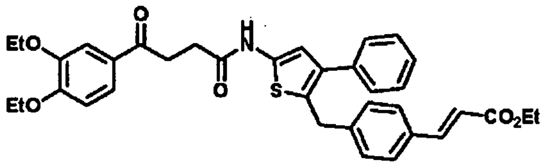

To a solution of N-[5-(4-bromobenzyl)-4-phenyl-2-thienyl]-4-(3,4-diethoxyphenyl)-4-oxobutanamide obtained in Example 47-(3) (600 mg) in DMF (5 mL) were added acetate palladium (224 mg), triphenylphosphine (520 mg), triethylamine (0.3 mL) and ethyl acrylate (0.23 mL), and the mixture was heated overnight at 100°C under nitrogen atmosphere. Water was added to the reaction mixture, and the mixture was extracted with ethyl acetate. The obtained organic layer was washed with water, dried over anhydrous magnesium sulfate, and concentrated under reduced pressure. The obtained residue was purified by silica gel column chromatography [developing solvent: hexane-ethyl acetate (2:1)] to give ethyl (2E)-3-{4-[(5-{[4-(3,4-diethoxyphenyl)-4-oxobutanoyl]amino}-3-phenyl-2-thienyl)methyl]phenyl}acrylate as crystals (24 mg).
$^1$H-NMR ($CDCl_3$) δ: 1.33(3H, t, J=7.8Hz), 1.45(6H, m), 2.79(2H, t, J=6.6Hz), 3.41 (2H, t, J=6.6Hz), 4.12 (2H, s), 4.18 (4H, m), 4.25(2H, m), 6.37(1H, d, J=15.6Hz), 6.64(1H, s), 6.88(1H, d, J=8.7Hz), 7.29-7.72(12H, m), 8.63(1H, br s)

Example 52

ethyl 5-{[4-(3,4-diethoxyphenyl)-4-oxobutanoyl]amino}-3-phenylthiophene-2-carboxylate

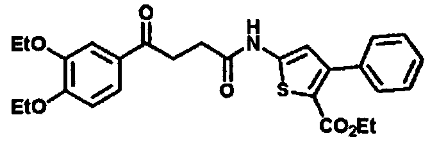

(1) ethyl 5-amino-3-phenylthiophene-2-carboxylate

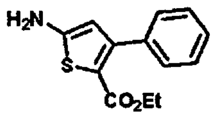

To a solution of ethyl benzoylacetate (10.0 g) in toluene (30 mL) were added cyanoacetic acid (4.4 g), ammonium acetate (0.6 g), acetic acid (1.2 mL) and piperidine (0.16 mL), and the mixture was heated under reflux overnight. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure. The obtained residue was dissolved in ethanol (30 mL), and sulfur (1.67 g) and diisopropyl ether (7.3 mL) were added. The mixture was heated at 70°C for 5 hr, and concentrated. The obtained residue was purified by silica gel column chromatography [developing solvent: hexane-ethyl acetate (2:1)] to give ethyl 5-amino-3-phenylthiophene-2-carboxylate (1.2 g) as crystals.
$^1$H-NMR ($CDCl_3$) δ: 1.26 (3H, t, J=7.2Hz), 4.14 (2H, q, J=7. 2Hz), 4.28 (2H, br s), 6.12 (1H, s), 7.32-7.44 (5H, m)
(2) ethyl 5-{[4-(3,4-diethoxyphenyl)-4-oxobutanoyl]amino}-3-phenylthiophene-2-carboxylate

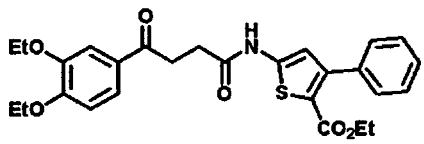

To a suspension of potassium 4-(3,4-diethoxyphenyl)-4,4-dimethoxybutanoate obtained in Example 8-(7) (350 mg) and 2,4,6-trichlorobenzoyl chloride (0.19 mL) in THF (10 mL) was added triethylamine (0.28 mL), and the mixture was stirred at room temperature for 4 hr. Ethyl 5-amino-3-phenylthiophene-2-carboxylate obtained in Example 52-(1) (240 mg) was added to the reaction mixture, and the mixture was heated overnight at 50°C. Diluted hydrochloric acid was added to the reaction mixture, and the mixture was stirred for 1 hr, and extracted with ethyl acetate. The organic layer was washed with water, dried over anhydrous magnesium sulfate, and concentrated under reduced pressure to give ethyl 5-{[4-(3,4-diethoxyphenyl)-4-oxobutanoyl]amino}-3-phenylthiophene-2-carboxylate (217 mg) as crystals.
$^1$H-NMR ($CDCl_3$) δ: 1.21(3H, t, J=7.2Hz), 1.48(6H, m), 2.86(2H, t, J=6.3Hz), 3.45(2H, t, J=6.3Hz), 4.09-4.22(6H, m), 6.61(1H, s), 6.89(1H, d, J=8.6Hz), 7.34-7.43(5H, m), 7.52(1H, d, J=2.1Hz), 7.61(1H, dd, J=8.4Hz, 2.1Hz), 9.33 (1H, br s)

Example 53

3-(5-{4-[(5-benzyl-4-phenyl-2-thienyl)amino]-4-oxobutanoyl}-2-methoxyphenyl)propyl acetate

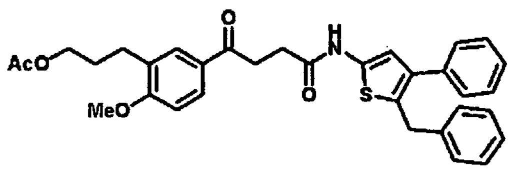

To a suspension of 5-benzyl-4-phenylthiophen-2-amine obtained in Example 17-(2) (2.0 g), 4-{3-[3-(acetyloxy)propyl]4-methoxyphenyl}-4-oxobutanoic acid (3.0 g) and HOBt (1.7 g) in acetonitrile (30 mL) was added WSC (2.0 mL), and the mixture was stirred at 50°C for 2 hr. The reaction solution was cooled under ice bath, and the precipitated solid was collected by filtration to give 3-(5-{4-[(5-benzyl-4-phenyl-2-thienyl)amino]-4-oxobutanoyl}-2-methoxyphenyl)propyl acetate (2.5 g).
$^1$H-NMR ($CDCl_3$) δ: 1.93 (2H, m), 2.06 (3H, s), 2. 69 (2H, t, J=7.8Hz), 2.80 (2H, t, J=6.3Hz), 3.40 (2H, t, J=6.3Hz), 3.88 (3H, s), 4.07 (2H, t, J=6.6Hz), 4.10 (2H, s), 6.64 (1H, s), 6.86 (1H, d, J=8.7Hz), 7.16-7.34 (10H, m), 7.79 (1H, d, J=2.4Hz), 7.87 (1H, dd, J = 9.0Hz, 2.4Hz), 8.96 (1H, br s)

Example 54

N-(5-benzyl-4-phenyl-2-thienyl)-4-[3-(3-hydroxypropyl)-4-methoxyphenyl]-4-oxobutanamide

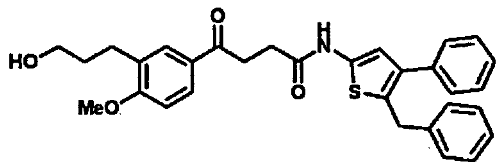

To a solution of 3-(5-{4-[(5-benzyl-4-phenyl-2-thienyl)amino]-4-oxobutanoyl}-2-methoxyphenyl)propyl acetate obtained in Example 53 (0.3 g) in a mixed solvent of methanol (50 mL)-THF (50 mL) was added 2N aqueous sodium hydroxide solution (15 mL), and the mixture was stirred overnight at room temperature. The reaction mixture was concentrated, and the obtained residue was acidified with diluted hydrochloric acid, and extracted with ethyl acetate. The obtained organic layer was washed with water, dried over anhydrous magnesium sulfate, and concentrated under reduced pressure to give N-(5-benzyl-4-phenyl-2-thienyl)-4-[3-(3-hydroxypropyl)-4-methoxyphenyl]-4-oxobutanamide (1.6 g) as crystals.
$^1$H-NMR ($CDCl_3$) δ: 1.84(2H, m), 2.73(2H, t, J=7.8Hz), 2.80 (2H, t, J=6.3Hz), 3.40(2H, t, J=6.3Hz), 3.62(2H, t, J=6.6Hz), 3.89(3H, s), 4.11(2H, s), 6.64(1H, s), 6.87(1H, d, J=8.7Hz), 7.16-7.39 (10H, m), 7.81 (1H, d, J=2.1Hz), 7.86(1H, dd, J=8.4Hz, 2.1Hz), 8.67 (1H, br s)
Anal. Calcd for $C_{31}H_{31}NO_4S$: C, 72.49; H, 6.08; N, 2.73.Found: C, 72.17; H, 6.14; N, 2.56.

Example 55

N-(5-benzyl-4-phenyl-2-thienyl)-4-(3-ethyl-4-methoxyphenyl)-4-oxobutanamide

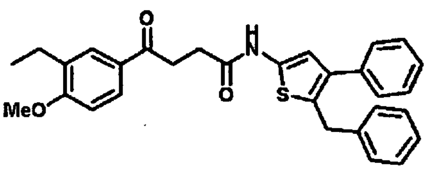

(1) 4-(3-ethyl-4-methoxyphenyl)-4-oxobutanoic acid

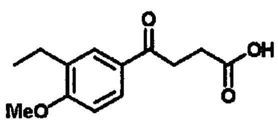

To a suspension of ethyl-2-methoxybenzene (9.9 g) and succinic anhydride (8.0 g) in dichloromethane (50 mL) was gradually added aluminum chloride (24.2 g) under ice-cooling, and the mixture was stirred for 0.5 hr. The reaction mixture was poured into concentrated hydrochloric acid (100 mL)-ice, and the mixture was stirred for 1 hr, and extracted with ethyl acetate. The obtained organic layer was washed with water, dried over anhydrous magnesium sulfate, and concentrated under reduced pressure to give 4-(3-ethyl-4-methoxyphenyl)-4-oxobutanoic acid (14.5 g)

as crude crystals.
$^1$H-NMR ($CDCl_3$) δ: 1.21(3H, t, J=7.8Hz), 2.65(2H, q, J=7.8Hz), 2.80(2H, t, J=6.6Hz), 3.29(2H, t, J=6.6Hz), 3.89 (3H, s), 6.86(1H, d, J=8.4Hz), 7.80(1H, m), 7.85(1H, m)
(2) N-(5-benzyl-4-phenyl-2-thienyl)-4-(3-ethyl-4-methoxyphenyl)-4-oxobutanamide

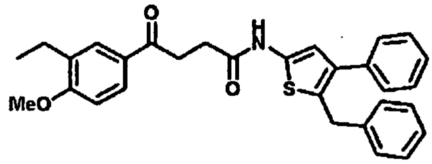

To a suspension of 5-benzyl-4-phenylthiophen-2-amine obtained in Example 17-(2) (265 mg), 4-(3-ethyl-4-methoxyphenyl)-4-oxobutanoic acid obtained in Example 55-(1) (307 mg) and HOBt (230 mg) in acetonitrile (5 mL) was added WSC (0.26 mL), and the mixture was stirred at 50°C for 2 hr. The reaction solution was cooled under ice bath, and the precipitated solid was collected by filtration to give N-(5-benzyl-4-phenyl-2-thienyl)-4-(3-ethyl-4-methoxyphenyl)-4-oxobutanamide (289 mg).
$^1$H-NMR ($CDCl_3$) δ: 1.19(3H, t, J=7.5Hz), 2.64(2H, q, J=7.5Hz), 2.81(2H, t, J=6.3Hz), 3.42(2H, t, J=6.3Hz), 3.89(3H, s), 4.11(2H, s), 6.64(1H, s), 6.85(1H, d, J=8.4Hz), 7.16-7.39(10H, m), 7.80(1H, m), 7.81(1H, m), 8.73(1H, br s)

Example 56

tert-butyl 5-{[4-(3,4-diethoxyphenyl)-4-oxobutanoyl]amino}-3-phenylthiophene-2-carboxylate

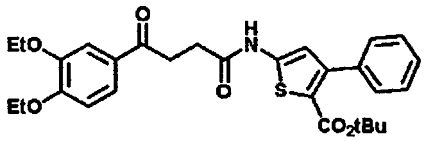

To a suspension of potassium 4-(3,4-diethoxyphenyl)-4,4-dimethoxybutanoate obtained in Example 8-(7) (350 mg) and 2,4,6-trichlorobenzoyl chloride (0.19 mL) in THF (10 mL) was added triethylamine (0.28 mL), and the mixture was stirred at room temperature for 4 hr. tert-Butyl 5-amino-3-phenylthiophene-2-carboxylate synthesized according to a method described in Chem. Pharm. Bull., 36, 4389-4402 (1988) (260 mg) was added to the reaction mixture, and the mixture was heated overnight at 50°C. Diluted hydrochloric acid was added to the reaction mixture, and the mixture was stirred for 1 hr, and extracted with ethyl acetate. The organic layer was washed with water, dried over anhydrous magnesium sulfate, and concentrated under reduced pressure. The obtained residue was purified by silica gel column chromatography [developing solvent: hexane-ethyl acetate (2:1)] to give tert-butyl 5-{[4-(3,4-diethoxyphenyl)-4-oxobutanoyl]amino}-3-phenylthiophene-2-carboxylate (120 mg) as crystals.
$^1$H-NMR ($CDCl_3$) δ: 1.41(9H, s), 1.48(6H, m), 2.85(2H, t, J=6.3Hz), 3.45(2H, t, J=6.3Hz), 4.09-4.21(4H, m), 6.58(1H, s), 6.89(1H, d, J=8.6Hz), 7.32-7.42(5H, m), 7.52(1H, d, J=2.1Hz), 7.60(1H, dd, J=8.4Hz, 2.1Hz), 9.00(1H, br s)

Example 57

N-(5-benzyl-4-phenyl-1,3-thiazol-2-yl)-4-(3,4-diethoxyphenyl)-3-methyl-4-oxobutanamide

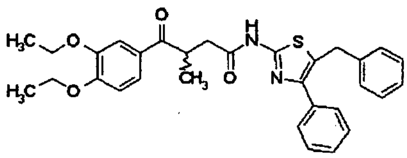

(1) ethyl 4-(3,4-diethoxyphenyl)-3-methyl-4-oxobutanoate

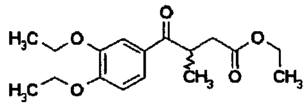

To a solution of 1,2-diethoxybenzene (8.31 g, 0.05 mol) in dichloromethane (80 mL) was slowly added aluminum chloride (8.0 g, 0.06 mol) under ice-cooling. Propionyl chloride (5.55 g, 0.06 mol) was added dropwise to the reaction mixture under ice-cooling, and the mixture was stirred for 30 min. The reaction mixture was poured into ice, concentrated hydrochloric acid (80 mL) was added, and the mixture was stirred for 1 hr. The reaction mixture was extracted with ethyl acetate, and the organic layer was washed with saturated aqueous sodium hydrogencarbonate solution and saturated brine, and dried over anhydrous magnesium sulfate. The extract was concentrated, and the residue was dissolved in THF (30 mL). A mixed solution of 15% potassium bis(trimethylsilyl)amide-toluene solution (79.8 g, 0.06 mol) and THF (100 mL) was slowly added dropwise at -78°C, and the mixture was stirred at the same temperature for 2 hr. Ethyl bromoacetate (6.65 mL, 0.06 mol) was added to the reaction mixture, and the mixture was stirred for 2 hr. 1N Aqueous hydrochloric acid solution (60 mL) was added to the reaction mixture, and the mixture was extracted with ethyl acetate. The organic layer was washed with water and saturated brine, dried over anhydrous sodium sulfate, and concentrated under reduced pressure. The residue was crudely purified by silica gel column chromatography to give ethyl 4-(3,4-diethoxyphenyl)-3-methyl-4-oxobutanoate (9.61 g). MASS m/z: 309.1 ($MH^+$)

(2) N-(5-benzyl-4-phenyl-1,3-thiazol-2-yl)-4-(3,4-diethoxyphenyl)-3-methyl-4-oxobutanamide

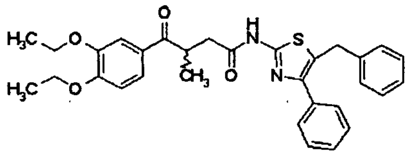

Using ethyl 4-(3,4-diethoxyphenyl)-3-methyl-4-oxobutanoate (9.61 g) as a starting material and in the same manners as in Example 5-(2) and (3), N-(5-benzyl-4-phenyl-1,3-thiazol-2-yl)-4-(3,4-diethoxyphenyl)-3-methyl-4-oxobutanamide (126 mg) was synthesized.

$^1H$-NMR (DMSO-$d_6$) δ : 1.11(3H, d, J=7.2Hz), 1.34(6H, q, J=7.1Hz), 2.52-2.61(1H, m), 2.92(1H, dd, J=16.0Hz, 8.9Hz), 4.02-4.16 (5H, m), 4.20(2H, s), 7.07(1H, d, J=8.5Hz), 7.17-7.24(3H, m), 7.27-7.39(3H, m), 7.42-7.48(3H, m), 7.59-7.68 (3H, m), 12.23(1H, s)

MASS m/z: 529.1 ($MH^+$)

Example 58

N-(5-butyl-4-phenyl-2-thienyl)-4-(3,4-diethoxyphenyl)-4-oxobutanamide

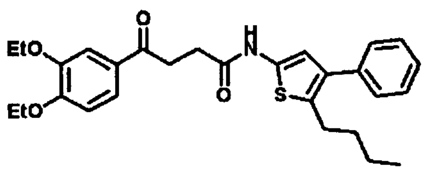

(1) tert-butyl (4-bromo-5-butyl-2-thienyl)carbamate

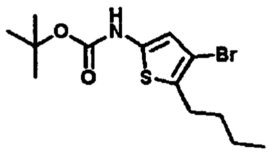

To a solution of 4,5-dibromothiophene-2-carboxylic acid (5.0 g) in THF (50 mL) was added dropwise 1.6M n-butyllithium-hexane solution (23 mL) at -78°C, and the mixture was stirred at the same temperature for 30 min. 1-Bromo-3-methylbutane (2.3 mL) was added, and the mixture was stirred at -78°C for 30 min. The reaction mixture was allow to warm to room temperature, diluted hydrochloric acid was added, and the mixture was extracted with ethyl acetate. The organic layer was washed with water, dried over anhydrous magnesium sulfate, and concentrated under reduced pressure. To a solution of the obtained residue in toluene (60 mL) were added triethylamine (2.8 mL) and DPPA (3.1 mL), and the mixture was stirred at room temperature for 1 hr. tert-Butanol (2.0 mL) was added, and the mixture was heated overnight at 90°C. The reaction mixture was concentrated. The obtained residue was purified by silica gel column chromatography [developing solvent: hexane-ethyl acetate (4:1)] to give tert-butyl (4-bromo-5-butyl-2-thienyl)carbamate (1.4 g) as an oil.

$^1$H-NMR ($CDCl_3$) δ: 0.92(3H, m), 1.37(2H, m), 1.62(2H, m), 2.68(2H, t, J=7.5Hz), 6.31(1H, s), 6.83(1H, br s)

(2) 4-bromo-5-butylthiophen-2-amine

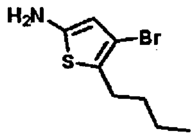

To tert-butyl (4-bromo-5-butyl-2-thienyl)carbamate obtained in Example 58-(1) (1.4 g) was added trifluoroacetic acid (3 mL), and the mixture was stirred at room temperature for 3 hr. The reaction mixture was concentrated, and the residue was alkalified with aqueous sodium hydrogencarbonate solution, and extracted with ethyl acetate. The organic layer was washed with water, dried over anhydrous magnesium sulfate, and concentrated under reduced pressure to give 4-bromo-5-butylthiophen-2-amine (0.8 g) as crystals. The crystals were used for the next step without purification.

(3) N-(4-bromo-5-butyl-2-thienyl)-4-(3,4-diethoxyphenyl)-4-oxobutanamide

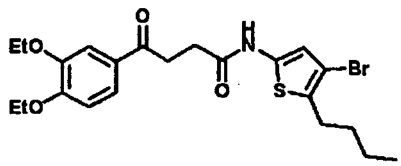

To a suspension of potassium 4-(3,4-diethoxyphenyl)-4,4-dimethoxybutanoate obtained in Example 8-(7) (1.3 g) and 2,4,6-trichlorobenzoyl chloride (0.64 mL) in THF (20 mL) was added triethylamine (0.95 mL), and the mixture was stirred at room temperature for 4 hr. 4-Bromo-5-butylthiophen-2-amine obtained in Example 58-(2) (0.8 g) was added to the reaction mixture, and the mixture was heated overnight at 50°C. Diluted hydrochloric acid was added to the reaction mixture, and the mixture was stirred for 1 hr, and extracted with ethyl acetate. The organic layer was washed with water, dried over anhydrous magnesium sulfate, and concentrated under reduced pressure. The obtained residue was purified by silica gel column chromatography [developing solvent: hexane-ethyl acetate (2:1)] to give N-(4-bromo-5-butyl-2-thienyl)-4-(3,4-diethoxyphenyl)-4-oxobutanamide (0.4 g) as crystals.

$^1$H-NMR ($CDCl_3$) δ: 0.92 (3H, t, J=7.2Hz), 1.38(2H, m), 1.47(3H, t, J=7.2 Hz), 1.49(3H, t, J=7.2Hz), 1.59(2H, m), 2.68(2H, t, J=7.5Hz), 2.80(2H, t, J=6.3 Hz), 3.41(2H, t, J=6.3Hz), 4.16(4H, m), 6.46 (1H, s), 6.88 (1H, d, J=8.7Hz), 7.52 (1H, d, J=2.7Hz), 7.59 (1H, dd, J=8.7Hz, 2.7Hz), 8.73(1H, br s)

(4) N-(5-butyl-4-phenyl-2-thienyl)-4-(3,4-diethoxyphenyl)-4-oxobutanamide

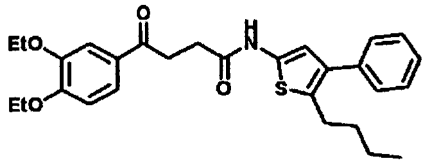

To a solution of N-(4-bromo-5-butyl-2-thienyl)-4-(3,4-diethoxyphenyl)-4-oxobutanamide obtained in Example 58-(3) (200 mg) in a mixed solvent of toluene (5 mL)-water (1 mL) were added phenylboronic acid (56 mg), tetrakis (triphenylphosphine)palladium (24 mg) and potassium carbonate (115 mg), and the mixture was heated under reflux overnight under nitrogen atmosphere. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure to give N-(5-butyl-4-phenyl-2-thienyl)-4-(3,4-diethoxyphenyl)-4-oxobutanamide (103 mg) as crystals.
$^1$H-NMR ($CDCl_3$) δ: 0.85(3H, t, J=7.2Hz), 1.29(2H, m), 1.47(3H, t, J=7.2Hz), 1.49(3H, t, J=7.2Hz), 1.59 (2H, m), 2.77 (4H, m), 3.43 (2H, t, J=6.3Hz), 4.16(4H, m), 6.58(1H, s), 6.88(1H, d, J=8.7Hz), 7.26-7.41(5H, m), 7.53(1H, d, J=2.7Hz), 7.60(1H, dd, J=8.7Hz, 2.7Hz), 8.55(1H, br s)

Example 59

Ethyl 3-(5-{[4-(3,4-dimethoxyphenyl)-4-oxobutanoyl]amino}-3-phenyl-2-thienyl)propionate

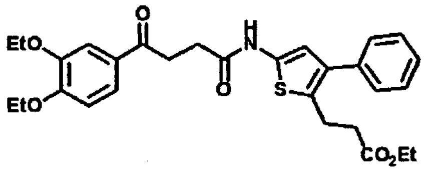

(1) tert-butyl 4-bromo-5-[(1E)-3-ethoxy-3-oxo-1-propen-1-yl]thiophene-2-carboxylate

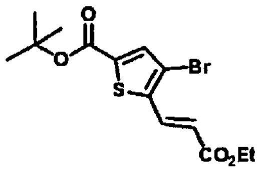

To a solution of ethyl diethylphosphonoacetate (2.9 g) in DMF (20 mL) was added sodium hydride (60%, in oil, 0.57 g), and the mixture was stirred for 0.5 hr under ice-cooling. tert-Butyl 4-bromo-5-formylthiophene-2-carboxylate (3.8 g) was added, and the mixture was stirred for 0.5 hr under ice-cooling. Water was added to the reaction mixture, and the mixture was extracted with ethyl acetate. The obtained organic layer was washed with water, dried over anhydrous magnesium sulfate, and concentrated under reduced pressure. The obtained residue was purified by silica gel column chromatography [developing solvent: hexane-ethyl acetate (10:1)] to give tert-butyl 4-bromo-5-[(1E)-3-ethoxy-3-oxo-l-propen-1-yl]thiophene-2-carboxylate (4.0 g) as an oil.
$^1$H-NMR ($CDCl_3$) δ: 1.34(3H, t, J=7.2Hz), 1.56(9H, s), 4.27(2H, q, J=7.2Hz), 6.40(1H, d, J=15.9Hz), 7.57(1H, s), 7.79(1H, d, J=15.9Hz)
(2) 4-bromo-5-[(1E)-3-ethoxy-3-oxo-1-propen-1-yl]thiophene-2-carboxylic acid

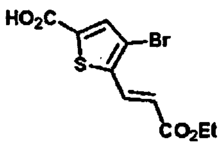

To tert-butyl 4-bromo-5-[(1E)-3-ethoxy-3-oxo-1-propen-1-yl]thiophene-2-carboxyate obtained in Example 59-(1) (4.0 g) was added trifluoroacetic acid (3 mL), and the mixture was stirred at room temperature for 1 hr. The reaction mixture was concentrated, and the residue was alkalified with aqueous sodium hydrogencarbonate solution, and extracted with ethyl acetate. The organic layer was washed with water, dried over anhydrous magnesium sulfate, and concentrated under reduced pressure to give 4-bromo-5-[(1E)-3-ethoxy-3-oxo-1-propen-1-yl]thiophene-2-carboxylic acid (3.0 g) as crystals.

$^1$H-NMR ($CDCl_3$) δ: 1.35 (3H, t, J=7.2Hz), 4.29 (2H, q, J=7.2Hz), 6.46(1H, d, J=16.2Hz), 7.76 (1H, s), 7.82 (1H, d, J=16.2Hz)

(3) ethyl (2E)-3-{3-bromo-5-[(tert-butoxycarbonyl)amino]-2-thienyl}acrylate

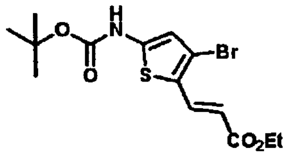

To a solution of 4-bromo-5-[(1E)-3-ethoxy-3-oxo-1-propen-1-yl]thiophene-2-carboxylic acid obtained in Example 59-(2) (1.0 g) in toluene (20 mL) were added triethylamine (0.7 mL) and DPPA (0.8 mL), and the mixture was stirred at room temperature for 1 hr. tert-Butanol (0.5 mL) was added, and the mixture was heated overnight at 90°C. The reaction mixture was concentrated. The obtained residue was purified by silica gel column chromatography [developing solvent: hexane-ethyl acetate (7:1)] to give ethyl (2E)-3-{3-bromo-5-[(tert-butoxycarbonyl)amino]-2-thienyl} acrylate (0.8 g) as crystals.

$^1$H-NMR ($CDCl_3$) δ: 1.32(3H, t, J=7.2Hz), 1.57 (9H, s), 4.24 (2H, q, J=7.2Hz), 6.12 (1H, d, J = 15.9Hz), 6.43 (1H, s), 7 .20 (1H, br s), 7.76(1H, d, J=15.9Hz)

(4) ethyl (2E)-3-{5-[(tert-butoxycarbonyl)amino]-3-phenyl-2-thienyl}acrylate

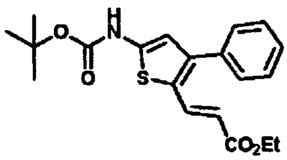

To a solution of ethyl (2E)-3-{3-bromo-5-[(tert-butoxycarbonyl)amino]-2-thienyl}acrylate obtained in Example 59-(3) (0.8 g) in a mixed solvent of toluene (20 mL)-water (4 mL) were added phenylboronic acid (0.3 g), tetrakis(triphenylphosphine)palladium (130 mg) and potassium carbonate (0.61 g), and the mixture was heated under reflux overnight under nitrogen atmosphere. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure. The obtained residue was purified by silica gel column chromatography [developing solvent: hexane-ethyl acetate (7: 1)] to give ethyl (2E)-3-{5-[(tert-butoxycarbonyl)amino]-3-phenyl-2-thienyl}acrylate (0.9 g) as an oil.

$^1$H-NMR ($CDCl_3$) δ: 1.27(3H, t, J=7.2Hz), 1.55(9H, s), 4.12(2H, q, J=7.2Hz), 6.13(1H, d, J=15.3Hz), 6.52(1H, s), 7.16(1H, br s), 7.33-7.46(5H, m), 7.77(1H, d, J=15.3 Hz)

(5) ethyl 3-{5-[(tert-butoxycarbonyl)amino]-3-phenyl-2-thienyl}propionate

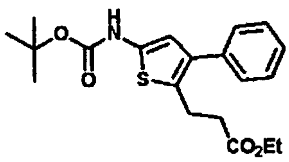

To a solution of ethyl (2E)-3-{5-[(tert-butoxycarbonyl)amino]-3-phenyl-2-thienyl}acrylate obtained in Example 59-(4) (0.9 g) in a mixed solvent of ethanol (10 mL)-THF (10 mL) was added 10% palladium-carbon (0.2 g), and the mixture was stirred overnight under hydrogen atmosphere. The reaction mixture was filtrated through celite, and the filtrate was concentrated to give ethyl 3-{5-[(tert-butoxycarbonyl)amino]-3-phenyl-2-thienyl}propionate as an oil. This was used for the next step without purification.

$^1$H-NMR ($CDCl_3$) δ: 1.27(3H, t, J=7.2Hz), 2.59(2H, t, J=8.7Hz), 3.11(2H, t, J=8.7Hz), 4.10(2H, q, J=7.2Hz), 6.45 (1H, s), 6.85 (1H, br s), 7.31-7.42(5H, m)

(6) ethyl 3-(5-amino-3-phenyl-2-thienyl)propionate

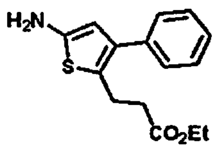

To ethyl 3-{5-[(tert-butoxycarbonyl)amino]-3-phenyl-2-thienyl}propionate obtained in Example 59-(5) was added trifluoroacetic acid (5 mL), and the mixture was stirred at room temperature for 1 hr. The reaction mixture was concentrated, and the residue was alkalified with aqueous sodium hydrogencarbonate solution, and extracted with ethyl acetate. The organic layer was washed with water, dried over anhydrous magnesium sulfate, and concentrated under reduced pressure to give ethyl 3-(5-amino-3-phenyl-2-thienyl) propionate (0.56 g) as an oil. This was used for the next step without purification.

(7) ethyl 3-(5-{[4-(3,4-dimethoxyphenyl)-4-oxobutanoyl]amino}-3-phenyl-2-thienyl)propionate

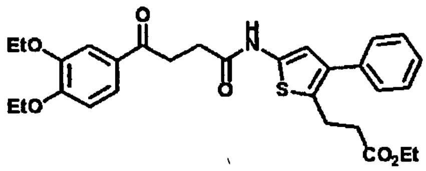

To a suspension of ethyl 3-(5-amino-3-phenyl-2-thienyl)propionate obtained in Example 59-(6) (0.56 g), 4-(4,5-diethoxy-2-methylphenyl)-4-oxobutanoic acid obtained in Example 3-(1) (0.70 g) and HOBt (0.47 g) in acetonitrile (10 mL) was added WSC (0.54 mL), and the mixture was stirred at 50°C for 2 hr. Water was added to the reaction mixture, and the mixture was extracted with ethyl acetate. The organic layer was washed successively with aqueous sodium hydrogencarbonate solution and water, and dried over anhydrous magnesium sulfate, and the solvent was evaporated under reduced pressure. The obtained residue was purified by silica gel column chromatography [developing solvent: hexane-ethyl acetate (3:1)] to give ethyl 3-(5-{[4-(3,4-dimethoxyphenyl)-4-oxobutanoyl]amino}-3-phenyl-2-thienyl)propionate (0.5 g) as crystals.

$^1$H-NMR ($CDCl_3$) δ: 1.22(3H, t, J=7.2Hz), 1.46(3H, t, J=7.8Hz), 1.49(3H, t, J=7.8Hz), 2.58(2H, t, J=7.5Hz), 2.80(2H, t, J=6.0Hz), 3.11(2H, t, J=7.5Hz), 3.42(2H, t, J=6.0Hz), 4.05-4.20(6H, m), 6.57(1H, s), 6.87(1H, d, J=8.4Hz), 7.28-7.40 (5H, m), 7.51(1H, d, J=2.1Hz), 7.59(1H, dd, J=8.4Hz, 2.1Hz), 8.56(1H, br s)

## Example 60

N-(2,5-diphenyl-3-thienyl)-4-(4-ethoxyphenyl)-4-oxobutanamide

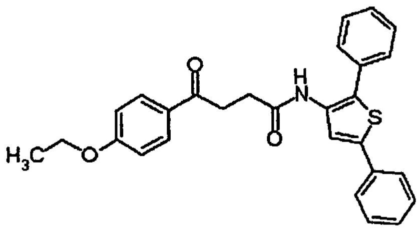

(1) methyl 4-(4-ethoxyphenyl)-4-oxobutanoate

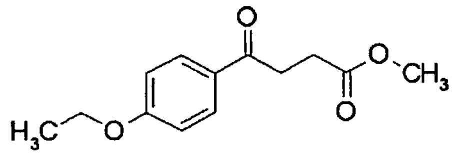

To a solution of phenetole (15.0 g) and methylsuccinyl chloride (18.0 mL) in dichloromethane (200 mL) was slowly added aluminum chloride (19.6 g) under ice-cooling, and the mixture was stirred for 30 min. The reaction mixture was poured into ice water, and the mixture was extracted with chloroform. The organic layer was washed with 5% aqueous potassium carbonate solution and saturated brine, dried over anhydrous sodium sulfate, and concentrated under reduced pressure. The residue was purified by silica gel column chromatography [developing solvent: n-hexane-ethyl acetate (3:1)], and the obtained crystals were recrystallized from ethyl acetate-n-hexane (1:3) to give methyl 4-(4-ethoxyphenyl)-4-oxobutanoate (18.1 g) as white crystals.

$^1$H-NMR ($CDCl_3$) δ : 1.44(3H, t, J=7.0Hz), 2.75 (2H, t, J=6.7Hz), 3.27 (2H, t, J=6.7Hz), 3.71(3H, s), 4.10(2H, q, J=7.0Hz), 6.92 (2H, d, J=8.6Hz), 7.96(2H, d, J=8.6Hz)

MASS m/z: 237.04 ($MH^+$)

(2) methyl 4-(4-ethoxyphenyl)-4,4-dimethoxybutanoate

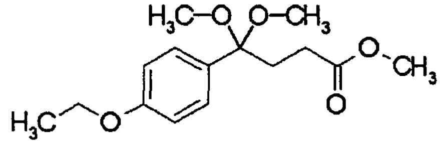

To a solution of methyl 4-(4-ethoxyphenyl)-4-oxobutanoate obtained in Example 60-(1) (17.7 g) in methanol (300 mL) were added trimethyl orthoformate (25 mL) and p-toluenesulfonic acid monohydrate (100 mg), and the mixture was stirred overnight at 50°C. Sodium hydrogencarbonate (2.5 g) was added to the reaction mixture, and the solvent was evaporated under reduced pressure. The residue was dissolved in ethyl acetate, and the mixture was washed with saturated aqueous sodium hydrogencarbonate solution and saturated brine, and dried over anhydrous sodium sulfate. After filtration, the solvent was evaporated to give methyl 4-(4-ethoxyphenyl)-4,4-dimethoxybutanoate (21.4 g).

$^1$H-NMR($CDCl_3$)δ: 1.41 (3H, t, J=7.0Hz) , 1.98-2.11(2H, m), 2.17-2.25 (2H, m), 3.14 (6H, s), 3.56 (3H, s), 4.03 (3H, q, J=7.0Hz), 6.86 (2H, d, J=8.9Hz), 7.34 (2H, d, J=8.9Hz)

(3) potassium 4-(4-ethoxyphenyl)-4,4-dimethoxybutanoate

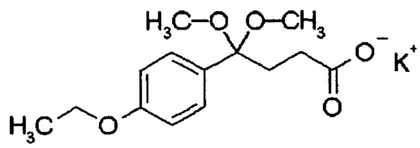

A solution of methyl 4-(4-ethoxyphenyl)-4,4-dimethoxybutanoate obtained in Example 60-(2) (21.4 g) and potassium hydroxide (5.0 g) in methanol (300 mL) was stirred overnight at 50°C, then at room temperature for 3 days, and at 50°C for 2 days. The solvent was evaporated under reduced pressure, and diisopropyl ether was added to the residue. The precipitate was collected by filtration, and washed with diisopropyl ether to give potassium 4-(4-ethoxyphenyl)-4,4-dimethoxybutanoate (23.8 g).

$^1$H-NMR($CDCl_3$) δ: 1.31(3H, t, J=7.0Hz), 1.31-1.38(2H, m), 1.87-1.96 (2H, m), 3.00(6H, s), 4.03(3H, q, J=7.0Hz), 6.86(2H, d, J=8.9Hz), 7.22(2H, d, J=8.9Hz)

(4) N-(2,5-diphenyl-3-thienyl)-4-(4-ethoxyphenyl)-4-oxobutanamide

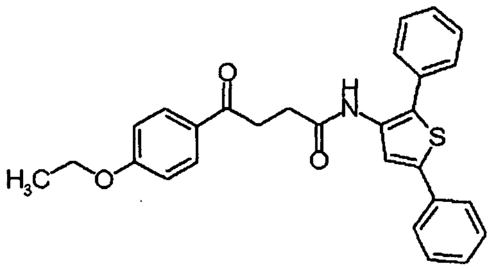

A solution of potassium 4-(4-ethoxyphenyl)-4,4-dimethoxybutanoate obtained in Example 60-(3) (238 mg), triethylamine (0.114 mL) and 2,4,6-trichlorobenzoyl chloride (0.121 mL) in THF (5 mL) was stirred at room temperature 5 hr. (2,5-Diphenyl-3-thienyl)amine obtained in Example 18-(4) (97.5 mg) was added to the reaction mixture, and the mixture was stirred at 60°C for 3 days. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (19:1→1:1)] to give a red liquid. This compound was dissolved in acetonitrile (10 mL), and trifluoroacetic acid (1 mL) was added. The mixture was stood still at room temperature for 5 min, and concentrated under reduced pressure. The residue was purified by silica gel column chromatography [developing solvent: n-hexane-ethyl acetate (4:1→4:6)], and the obtained crystals were recrystallized from chloroform-n-hexane (1:3) to give N-(2,5-diphenyl-3-thienyl)-4-(4-ethoxyphenyl)-4-oxobutanamide (177 mg) as white crystals.
$^1$H-NMR ($CDCl_3$) δ : 1.35 (3H, t, J=7.1Hz), 2.68 (2H, t, J=6.4Hz), 3.22-3.30 (2H, m), 4.13 (2H, q, J=7.1Hz), 7.04 (2H, d, J=8.7Hz), 7.28-7.52(6H, m), 7.58 (1H, s), 7.60-7.68(4H, m), 7.98(2H, d, J=8.7Hz), 9.79 (1H, s)
MASS m/z: 456.03 ($MH^+$)

Example 61

N-{2-[(4-ethoxybenzoyl)amino]ethyl}-1-phenyl-3-(trifluoromethyl)-1H-pyrazole-4-carboxamide

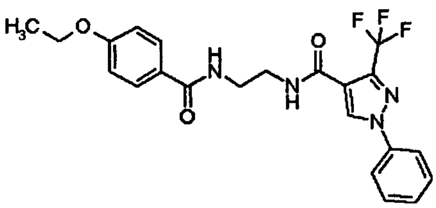

(1) N-(2-aminoethyl)-4-ethoxybenzamide hydrochloride

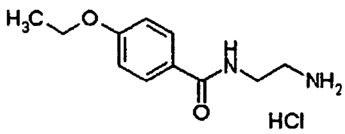

A solution of 4-ethoxybenzoic acid (3.0 g, 0.018 mol), tert-butyl (2-aminoethyl)carbamate (3.18 g, 0.0199 mol), 1-ethyl-3-(3-dimethylaminopropyl)carbodiimide hydrochloride (5.19 g, 0.027 mol) and HOBt (4.15 g, 0.027 mmol) in acetonitrile (30 mL) was stirred at room temperature for 4 hr. The reaction mixture was extracted with ethyl acetate, and the organic layer was washed with aqueous sodium hydrogencarbonate solution and saturated brine, dried over anhydrous magnesium sulfate, and concentrated under reduced pressure. The residue was dissolved in ethyl acetate (50 mL), 4N hydrochloric acid-ethyl acetate solution (19 ml, 0.0762 mol) was added, and the mixture was stirred for 24 hr. The reaction mixture was concentrated under reduced pressure, and the obtained residue was collected by filtration, and washed with ethyl acetate to give N-(2-aminoethyl)-4-ethoxybenzamide hydrochloride (3.12 g).

$^1$H-NMR($CDCl_3$)δ: 1.34(3H, t, J=7.0Hz), 2.98(2H, t, J=6.2Hz), 3.52(2H, q, J=6.0 Hz), 4.08(2H, q, J=7.0 Hz), 6.98 (2H, ddd, J=9.4, 2.8, 2.4 Hz), 7.88-7.94(2H, m), 8.20(3H, s), 8.69(1H, t, J=5.5Hz)

MASS m/ z : 209.15(MH$^+$)

(2) ethyl 3-(trifluoromethyl)-1H-pyrazole-4-carboxylate

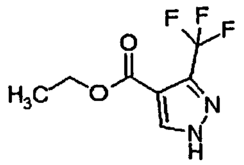

A mixture of ethyl 4,4,4-trifluoro-3-oxobutanoate (64.8 g, 0.352 mol), triethyl orthoformate (117ml, 0.7039 mol) and acetic anhydride (100 mL, 1.06 mol) was stirred at 120°C for 2 hr, and heated under reflux for 5 hr. The reaction mixture was concentrated under reduced pressure. Hydrazine monohydrate (25.6 ml, 0.528 mol) and methanol (500 mL) were added to the residue, and the mixture was heated under reflux for 3 hr. The reaction mixture was concentrated under reduced pressure, and the residue was extracted with ethyl acetate. The organic layer was washed with water and saturated brine, and dried over anhydrous magnesium sulfate. The extract concentrated under reduced pressure. The residue was recrystallized from ethyl acetate-hexane to give ethyl 3-(trifluoromethyl)-1H-pyrazole-4-carboxylate (49.66 g) as crystals.

$^1$H-NMR ($CDCl_3$) δ: 1.28(4H, t, J=7.1Hz) 4.25 (2H, q, J=7.0Hz) 8.59 (1H, s) 14.13 (1H, t)

MASS m/z: 208.97 (MH$^+$)

(3) 1-phenyl-3-(trifluoromethyl)-1H-pyrazole-4-carboxylic acid

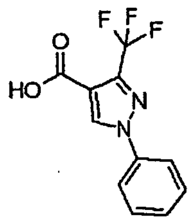

To a solution of ethyl 3-(trifluoromethyl)-1H-pyrazole-4-carboxylate obtained in Example 61-(2) (10.41 g, 0.05 mol) in N,N-dimethylformamide (100 mL) were added phenylboronic acid (12.19 g, 0.1 mol), copper(II) acetate (13.62 g, 0.075 mol) and pyridine (8.09ml, 0.1 mol), and the mixture was stirred at room temperature for 24 hr. The reaction mixture was filtrated through celite, the filtrate was concentrated under reduced pressure, and the residue was extracted with ethyl acetate. The organic layer was washed with saturated aqueous sodium hydrogencarbonate solution and saturated brine, and dried over anhydrous sodium sulfate. The extract was concentrated under reduced pressure. To a solution of the residue in a mixed solvent of ethanol (100 mL)-THF (20 mL) was added 2N aqueous sodium hydroxide solution (75 ml, 0.15 mol), and the mixture was stirred at room temperature 5 hr, then at 50°C for 1 hr, and heated under reflux for 2 hr. The reaction mixture was concentrated under reduced pressure, and the residue was extracted with water, and the aqueous layer was washed with diethyl ether. The extract was neutralized with 6N aqueous hydrochloric acid solution, and extracted with ethyl acetate. The organic layer was washed with saturated brine, and dried over anhydrous sodium sulfate. The extract was concentrated under reduced pressure, and the obtained crystals were recrystallized from ethyl acetate-hexane to give 1-phenyl-3-(trifluoromethyl)-1H-pyrazole-4-carboxylic acid (10.06 g) as crystals.

$^1$H-NMR($CDCl_3$)δ: 7.47(1H, t, J=7.3Hz) 7.54-7.61(2H, m) 7.91-7.98(2H, m) 9.23 (1H, d, J=0.9Hz) 13.23 (1H, s)

MASS m/z: 279.03 (MNa$^+$)

(4) N-{2-[(4-ethoxybenzoyl)amino]ethyl}-1-phenyl-3-(trifluoromethyl)-1H-pyrazole-4-carboxamide

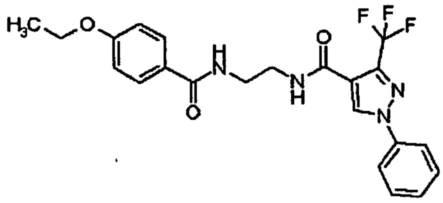

A solution of N-(2-aminoethyl)-4-ethoxybenzamide hydrochloride obtained in Example 61-(1) (98 mg, 0.4 mmol), 1-phenyl-3-(trifluoromethyl)-1H-pyrazole-4-carboxylic acid obtained in Example 61-(3) (102 mg, 0.4 mmol), 1-ethyl-3-(3-dimethylaminopropyl)carbodiimide hydrochloride (115 mg, 0.6 mmol), HOBt (92 mg, 0.6 mmol) and triethylamine (0.112 ml, 0.8 mmol) in N,N-dimethylformamide (5 mL) was stirred at room temperature for 24 hr. The reaction mixture was extracted with ethyl acetate, and the organic layer was washed with aqueous sodium hydrogencarbonate solution and saturated brine, and dried over magnesium sulfate. The extract was concentrated under reduced pressure, and the residue was recrystallized from ethyl acetate-hexane to give N-{2-[(4-ethoxybenzoyl)amino]ethyl}-1-phenyl-3-(trifluoromethyl)-1H-pyrazole-4-carboxamide (148 mg) as crystals.
$^1$H-NMR ($CDCl_3$) $\delta$ : 1.42 (3H, t, J=7.0Hz), 3.57-3.77 (4H, m), 4.06 (2H, q, J=6.9Hz), 6.85-6.98(2H, m), 7.36-7.45(1H, m), 7.46-7.61(3H, m), 7.66-7.74 (2H, m), 7.74-7.89 (3H, m), 8.55(1H, s)
MASS m/z: 446.94 ($MH^+$)

Example 62

N-{2-[(4-ethoxybenzoyl)amino]ethyl}-2-methyl-5-phenyl-1-[4-(trifluoromethoxy)phenyl]-1H-pyrrole-3-carboxamide

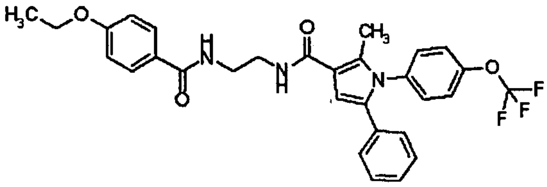

(1) ethyl 2-acetyl-4-oxo-4-phenylbutanoate

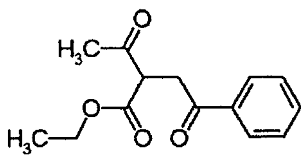

To a suspension of 60% sodium hydride (5.28 g, 0.132 mol) in THF (120 mL) was added dropwise a solution of ethyl acetoacetate (15.62 g, 0.12 mol) in THF (30 mL) under ice-cooling, and the mixture was stirred for 80 min under ice-cooling. 2-Bromo-1-phenylethanone (26.27 g, 0.132 mol) was gradually added to the reaction mixture under ice-cooling, and the mixture was stirred overnight at room temperature. The reaction mixture was extracted with ethyl acetate, and the organic layer was washed with aqueous ammonium chloride solution and saturated brine, and dried over anhydrous magnesium sulfate. The extract was concentrated under reduced pressure, and the residue was purified by silica gel column chromatography to give ethyl 2-acetyl-4-oxo-4-phenylbutanoate (27.29 g) as an oil. MASS m/z: 271.02 ($MNa^+$)
(2) ethyl 2-methyl-5-phenyl-1-[4-(trifluoromethoxy)phenyl]-1H-pyrrole-3-carboxylate

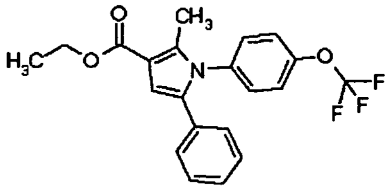

A solution of ethyl 2-acetyl-4-oxo-4-phenylbutanoate obtained in Example 62-(1) (3.5 g, 0.0141 mol) and 4-(trifluoromethoxy)aniline (2.1 ml, 0.0155 mol) in acetic acid (35 mL) was heated under reflux for 1 hr. The reaction mixture was concentrated under reduced pressure, and the residue was extracted with ethyl acetate. The organic layer was washed with saturated aqueous sodium hydrogencarbonate solution and saturated brine, and dried over anhydrous magnesium sulfate. The extract was concentrated under reduced pressure, and the residue was purified by silica gel column chromatography to give ethyl 2-methyl-5-phenyl-1-[4-(trifluoromethoxy)phenyl]-1H-pyrrole-3-carboxylate (4.71 g) as a solid.

$^1$H-NMR ($CDCl_3$) δ: 1.38 (3H, t, J=7.2Hz), 2.42 (3H, s), 4.33 (2H, q, J=7.0Hz), 6.79(1H, s), 7.00-7.05(2H, m), 7.13-7.20 (5H, m), 7.24(2H, d, J=9.8Hz)

MASS m/z: 389.99 ($MH^+$)

(3) 2-methyl-5-phenyl-1-[4-(trifluoromethoxy)phenyl]-1H-pyrrole-3-carboxylic acid

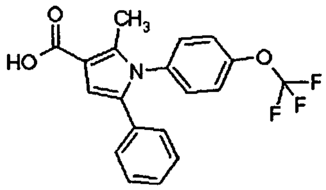

To a solution of ethyl 2-methyl-5-phenyl-1-[4-(trifluoromethoxy)phenyl]-1H-pyrrole-3-carboxylate obtained in Example 62-(2) (4.71 g, 0.0121 mol) in ethanol (60 mL) was added 2N aqueous sodium hydroxide solution (12 ml, 0.0242 mol), and the mixture was heated under reflux for 12 hr. The reaction mixture was acidified with 6N aqueous hydrochloric acid solution, and extracted with ethyl acetate. The organic layer was washed with water and saturated brine, and dried over anhydrous magnesium sulfate. The extract was concentrated under reduced pressure, and the residue was recrystallized from ethyl acetate-hexane to give 2-methyl-5-phenyl-1-[4-(trifluoromethoxy)phenyl]-1H-pyrrole-3-carboxylic acid (3.87 g) as crystals.

$^1$H-NMR ($CDCl_3$) δ: 2.32(3H, s), 6.68 (1H, s), 7.00-7.07 (2H, m), 7.13-7 .23 (3H, m) 7.39-7.49(4H, m), 12 .02 (1H, s)

MASS m/z: 361.95 ($MH^+$)

(4) N-{2-[(4-ethoxybenzoyl)amino]ethyl}-2-methyl-5-phenyl-1-[4-(trifluoromethoxy)phenyl]-1H-pyrrole-3-carboxamide

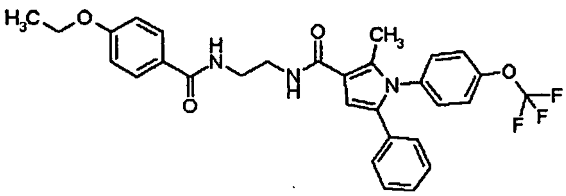

Using 2-methyl-5-phenyl-1-[4-(trifluoromethoxy)phenyl]-1H-pyrrole-3-carboxylic acid obtained in Example 62-(3) (181 mg, 0.5 mmol) as a starting material and in the same manner as in Example 61-(4), N-{2-[(4-ethoxybenzoyl) amino]ethyl}-2-methyl-5-phenyl-1-[4-(trifluoromethoxy)phenyl]-1H-pyrrole-3-carboxamide (253 mg) was obtained.

$^1$H-NMR ($CDCl_3$) δ: 1.33 (3H, t, J=7.0Hz), 2 .33 (3H, s), 3.40 (4H, s), 4 .07 (2H, q, J=7.0Hz), 6. 85 (1H, s), 6.94-7.04 (4H, m), 7.12-7.24(3H, m), 7.35-7.41(2H, m), 7.42-7.48(2H, m), 7.83(2H, d, J=8.9Hz), 8.04 (1H, s), 8.45(1H, s)

MASS m/z: 552.07 ($MH^+$)

Example 63

1-benzyl-N-{2-[(4-ethoxybenzoyl)amino]ethyl}-2-methyl-5-phenyl-1H-pyrrole-3-carboxamide

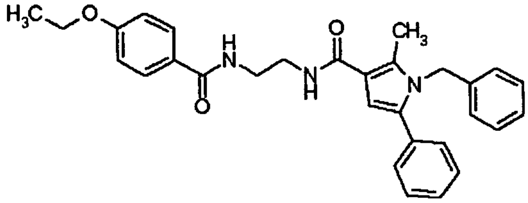

(1) 1-benzyl-2-methyl-5-phenyl-1H-pyrrole-3-carboxylic acid

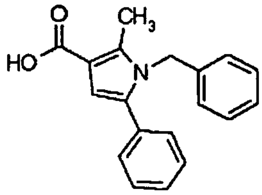

Using benzylamine (1.69 ml, 0.0155 mol) as a starting material and in the same manners as in Example 62-(2) and (3), 1-benzyl-2-methyl-5-phenyl-1H-pyrrole-3-carboxylic acid (3.33 g) was obtained. MASS m/z: 292.0 ($MH^+$)
(2) 1-benzyl-N-{2-[(4-ethoxybenzoyl)amino]ethyl}-2-methyl-5-phenyl-1H-pyrrole-3-carboxamide

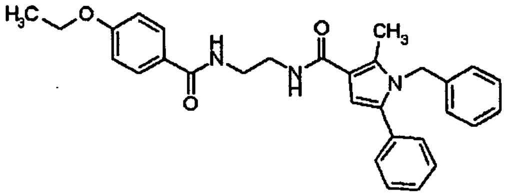

Using 1-benzyl-2-methyl-5-phenyl-1H-pyrrole-3-carboxylic acid obtained in Example 63-(1) (116 mg, 0.4 mmol) as a starting material and in the same manner as in Example 61-(4), 1-benzyl-N-{2-[(4-ethoxybenzoyl)amino]ethyl}-2-methyl-5-phenyl-1H-pyrrole-3-carboxamide (172 mg) was obtained.
$^1$H-NMR ($CDCl_3$) δ: 1.41 (3H, t, J=7.0Hz), 2.47 (3H, s), 3.59-3.74(4H, m), 4.04 (2H, q, J=7.0Hz), 5.11 (2H, s), 6.39 (1H, s), 6.53 (1H, s), 6.83-6.98(4H, m), 7.21-7.36(8H, m), 7.56(1H, s), 7.81(2H, d,
J=8.9Hz)
MASS m/z: 482.02 ($MH^+$)

Example 64

N-{2-[(4-ethoxybenzoyl)amino]ethyl}-1-(4-methoxyphenyl)-2-methyl-5-phenyl-1H-pyrrole-3-carboxamide

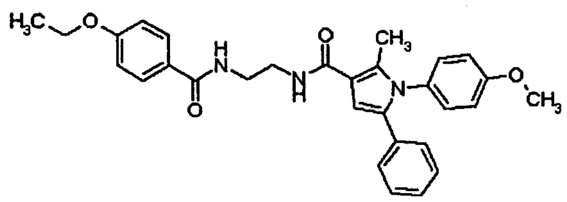

(1) 1-(4-methoxyphenyl)-2-methyl-5-phenyl-1H-pyrrole-3-carboxylic acid

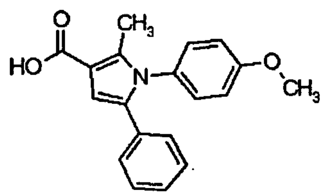

Using 4-methoxyaniline (1.09 g, 8.86 mmol) as a starting material and in the same manners as in Example 62-(2) and (3), 1-(4-methoxyphenyl)-2-methyl-5-phenyl-1H-pyrrole-3-carboxylic acid (2.05 g) was obtained. MASS m/z: 307.97 ($MH^+$)

(2) N-{2-[(4-ethoxybenzoyl)amino]ethyl}-1-(4-methoxyphenyl)-2-methyl-5-phenyl-1H-pyrrole-3-carboxamide

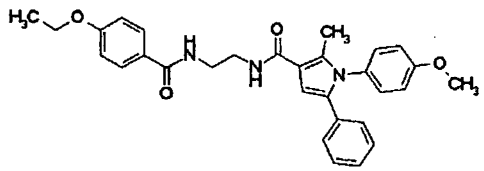

Using 1-(4-methoxyphenyl)-2-methyl-5-phenyl-1H-pyrrole-3-carboxylic acid obtained in Example 64-(1) (154 mg, 0.5 mmol) as a starting material and in the same manner as in Example 61-(4), N-{2-[(4-ethoxybenzoyl)amino]ethyl}-1-(4-methoxyphenyl)-2-methyl-5-phenyl-1H-pyrrole-3-carboxamide (205 mg) was obtained.
$^1$H-NMR($CDCl_3$)δ: 1.41(3H, t, J=7.0Hz), 2.39(3H, s), 3.62-3.73(4H, m), 3.82(3H, s), 4.04 (2H, q, J=7.0Hz), 6.51(1H, s), 6.62(1H, t, J=4.9Hz), 6.87(4H, dd, J=8.9, 1.7Hz), 6.99-7 .07 (4H, m), 7.11-7.19(3H, m), 7.57(1H, s), 7.82(2H, d, J=8.9 Hz)
MASS m/z: 498.05 ($MH^+$)

Example 65

N-{2-[(4-ethoxybenzoyl)amino]ethyl}-2-methyl-1-[4-(methylthio)phenyl]-5-phenyl-1H-pyrrole-3-carboxamide

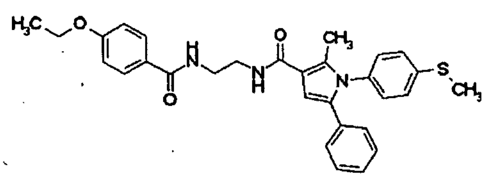

Using 2-methyl-1-[4-(methylthio)phenyl]-5-phenyl-1H-pyrrole-3-carboxylic acid as a starting material and in the same manner as in Example 61-(4), N-{2-[(4-ethoxybenzoyl)amino]ethyl}-2-methyl-1-[4-(methylthio)phenyl]-5-phenyl-1H-pyrrole-3-carboxamide (39 mg) was synthesized.
MASS m/z: 514.32 ($MH^+$)

Example 66

4-(4-ethoxyphenyl)-N-(2,4-diphenyl-1,3-thiazol-5-yl)-4-oxobutanamide

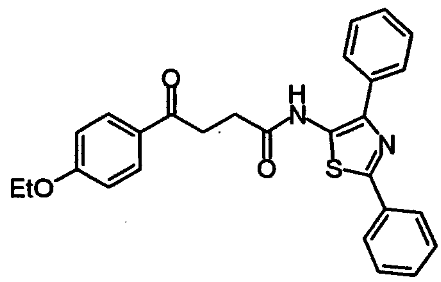

To a suspension of 4-(4-ethoxyphenyl)-4-oxobutanoic acid obtained in Example 1-(1) (306 mg) and 2,4,6-trichlorobenzoyl chloride (0.17 mL) in THF (5 mL) was added triethylamine (0.28 mL), and the mixture was stirred at room temperature for 4 hr. 2,4-Diphenyl-1,3-thiazol-5-amine obtained in Example 16-(1) (240 mg) was added to the reaction mixture, and the mixture was heated at 50°C for 2 days. Diluted hydrochloric acid was added to the reaction mixture, and the mixture was stirred for 1 hr, and extracted with ethyl acetate. The organic layer was washed with water, dried (over magnesium sulfate), and concentrated under reduced pressure to give the title compound as crystals (63 mg).
$^1$H-NMR ($CDCl_3$) δ: 1.45(3H, t, J = 6.9 Hz), 2.85(2H, t, J = 6.6 Hz), 3.40(2H, t, J = 6.6 Hz), 4.12(2H, q, J = 6.9 Hz), 6.93 (2H, d, J = 8.7 Hz), 7.37-7.52(4H, m), 7.55(2H, m), 7.75(2H, d, J = 8.1 Hz), 7.94-7.97(4H, m), 8.71 (1H, brs).
mp. 169-170°C

Example 67

N-(5-benzyl-4-phenyl-1,3-thiazol-2-yl)-4-(2,3-dihydro-1,4-benzodioxin-6-yl)-4-oxobutanamide

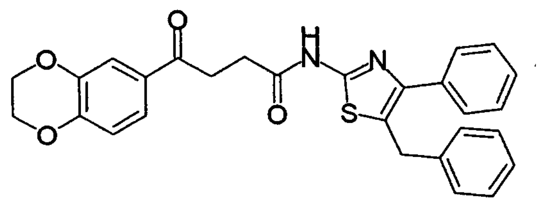

A suspension of 5-benzyl-4-phenyl-1,3-thiazol-2-amine obtained in Example 1-(2) (26.6 mg), 4-(2,3-dihydro-1,4-benzodioxin-6-yl)-4-oxobutanoic acid (23.6 mg), WSC (0.0351 mL) and HOBt (27.0 mg) in dichloromethane (2.3 mL) was stirred overnight at room temperature. 4-(2,3-Dihydro-1,4-benzodioxin-6-yl)-4-oxobutanoic acid (18.9 mg) was added, and the mixture was stirred overnight at 35°C, and extracted with dichloromethane. The organic layer was wash with water, 1N hydrochloric acid and saturated aqueous sodium chloride solution, dried (over sodium sulfate), and concentrated under reduced pressure. The residue was dissolved in dimethylsulfoxide, and the mixture was purified by preparative HPLC to give the title compound (31.4 mg).
LC-MS: 485 ($MH^+$)

Example 68

N-(5-benzyl-4-phenyl-1,3-thiazol-2-yl)-4-(3,4-dihydro-2H-1,5-benzodioxepin-7-yl)-4-oxobutanamide

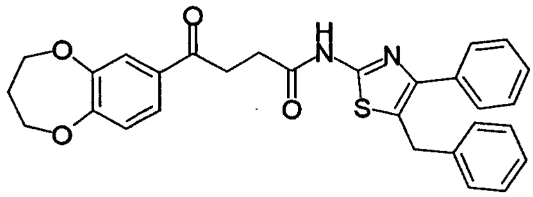

A suspension of 5-benzyl-4-phenyl-1,3-thiazol-2-amine obtained in Example 1-(2) (26.6 mg), 4-(3,4-dihydro-2H-1,5-benzodioxepin-7-yl)-4-oxobutanoic acid (45.0 mg), WSC (0.0351 mL) and HOBt (27.0 mg) in acetonitrile (1.0 mL) was stirred overnight at 70°C. The mixture was extracted with dichloromethane, and the organic layer was wash with saturated aqueous sodium hydrogencarbonate, 1N hydrochloric acid, and saturated aqueous sodium chloride solution, and concentrated under reduced pressure. The residue was dissolved in dimethylsulfoxide, and the mixture was purified by preparative HPLC to give the title compound (29.8 mg).
$^1$H-NMR ($CDCl_3$) δ: 2.23 (2H, quint, J = 5.8 Hz), 2.36-2.50 (2H, br), 3.04-3.16 (2H, br), 4.18 (2H, s), 4.25 (2H, t, J = 5.8 Hz), 4.31 (2H, t, J = 5.8 Hz), 6.97 (1H, d, J = 8.3 Hz), 7.15-7.35 (6H, m), 7.41 (2H, t, J = 7.4 Hz), 7.48-7.60 (4H, m).

Example 69

N-[5-(4-butylbenzoyl)-4-phenyl-1,3-thiazol-2-yl]-4-(4,5-diethoxy-2-methylphenyl)-4-oxobutanamide

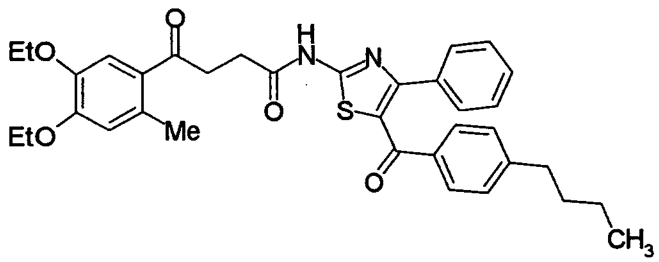

(1) (2-amino-4-phenyl-1,3-thiazol-5-yl)(4-butylphenyl)methanone

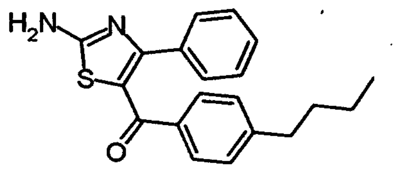

To a solution of benzamidine hydrochloride (15.0 g) in DMF (150 mL) were added successively ethoxycarbonylisothiocyanate (11.9 mL) and diisopropylethylamine (17.5 mL), and the mixture was stirred at room temperature for 1 hr. 2-Bromo-1-(4-butylphenyl)ethanone (25.7 g) and diisopropylethylamine (35 mL) were added successively, and the mixture was stirred at room temperature for 1 hr. Water was added to the reaction mixture, and the mixture was extracted with ethyl acetate. The obtained organic layer was washed with water, dried (over magnesium sulfate), and concentrated under reduced pressure. The obtained residue was purified by silica gel column chromatography (developing solvent: hexane-ethyl acetate (3:1)), acetic acid (150 mL) and 12N hydrochloric acid (100 mL) were added to the obtained resultant product, and the mixture was heated under reflux for 2 days. The reaction mixture was concentrated, and water was added to the obtained residue. The mixture was alkalified with aqueous saturated $NaHCO_3$ solution, and extracted with ethyl acetate. The obtained organic layer was washed successively with saturated aqueous $NaHCO_3$ solution and water, and dried (over magnesium sulfate), and the solvent was evaporated under reduced pressure to give (2-amino-4-phenyl-1,3-thiazol-5-yl)(4-butylphenyl)methanone (1.9 g) as crystals.
$^1$H-NMR ($CDCl_3$) δ: 0.90 (3H, t, J=7.2Hz), 1.23 (2H, m), 1.47(2H, m), 2.49(2H, t, J=7.5Hz), 5.54(2H, br s), 6.88(2H, d, J=8.1Hz), 7.06(3H, m), 7.26(2H, m), 7.38(2H, d, J=7.8Hz).

(2) N-[5-(4-butylbenzoyl)-4-phenyl-1,3-thiazol-2-yl]-4-(4,5-diethoxy-2-methylphenyl)-4-oxobutanamide

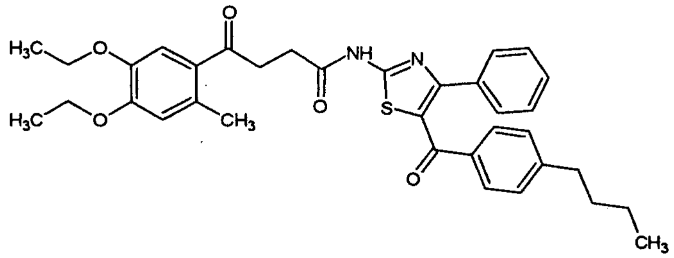

A suspension of (2-amino-4-phenyl-1,3-thiazol-5-yl)(4-butylphenyl)methanone obtained in Example 69-(1) (33.6 mg), 4-(4,5-diethoxy-2-methylphenyl)-4-oxobutanoic acid obtained in Example 4-(1) (50.5 mg), WSC (0.0351 mL) and HOBt (27.0 mg) in acetonitrile (0.50 mL) was stirred at 50°C for 5 hr. The mixture was extracted with dichloromethane, and the organic layer was wash with water, 1N hydrochloric acid, saturated aqueous sodium hydrogencarbonate and saturated aqueous sodium chloride solution, and concentrated under reduced pressure. The residue was dissolved in dimethylsulfoxide, and the mixture was purified by preparative HPLC to give the title compound (32.4 mg).
$^1$H-NMR ($CDCl_3$) δ: 0.90 (3H, t, J = 7.3 Hz), 1.13-1.35 (2H, m), 1.38-1.56 (8H, m), 2.49 (3H, s), 2.43-2.59 (2H, t, J = 7.6 Hz), 3.02 (2H, t, J = 6.0 Hz), 3.41 (2H, t, J = 6.0 Hz), 4.13 (2H, q, J = 7.0 Hz), 4.15 (2H, q, J = 7.0 Hz), 6.71 (1H, s), 6.99 (2H, d, J = 8.4 Hz), 7.17-7.35 (5H, m), 7.37 (1H, s), 7.47 (2H, d, J = 8.4 Hz).

Example 70

N-(5-benzyl-4-phenyl-2-thienyl)-4-(3-chloro-4-ethoxyphenyl)-4-oxobutanamide

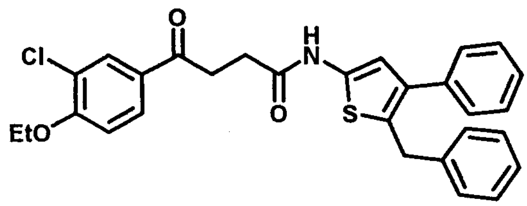

To a suspension of 5-benzyl-4-phenylthiophen-2-amine obtained in Example 17-(2) (265 mg), 4-(3-chloro-4-ethoxyphenyl)-4-oxobutanoic acid obtained in Example 36-(1) (460 mg) and HOBt (306 mg) in acetonitrile (10 mL) was added WSC (0.35 mL), and the mixture was stirred at 50°C for 2 hr. Water was added to the reaction mixture, and the mixture was extracted with ethyl acetate. The organic layer was washed successively with saturated aqueous $NaHCO_3$ solution and water, and dried (over magnesium sulfate), and the solvent was evaporated under reduced pressure to give N-(5-benzyl-4-phenyl-2-thienyl)-4-(3-chloro-4-ethoxyphenyl)-4-oxobutanamide (192 mg) as crystals.
$^1$H-NMR ($CDCl_3$) δ: 1.50(3H, t, J=6.6Hz), 2.79(2H, t, J=6.0Hz), 3.73(2H, t, J=6.0Hz), 4.10(2H, s), 4.17(2H, q, J=6.6Hz), 6.63(1H, s), 6.92(1H, d, J=8.7Hz), 7.15-7.39(10H, m), 7.85(1H, dd, J=8.7Hz, J=2.1Hz), 7.86(1H, d, J=2.1Hz), 8.40(1H, br s).

Example 71

N-(5-benzyl-4-phenyl-1,3-thiazol-2-yl)-4-(3,4-dimethoxyphenyl)-4-oxobutanamide

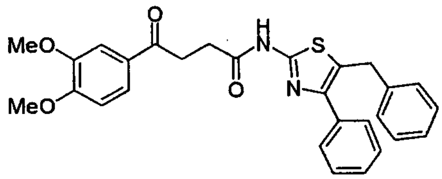

(1) 4-(3,4-dimethoxyphenyl)-4-oxobutanoic acid

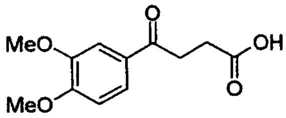

To a solution of 1,2-dimethoxybenzene (7.48 g) and succinic anhydride (6.5 g) in dichloromethane (80 mL) was slowly added aluminum chloride (18.05 g) under ice-cooling. The mixture was stirred for 30 min under ice-cooling, and poured into ice, and concentrated hydrochloric acid (20 mL) was added. The reaction mixture was extracted with ethyl acetate, and the organic layer was washed with saturated brine, dried over magnesium sulfate, and concentrated under reduced pressure. The residue was collected by filtration, and washed with isopropyl ether to give 4-(3,4-dimethoxyphenyl)-4-oxobutanoic acid (4.47 g).
LCMS 239.1(MH+)
(2) N-(5-benzyl-4-phenyl-1,3-thiazol-2-yl)-4-(3,4-dimethoxyphenyl)-4-oxobutanamide

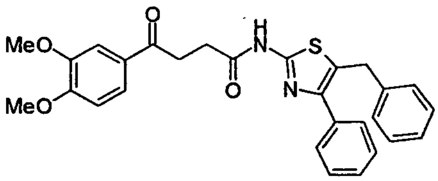

A solution of 4-(3,4-dimethoxyphenyl)-4-oxobutanoic acid obtained in Example 71-(1) (0.358 g), WSC (0.237 mL), HOBt (0.207 g) and 5-benzyl-4-phenyl-1,3-thiazol-2-amine obtained in Example 1-(2) (0.20 g) in acetonitrile (5 mL) was stirred at 50°C for 15 hr. The reaction mixture was extracted with ethyl acetate, and the organic layer was washed with saturated aqueous sodium hydrogencarbonate solution and saturated brine, dried over magnesium sulfate, and crudely purified by filtration through silica gel. The filtrate was concentrated under reduced pressure. The residue was recrystallized from ethyl acetate to give N-(5-benzyl-4-phenyl-1,3-thiazol-2-yl)-4-(3,4-dimethoxyphenyl)-4-oxobutanamide (28 mg) as crystals.
$^1$H-NMR(300 MHz, DMSO-$d_6$) δppm 2.76 (2 H, t, J=6.3 Hz) 3.30 - 3.35 (2 H, m) 3.81 (3 H, s) 3.85 (3 H, s) 4.22 (2 H, s) 7.07 (1 H, d, J=8.5 Hz) 7.18- 7.25 (3 H, m) 7.28 - 7.39 (3 H, m) 7.42 - 7.48 (3 H, m) 7.61 - 7.69 (3 H, m) 12.23 (1 H, s)

Example 72

N-(5-benzyl-4-phenyl-1,3-thiazol-2-yl)-4-(5-ethyl-2-thienyl)-4-oxobutanamide

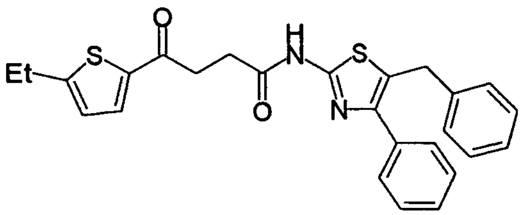

4-(5-Ethyl-2-thienyl)-4-oxobutanoic acid (212 mg), 5-benzyl-4-phenyl-1,3-thiazol-2-amine obtained in Example 1-(2) (266 mg), WSC (155 mg) and HOBt (135 mg) were dissolved in acetonitrile (10 mL), and the mixture was stirred overnight at 50°C. The reaction mixture was ice-cooled, and the precipitated solid was collected by filtration to give the title compound (86 mg).
$^1$H-NMR(300 MHz, DMSO-$d_6$) δppm 1.25 (3 H, t, J=7.5 Hz) 2.76 (2 H, t, J=6.4 Hz) 2.86 (2 H, q, J=7.5 Hz) 3.25 (2 H, t, J=6.4 Hz) 4.22 (2 H, s) 7.00 (1 H, d, J=3.8 Hz) 7.16 - 7.50 (8 H, m) 7.59 - 7.66 (2 H, m) 7.83 (1 H, d, J=3.8 Hz) 12.23 (1 H, s)

Example 73

N-(6-chloro-4-phenylquinolin-2-yl)-4-(4,5-diethoxy-2-methylphenyl)-4-oxobutanamide

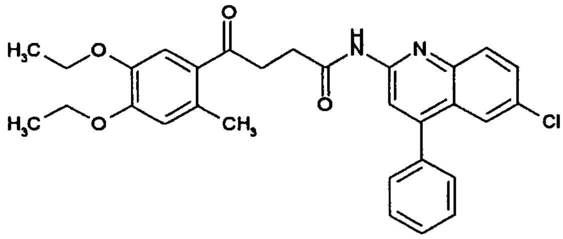

4-(4,5-Diethoxy-2-methylphenyl)-4-oxobutanoic acid obtained in Example 4-(1) (140 mg), 6-chloro-4-phenylquinolin-2-amine synthesized according to J. Synth. Org. Chem. Jpn, 1973, 31, 328 (127 mg), WSC (0.177 mL) and HOBt (135 mg) were dissolved in acetonitrile (5 mL), and the mixture was stirred overnight at 50°C. The solvent was evaporated under reduced pressure, and the residue was purified by silica gel column chromatography to give the title compound (45 mg).
$^1$H-NMR (300 MHz, CDCl3) δppm 1.46 (6 H, q, J=7.1 Hz) 2.51 (3 H, s) 2.88 (2 H, t, J=6.4 Hz) 3.36 (2 H, t, J=6.4 Hz) 4.06 - 4.18 (4 H, m) 6.70 (1 H, s) 7.38 (1 H, s) 7.44 - 7.55 (5 H, m) 7.60 (1 H, dd, J=8.9, 2.4 Hz) 7.79 - 7.85 (2 H, m) 8.39 (1 H, s) 8.42 (1 H, s)

Example 74

4-(3,4-diethoxyphenyl)-4-oxo-N-(4-phenylquinolin-2-yl)butanamide

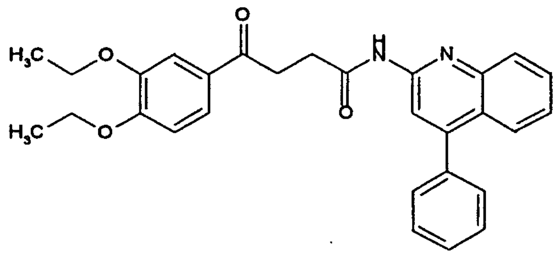

Potassium 4-(3,4-diethoxyphenyl)-4,4-dimethoxybutanoate obtained in Example 8-(7) (350 mg), 4-phenylquinolin-2-amine synthesized according to J. Synth. Org. Chem. Jpn, 1973, 31, 328 (220 mg), WSC (0.177 mL) and HOBt (153 mg) were dissolved in DMF (2 mL), and the mixture was stirred overnight at 50°C. The solvent was evaporated under reduced pressure, and the residue was purified by column chromatography, and the obtained crystals were recrystallized from acetonitrile-water to give the title compound (30 mg).
$^1$H-NMR (300 MHz, DMSO-$d_6$) 5ppm 1.27 - 1.41 (6 H, m) 2.84 (2 H, t, J=6.2 Hz) 3.31 (2 H, t, J=6.2 Hz) 4.01 - 4.17 (4 H, m) 7.06 (1 H, d, J=8.5 Hz) 7.42 - 7.69 (8 H, m) 7.70 - 7.79 (2 H, m) 7.86 - 7.95 (1 H, m) 8.25 (1 H, s) 11.02 (1 H, s)

Example 75

N-(6-chloro-4-phenylquinolin-2-yl)-4-oxo-4-(5,6,7,8-tetrahydronaphthalen-2-yl)butanamide

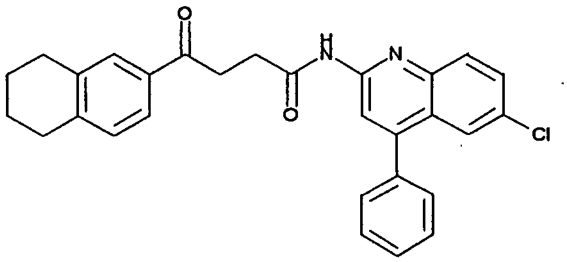

Using 4-oxo-4-(5,6,7,8-tetrahydronaphthalen-2-yl)butanoic acid obtained in Example 7-(1) (465 mg) and 6-chloro-4-phenylquinolin-2-amine synthesized according to J. Org. Chem. 1980, 45, 4767 (255 mg) and in the same manner as in Example 72, the title compound (124 mg) was obtained. $^1$H-NMR(300 MHz, DMSO-$d_6$) δppm 1.70 - 1.80 (4 H, m) 2.77 (4 H, br) 2.85 (2 H, t, J=6.3 Hz) 3.30 (2 H, t, J=6.3 Hz) 7.19 (1 H, d, J=8.5 Hz) 7.49 - 7.71 (8 H, m) 7.77 (1 H, dd, J=9.0, 2.3 Hz) 7.92 (1 H, d, J=9.0 Hz) 8.28 (1 H, s) 11.10 (1 H, s)

Example 76

N-[6-bromo-4-(4-methylphenyl)quinolin-2-yl]-4-(3,4-diethoxyphenyl)-4-oxobutanamide

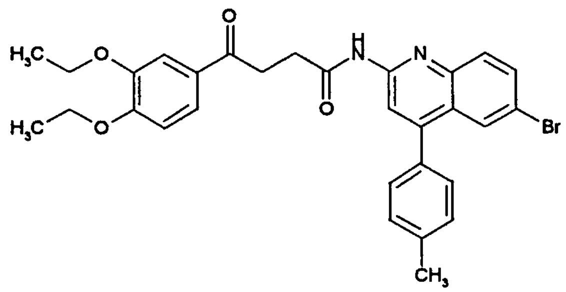

(1) (2-Amino-5-bromophenyl)(4-methylphenyl)methanone synthesized according to US 3239564 (2.9 g) and acetonitrile (3.1 mL) were dissolved in pyridine (100 mL), and 60% sodium hydride (0.6 g) was added. The reaction mixture was refluxed overnight, and cooled. Water was added, and the mixture was extracted with ethyl acetate. The organic layer was washed with saturated brine, and dried over anhydrous magnesium sulfate, and the solvent was evaporated under reduced pressure. The obtained solid was washed with diisopropyl ether to give 6-bromo-4-(4-methylphenyl)quinolin-2-amine (3.6 g).
$^1$H-NMR(300 MHz, DMSO-$d_6$) δppm 2.41 (3 H, s) 6.67 (2 H, br) 6.70 (1 H, s) 7.32 - 7.63 (7 H, m)
(2) Potassium 4-(3,4-diethoxyphenyl)-4,4-dimethoxybutanoate obtained in Example 8-(7) (2.1 g), 2,4,6-trichlorobenzoyl chloride (1.0 mL) and triethylamine (0.92 mL) were dissolved in THF (90 mL), and the mixture was stirred for 4 hr. 6-Bromo-4-(4-methylphenyl)quinolin-2-amine obtained in Example 76-(1) (1.9 g) was added to the reaction mixture, and the mixture was stirred overnight at 50°C. 1N Hydrochloric acid was added to the reaction mixture, and the mixture was extracted with ethyl acetate. The organic layer was washed with saturated brine, and dried over anhydrous magnesium sulfate, and the solvent was evaporated under reduced pressure. The residue was recrystallized from DMSO-acetonitrile to give the title compound (2.2 g).
$^1$H-NMR (300 MHz, DMSO-$d_6$) δppm 1.34 (6 H, q, J=6.8 Hz) 2.42 (3 H, s) 2.84 (2 H, t, J=6.2 Hz) 3.30 (2 H, t, J=6.2 Hz) 4.02 - 4.16 (4 H, m) 7.06 (1 H, d, J=8.4 Hz) 7.39 - 7.46 (5 H, m) 7.65 (1 H, dd, J=8.4 2.0) 7.80 - 7.89 (3 H, m) 8.26 (1 H, s) 11.07 (1 H, s)

Example 77

ethyl (2E)-3-[2-{[4-(3,4-diethoxyphenyl)-4-oxobutanoyl]amino}-4-(4-methylphenyl)quinoline-6-yl]acrylate

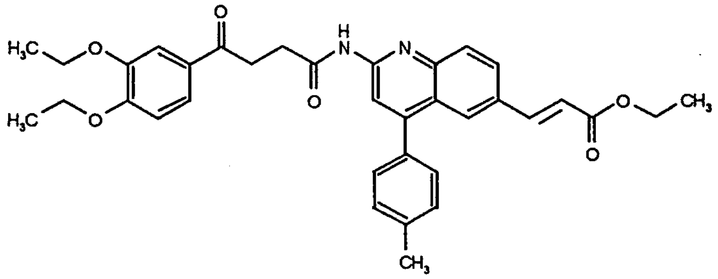

N-[6-Bromo-4-(4-methylphenyl)quinolin-2-yl]-4-(3,4-diethoxyphenyl)-4-oxobutanamide obtained in Example 76 (560 mg), ethyl vinylacetate (0.22 mL), palladium acetate (11 mg), tributylphosphine (0.025 mL) and triethylamine (0.28 mL) were dissolved in DMF (5.6 mL), and the mixture was stirred overnight at 100°C. 1N Hydrochloric acid was added to the reaction mixture, and the mixture was extracted with ethyl acetate. The organic layer was washed with saturated brine, and dried over anhydrous magnesium sulfate, and the solvent was evaporated under reduced pressure. The residue was purified by column chromatography to give the title compound (350 mg).
$^1$H-NMR(300 MHz, DMSO-$d_6$) δppm 1.25 (3 H, t, J=7.2 Hz) 1.34 (6 H, q, J=6.8 Hz) 2.43 (3 H, s) 2.84 (2 H, t, J=5.9 Hz) 3.31 (2 H, t, J=5.9 Hz) 4.02 - 4.24 (6 H, m) 6.64 (1 H, d, J=16.0 Hz) 7.06 (1 H, d, J=8.5 Hz) 7.37 - 7.49 (5 H, m) 7.65 (1 H, dd, J=8.5, 1.9 Hz) 7.73 (1 H, d, J=16.0 Hz) 7.87 (1 H, d, J=8.8 Hz) 7.96 (1 H, d, J=1.3 Hz) 8.16 (1 H, dd, J=8.8, 1.7 Hz) 8.26 (1 H, s) 11.09 (1 H, s)

Example 78

tert-butyl (2E)-3-[2-{[4-(3,4-diethoxyphenyl)-4-oxobutanoyl]amino}-4-(4-methylphenyl)quinoline-6-yl]acrylate

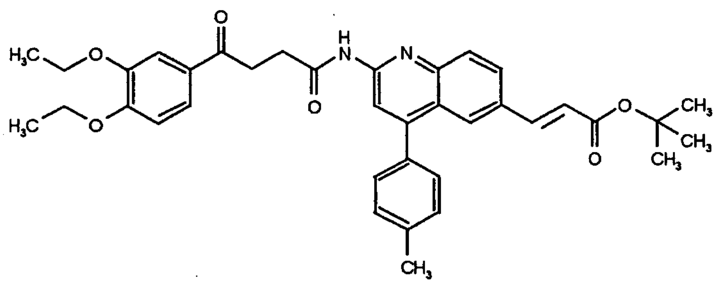

N-[6-Bromo-4-(4-methylphenyl)quinolin-2-yl]-4-(3,4-diethoxyphenyl)-4-oxobutanamide obtained in Example 76 (560 mg), t-butyl vinylacetate (0.29 mL), palladium acetate (22 mg), triphenylphosphine (54 mg) and triethylamine (0.28 mL) were dissolved in DMF (10 mL), and the mixture was stirred overnight at 100°C. 1N Hydrochloric acid was added to the reaction mixture, and the mixture was extracted with ethyl acetate. The organic layer was washed with saturated brine, and dried over anhydrous magnesium sulfate, and the solvent was evaporated under reduced pressure. The residue was recrystallized from acetate-diethyl ether to give the title compound (250 mg).
$^1$H-NMR(300 MHz, DMSO-$d_6$) δppm 1.28 - 1.40 (6 H, m) 1.48 (9 H, s) 2.43 (3 H, s) 2.84 (2 H, t, J=6.2 Hz) 3.30 (2 H, t, J=6.2 Hz) 4.01 - 4.17 (4 H, m) 6.52 (1 H, d, J=15.8 Hz) 7.06 (1 H, d, J=8.5 Hz) 7.36 - 7.48 (5 H, m) 7.57 - 7.69 (2 H, m) 7.86 (1 H, d, J=8.9 Hz) 7 . 92 (1 H, d, J=1.5 Hz) 8.13 (1 H, dd, J=8.9, 1.8 Hz) 8.25 (1 H, s) 11.09 (1 H, s)

Example 79

4-(4-ethoxyphenyl)-4-oxo-N-(4-phenylquinolin-2-yl)butanamide

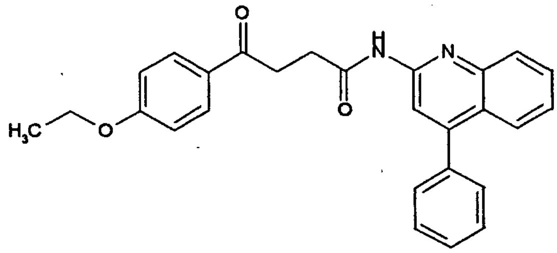

4-(4-Ethoxyphenyl)-4-oxobutanoic acid obtained in Example 1-(1) (220 mg), 4-phenylquinolin-2-amine synthesized according to J. Synth. Org. Chem. Jpn, 1973, 31, 328 (110 mg), WSC (0.177 mL) and HOBt (153 mg) were dissolved in a mixed solvent of DMF (5 mL) and acetonitrile (10 mL), and the mixture was stirred overnight at 50°C. The reaction mixture was ice-cooled, and the precipitated solid was collected by filtration to give the title compound (75 mg).
$^1$H-NMR (300 MHz, DMSO-$d_6$) δppm 1.35 (3 H, t, J=6.97 Hz) 2.85 (2 H, t, J=6.2 Hz) 3.29 (2 H, t, J=6.2 Hz) 4.12 (2 H, q, J=6.9 Hz) 7.03 (2 H, d, J=8.8 Hz) 7.43 - 7.62 (6 H, m) 7.71 - 7.78 (2 H, m) 7.90 (1 H, dd, J=8.8, 1.1 Hz) 7.96 (2 H, d, J=8.8 Hz) 8.24 (1 H, d) 11.02 (1 H, s)

Example 80

4-(3,4-diethoxyphenyl)-N-[4-(3-methoxyphenyl)quinolin-2-yl]-4-oxobutanamide

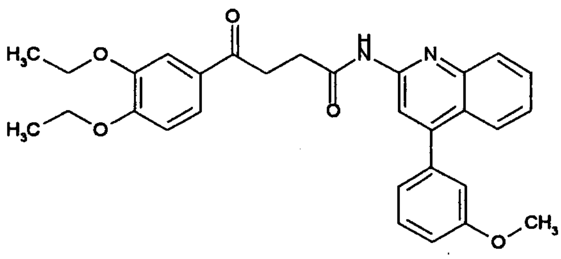

(1) (2-Aminophenyl)(3-methoxyphenyl)methanone (1.0 g) synthesized according to J. Chem. Soc. Perkin Trans. 1972, 1, 2524 (0.49 g) and acetonitrile was dissolved in pyridine (20 mL), and 60% sodium hydride (0.24 g) was added. The reaction mixture was refluxed overnight, and cooled. Water was added, and the mixture was extracted with ethyl acetate. The organic layer was washed with saturated brine, and dried over anhydrous magnesium sulfate, and the solvent was evaporated under reduced pressure. The residue was purified by column chromatography to give 4-(3-methoxyphenyl)quinolin-2-amine (0.90 g).
$^1$H-NMR (300 MHz, $CDCl_3$) δppm 3.86 (3 H, s) 4.81 (2 H, s) 6.67 (1 H, br) 6.97 - 7.09 (3 H, m) 7.18 - 7.24 (1 H, m) 7.37 - 7.44 (1 H, m) 7.52 - 7.60 (1 H, m) 7.67 - 7.75 (2 H, m)
(2) 4-(3,4-Diethoxyphenyl)-4-oxobutanoic acid obtained in Example 3-(1) (266 mg), 4-(3-methoxyphenyl)quinolin-2-amine obtained in Example 80-(1) (250 mg), WSC (155 mg) and HOBt (135 mg) were dissolved in acetonitrile (10 mL), and the mixture was stirred overnight at 50°C. The reaction mixture was ice-cooled, and the precipitated solid was collected by filtration to give the title compound (121 mg).
$^1$H-NMR(300 MHz, DMSO-$d_6$) δppm 1.34 (6 H, q, J=6.9 Hz) 2.84 (2 H, t, J=6.2 Hz) 3.30 (2 H, t, J=6.2 Hz) 3.81 (3 H, s) 4.02 - 4.17 (4 H, m) 7.01 - 7.14 (4 H, m) 7.42 - 7.53 (3 H, m) 7.65 (1 H, dd, J=8.4, 1.8 Hz) 7.70 - 7.80 (2 H, m) 7.85 - 7.93 (1 H, m) 8.24 (1 H, s) 11.01 (1 H, s)

Example 81

N-(7-bromo-4-phenylquinolin-2-yl)-4-(3,4-diethoxyphenyl)-4-oxobutanamide

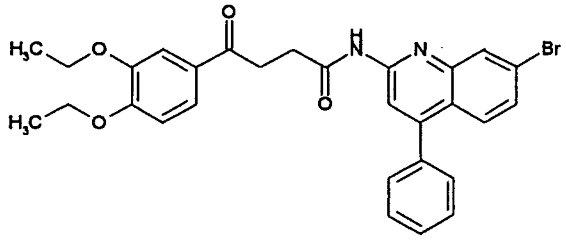

(1) (2-Amino-4-bromophenyl)(phenyl)methanone (5.0 g) and acetonitrile (2.8 mL) were dissolved in pyridine (100 mL), and 60% sodium hydride (1.1 g) was added. The reaction mixture was refluxed overnight, and cooled. Water was added, and the mixture was extracted with ethyl acetate. The organic layer was washed with saturated brine, and dried over anhydrous magnesium sulfate, and the solvent was evaporated under reduced pressure. The obtained crystals were washed with diisopropyl ether to give 7-bromo-4-phenylquinolin-2-amine (3.3 g).
$^1$H-NMR(300 MHz, DMSO-$d_6$) δppm 6.52 (2 H, s) 6.67 (1 H, s) 7.07 - 7.29 (2 H, m) 7.39 - 7.58 (4 H, m) 7.70 - 7.79 (2 H, m)
(2) 4-(3,4-Diethoxyphenyl)-4-oxobutanoic acid obtained in Example 3-(1) (2.1 g), 7-bromo-4-phenylquinolin-2-amine obtained in Example 81-(1) (1.2 g), WSC (1.2 g) and HOBt (1.2 g) were dissolved in a mixed solvent of acetonitrile (10 mL) and DMF (5 mL), and the mixture was stirred overnight at 50°C. The reaction mixture was ice-cooled, and the precipitated solid was collected by filtration to give the title compound (1.5 g).
$^1$H-NMR(300 MHz, DMSO-$d_6$) δppm 1.34 (6 H, q, J=6.9 Hz) 2.84 (2 H, t, J=6.2 Hz) 3.30 (2 H, t, J=6.2 Hz) 4.02 - 4.17 (4 H, m) 7.06 (1H, d, J=8.4 Hz) 7.44 - 7.51 (4 H, m) 7.65 (1 H, dd, J=8.4, 1.8 Hz) 7.69 - 7.80 (4 H, m) 7.87 - 7.94 (1 H, m) 8.24 (1 H, s) 11.03 (1 H, s)

Example 82

tert-butyl (2E)-3-(2-{[4-(3,4-diethoxyphenyl)-4-oxobutanoyl]amino}-4-phenylquinoline-7-yl)acrylate

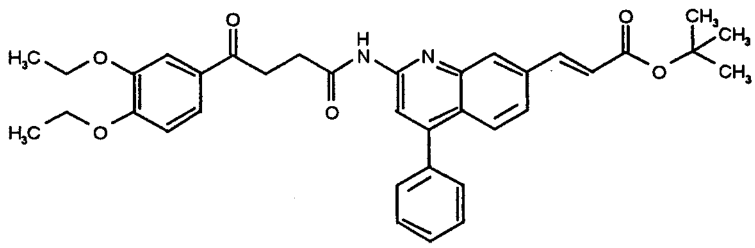

N-(7-Bromo-4-phenylquinolin-2-yl)-4-(3,4-diethoxyphenyl)-4-oxobutanamide obtained in Example 81-(2) (550 mg), t-butyl vinylacetate (0.26 mL), palladium acetate (22 mg), triphenylphosphine (52 mg) and triethylamine (0.28 mL) were dissolved in DMF (10 mL), and the mixture was stirred at 100°C for 2 days. Water was added to the reaction mixture, and the mixture was extracted with ethyl acetate. The organic layer was washed with saturated brine, and dried over anhydrous magnesium sulfate, and the solvent was evaporated under reduced pressure. The residue was purified by column chromatography to give the title compound (290 mg).
$^1$H-NMR(300 MHz, DMSO-$d_6$) δppm 1.34 (6 H, q, J=6.9 Hz) 1.51 (9 H, s) 2.84 (2 H, t, J=6.1 Hz) 3.30 (2 H, t, J=6.1 Hz) 4.01 - 4.17 (4 H, m) 6.64 (1 H, d, J=16.0 Hz) 7.06 (1 H, d, J=8.4 Hz) 7.42 - 7.80 (8 H, m) 7.86 - 7.94 (3 H, m) 8.26 (1 H, s) 11.03 (1 H, s)

Example 83

(2E)-3-(2-{[4-(3,4-diethoxyphenyl)-4-oxobutanoyl]amino}-4-phenylquinoline-7-yl)acrylic acid

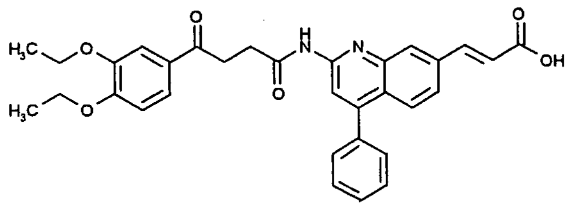

To tert-butyl (2E)-3-(2-{[4-(3,4-diethoxyphenyl)-4-oxobutanoyl]amino}-4-phenylquinoline-7-yl)acrylate obtained in Example 82 (200 mg) was added trifluoroacetic acid (2 mL), and the mixture was stirred for 30 min. Trifluoroacetic acid was evaporated under reduced pressure, and the obtained crystals were recrystallized from acetonitrile, THF and ethyl acetate to give the title compound (61 mg).
$^1$H-NMR (300 MHz, DMSO-$d_6$) δppm 1.29 - 1.40 (6 H, m) 2.85 (2 H, t, J=6.2 Hz) 3.31 (2 H, t, J=6.2 Hz) 4.01 - 4.16 (4 H, m) 6.65 (1 H, d, J=16.2 Hz) 7.06 (1 H, d, J=8.6 Hz) 7.44 - 7.79 (8 H, m) 7.85 - 7.95 (3 H, m) 8.26 (1 H, s) 11.04 (1 H, s)

Example 84

4-(3,4-diethoxyphenyl)-4-oxo-N-(1-phenylisoquinolin-3-yl)butanamide

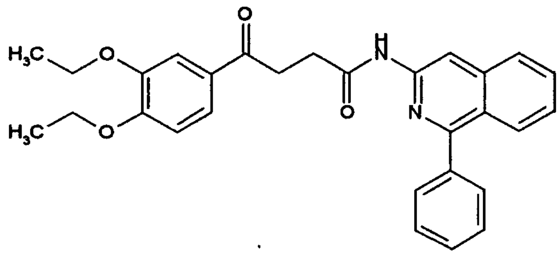

4-(3,4-Diethoxyphenyl)-4-oxobutanoic acid obtained in Example 3-(1) (266 mg), 1-phenylisoquinolin-3-amine synthesized according to Heterocycles, 2000, 53, 291 (220 mg), WSC (155 mg) and HOBt (153 mg) were dissolved in acetonitrile (5 mL), and the mixture was stirred overnight at 50°C. The reaction mixture was ice-cooled, and the precipitated solid was collected by filtration to give the title compound (21 mg).
$^1$H-NMR(300 MHz, DMSO-$d_6$) δppm 1.31 - 1.40 (6 H, m) 2.82 (2 H, t, J=6.3 Hz) 3.32 (2 H, t, J=6.3 Hz) 4.03 - 4.18 (4 H, m) 7.08 (1 H, d, J=8.4 Hz) 7.43 - 7.73 (9 H, m) 7.90 (2 H, dd, J=12.1, 8.4 Hz) 8.45 (1 H, s) 10.75 (1 H, s)

Example 85

4-(3,4-diethoxyphenyl)-4-oxo-N-[1-(piperidin-1-yl)isoquinolin-3-yl]butanamide

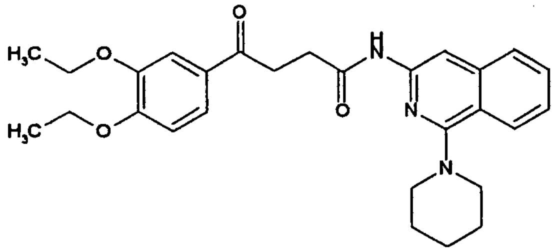

4-(3,4-Diethoxyphenyl)-4-oxobutanoic acid obtained in Example 3-(1) (266 mg), 1-(piperidin-1-yl)isoquinolin-3-amine synthesized according to Heterocycles, 2000, 53, 291 (227 mg), WSC (155 mg) and HOBt (153 mg) were dissolved in acetonitrile (5 mL), and the mixture was stirred overnight at 50°C. The reaction mixture was ice-cooled, and the precipitated solid was collected by filtration to give the title compound (62 mg).
$^1$H-NMR (300 MHz, DMSO-$d_6$) δppm 1.31- 1.40 (6 H, m) 1.59 - 1.84 (6 H, m) 2.80 (2 H, t, J=6.3 Hz) 3.25- 3.35 (6 H, m) 4.03 - 4.18 (4 H, m) 6.55 (1 H, s) 7.07 (1 H, d, J=8.4 Hz) 7.36 - 7.43 (1 H, m) 7.47 (1 H, d, J=2.0 Hz) 7.53 - 7.61 (1 H, m) 7.64 - 7.73 (2 H, m) 7.91 - 7.98 (1 H, m) 10.17 (1 H, s)

Example 86

N-{7-[(E)-2-cyanovinyl]-4-phenylquinolin-2-yl}-4-(3,4-diethoxyphenyl)-4-oxobutanamide

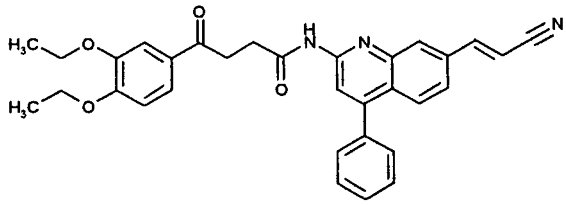

N-(7-Bromo-4-phenylquinolin-2-yl)-4-(3,4-diethoxyphenyl)-4-oxobutanamide obtained in Example 81 (0.55 g), acrylonitrile (0.13 mL), palladium acetate (22 mg), triphenylphosphine (52 mg), triethylamine (0.28 mL) were dissolved in DMF (10 mL), and the mixture was stirred at 100°C for 2 days. Water was added to the reaction mixture, and the mixture was extracted with ethyl acetate. The organic layer was washed with saturated brine, and dried over anhydrous magnesium sulfate, and the solvent was evaporated under reduced pressure. The residue was purified by column chromatography to give the title compound (290 mg).
$^1$H-NMR(300 MHz, DMSO-$d_6$) δppm 1.34 (6 H, q, J=6.9 Hz) 2.84 (2 H, t, J=6.1 Hz) 3.30 (2 H, t, J=6.1 Hz) 4.00 - 4.18 (4 H, m) 6.60 (1 H, d, J=16.7 Hz) 7.06 (1 H, d, J=8.4 Hz) 7.42 - 8.07 (11 H, m) 8.26 (1 H, s) 11.04 (1 H, s)

Example 87

tert-butyl 3-(2-{[4-(3,4-diethoxyphenyl)-4-oxobutanoyl]amino}-4-phenylquinoline-7-yl)propanoate

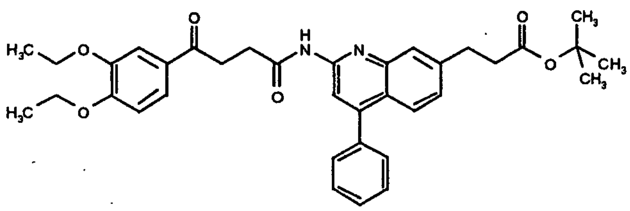

tert-Butyl (2E)-3-(2-{[4-(3,4-diethoxyphenyl)-4-oxobutanoyl]amino}-4-phenylquinoline-7-yl)acrylate obtained in Example 82 (300 mg) and 10% palladium carbon (300 mg) were suspended in ethyl acetate (10 mL), and the mixture was stirred overnight under hydrogen atmosphere. 10% Palladium carbon was removed by filtration, and the solvent was evaporated under reduced pressure to give the title compound (260 mg).
LC-MS(ES+) 597.1

Example 88

4-(3,4-diethoxyphenyl)-N-(3-methyl-4-phenylquinolin-2-yl)-4-oxobutanamide

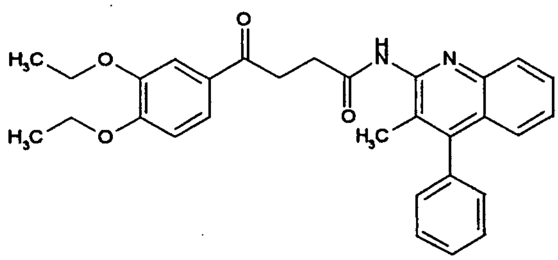

4-(3,4-Diethoxyphenyl)-4-oxobutanoic acid obtained in Example 3-(1) (266 mg), 3-methyl-4-phenylquinolin-2-amine synthesized according to J. Synth. Org. Chem. Jpn, 1973, 31, 328 (234 mg), WSC (155 mg) and HOBt (153 mg) were dissolved in acetonitrile (5 mL), and the mixture was stirred overnight at 50°C. The solvent was evaporated under reduced pressure, the residue was purified by column chromatography, and the obtained crystals were recrystallized from diethyl ether and diisopropyl ether to give the title compound (133 mg).
$^1$H-NMR (300 MHz, $CDCl_3$) δppm 1.43 - 1.52 (6 H, m) 2.16 (3 H, s) 3.12 - 3.24 (2 H, m) 3.43 - 3.50 (2 H, m) 4.09 - 4.22 (4 H, m) 6.88 (1 H, d, J=8.3 Hz) 7.23 - 7.35 (4 H, m) 7.44 - 7.67 (6 H, m) 7.96 (1 H, d, J=8.3 Hz) 8.10 (1 H, s)

Example 89

4-(3,4-diethoxyphenyl)-4-oxo-N-[4-(pyridin-2-yl)quinolin-2-yl]butanamide

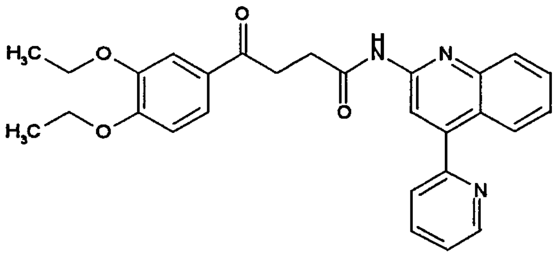

(1) (2-Aminophenyl)(pyridin-2-yl)methanone (5.0 g) and acetonitrile (3.5 mL) were dissolved in pyridine (100 mL), and 60% sodium hydride (1.5 g) was added. The reaction mixture was refluxed overnight, and cooled. Water was added, and the mixture was extracted with ethyl acetate. The organic layer was washed with saturated brine, and dried over anhydrous magnesium sulfate, and the solvent was evaporated under reduced pressure. The residue was purified by column chromatography to give 4-(pyridin-2-yl)quinolin-2-amine (1.9 g).
$^1$H-NMR (300 MHz, DMSO-$d_6$) δppm 6.54 (2 H, br) 6.84 (1 H, s) 7.09 - 7.17 (1 H, m) 7.45 - 7.57 (3 H, m) 7.61 - 7.75 (2 H, m) 7.95 - 8.04 (1 H, m) 8.73 - 8.80 (1 H, m)
(2) Potassium 4-(3,4-diethoxyphenyl)-4,4-dimethoxybutanoate obtained in Example 8-(7) (0.35 g), 2,4,6-trichlorobenzoyl chloride (0.27 mL) and triethylamine (0.15 mL) were dissolved in THF (5 mL), and the mixture was stirred for 4 hr. 4-(Pyridin-2-yl)quinolin-2-amine obtained in Example 89-(2) (0.22 g) was added to the reaction mixture, and the mixture was stirred overnight at 50°C. 2N Hydrochloric acid was added to the reaction mixture, and the

mixture was washed with ethyl acetate. 4N Aqueous sodium hydroxide solution was added to the separated aqueous layer, and the mixture was extracted with ethyl acetate. The organic layer was washed with saturated brine, and dried over anhydrous magnesium sulfate, and the solvent was evaporated under reduced pressure. The residue was purified by column chromatography, and the obtained crystals were recrystallized from ethyl acetate and diethyl ether to give the title compound (0.14 g).
$^1$H-NMR(300 MHz, DMSO-$d_6$) δppm 1.34 (6 H, q, J=6.9 Hz) 2.85 (2 H, t, J=6.2 Hz) 3.31 (2 H, t, J=6.2 Hz) 4.01 - 4.18 (4 H, m) 7.06 (1 H, d, J=8.6 Hz) 7.44 - 7.59 (3 H, m) 7.66 (1 H, dd, J=8.6, 1.8 Hz) 7.70 - 7.79 (2 H, m) 7.91 (1 H, d, J=7.7 Hz) 7.97 - 8.07 (2 H, m) 8.40 (1 H, s) 8.77 - 8.83 (1 H, m) 11.06 (1 H, s)

Example 90

4-(3,4-diethoxyphenyl)-N-{4-[4-(2-(morpholin-4-yl)ethoxy)phenyl]quinolin-2-yl}-4-oxobutanamide

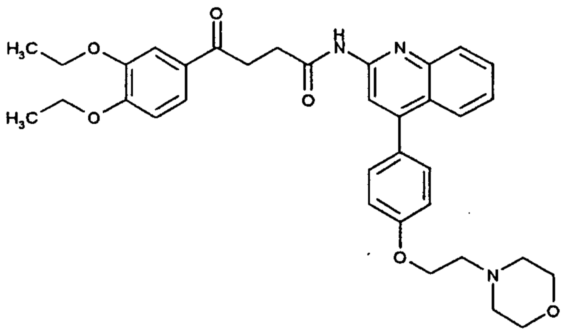

To 4-(3,4-diethoxyphenyl)-N-[4-(4-hydroxyphenyl)quinolin-2-yl]-4-oxobutanamide obtained in Example 22 (100 mg), 4-(2-chloroethyl)morpholine hydrochloride (58 mg), potassium carbonate (57 mg) and potassium iodide (51 mg) was added DMF (3 mL), and the mixture was stirred overnight. Water was added, and the mixture was extracted with ethyl acetate. The organic layer was washed with saturated brine, and dried over anhydrous magnesium sulfate, and the solvent was evaporated under reduced pressure. The residue was purified by column chromatography, and the obtained crystals were recrystallized from diethyl ether to give the title compound (57 mg).
$^1$H-NMR(300 MHz, DMSO-$d_6$) δppm 1.34 (6 H, q, J=6.9 Hz) 2.45 - 2.55 (4 H, m) 2.74 (2 H, t, J=5.7 Hz) 2.84 (2 H, t, J=6.1 Hz) 3.30 (2 H, t, J=6.1 Hz) 3.55 - 3.63 (4 H, m) 4.01 - 4.22 (6 H, m) 7.03 - 7.09 (1 H, m, J=8.4 Hz) 7.13 (2 H, d, J=8.6 Hz) 7.41 - 7.52 (4 H, m) 7.65 (1 H, dd, J=8.4, 1.8 Hz) 7.69 - 7.77 (1 H, m) 7.81 (1 H, d, J=8.4 Hz) 7.88 (1 H, d, J=7.9 Hz) 8.22 (1 H, s) 10.97 (1 H, s)

Example 91

4-(3,4-diethoxyphenyl)-4-oxo-N-[4-(pyridin-4-yl)quinolin-2-yl]butanamide

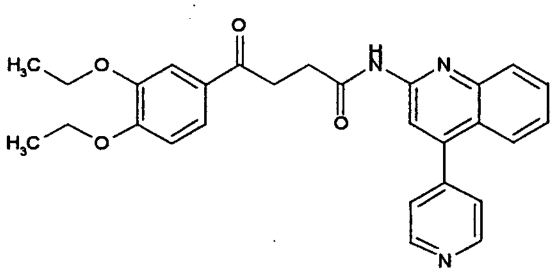

4-(3,4-Diethoxyphenyl)-4-oxobutanoic acid obtained in Example 3-(1) (266 mg), 4-(pyridin-4-yl)quinolin-2-amine synthesized according to J. Chem. Soc. 1952, 589 (221 mg), WSC (354 mg) and HOBt (306 mg) were dissolved

in acetonitrile (5 mL), and the mixture was stirred overnight at 50°C. Water was added to the reaction mixture, and the mixture was extracted with ethyl acetate. The organic layer was washed with saturated brine, and dried over anhydrous magnesium sulfate, and the solvent was evaporated under reduced pressure. The residue was purified by column chromatography to give the title compound (170 mg).
$^1$H-NMR(300 MHz, DMSO-$d_6$) δppm 1.34 (6 H, q, J=6.8 Hz) 2.85 (2 H, t, J=6.0 Hz) 3.31 (2 H, t, J=6.0 Hz) 4.01 - 4.16 (4 H, m) 7.06 (1 H, d, J=8.4 Hz) 7.43 - 7.82 (7 H, m) 7.93 (1 H, d, J=8.4 Hz) 8.27 (1 H, s) 8.77 (2 H, d, J=5.8 Hz) 11.10 (1 H, s)

Example 92

tert-butyl {2-[4-(2-{[4-(3,4-diethoxyphenyl)-4-oxobutanoyl]amino}quinolin-4-yl)phenoxy]ethyl}carbamate

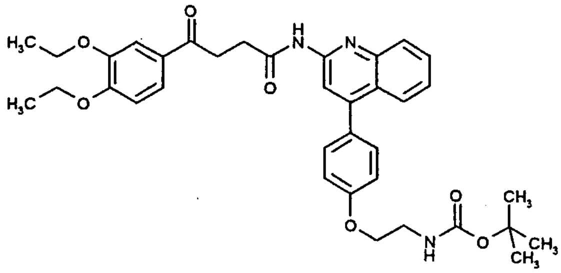

To 4-(3,4-diethoxyphenyl)-N-[4-(4-hydroxyphenyl)quinolin-2-yl]-4-oxobutanamide obtained in Example 22 (100 mg), tert-butyl (2-bromoethyl)carbamate (69 mg), potassium carbonate (57 mg) and potassium iodide (51 mg) was added DMF (3 mL), and the mixture was stirred overnight. Water was added, and the mixture was extracted with ethyl acetate. The organic layer was washed with saturated brine, and dried over anhydrous magnesium sulfate, and the solvent was evaporated under reduced pressure. The residue was purified by column chromatography, and the obtained crystals were recrystallized from diethyl ether to give the title compound (125 mg).
$^1$H-NMR (300 MHz, DMSO-$d_6$) δppm 1.30 - 1.42 (15 H, m) 2.83 (2 H, t, J=6.1 Hz) 3.27- 3.38 (4 H, m) 4.01 - 4.17 (6 H, m) 7.03 - 7.16 (4 H, m) 7.42 - 7.50 (4 H, m) 7.62 - 7.91 (4 H, m) 8.22 (1 H, s) 10.97 (1 H, s)

Example 93

4-(3,4-diethoxyphenyl)-N-(4-{4-[3-(dimethylamino)propoxy]phenyl}quinolin-2-yl)-4-oxobutanamide

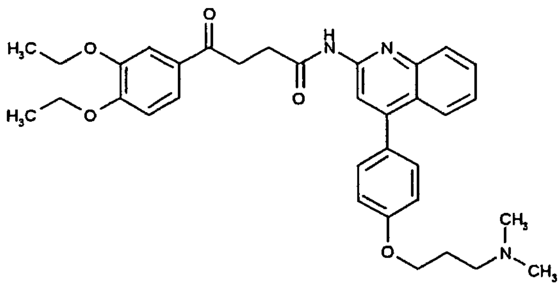

To 4-(3,4-diethoxyphenyl)-N-[4-(4-hydroxyphenyl)quinolin-2-yl]-4-oxobutanamide obtained in Example 22 (100 mg), 3-chloro-N,N-dimethylpropan-1-amine hydrochloride (49 mg), potassium carbonate (57 mg) and potassium iodide (51 mg) was added DMF (3 mL), and the mixture was stirred overnight. Water was added, and the mixture was extracted with ethyl acetate. The organic layer was washed with saturated brine, and dried over anhydrous magnesium sulfate, and the solvent was evaporated under reduced pressure. The residue was purified by column chromatography, and the obtained crystals were recrystallized from diethyl ether to give the title compound (21 mg).
$^1$H-NMR(300 MHz, DMSO-$d_6$) δppm 1.34 (6 H, q, J=6.9 Hz) 1.82 - 1.96 (2 H, m) 2.16 (6 H, s) 2.38 (2 H, t, J=7.0 Hz)

2.83 (2 H, t, J=6.3 Hz) 3.30 (2 H, t, J=6.3 Hz) 4.01 - 4.18 (6 H, m) 7.02 - 7.15 (3 H, m) 7.41 - 7.51 (4 H, m) 7.60 - 7.92 (4 H, m) 8.22 (1 H, s) 10.96 (1 H, s)

Example 94

N-{4-[4-(2-aminoethoxy)phenyl]quinolin-2-yl}-4-(3,4-diethoxyphenyl)-4-oxobutanamide

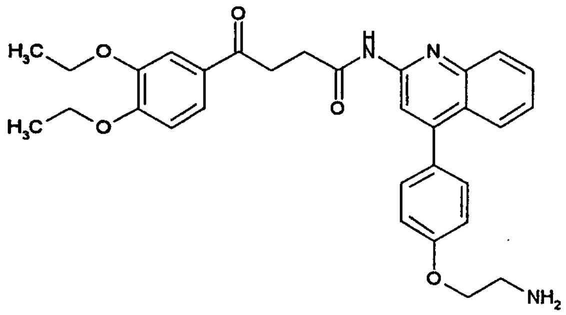

To 4-(3,4-diethoxyphenyl)-N-(4-{4-[3-(dimethylamino)propoxy]phenyl}quinolin-2-yl)-4-oxobutanamide obtained in Example 93 (100 mg) was added trifluoroacetic acid (2 mL), and the mixture was stirred for 1 hr. Trifluoroacetic acid was evaporated under reduced pressure to give the title compound (76 mg) .
$^1$H-NMR(300 MHz, DMSO-$d_6$) δppm 1.34 (6 H, q, J=6.7 Hz) 1.86 (2 H, s) 2.84 (2 H, t, J=6.2 Hz) 2.93 (2 H, t, J=5.1 Hz)-3.30 (2 H, t, J=6.2 Hz) 3.97 - 4.17 (6 H, m) 7.06 (1 H, d, J=8.4 Hz) 7.13 (2 H, d, J=8.8 Hz) 7.42 - 7.50 (4 H, m) 7.62 - 7.92 (4 H, m) 8.22 (1 H, s) 10.97 (1 H, s)

Example 95

N-(6-benzyl-4-phenylpyridin-2-yl)-4-(3,4-diethoxyphenyl)-4-oxobutanamide

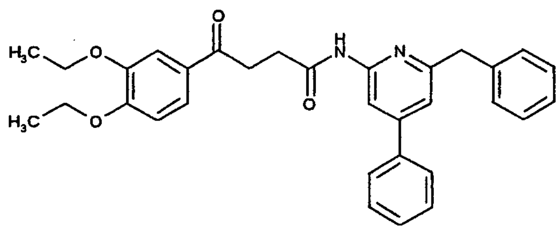

Potassium 4-(3,4-diethoxyphenyl)-4,4-dimethoxybutanoate obtained in Example 8-(7) (0.42 g), 2,4,6-trichlorobenzoyl chloride (0.23 mL) and triethylamine (0.33 mL) were dissolved in THF (5 mL), and the mixture was stirred for 4 hr. 6-Benzyl-4-phenylpyridin-2-amine synthesized according to Chem. Ber. 1961, 94, 698 (0.31 g) was added to the reaction mixture, and the mixture was stirred overnight at 50°C. Diluted hydrochloric acid was added to the reaction mixture, and the mixture was extracted with ethyl acetate. The organic layer was washed with water, dried (over magnesium sulfate), and concentrated under reduced pressure to give the title compound (0.21 g).
$^1$H-NMR (300 MHz, DMSO-$d_6$) δppm 1.30 - 1.39 (6 H, m) 2.78 (2 H, t, J=6.4 Hz) 3.28 (2 H, t, J=6.4 Hz) 4.02 - 4.16 (6 H, m) 7.06 (1 H, d, J=8.4 Hz) 7.17 - 7.37 (6 H, m) 7.43 - 7.54 (4 H, m) 7.62 - 7.68 (3 H, m) 8.23 (1 H, s) 10.63 (1 H, s)

Example 96

ethyl 5-benzyl-2-{[4-(3,4-diethoxyphenyl)-4-oxobutanoyl]amino}-4-phenylthiophene-3-carboxylate

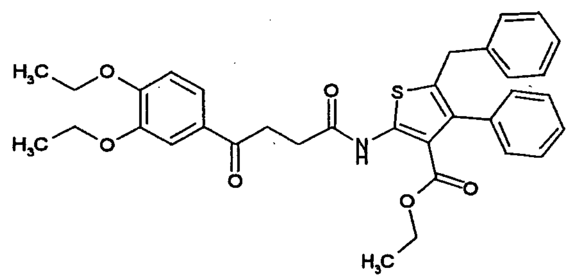

Potassium 4-(3,4-diethoxyphenyl)-4,4-dimethoxybutanoate obtained in Example 8-(7) (0.70 g), 2,4,6-trichlorobenzoyl chloride (0.34 mL) and triethylamine (0.31 mL) were dissolved in THF (30 mL), and the mixture was stirred for 4 hr. Ethyl 2-amino-5-benzyl-4-phenylthiophene-3-carboxylate obtained in Example 17-(1) (0.68 g) was added to the reaction mixture, and the mixture was stirred overnight at 50°C. Diluted hydrochloric acid was added to the reaction mixture, and the mixture was extracted with ethyl acetate. The organic layer was washed with water, dried (over magnesium sulfate), and concentrated under reduced pressure. The residue was purified by column chromatography, and the obtained crystals were recrystallized from ethanol and water to give the title compound (0.29 g).
$^1$H-NMR(300 MHz, DMSO-$d_6$) δppm 0.78 (3 H, t, J=7.1 Hz) 1.29 - 1.40 (6 H, m) 2.83 (2 H, t, J=6.1 Hz) 3.29 - 3.38 (2 H, m) 3.80 (2 H, s) 3.94 (2 H, q, J=7.1 Hz) 4.02 - 4.17 (2 H, m) 7.01 - 7.10 (3 H, m) 7 .13 - 7.29 (5 H, m) 7.34 - 7.47 (4 H, m) 7.64 (1 H, dd, J=8.4, 2.0 Hz) 11.08 (1 H, s)

Example 97

N-(5-benzyl-4-phenyl-2-thienyl)-4-(4-ethoxy-3-propylphenyl)-4-oxobutanamide

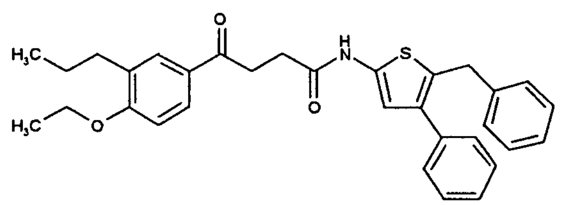

4-(4-Ethoxy-3-propylphenyl)-4-oxobutanoic acid obtained in Example 24-(1) (0.53 g), 5-benzyl-4-phenylthiophen-2-amine obtained in Example 17-(2) (0.27 g), WSC (0.35 mL) and HOBt (0.31 g) were dissolved in acetonitrile (20 mL), and the mixture was stirred overnight at 50°C. Water was added to the reaction mixture, and the mixture was extracted with ethyl acetate. The organic layer was washed with saturated brine, and dried over anhydrous magnesium sulfate, and the solvent was evaporated under reduced pressure. The residue was purified by column chromatography, and the obtained crystals were recrystallized from ethanol and water to give the title compound (0.073 g).
$^1$H-NMR(300 MHz, DMSO-$d_6$) δppm 0.89 (2 H, t, J=7.35 Hz) 1.36 (3 H, t, J=6.9 Hz) 1.49 - 1.65 (2 H, m) 2.54 - 2.61 (2 H, m) 2.67 (2 H, t, J=6.4 Hz) 3.28 (2 H, t, J=6.4 Hz) 4.04 - 4.19 (4 H, m) 6.60 (1 H, s) 7.04 (1 H, d, J=8.6 Hz) 7.09 - 7.47 (11 H, m) 7.75 (1 H, d, J=2.1 Hz) 7.85 (1 H, dd, J=8.6, 2.1 Hz) 11.19 (1 H, s)

Example. 98

N-(5-benzyl-4-phenyl-2-thienyl)-4-(3-bromo-4-ethoxyphenyl)-4-oxobutanamide

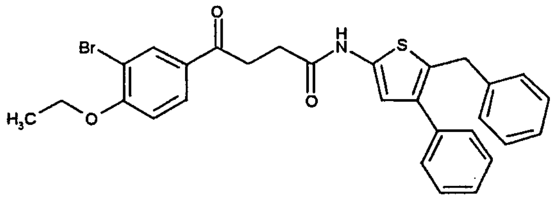

4-(3-Bromo-4-ethoxyphenyl)-4-oxobutanoic acid obtained in Example 25-(1) (0.60 g), 5-benzyl-4-phenylthiophen-2-amine obtained in Example 17-(2) (0.27 g), WSC (0.35 mL) and HOBt (0.31 g) were dissolved in acetonitrile (20 mL), and the mixture was stirred overnight at 50°C. Water was added to the reaction mixture, and the mixture was extracted with ethyl acetate. The organic layer was washed with saturated brine, and dried over anhydrous magnesium sulfate, and the solvent was evaporated under reduced pressure. The residue was purified by column chromatography, and the obtained crystals were recrystallized from ethanol and water to give the title compound (0.19 g).
$^1$H-NMR(300 MHz, DMSO-$d_6$) δppm 1.39 (3 H, t, J=6.9 Hz) 2.68 (2 H, t, J=6.2 Hz) 3.29 (2 H, t, J=6.2 Hz) 4.22 (2 H, q, J=6.9 Hz) 6.60 (1 H, s) 7.09 - 7.48 (11 H, m) 8.00 (1 H, dd, J=8.6, 2.1 Hz) 8.13 (1 H, d, J=2.1 Hz) 11.19 (1 H, s)

Example 99

N-(5-benzyl-4-phenyl-2-thienyl)-4-oxo-4-(5,6,7,8-tetrahydronaphthalen-2-yl)butanamide

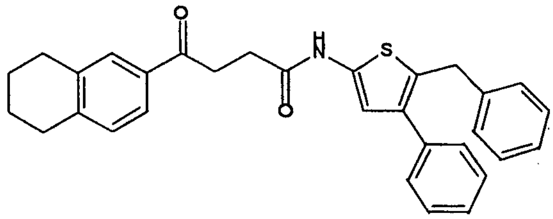

4-Oxo-4-(5,6,7,8-tetrahydronaphthalen-2-yl)butanoic acid obtained in Example 7-(1) (0.47 g), 5-benzyl-4-phenylthiophen-2-amine obtained in Example 17-(2) (0.27 g), WSC (0.35 mL) and HOBt (0.31 g) were dissolved in acetonitrile (20 mL), and the mixture was stirred overnight at 50°C. Water was added to the reaction mixture, and the mixture was extracted with ethyl acetate. The organic layer was washed with saturated brine, and dried over anhydrous magnesium sulfate, and the solvent was evaporated under reduced pressure. The residue was purified by column chromatography, and the obtained crystals were recrystallized from ethanol and water to give the title compound (0.21 g).
$^1$H-NMR (300 MHz, DMSO-$d_6$) δppm 1.70 - 1.79 (4 H, m) 2.68 (2 H, t, J=6.4 Hz) 2.78 (4 H, s) 3.29 (2 H, t, J=6.4 Hz) 4.08 (2 H, br) 6.60 (1 H, s) 7.10 - 7.47 (11 H, m) 7.64 - 7.71 (2 H, m) 11.19 (1 H, s)

Example 100

N-[3-tert-butyl-1-(4-tert-butylphenyl)-1H-pyrazol-5-yl]-4-(3,4-diethoxyphenyl)-4-oxobutanamide

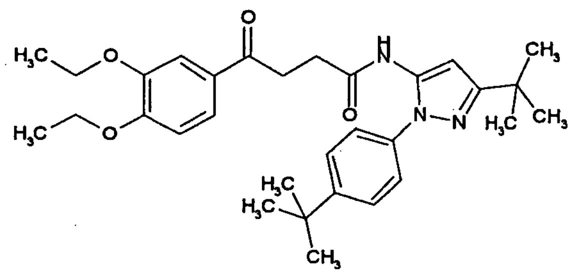

(1) A solution of pivaloylacetonitrile (4.60 g) and 4-tert-butylphenylhydrazine hydrochloride (3.167 g) in a mixed solvent of ethyl acetate (63 mL) and methanol (7 mL) was heated under reflux for 12 hr. The solvent of the reaction mixture was evaporated, and the residue was poured into ethyl acetate. The mixture was washed with saturated aqueous sodium hydrogencarbonate solution and saturated brine, and dried, and the solvent was evaporated. The residue was purified by column chromatography to give 3-tert-butyl-1-(4-tert-butylphenyl)-1H-pyrazol-5-amine (3.54 g).

$^1$H-NMR (300 MHz, $CDCl_3$) δppm 1.21 (9 H, s) 1.31 (9 H, s) 5.15 (2 H, br s) 5.36 (1 H, s) 7.46 (4 H, m)

(2) Potassium 4-(3,4-diethoxyphenyl)-4,4-dimethoxybutanoate obtained in Example 8-(7) (0.18 g), 2,4,6-trichlorobenzoyl chloride (0.095 mL) and triethylamine (0.14 mL) were dissolved in THF (5 mL), and the mixture was stirred for 4 hr. THF was evaporated under reduced pressure, and 1,2-dichloroethane (2 mL), triethylamine (0.14 mL) and 3-tert-butyl-1-(4-tert-butylphenyl)-1H-pyrazol-5-amine obtained in Example 100-(1) (0.068 g) were added, and the mixture was stirred overnight at 50°C. Saturated aqueous sodium hydrogencarbonate was added to the reaction mixture, and the mixture was extracted with ethyl acetate. The organic layer was washed with water, dried (over magnesium sulfate), and concentrated under reduced pressure. The residue was purified by column chromatography to give the title compound (0.073 g).

$^1$H-NMR (300 MHz, DMSO-$d_6$) δppm 1.26 (9 H, s) 1.31 (9 H, s) 1.32 - 1.39 (6 H, m) 2.62 (2 H, t, J=6. 2 Hz) 3.24 (2 H, t, J=6.2 Hz) 4.04 - 4.17 (4 H, m) 6.26 (1 H, s) 7.06 (1 H, d, J=8.4 Hz) 7.39 - 7.51 (5 H, m) 7.64 (1 H, dd, J=8.4, 1.8 Hz) 9.94 (1 H, s)

Example 101

N-[3-(2-chlorophenyl)-1-(4-methylphenyl)-1H-pyrazol-5-yl]-4-(3,4-diethoxyphenyl)-4-oxobutanamide

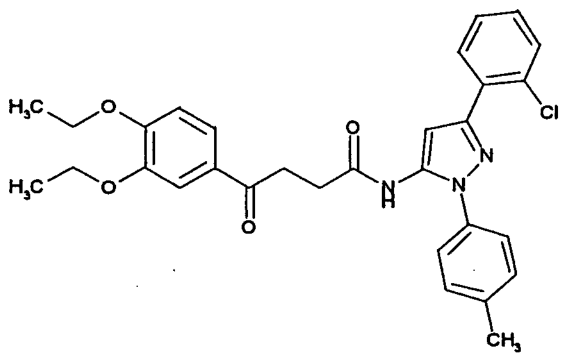

Potassium 4-(3,4-diethoxyphenyl)-4,4-dimethoxybutanoate obtained in Example 8-(7) (0.070 g), 2,4,6-trichlorobenzoyl chloride (0.038 mL) and triethylamine (0.056 mL) was dissolved in 1,2-dichloroethane (2 mL), and the mixture was stirred for 4 hr. 3-(2-Chlorophenyl)-1-(4-methylphenyl)-1H-pyrazol-5-amine (0.028 g) was added, and the mixture was stirred overnight at 50°C. Saturated aqueous sodium hydrogencarbonate was added to the reaction mixture, and the mixture was extracted with dichloromethane. The organic layer was washed with water, dried (over magnesium sulfate), and concentrated under reduced pressure. The residue was purified by column chromatography to give the title compound (0.025 g).

LC-MS(ES+) 532.06

Example 102

N-(4,6-diphenylpyridin-2-yl)-4-(4-ethoxyphenyl)-4-oxobutanamide

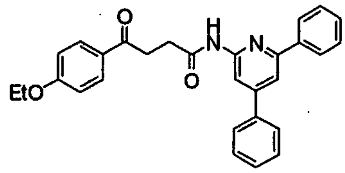

A solution of potassium 4-(4-ethoxyphenyl)-4,4-dimethoxybutanoate obtained in Example 60-(3) (297 mg), triethylamine (0.145 mL) and 2,4,6-trichlorobenzoyl chloride (0.152 mL) in THF (5 mL) was stirred at room temperature for 5 hr. 4,6-Diphenylpyridin-2-amine (0.12 g, synthesized according to a method described in Chem Ber, 1961, 94, 698-704) was added to the reaction mixture, and the mixture was stirred overnight at 50°C. 2N Hydrochloric acid was added to the reaction mixture, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (9:1→1:1)] to give N-(4,6-diphenylpyridin-2-yl)-4-(4-ethoxyphenyl)-4-oxobutanamide (76 mg) as white crystals.
$^1$H-NMR ($CDCl_3$) δ: 1.23 (3 H, t, J=7.16 Hz), 4.23 (2 H, q, J=7.16 Hz), 4.52 - 4.68 (2 H, m), 6.16 (1 H, s), 6.98 - 7.12 (2 H, m), 7.14 - 7.20 (1 H, m), 7.42 (1 H, td, J=7.77, 1.79 Hz), 8.11 (1 H, s)

Example 103

N-(4,6-diphenylpyridin-2-yl)-4-(3-ethoxyphenyl)-4-oxobutanamide

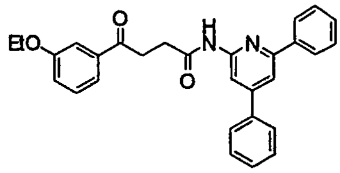

(1) potassium 4-(3-ethoxyphenyl)-4,4-dimethoxybutanoate

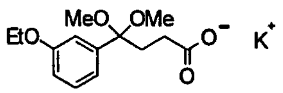

To a solution of 4-(3-ethoxyphenyl)-4-oxobutanoic acid obtained in Example 5-(2) (1.27 g) in a mixed solvent of trimethyl orthoformate (2.5 mL)-methanol (20 mL) was added p-toluenesulfonic acid (11 mg), and the mixture was heated under reflux overnight. The reaction mixture was concentrated under reduced pressure, and the residue was extracted with ethyl acetate. The organic layer was washed with saturated aqueous sodium hydrogencarbonate solution and saturated brine, and dried over anhydrous magnesium sulfate. The extract was concentrated under reduced pressure, and residue was dissolved in methanol (15 mL). Potassium hydroxide (0.32 g) was added, and the mixture was stirred for 1 hr, and concentrated. The residue was poured into diethyl ether (100 mL) to give potassium 4-(3-ethoxyphenyl)-4,4-dimethoxybutanoate (1.2 g).
$^1$H-NMR(DMSO-$d_6$) δ: 1.28 - 1.42 (5 H, m), 1.94 (2 H, s), 3.03 (6 H, s), 4.00 (2 H, q, J=6.78 Hz), 6.79 - 6.88 (2 H, m), 6.88 - 6.98 (1 H, m), 7.24 (1 H, t, J=7.63 Hz)
(2) N-(4,6-diphenylpyridin-2-yl)-4-(3-ethoxyphenyl)-4-oxobutanamide

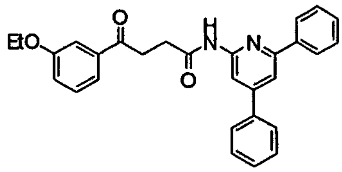

A solution of potassium 4-(3-ethoxyphenyl)-4,4-dimethoxybutanoate obtained in Example 103-(1) (297 mg), triethylamine (0.145 mL) and 2,4,6-trichlorobenzoyl chloride (0.152 mL) in THF (5 mL) was stirred at room temperature for 70 hr. 4,6-Diphenylpyridin-2-amine (0.12 g, synthesized according to a method described in Chem Ber, 1961, 94, 698-704) was added to the reaction mixture, and the mixture was stirred overnight at 70°C. 2N Hydrochloric acid was added to the reaction mixture, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (9:1→1:1)] to give N-(4,6-diphenylpyridin-2-yl)-4-(3-ethoxyphenyl)-4-oxobutanamide (41 mg) as white crystals.
$^1$H-NMR ($CDCl_3$) δ: 1.34 - 1.48 (3 H, m), 2.92 (2 H, t, J=6.50 Hz), 3.46 (2 H, t, J=6.50 Hz), 4.08 (2 H, q, J=6.97 Hz), 7.04 - 7.16 (1 H, m), 7.34 - 7.38 (1 H, m), 7.40 - 7.54 (7 H, m), 7.60 (1 H, d, J=6.78 Hz), 7.65 - 7.75 (3 H, m), 7.94 - 8.04 (2 H, m), 8.29 (1H, s), 8.42 (1H, s)

<u>Example 104</u>

4-(2,3-dihydro-1-benzofuran-5-yl)-N-(4,6-diphenylpyridin-2-yl)-4-oxobutanamide

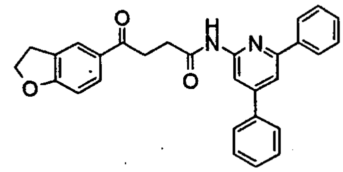

(1) potassium 4-(2,3-dihydro-1-benzofuran-5-yl)-4,4-dimethoxybutanoate

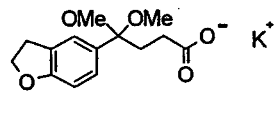

To a solution of 4-(2,3-dihydro-1-benzofuran-5-yl)-4-oxobutanoic acid obtained in Example 27-(1) (3.2 g) in a mixed solvent of trimethyl orthoformate (8.0 mL)-methanol (50 mL) was added p-toluenesulfonic acid (70 mg), and the mixture was heated under reflux overnight. The reaction mixture was concentrated under reduced pressure, and the residue was extracted with ethyl acetate. The organic layer was washed with saturated aqueous sodium hydrogencarbonate solution and saturated brine, and dried over anhydrous magnesium sulfate. The extract was concentrated under reduced pressure, and residue was dissolved in methanol (25 mL). Potassium hydroxide (0.83 g) was added, and the mixture was stirred overnight, and concentrated. The residue was poured into diethyl ether (100 mL) to give potassium 4-(2,3-dihydro-1-benzofuran-5-yl)-4,4-dimethoxybutanoate (3.0 g).
$^1$H-NMR (DMSO-$d_6$) δ: 1.34 - 1.44 (2 H, m), 1.92 (2 H, ddd, J=8.48, 4.52, 4.33 Hz), 2.94 - 3.04 (6 H, m), 3.16 (2 H, t, J=8.67 Hz), 4.51 (2 H, t, J=8.76 Hz), 6.69 (1 H, d, J=8.29 Hz), 7.05 (1 H, dd, J=8.38, 1.79 Hz), 7.19 (1 H, d, J=1.13 Hz)
(2) 4-(2,3-dihydro-1-benzofuran-5-yl)-N-(4,6-diphenylpyridin-2-yl)-4-oxobutanamide

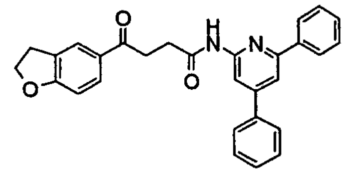

A solution of potassium 4-(2,3-dihydro-1-benzofuran-5-yl)-4,4-dimethoxybutanoate obtained in Example 104-(1) (298 mg), triethylamine (0.152 mL) and 2,4,6-trichlorobenzoyl chloride (0.153 mL) in THF (2 mL) was stirred at room temperature for 18 hr. 4,6-Diphenylpyridin-2-amine (0.12 g, synthesized according to a method described in Chem Ber, 1961, 94, 698-704) was added to the reaction mixture, and the mixture was stirred overnight at 70°C. 2N Hydrochloric acid was added to the reaction mixture, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (9:1-1:1)] to give 4-(2,3-dihydro-1-benzofuran-5-yl)-N-(4,6-diphenylpyridin-2-yl)-4-oxobutanamide (6.4 mg) as white crystals.
$^1$H-NMR($CDCl_3$) δ : 2.90 (2 H, t, J=6.41 Hz), 3.20 - 3.30 (2 H, m), 3.41 (2 H, t, J=6.50 Hz), 4.67 (2 H, t, J=8.76 Hz), 6.82 (1 H, d, J=8.29 Hz), 7 .40 - 7.53 (6 H, m), 7.71 (3 H, td, J=7.35, 1.32 Hz), 7.88 (2 H, d, J=8.48 Hz), 7.99 - 8.04 (2 H, m), 8.31 (1 H, s), 8.42 (1 H, s)

Example 105

N-(4,6-diphenylpyridin-2-yl)-4-oxo-4-(5,6,7,8-tetrahydronaphthalen-2-yl)butanamide

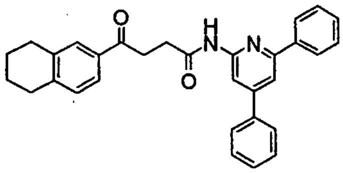

(1) potassium 4,4-dimethoxy-4-(5,6,7,8-tetrahydronaphthalen-2-yl)butanoate

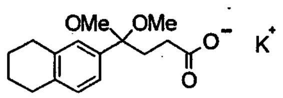

To a solution of 4-oxo-4-(5,6,7,8-tetrahydronaphthalen-2-yl)butanoic acid obtained in Example 7-(1) (3.86 g), methanol (10 mL) and methyl orthoformate (30 mL) was added concentrated sulfuric acid (4 drops), and the mixture was heated under reflux for 22 hr. The reaction mixture was concentrated under reduced pressure, and diluted with saturated aqueous sodium hydrogencarbonate solution, and the mixture was extracted with ethyl acetate. The organic layer was washed with saturated brine, dried over magnesium sulfate, and concentrated under reduced pressure. The residue was dissolved in methanol (20 mL), potassium hydroxide (1.1 g) was added, and the mixture was stirred for 4 days. The reaction mixture was concentrated under reduced pressure, acetone was added to the residue, and the obtained solid was collected by filtration to give potassium 4,4-dimethoxy-4-(5, 6, 7, 8-tetrahydronaphthalen-2-yl)butanoate (4.2 g) as a crude solid.
$^1$H-NMR (DMSO-$d_6$) δ: 1.42 (2 H, dt, J=8.6, 4.4 Hz), 1.73 (4 H, t, J=2.5 Hz), 1.94 (2 H, ddd, J=8.5, 4.5, 4.3 Hz), 2.70 (4 H, d, J=4.5 Hz), 3.01 (6 H, s), 6.97 - 7.05 (3 H, m)
(2) N-(4, 6-diphenylpyridin-2-yl)-4-oxo-4-(5,6,7,8-tetrahydronaphthalen-2-yl)butanamide

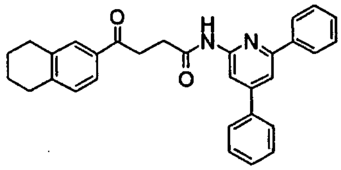

A solution of potassium 4,4-dimethoxy-4-(5,6,7,8-tetrahydronaphthalen-2-yl)butanoate obtained in Example 105-(1) (310 mg), triethylamine (0.152 mL) and 2,4,6-trichlorobenzoyl chloride (0.153 mL) in THF (2 mL) was stirred at room temperature for 24 hr. 4,6-Diphenylpyridin-2-amine (0.12 g, synthesized according to a method described in Chem Ber, 1961, 94, 698-704) was added to the reaction mixture, and the mixture was stirred overnight at 70°C. 2N Hydrochloric acid was added to the reaction mixture, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (9:1→1:1)] to give N-(4,6-diphenylpyridin-2-yl)-4-oxo-4-(5,6,7,8-tetrahydronaphthalen-2-yl)butanamide (99.4 mg) as white crystals.
$^1$H-NMR ($CDCl_3$) δ: 1.73 - 1.93 (4 H, m), 2 .76 - 2.84 (4 H, m), 2.90 (2 H, t, J=6.50 Hz), 3.44 (2 H, t, J=6.50 Hz), 7.15 (1 H, d, J=8.29 Hz), 7.39 - 7.53 (6 H, m), 7.67 - 7.76 (5 H, m, J=8.24, 6.45, 1.51 Hz), 8.01 (2 H, dd, J=8.10, 1.32 Hz), 8.32 (1 H, s), 8.42 (1 H, s)

Example 106

4-(3,4-diethoxyphenyl)-N-[4-(3-methoxyphenyl)-6-phenylpyridin-2-yl]-4-oxobutanamide

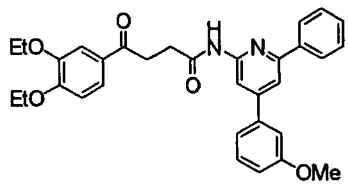

To a solution of N-(4-chloro-6-phenylpyridin-2-yl)-4-(3,4-diethoxyphenyl)-4-oxobutanamide obtained in Example 43-(3) (200 mg) in DME (2 mL) were added palladium acetate (10 mg), tri-o-tolylphosphine (54 mg), 2N aqueous potassium carbonate solution (0.6 mL) and 3-methoxyphenylboronic acid (147 mg), and the mixture was heated overnight at 100°C under nitrogen atmosphere. Water was added to the reaction mixture, and the mixture was extracted with ethyl acetate. The obtained organic layer was washed with 1N hydrochloric acid, saturated aqueous sodium hydrogencarbonate solution and saturated brine, dried over anhydrous magnesium sulfate, and concentrated under reduced pressure. The obtained residue was purified by silica gel column chromatography [developing solvent: hexane-ethyl acetate (3: 1)] to give 4-(3,4-diethoxyphenyl)-N-[4-(3-methoxyphenyl)-6-phenylpyridin-2-yl]-4-oxobutanamide (69 mg) as crystals.
$^1$H-NMR ($CDCl_3$) δ: 1.46 (3 H, t, J=6.97 Hz), 1.49 (3 H, t, J=6.97 Hz), 2.89 (2 H, t, J=6.50 Hz), 3.43 (2 H, t, J=6.50 Hz), 3.87 (3 H, s), 4.15 (2 H, q, J=7.10 Hz), 4.17 (2 H, q, J=6.97 Hz), 6.89 (1 H, d, J=8.48 Hz), 6.97 (1 H, ddd, J=8.10, 2.54, 1.04 Hz), 7.21 - 7.25 (1 H, m), 7.30 (1 H, dt, J=7.72, 1.32 Hz), 7.37 (1 H, d, J=7.91 Hz), 7.40 - 7.53 (3 H, m), 7.56 (1 H, d, J=1.88 Hz), 7 . 61 - 7.68 (2 H, m), 8.01 (2 H, ddd, J=6.45, 1.65, 1.51 Hz), 8.31 (1 H, s), 8.40 (1 H, s)

Example 107

N-[4-(2-butoxyethoxy)-6-phenylpyridin-2-yl]-4-(3,4-diethoxyphenyl)-4-oxobutanamide

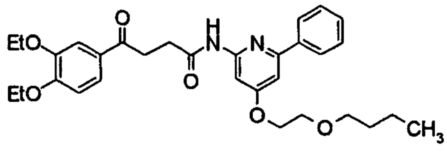

(1) 4-(2-butoxyethoxy)-6-phenylpyridin-2-amine

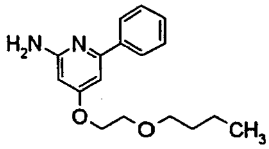

To a solution of 4-oxo-6-phenyl-1,4-dihydropyridine-2-carboxylic acid synthesized according to a method described in J. Med. Chem. 26, 1499-1504 (1983) (2.0 g) in DMF (80 mL) were added potassium carbonate (3.9 g) and 1-(2-chloroethoxy)butane (5.3 mL), and the mixture was heated at 80°C for 12 hr. Water was added to the reaction mixture, and the mixture was extracted with ethyl acetate. The organic layer was washed with water, and dried over anhydrous magnesium sulfate, and the solvent was evaporated under reduced pressure. The obtained residue was purified by silica gel column chromatography [developing solvent: hexane-ethyl acetate (3:1)] to give 2-butoxyethyl 4-(2-butoxyethoxy)-6-phenylpyridine-2-carboxylate as an oil. This compound was dissolved in methanol (50 mL), 2N aqueous sodium hydroxide solution (14 mL) was added, and the mixture was stirred at room temperature for 18 hr, and concentrated. The obtained residue was acidified with 1N hydrochloric acid, and extracted with ethyl acetate. The organic layer was washed with water, and dried over anhydrous magnesium sulfate, and the solvent was evaporated under reduced pressure to give 4-(2-butoxyethoxy)-6-phenylpyridine-2-carboxylic acid (1.3 g) as an oil. To a solution of this compound in toluene (25 mL) were added triethylamine (0.75 mL) and DPPA (1.1 mL), and the mixture was stirred at room temperature for 2 hr. tert-Butanol (2 mL) was added, and the mixture was heated overnight at 50°C, and concentrated. The obtained residue was purified by silica gel column chromatography [developing solvent: hexane-ethyl acetate (4:1)] to give tert-butyl [4-(2-butoxyethoxy)-6-phenylpyridin-2-yl]carbamate (0.70 g) as an oil. Trifluoroacetic acid (5 mL) was added thereto, and the mixture was stirred at room temperature for 18 hr. The reaction mixture was concentrated, and the residue was alkalified with aqueous sodium hydrogencarbonate solution, and extracted with ethyl acetate. The organic layer was washed with water, dried over anhydrous magnesium sulfate, and concentrated under reduced pressure to give 4-(2-butoxyethoxy)-6-phenylpyridin-2-amine (0.35 g) as crystals. The crystals were used for the next step without purification.

$^1$H-NMR ($CDCl_3$) δ: 0.85 - 0.96 (3 H, m), 1.32 - 1.45 (2 H, m), 1.49 - 1.78 (2 H, m), 3.54 (2 H, t, J=6.69 Hz), 3.76 - 3. 86 (2 H, m), 4. 08 - 4.19 (2 H, m), 4.45 (2 H, s), 5.98 (1 H, d, J=1.88 Hz), 6.73 (1H, d, J=2.07 Hz), 7.33 - 7.47 (3 H, m), 7.85 - 7.92 (2 H, m)

(2)N-[4-(2-butoxyethoxy)-6-phenylpyridin-2-yl]-4-(3,4-diethoxyphenyl)-4-oxobutanamide

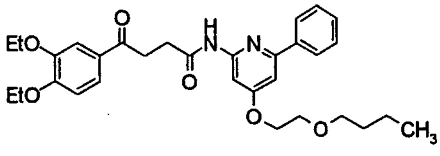

To a suspension of potassium 4-(3,4-diethoxyphenyl)-4,4-dimethoxybutanoate obtained in Example 8-(7) (0.28 g) and 2,4,6-trichlorobenzoyl chloride (0.13 mL) in THF (5 mL) was added triethylamine (0.15 mL), and the mixture was stirred at 50°C for 24 hr. 4-(2-Butoxyethoxy)-6-phenylpyridin-2-amine obtained in Example 107-(1) (0.12 g) was added to the reaction mixture, and the mixture was heated overnight at 70°C. Diluted hydrochloric acid was added to the reaction mixture, and the mixture was stirred for 1 hr, and extracted with ethyl acetate. The organic layer was washed with water, dried over anhydrous magnesium sulfate, and concentrated under reduced pressure. The obtained residue was purified by silica gel column chromatography [developing solvent: hexane-ethyl acetate (2:1)]

to give N-[4-(2-butoxyethoxy)-6-phenylpyridin-2-yl]-4-(3,4-diethoxyphenyl)-4-oxobutanamide (0.1 g) as crystals.
$^1$H-NMR ($CDCl_3$) δ: 0.92 (3 H, t, J=7.35 Hz), 1.34 - 1.42 (2 H, m), 1.44 - 1.52 (6 H, m), 1.54 - 1.65 (2 H, m), 2.83 (2 H, t, J=6.50 Hz), 3.39 (2 H, t, J=6.50 Hz), 3.53 (2 H, t, J = 6.59 Hz), 3.73 - 3.83 (2 H, m), 4.11 - 4.25 (6 H, m), 6.89 (1 H, d, J=8.48 Hz), 7.05 (1 H, d, J=2.07 Hz), 7.37 - 7.48 (3 H, m), 7.52 - 7.65 (2 H, m), 7.79 (1 H, d, J=1.32 Hz), 7.90 (2 H, dd, J=7.91, 1.51 Hz), 8.34 (1 H, s)

Example 108

4-(3,4-diethoxyphenyl)-N-[3-(4-fluorophenyl)-1-phenyl-1H-pyrazol-4-yl]-4-oxobutanamide

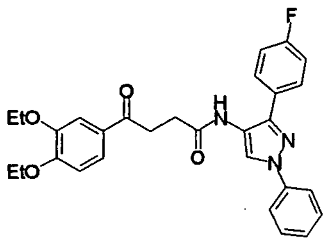

(1) 3-(4-fluorophenyl)-1-phenyl-1H-pyrazol-4-amine

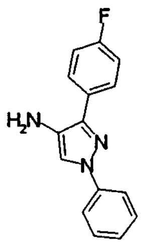

To a solution of 3-(4-fluorophenyl)-1-phenyl-1H-pyrazole-4-carboxylic acid (500.3 mg) in tetrahydrofuran (10 mL) were added triethylamine (0.371 mL) and diphenylphosphoryl azide (0.42 mL), and the mixture was heated under reflux for 2 hr. After cooling the reaction mixture to room temperature, 2-(trimethylsilyl)ethanol (0.762 mL) was added, and the mixture was heated under reflux for 2 hr. The reaction mixture was poured into a mixture of water and ethyl acetate, and the precipitated crystals were filtered off. The ethyl acetate layer was washed with saturated brine, and dried, and the solvent was evaporated. The residue was purified by silica gel column chromatography [developing solvent: n-hexane-ethyl acetate (1:19-3:7)] to give colorless crystals (64.7 mg). This colorless crystals were dissolved in a mixed solvent of tetrahydrofuran (10 mL) and water (1 mL), 1M solution (0.20 mL) of tri(n-butyl)ammonium fluoride in tetrahydrofuran was added, and the mixture was heated under reflux for 3 days. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (3:7)-ethyl acetate] to give 3-(4-fluorophenyl)-1-phenyl-1H-pyrazol-4-amine (23.1 mg) as a colorless solid.
$^1$H-NMR ($CDCl_3$) δ : 7.15 (2 H, t, J=8.8 Hz), 7.44 (2 H, t, J=7.9 Hz), 7.65 - 7.70 (3 H, m), 7.81 - 7.87 (2 H, m)
(2) 4-(3,4-diethoxyphenyl)-N-[3-(4-fluorophenyl)-1-phenyl-1H-pyrazol-4-yl]-4-oxobutanamide

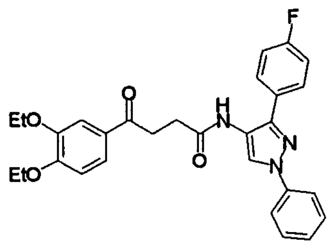

To a suspension of potassium 4-(3,4-diethoxyphenyl)-4,4-dimethoxybutanoate obtained in Example 8-(7) (64 mg) and 2,4,6-trichlorobenzoyl chloride (0.029 mL) in THF (10 mL) was added triethylamine (0.027 mL), and the mixture was stirred at room temperature for 5 hr. 3-(4-Fluorophenyl)-1-phenyl-1H-pyrazol-4-amine obtained in Example 108-(1) (23.1 mg) was added to the reaction mixture, and the mixture was stirred at 60°C for 2 days. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (9:1-1:4)]. The obtained colorless liquid was dissolved in dichloromethane (5 mL), and trifluoroacetic acid (1 mL) was added. The mixture was stirred at room temperature for 5 min, and the solvent was evaporated. The residue was crystallized from diethyl ether-n-hexane (1:2) to give 4-(3,4-diethoxyphenyl)-N-[3-(4-fluorophenyl)-1-phenyl-1H-pyrazol-4-yl]-4-oxobutanamide (4.2 mg) as colorless crystals.
$^1$H-NMR ($CDCl_3$) δ: 1.42 - 1.53 (6 H, m), 2.81 (2 H, t, J=6.2 Hz), 3.39 - 3.54 (2 H, m), 4.16 (4 H, qd, J=7.3, 7.1 Hz), 6.90 (1 H, d, J=8.5 Hz), 7.21 (2 H, ddd, J=8.7, 6.7, 2.0 Hz), 7.25 (1 H, s), 7.27 - 7.30 (1 H, m), 7.44 (2 H, t, J=7.9 Hz), 7.52 (1 H, d, J=1.9 Hz), 7.59 - 7.63 (1 H, m), 7.67 - 7.76 (3 H, m), 7.85 (1 H, s), 8.65 (1 H, s)
melting point :134.7-135.6°C

Example 109

N-(1-benzyl-3-phenyl-1H-pyrazol-4-yl)-4-(4-ethoxyphenyl)-4-oxobutanamide

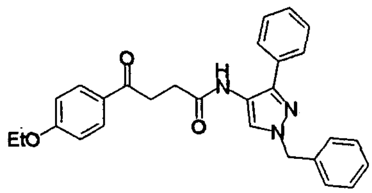

To a solution of potassium 4-(4-ethoxyphenyl)-4,4-dimethoxybutanoate obtained in Example 60-(3) (244 mg) and 2,4,6-trichlorobenzoyl chloride (0.124 mL) in tetrahydrofuran (10 mL) was added triethylamine (0.117 mL), and the mixture was stirred at room temperature for 5 hr. 1-Benzyl-3-phenyl-1H-pyrazol-4-amine obtained in Example 20-(4) (92.0 mg) was added to the reaction mixture, and the mixture was stirred overnight at 60°C. The reaction mixture was poured into water, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (4:1-2:3)] to give a colorless liquid. This colorless liquid was dissolved in acetonitrile (5 mL), and trifluoroacetic acid (1.0 mL) was added. The mixture was stirred at room temperature for 2 min, and concentrated under reduced pressure. The residue was purified by silica gel column chromatography [developing solvent: n-hexane-ethyl acetate (9:1-2:3)], and the obtained crystals were recrystallized from ethyl acetate-n-hexane (1:3) to give N-(1-benzyl-3-phenyl-1H-pyrazol-4-yl)-4-(4-ethoxyphenyl)-4-oxobutanamide (114.4 mg) as colorless crystals.
$^1$H-NMR ($CDCl_3$) δ: 1.44 (3 H, t, J=7.0 Hz), 2.75 (2 H, t, J=6.3 Hz), 3.37 (2 H, t, J=6.3 Hz), 4.10 (2 H, q, J=7.0 Hz), 5.29 (2 H, s), 6.89 - 6.95 (2 H, m), 7.28 - 7.42 (6 H, m), 7 .45 - 7.51 (2 H, m), 7.59 - 7.74 (3 H, m), 7.95 (2 H, ddd, J=9.4, 2.8, 2.4 Hz), 8.08 (1H, s)
melting point :138.4-140.6°C

Example 110

N-[4-(4-cyclohexylphenyl)-6-phenylpyridin-2-yl]-4-(3,4-diethoxyphenyl)-4-oxobutanamide

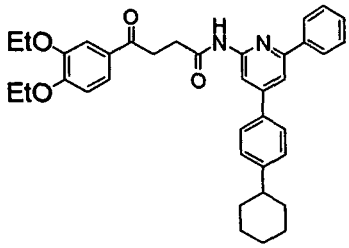

To a solution of N-(4-chloro-6-phenylpyridin-2-yl)-4-(3,4-diethoxyphenyl)-4-oxobutanamide obtained in Example 43-(3) (200 mg) in DME (2 mL) were added palladium acetate (10 mg), tri-o-tolylphosphine (54 mg), 2N aqueous potassium carbonate solution (0.6 mL) and 4-cyclohexylphenylboronic acid (108 mg), and the mixture was heated overnight at 100°C under nitrogen atmosphere. Water was added to the reaction mixture, and the mixture was extracted with ethyl acetate. The obtained organic layer was washed with 1N hydrochloric acid, saturated aqueous sodium hydrogencarbonate solution and saturated brine, dried over anhydrous magnesium sulfate, and concentrated under reduced pressure. The obtained residue was purified by silica gel column chromatography [developing solvent: hexane-ethyl acetate (3:1)] to give N-[4-(4-cyclohexylphenyl)-6-phenylpyridin-2-yl]-4-(3,4-diethoxyphenyl)-4-oxobutanamide (29 mg) as crystals.
$^1$H-NMR ($CDCl_3$) δ: 1.14 - 1.60 (11 H, m), 1.71 - 1.83 (1 H, m), 1 .85 - 1.94 (4 H, m), 2.55 - 2.64 (1 H, m), 3.09 - 3.14 (2 H, m), 3.40 - 3.46 (2 H, m), 4.08-4.23 (4H, m), 6.90 (1 H, d, J=8.48 Hz), 7.40 (2 H, d, J=8.29 Hz), 7.53 - 7.65 (6 H, m), 7.71 - 7.81 (5 H, m), 8.42 (1 H, d, J=1.51 Hz)

Example 111

4-(3,4-dimethylphenyl)-N-(4,6-diphenylpyridin-2-yl)-4-oxobutanamide

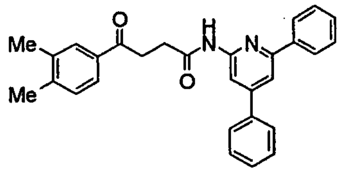

(1) ethyl 4-(3,4-dimethylphenyl)-4-oxobutanoate

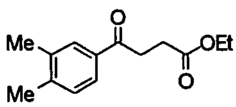

To a solution of o-xylene (7.3 mL) and ethyl 4-chloro-4-oxobutanoate (11.3 mL) in dichloromethane (100 mL) was gradually added aluminum chloride (17.6 g) under ice-cooling, and the mixture was stirred for 30 min under ice-cooling. The reaction mixture was poured into ice, concentrated hydrochloric acid (100 mL) was added, and the mixture was stirred for 30 min. The reaction mixture was extracted with ethyl acetate, and the organic layer was washed with saturated brine, dried over anhydrous magnesium sulfate, and concentrated under reduced pressure

to give ethyl 4-(3,4-dimethylphenyl)-4-oxobutanoate (15.7 g).
$^1$H-NMR ($CDCl_3$) δ: 1.27 (3 H, t, J=7.16 Hz), 2.32 (6 H, s), 2.74 (2 H, t, J=6.69 Hz), 3.29 (2 H, t, J=6.69 Hz), 4.09 - 4.20 (2 H, m), 7.21 (1 H, d, J=7.72 Hz), 7.72 (1 H, dd, J=7.82, 1.98 Hz), 7.76 (1 H, s)
(2) potassium 4-(3,4-dimethylphenyl)-4,4-dimethoxybutanoate

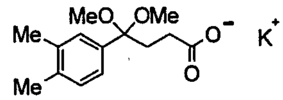

To a solution of ethyl 4-(3,4-dimethylphenyl)-4-oxobutanoate obtained in Example 111-(1) (14 g) in a mixed solvent of trimethyl orthoformate (33 mL)-methanol (100 mL) was added p-toluenesulfonic acid (280 mg), and the mixture was heated under reflux overnight at 50°C. The reaction mixture was concentrated under reduced pressure, and the residue was extracted with ethyl acetate. The organic layer was washed with saturated aqueous sodium hydrogencarbonate solution and saturated brine, and dried over anhydrous magnesium sulfate. The extract was concentrated under reduced pressure, and residue was dissolved in methanol (100 mL). Potassium hydroxide (3.4 g) was added, and the mixture was stirred for 1 hr. The reaction mixture was concentrated, and the residue was poured into diethyl ether to give potassium 4-(3,4-dimethylphenyl)-4,4-dimethoxybutanoate (11.0 g).
$^1$H-NMR (DMSO-$d_6$) δ: 1.32 - 1.43 (2 H, m), 1.93 (2 H, ddd, J=8.48, 4.52, 4.33 Hz), 2.19 (3 H, s), 2.22 (3 H, s), 3.00 (6 H, s), 7.02 - 7.14 (3 H, m)
(3) 4-(3,4-dimethylphenyl)-N-(4,6-diphenylpyridin-2-yl)-4-oxobutanamide

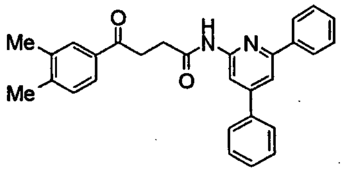

A solution of potassium 4-(3,4-dimethylphenyl)-4,4-dimethoxybutanoate obtained in Example 111-(2) (212 mg), triethylamine (0.124 mL) and 2,4,6-trichlorobenzoyl chloride (0.114 mL) in THF (5 mL) was stirred for 42 hr. 4,6-Diphenylpyridin-2-amine (0.12 g, synthesized according to a method described in Chem Ber, 1961, 94, 698-704) was added to the reaction mixture, and the mixture was stirred overnight at 70°C. 2N Hydrochloric acid was added to the reaction mixture, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (9:1→1:1)] to give 4-(3,4-dimethylphenyl)-N-(4,6-diphenylpyridin-2-yl)-4-oxobutanamide (40 mg) as white crystals.
$^1$H-NMR ($CDCl_3$) δ: 2.32 (6 H, s), 2.90 (2 H, t, J=6.50 Hz), 3.45 (2 H, t, J=6.59 Hz), 7.23 (1 H, d, J=7.72 Hz), 7.39 - 7.53 (6 H, m), 7.68 - 7.81 (5 H, m), 8.01 (2 H, ddd, J=6.55, 1.65, 1.41 Hz), 8.30 (1 H, s), 8.42 (1 H, s)

Example 112

methyl 4-(2-{[4-(3,4-diethoxyphenyl)-4-oxobutanoyl]amino}-6-phenylpyridin-4-yl)benzoate

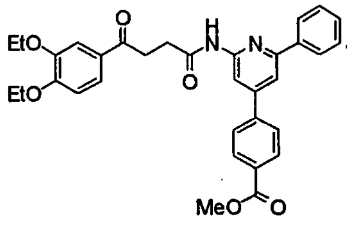

To a solution of N-(4-chloro-6-phenylpyridin-2-yl)-4-(3,4-diethoxyphenyl)-4-oxobutanamide obtained in Example 43-(3) (190 mg) in toluene (5 mL) were added tris(dibenzylideneacetone)dipalladium (9 mg), biphenyl-2-yl(dicyclohexyl)phosphine (22 mg), aqueous potassium phosphate solution (267 mg) and [4-(methoxycarbonyl)phenyl]boronic acid (151 mg), and the mixture was heated overnight at 100°C under nitrogen atmosphere. Water was added to the reaction mixture, and the mixture was extracted with ethyl acetate. The obtained organic layer was washed with 1N hydrochloric acid, saturated aqueous sodium hydrogencarbonate solution and saturated brine, dried over anhydrous magnesium sulfate, and concentrated under reduced pressure. The obtained crystals were recrystallized from ethyl acetate-hexane to give methyl 4-(2-{[4-(3,4-diethoxyphenyl)-4-oxobutanoyl]amino}-6-phenylpyridin-4-yl)benzoate (131 mg) as crystals.

$^1$H-NMR ($CDCl_3$) δ: 1.43 - 1.54 (6 H, m, J=8.76, 7.02, 7.02 Hz), 2.90 (2 H, t, J=6.41 Hz), 3.44 (2 H, t, J=6.41 Hz), 3.95 (3 H, s), 4 .10 - 4.21 (4 H, m, J=7.16, 7.16, 7.16, 7.16 Hz), 6.89 (1 H, d, J=8.48 Hz), 7.42 - 7.57 (4 H, m), 7.61 - 7.72 (2 H, m), 7.78 (2 H, d, J=8.48 Hz), 7.99 - 8.10 (2 H, m), 8.13 (2 H, d, J=8.48 Hz), 8.35 (1 H, s), 8.44 (1 H, s)

Example 113

4-(2-{[4-(3,4-diethoxyphenyl)-4-oxobutanoyl]amino}-6-phenylpyridin-4-yl)benzoic acid

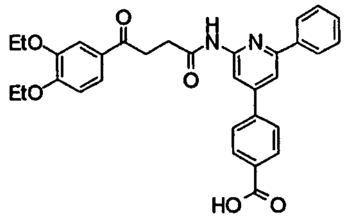

A mixture of methyl 4-(2-{[4-(3,4-diethoxyphenyl)-4-oxobutanoyl]amino}-6-phenylpyridin-4-yl)benzoate obtained in Example 112 (80 mg), 2N aqueous sodium hydroxide solution (0.4 mL), tetrahydrofuran (5 mL) and methanol (5 mL) was stirred at room temperature for 2 hr. The reaction mixture was concentrated under reduced pressure, and the residue was diluted with water. The mixture was acidified with 1N hydrochloric acid, and extracted with ethyl acetate. The extract was washed with water and saturated brine, dried over magnesium sulfate, and concentrated under reduced pressure. The obtained residue was recrystallized from ethyl acetate to give 4-(2-{[4-(3,4-diethoxyphenyl)-4-oxobutanoyl]amino}-6-phenylpyridin-4-yl)benzoic acid (56 mg) as white crystals.

$^1$H NMR (DMSO-$d_6$) δ: 1 .30 - 1.40 (6 H, m), 2.87 (2 H, t), 3.27 - 3.38 (2 H, m), 4.02 - 4.17 (4 H, m), 7.08 (1 H, d, J=8.48 Hz), 7.45 - 7.57 (4 H, m), 7.67 (1 H, dd, J=8.48, 1.88 Hz), 7.92 - 8.01 (3 H, m), 8. 04 - 8.13 (2 H, m), 8.19 - 8.27 (2 H, m), 8.39 (1 H, s), 10.77 (1 H, s), 12.71 - 13.52 (1 H, m)

Example 114

4-(2,3-dihydro-1-benzofuran-5-yl)-4-oxo-N-(6'-phenyl-3,4'-bipyridine-2'-yl)butanamide

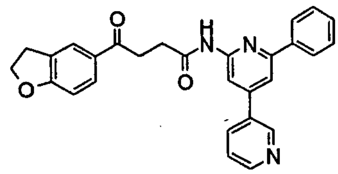

(1) N-(4-chloro-6-phenylpyridin-2-yl)-4-(2,3-dihydro-1-benzofuran-5-yl)-4-oxobutanamide

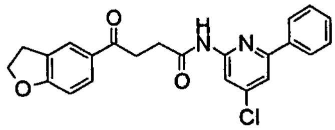

A solution of potassium 4-(2,3-dihydro-1-benzofuran-5-yl) -4,4-dimethoxybutanoate obtained in Example 104-(1) (8.3 g), triethylamine (7.7 mL) and 2,4,6-trichlorobenzoyl chloride (5.1 mL) in THF (80 mL) was stirred at 40°C for 20 hr. 4-Chloro-6-phenylpyridin-2-amine obtained in Example 43-(2) (2.4 g) was added to the reaction mixture, and the mixture was stirred overnight at 50°C. 2N Hydrochloric acid was added to the reaction mixture, and the mixture was extracted with ethyl acetate. The organic layer was washed with saturated aqueous sodium hydrogencarbonate solution and saturated brine, dried over anhydrous sodium sulfate, and filtered, and the solvent was evaporated. The residue was purified by silica gel column chromatography [developing solvent: n-hexane-ethyl acetate (9:1→1:1)] to give N-(4-chloro-6-phenylpyridin-2-yl)-4-(2,3-dihydro-1-benzofuran-5-yl)-4-oxobutanamide (2.3 g) as white crystals.

$^1$H-NMR ($CDCl_3$) δ: 2.87 (2 H, t, J=6.41 Hz), 3.25 (2 H, t, J=8.67 Hz), 3.39 (2 H, t, J-6.31 Hz), 4.67 (2 H, t, J=8.85 Hz), 6.82 (1 H, d, J=8.29 Hz), 7.43 - 7.51 (4 H, m), 7.84 - 7.96 (4 H, m), 8.21 (1 H, d, J=1.32 Hz), 8.34 (1 H, s)

(2) 4-(2,3-dihydro-1-benzofuran-5-yl)-4-oxo-N-(6' -phenyl-3,4' - bipyridine-2'-yl)butanamide

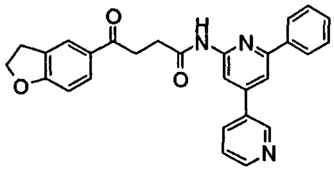

To a solution of N-(4-chloro-6-phenylpyridin-2-yl)-4-(2,3-dihydro-1-benzofuran-5-yl)-4-oxobutanamide obtained in Example 114-(1) (200 mg) in DME (3 mL) were added tris(dibenzylideneacetone)dipalladium (92 mg), biphenyl-2-yl(dicyclohexyl)phosphine (105 mg), 2N aqueous potassium carbonate solution (0.2 mL) and 3-pyridylboronic acid (122 mg), and the mixture was reacted using microwave (250 W) at 150°C for 5 min. Water was added to the reaction mixture, and the mixture was extracted with chloroform. The obtained organic layer was washed with water and saturated brine, dried over anhydrous magnesium sulfate, and concentrated under reduced pressure. The residue was purified by silica gel column chromatography [developing solvent: n-hexane-ethyl acetate (9:1→1:1)], and the obtained crystals were recrystallized from chloroform to give 4-(2,3-dihydro-1-benzofuran-5-yl)-4-oxo-N-(6'-phenyl-3,4'-bipyridine-2'-yl) butanamide (78 mg) as crystals.

$^1$H-NMR ($CDCl_3$) δ: 2.91 (2 H, t, J=6.41 Hz), 3.26 (2 H, t, J=8.85 Hz), 3.42 (2 H, t, J=6. 31 Hz), 4.67 (2 H, t, J=8.76 Hz), 6.82 (1 H, d, J=8.29 Hz), 7.39 - 7.54 (4 H, m), 7.67 (1 H, d, J=1.32 Hz), 7.86 - 7.92 (2 H, m), 7 .96 - 8.09 (3 H, m), 8.39 (2 H, d, J=12.43 Hz), 8.68 (1 H, dd, J=4.90, 1.51 Hz), 8.96 (1 H, d, J=2.26 Hz)

Example 115

4-(2,3-dihydro-1-benzofuran-5-yl)-N-[4-(3-furyl)-6-phenylpyridin-2-yl]-4-oxobutanamide

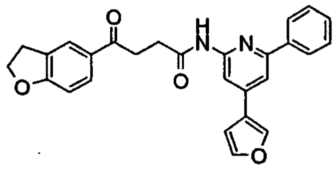

To a solution of N-(4-chloro-6-phenylpyridin-2-yl)-4-(2,3-dihydro-1-benzofuran-5-yl)-4-oxobutariamide obtained in Example 114-(1) (200 mg) in DME (1.5 mL) were added tris(dibenzylideneacetone)dipalladium (11 mg), biphenyl-2-yl(dicyclohexyl)phosphine (14 mg), 2N aqueous potassium carbonate solution (0.2 mL) and 3-furylboronic acid (112 mg), and the mixture was reacted using microwave (250 W) at 150°C for 5 min. Water was added to the reaction mixture, and the mixture was extracted with chloroform. The obtained organic layer was washed with water and saturated brine, dried over anhydrous magnesium sulfate, and concentrated under reduced pressure. The residue was purified by silica gel column chromatography [developing solvent: n-hexane-ethyl acetate (9:1→1:1)], and the obtained crystals were recrystallized from chloroform to give 4-(2,3-dihydro-1-benzofuran-5-yl)-N-[4-(3-furyl)-6-phenylpyridin-2-yl]-4-oxobutanamide (80 mg) as crystals.
$^1$H-NMR ($CDCl_3$) δ: $^1$H-NMR (300 MHz, CHLOROFORM-d) δppm 2.89 (2 H, t, J=6.50 Hz), 3.26 (2 H, t, J=8.76 Hz), 3.41 (2 H, t, J=6.50 Hz), 4.67 (2 H, t, J=8.76 Hz), 6.80 - 6.84 (2 H, m), 7.41 - 7.55 (5 H, m), 7.86 - 8.00 (5 H, m), 8.28 (2 H, s)

Example 116

4-(2,3-dihydro-1-benzofuran-5-yl)-4-oxo-N-(6-phenyl-4,4'-bipyridin-2-yl)butanamide

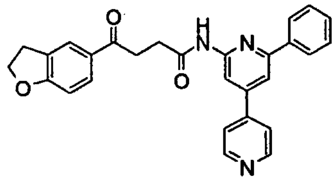

To a solution of N-(4-chloro-6-phenylpyridin-2-yl)-4-(2,3-dihydro-1-benzofuran-5-yl)-4-oxobutanamide obtained in Example 114-(1) (200 mg) in DME (2.5 mL) were added tris(dibenzylideneacetone)dipalladium (23 mg), biphenyl-2-yl(dicyclohexyl)phosphine (26 mg), 2N aqueous potassium carbonate solution (0.2 mL) and 4-pyridylboronic acid (122 mg), and the mixture was reacted using microwave (250 W) at 150°C for 5 min. Water was added to the reaction mixture, and the mixture was extracted with chloroform. The obtained organic layer was washed with water and saturated brine, dried over anhydrous magnesium sulfate, and concentrated under reduced pressure. The residue was purified by silica gel column chromatography [developing solvent: n-hexane-ethyl acetate (9:1→1:1)], and the obtained crystals were recrystallized from chloroform to give 4-(2,3-dihydro-1-benzofuran-5-yl)-4-oxo-N-(6-phenyl-4,4'-bipyridin-2-yl)butanamide (29 mg) as crystals.
$^1$H-NMR ($CDCl_3$) δ: 2.91 (2 H, t, J=6.31 Hz), 3.25 (2 H, t, J=8.76 Hz), 3.42 (2 H, t, J=6.41 Hz), 4.67 (2 H, t, J=8.76 Hz), 6.82 (1 H, d, J=8.29 Hz), 7.42 - 7.55 (3 H, m), 7.58 - 7.64 (2 H, m), 7.68 (1 H, d, J=1.32 Hz), 7.78 - 7.96 (2 H, m), 7.97 - 8.05 (2 H, m), 8.39 - 8.50 (2 H, m), 8.70 - 8.77 (2 H, m)

Example 117

4-(3,4-diethoxyphenyl)-N-[4-(3-furyl)-6-phenylpyridin-2-yl]-4-oxobutanamide

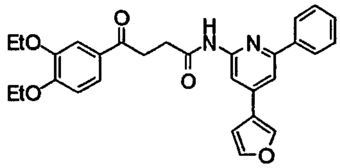

To a solution of N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg) and biphenyl-2-yl(dicyclohexyl)phosphine (1.8 mg) in DME (1 mL) were added 1N aqueous potassium carbonate solution (0.1 mL) and 3-furylboronic acid (14.0 mg), and the mixture was reacted using microwave (250 W) at 150°C for 5 min. Water was added to the reaction mixture, and the mixture was extracted with ethyl acetate. The organic layer was washed with water and saturated brine, and concentrated under reduced pressure. The obtained residue was subjected to a reversed-phase preparative high performance liquid chromatography (Gilson UniPoint System, Column Capcellpak C18 UG120 20 x 50 mm, manufactured by

SHISEIDO), and the fraction eluted with acetonitrile (containing 0.1 % trifluoroacetic acid)-water (20:80, v/v) was concentrated under reduced pressure to give 4-(3,4-diethoxyphenyl)-N-[4-(3-furyl)-6-phenylpyridin-2-yl]-4-oxobutanamide (3.7 mg). HPLC (220 nm) purity 89% (retention time 1.98 min)
MS (ESI+, m/e) 485 (M+H)

Example 118

tert-butyl 2-(2-{[4-(3,4-diethoxyphenyl)-4-oxobutanoyl]amino}-6-phenylpyridin-4-yl)-1H-pyrrole-1-carboxylate

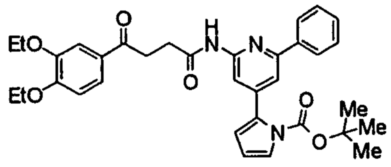

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [1-(tert-butoxycarbonyl)-1H-pyrrol-2-yl] boronic acid (26.4 mg) as starting materials and in the same manner as in Example 117, tert-butyl 2-(2-{[4-(3,4-diethoxyphenyl)-4-oxobutanoyl]amino}-6-phenylpyridin-4-yl)-1H-pyrrole-1-carboxylate (1.6 mg) was obtained.
HPLC (220 nm) purity 84% (retention time 2.17 min)
MS (ESI+, m/e) 584 (M+H)

Example 119

4-(3,4-diethoxyphenyl)-N-[4-(1-methyl-1H-pyrazol-4-yl)-6-phenylpyridin-2-yl]-4-oxobutanamide

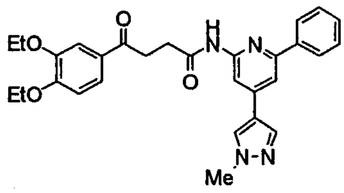

To a solution of N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg) and biphenyl-2-yl(dicyclohexyl)phosphine (1.8 mg) in DME (1 mL) were added 1N aqueous potassium carbonate solution (0.1 mL) and 1-methyl-4-(4,4,5,5-tetramethyl-1,3,2-dioxaborolan-2-yl)-1H-pyrazole (26.0 mg), and the mixture was reacted using microwave (250 W) at 150°C for 5 min. Water was added to the reaction mixture, and the mixture was extracted with ethyl acetate. The organic layer was washed with water and saturated brine, and concentrated under reduced pressure. The obtained residue was subject to a reversed-phase preparative high performance liquid chromatography (Gilson, UniPoint System, Column Capcellpak C18 UG120 20 x 50 mm, manufactured by SHISEIDO), and the fraction eluted with acetonitrile (containing 0.1% formic acid)-water (20:80, v/v) was concentrated under reduced pressure to give 4-(3,4-diethoxyphenyl)-N-[4-(1-methyl-1H-pyrazol-4-yl)-6-phenylpyridin-2-yl]-4-oxobutanamide (6.9 mg). HPLC (220 nm) purity 97% (retention time 1.75 min)
MS (ESI+, m/e) 499 (M+H)

Example 120

N-[4-(1,3-benzodioxol-5-yl)-6-phenylpyridin-2-yl]-4-(3,4-diethoxyphenyl)-4-oxobutanamide

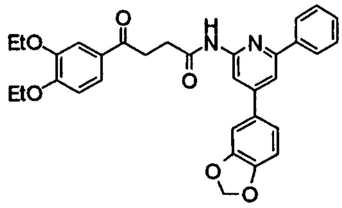

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (1,3-benzodioxol-5-yl)boronic acid (20.7 mg) as starting materials and in the same manner as in Example 119, N-[4-(1,3-benzodioxol-5-yl)-6-phenylpyridin-2-yl]-4-(3,4-diethoxyphenyl)-4-oxobutanamide (9.7 mg) was obtained. HPLC (220 nm) purity 98% (retention time 2.04 min)
MS (ESI+, m/e) 539 (M+H)

Example 121

4-(3,4-diethoxyphenyl)-N-[4-(1H-indol-5-yl)-6-phenylpyridin-2-yl]-4-oxobutanamide

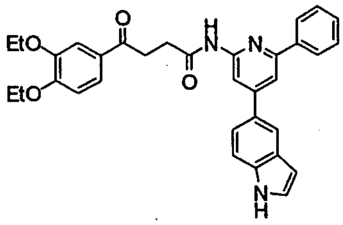

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and 5-(4,4,5,5-tetramethyl-1,3,2-dioxaborolan-2-yl)-1H-indole (30.3 mg) as starting materials and in the same manner as in Example 119, 4-(3,4-diethoxyphenyl)-N-[4-(1H-indol-5-yl)-6-phenylpyridin-2-yl]-4-oxobutanamide (7.1 mg) was obtained.
HPLC (220 nm) purity 89% (retention time 1.90 min)
MS (ESI+, m/e) 534 (M+H)

Example 122

N-[4-(4-cyanophenyl)-6-phenylpyridin-2-yl]-4-(3,4-diethoxyphenyl)-4-oxobutanamide

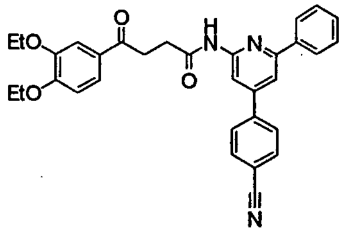

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous

potassium carbonate solution (0.1 mL) and (4-cyanophenyl)boronic acid (18.3 mg) as starting materials and in the same manner as in Example 119, N-[4-(4-cyanophenyl)-6-phenylpyridin-2-yl]-4-(3,4-diethoxyphenyl)-4-oxobutanamide (6.8 mg) was obtained.
HPLC (220 nm) purity 99% (retention time 2.12 min)
MS (ESI+, m/e) 520 (M+H)

Example 123

4-(3,4-diethoxyphenyl)-N-[4-(4-hydroxyphenyl)-6-phenylpyridin-2-yl]-4-oxobutanamide

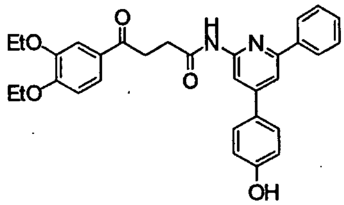

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (4-hydroxyphenyl)boronic acid (17.2 mg) as starting materials and in the same manner as in Example 119, 4-(3,4-diethoxyphenyl)-N-[4-(4-hydroxyphenyl)-6-phenylpyridin-2-yl]-4-oxobutanamide (4.6 mg) was obtained.
HPLC (220 nm) purity 82% (retention time 2.12 min)
MS (ESI+, m/e) 511 (M+H)

Example 124

4-(3,4-diethoxyphenyl)-N-{4-[4-(hydroxymethyl)phenyl]-6-phenylpyridin-2-yl}-4-oxobutanamide

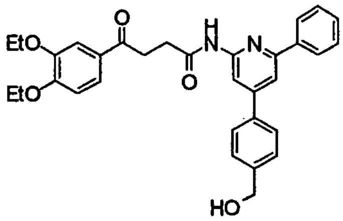

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (4-hydroxymethylphenyl)boronic acid (19.0 mg) as starting materials and in the same manner as in Example 119, 4-(3,4-diethoxyphenyl)-N-{4-[4-(hydroxymethyl)phenyl]-6-phenylpyridin-2-yl}-4-oxobutanamide (11.7 mg) was obtained.
HPLC (220 nm) purity 95% (retention time 1.89 min)
MS (ESI+, m/e) 525 (M+H)

Example 125

3-[4-(2-{[4-(3,4-diethoxyphenyl)-4-oxobutanoyl]amino}-6-phenylpyridin-4-yl)phenyl]propanoic acid

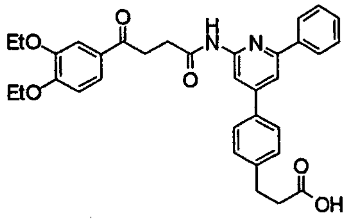

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [4-(2-carboxyethyl)phenyl]boronic acid (24.2 mg) as starting materials and in the same manner as in Example 119, 3-[4-(2-{[4-(3,4-diethoxyphenyl)-4-oxobutanoyl]amino}-6-phenylpyridin-4-yl)phenyl]propanoic acid (2.9 mg) was obtained.
HPLC (220 nm) purity 80% (retention time 1.92 min)
MS (ESI+, m/e) 567 (M+H)

Example 126

methyl 3-[4-(2-{[4-(3,4-diethoxyphenyl)-4-oxobutanoyl]amino}-6-phenylpyridin-4-yl)phenyl]propanoate

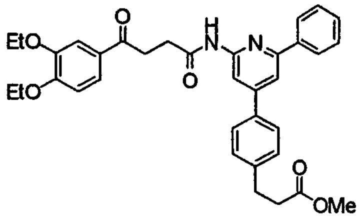

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [4-(2-methoxycarboxyethyl)phenyl]boronic acid (26.0 mg) as starting materials and in the same manner as in Example 119, methyl 3-[4-(2-{[4-(3,4-diethoxyphenyl)-4-oxobutanoyl]amino}-6-phenylpyridin-4-yl)phenyl]propanoate (11.1 mg) was obtained. HPLC (220 nm) purity 97% (retention time 2.08 min)
MS (ESI+, m/e) 581 (M+H)

Example 127

methyl (2E)-3-[4-(2-{[4-(3,4-diethoxyphenyl)-4-oxobutanoyl]amino}-6-phenylpyridin-4-yl)phenyl]acrylate

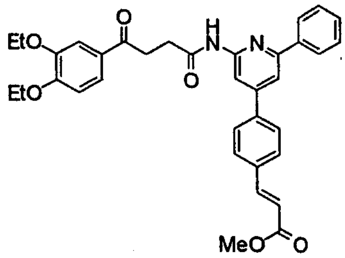

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [4-(E-3-methoxy-3-oxo-1-propen-1-yl)phenyl]boronic acid (25.8 mg) as starting materials and in the same manner as in Example 119, methyl (2E)-3-[4-(2-{[4-(3,4-diethoxyphenyl)-4-oxobutanoyl]amino}-6-phenylpyridin-4-yl)phenyl]acrylate (15.8 mg) was obtained.
HPLC (220 nm) purity 96% (retention time 2.16 min)
MS (ESI+, m/e) 579 (M+H)

Example 128

4-(2-{[4-(3,4-diethoxyphenyl)-4-oxobutanoyl]amino}-6-phenylpyridin-4-yl)benzamide

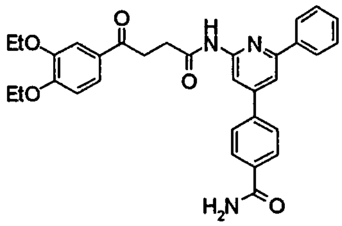

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (4-aminocarbonylphenyl)boronic acid (20.6 mg) as starting materials and in the same manner as in Example 119, 4-(2-{[4-(3,4-diethoxyphenyl)-4-oxobutanoyl]amino}-6-phenylpyridin-4-yl)benzamide (10.5 mg) was obtained.
HPLC (220 nm) purity 99% (retention time 1.85 min)
MS (ESI+, m/e) 538 (M+H)

Example 129

4-(3,4-diethoxyphenyl)-N-[4-(4-(morpholin-4-yl)phenyl)-6-phenylpyridin-2-yl]-4-oxobutanamide

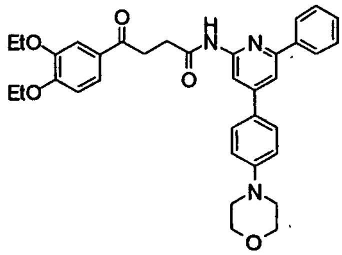

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and 4-[4-(4,4,5,5-tetramethyl-1,3,2-dioxaborolan-2-yl)phenyl]morpholine (36.1 mg) as starting materials and in the same manner as in Example 119, 4-(3,4-diethoxyphenyl)-N-[4-(4-(morpholin-4-yl)phenyl)-6-phenylpyridin-2-yl]-4-oxobutanamide (12.8 mg) was obtained.
HPLC (220 nm) purity 90% (retention time 1.93 min)
MS (ESI+, m/e) 580 (M+H)

Example 130

4-(3,4-diethoxyphenyl)-N-{4-[4-(morpholin-4-ylmethyl)phenyl]-6-phenylpyridin-2-yl}-4-oxobutanamide

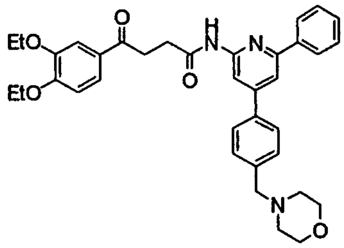

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and 4-[4-(4,4,5,5-tetramethyl-1,3,2-dioxaborolan-2-yl)benzyl]morpholine (37.9 mg) as starting materials and in the same manner as in Example 119, 4-(3,4-diethoxyphenyl)-N-{4-[4-(morpholin-4-ylmethyl)phenyl]-6-phenylpyridin-2-yl}-4-oxobutanamide (11.0 mg) was obtained.
HPLC (220 nm) purity 96% (retention time 1.67 min)
MS (ESI+, m/e) 594 (M+H)

Example 131

4-(3,4-diethoxyphenyl)-N-{4-[4-(dimethylamino)phenyl]-6-phenylpyridin-2-yl}-4-oxobutanamide

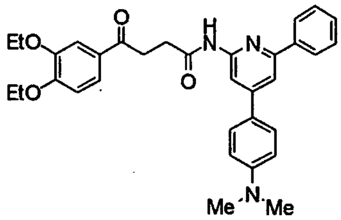

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (4-dimethylaminophenyl)boronic acid (20.6 mg) as starting materials and in the same manner as in Example 119, 4-(3,4-diethoxyphenyl)-N-{4-[4-(dimethylamino)phenyl]-6-phenylpyridin-2-yl}-4-oxobutanamide (3.9 mg) was obtained.
HPLC (220 nm) purity 92% (retention time 1.90 min)
MS (ESI+, m/e) 538 (M+H)

Example 132

N-[4-(3-cyanophenyl)-6-phenylpyridin-2-yl]-4-(3,4-diethoxyphenyl)-4-oxobutanamide

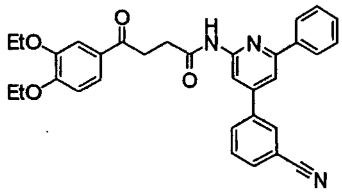

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3-cyanophenyl)boronic acid (18.4 mg) as starting materials and in the same manner as in Example 119, N-[4-(3-cyanophenyl)-6-phenylpyridin-2-yl]-4-(3,4-diethoxyphenyl)-4-oxobutanamide (15.4 mg) was obtained.
HPLC (220 nm) purity 90% (retention time 2.11 min)
MS (ESI+, m/e) 520 (M+H)

Example 133

N-{4-[3-(cyanomethyl)phenyl]-6-phenylpyridin-2-yl}-4-(3,4-diethoxyphenyl)-4-oxobutanamide

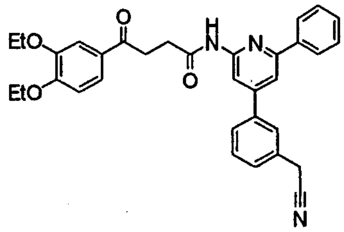

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(4,4,5,5-tetramethyl-1,3,2-dioxaborolan-2-yl)phenyl]acetonitrile (30.4 mg) as starting materials and in the same manner as in Example 119, N-{4-[3-(cyanomethyl)phenyl]-6-phenylpyridin-2-yl}-4-(3,4-diethoxyphenyl)-4-oxobutanamide (12.0 mg) was obtained.
HPLC (220 nm) purity 81% (retention time 2.04 min)
MS (ESI+, m/e) 534 (M+H)

Example 134

4-(3,4-diethoxyphenyl)-N-[4-(3-hydroxyphenyl)-6-phenylpyridin-2-yl]-4-oxobutanamide

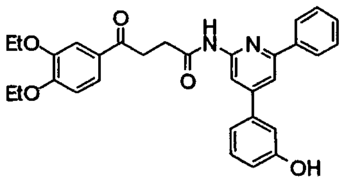

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3-hydroxyphenyl)boronic acid (17.2 mg) as starting materials and in the

same manner as in Example 119, 4-(3,4-diethoxyphenyl)-N-[4-(3-hydroxyphenyl)-6-phenylpyridin-2-yl]-4-oxobutanamide (11.0 mg) was obtained.
HPLC (220 nm) purity 91% (retention time 1.94 min)
MS (ESI+, m/e) 511 (M+H)

Example 135

N-[4-(3-butoxyphenyl)-6-phenylpyridin-2-yl]-4-(3,4-diethoxyphenyl)-4-oxobutanamide

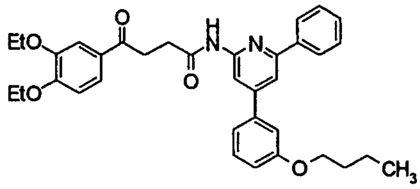

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3-butoxyphenyl)boronic acid (24.3 mg) as starting materials and in the same manner as in Example 119, N-[4-(3-butoxyphenyl)-6-phenylpyridin-2-yl]-4-(3,4-diethoxyphenyl)-4-oxobutanamide (14.9 mg) was obtained.
HPLC (220 nm) purity 96% (retention time 2.for 35 min)
MS (ESI+, m/e) 567 (M+H)

Example 136

4-(3,4-diethoxyphenyl)-N-{4-[3-(hydroxymethyl)phenyl]-6-phenylpyridin-2-yl}-4-oxobutanamide

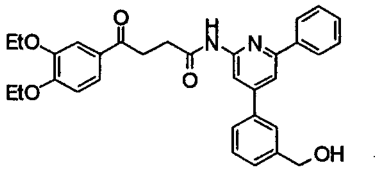

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3-hydroxymethylphenyl)boronic acid (19.0 mg) as starting materials and in the same manner as in Example 119, 4-(3,4-diethoxyphenyl)-N-{4-[3-(hydroxymethyl)phenyl]-6-phenylpyridin-2-yl}-4-oxobutanamide (15.6 mg) was obtained.
HPLC (220 nm) purity 98% (retention time 1.91 min)
MS (ESI+, m/e) 525 (M+H)

Example 137

4-(3,4-diethoxyphenyl)-N-{4-[3-(3-hydroxypropyl)phenyl]-6-phenylpyridin-2-yl}-4-oxobutanamide

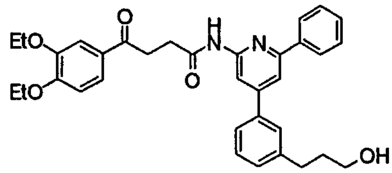

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(3-hydroxypropyl)phenyl]boronic acid (22.5 mg) as starting materials and in the same manner as in Example 119, 4-(3,4-diethoxyphenyl)-N-{4-[3-(3-hydroxypropyl)phenyl]-6-phenylpyridin-2-yl}-4-oxobutanamide (11.3 mg) was obtained.
HPLC (220 nm) purity 100% (retention time 2.22 min)
MS (ESI+, m/e) 553 (M+H)

Example 138

3-(2-{[4-(3,4-diethoxyphenyl)-4-oxobutanoyl]amino}-6-phenylpyridin-4-yl)benzoic acid

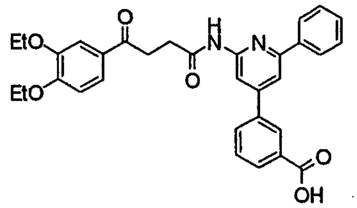

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and 3-(dihydroxyboryl)benzoic acid (20.7 mg) as starting materials and in the same manner as in Example 119, 3-(2-{[4-(3,4-diethoxyphenyl)-4-oxobutanoyl]amino}-6-phenylpyridin-4-yl)benzoic acid (3.4 mg) was obtained.
HPLC (220 nm) purity 86% (retention time 2.16 min)
MS (ESI+, m/e) 539 (M+H)

Example 139

methyl 3-(2-{[4-(3,4-diethoxyphenyl)-4-oxobutanoyl]amino}-6-phenylpyridin-4-yl)benzoate

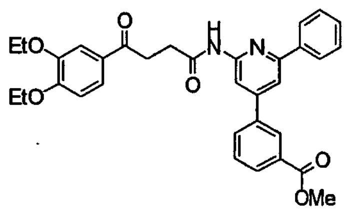

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(methoxycarbonyl)phenyl]boronic acid (22.5 mg) as starting materials and in the same manner as in Example 119, methyl 3-(2-{[4-(3,4-diethoxyphenyl)-4-oxobutanoyl]amino}-6-phenylpyridin-4-yl)benzoate (13.9 mg) was obtained.
HPLC (220 nm) purity 97% (retention time 2.14 min)
MS (ESI+, m/e) 553 (M+H)

Example 140

N-[4-(3-acetylphenyl)-6-phenylpyridin-2-yl]-4-(3,4-diethoxyphenyl)-4-oxobutanamide

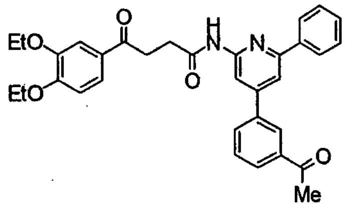

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3-acetylphenyl)boronic acid (20.5 mg) as starting materials and in the same manner as in Example 119, N-[4-(3-acetylphenyl)-6-phenylpyridin-2-yl]-4-(3,4-diethoxyphenyl)-4-oxobutanamide (14.1 mg) was obtained.
HPLC (220 nm) purity 96% (retention time 2.08 min)
MS (ESI+, m/e) 537 (M+H)

Example 141

3-[3-(2-{[4-(3,4-diethoxyphenyl)-4-oxobutanoyl]amino}-6-phenylpyridin-4-yl)phenyl]propanoic acid

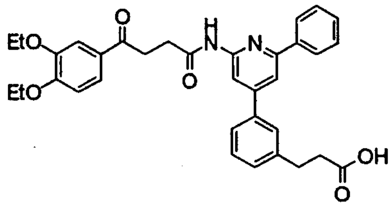

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(2-carboxyethyl)phenyl]boronic acid (24.2 mg) as starting materials and in the same manner as in Example 119, 3-[3-(2-{[4-(3,4-diethoxyphenyl)-4-oxobutanoyl]amino}-6-phenylpyridin-4-yl)phenyl]propanoic acid (11.6 mg) was obtained.
HPLC (220 nm) purity 91% (retention time 1.95 min)
MS (ESI+, m/e) 567 (M+H)

Example 142

methyl 3-[3-(2-{[4-(3,4-diethoxyphenyl)-4-oxobutanoyl]amino}-6-phenylpyridin-4-yl)phenyl]propanoate

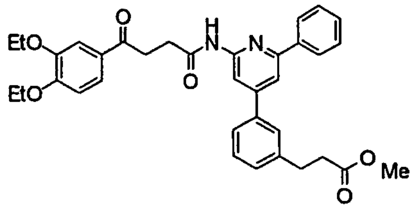

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(2-methoxycarboxyethyl)phenyl]boronic acid (26.0 mg) as starting materials and in the same manner as in Example 119, methyl 3-[3-(2-{[4-(3,4-diethoxyphenyl)-4-oxobutanoyl]amino}-6-phenylpyridin-4-yl)phenyl]propanoate (16.7 mg) was obtained. HPLC (220 nm) purity 93% (retention time 2.11 min)
MS (ESI+, m/e) 581 (M+H)

Example 143

4-(3,4-diethoxyphenyl)-4-oxo-N-{6-phenyl-4-[3-(trifluoromethoxy)phenyl]pyridin-2-yl}butanamide

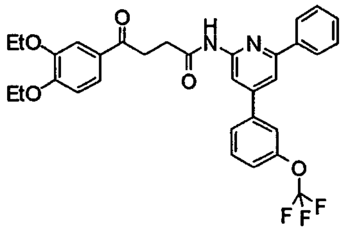

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(trifluoromethoxy)phenyl]boronic acid (25.7 mg) as starting materials and in the same manner as in Example 119, 4-(3,4-diethoxyphenyl)-4-oxo-N-{6-phenyl-4-[3-(trifluoromethoxy)phenyl]pyridin-2-yl}butanamide (15.0 mg) was obtained.
HPLC (220 nm) purity 94% (retention time 2.28 min)
MS (ESI+, m/e) 579 (M+H)

Example 144

N-[4-(2-cyanophenyl)-6-phenylpyridin-2-yl]-4-(3,4-diethoxyphenyl)-4-oxobutanamide

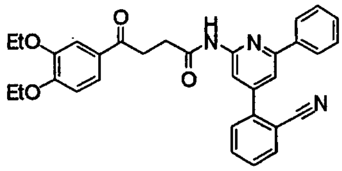

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (2-cyanophenyl)boronic acid (18.4 mg) as starting materials and in the same

manner as in Example 119, N-[4-(2-cyanophenyl)-6-phenylpyridin-2-yl]-4-(3,4-diethoxyphenyl)-4-oxobutanamide (9.0 mg) was obtained.
HPLC (220 nm) purity 94% (retention time 2.10 min)
MS (ESI+, m/e) 520 (M+H)

Example 145

4-(3,4-diethoxyphenyl)-N-[4-(2-hydroxyphenyl)-6-phenylpyridin-2-yl]-4-oxobutanamide

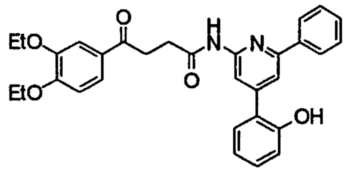

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (2-hydroxyphenyl)boronic acid (17.2 mg) as starting materials and in the same manner as in Example 119, 4-(3,4-diethoxyphenyl)-N-[4-(2-hydroxyphenyl)-6-phenylpyridin-2-yl]-4-oxobutanamide (5.1 mg) was obtained.
HPLC (220 nm) purity 92% (retention time 1.89 min)
MS (ESI+, m/e) 511 (M+H)

Example 146

4-(3,4-diethoxyphenyl)-N-[4-(2-methoxyphenyl)-6-phenylpyridin-2-yl]-4-oxobutanamide

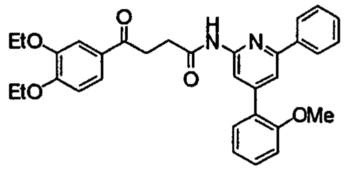

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (2-methoxyphenyl)boronic acid (19.0 mg) as starting materials and in the same manner as in Example 119, 4-(3,4-diethoxyphenyl)-N-[4-(2-methoxyphenyl)-6-phenylpyridin-2-yl]-4-oxobutanamide (7.5 mg) was obtained.
HPLC (220 nm) purity 95% (retention time 2.05 min)
MS (ESI+, m/e) 525 (M+H)

Example 147

4-(3,4-diethoxyphenyl)-N-{4-[2-(hydroxymethyl)phenyl]-6-phenylpyridin-2-yl}-4-oxobutanamide

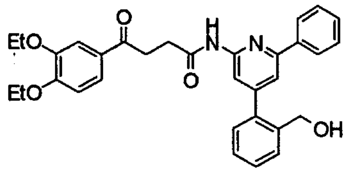

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and [2-(hydroxymethyl)phenyl]boronic acid (19.0 mg) as starting materials and in the same manner as in Example 119, 4-(3,4-diethoxyphenyl)-N-{4-[2-(hydroxymethyl)phenyl]-6-phenylpyridin-2-yl}-4-oxobutanamide (12.0 mg) was obtained.
HPLC (220 nm) purity 98% (retention time 1.95 min)
MS (ESI+, m/e) 525 (M+H)

Example 148

2-(2-{[4-(3,4-diethoxyphenyl)-4-oxobutanoyl]amino}-6-phenylpyridin-4-yl)benzoic acid

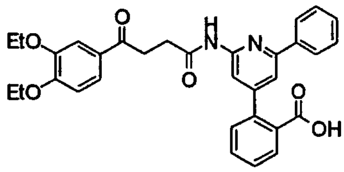

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and 2-(dihydroxyboryl)benzoic acid (20.7 mg) as starting materials and in the same manner as in Example 119, 2-(2-{[4-(3,4-diethoxyphenyl)-4-oxobutanoyl]amino}-6-phenylpyridin-4-yl)benzoic acid (4.4 mg) was obtained.
HPLC (220 nm) purity 99% (retention time 1.93 min)
MS (ESI+, m/e) 539 (M+H)

Example 149

4-(3,4-diethoxyphenyl)-N-[4-(4-hydroxy-3-methoxyphenyl)-6-phenylpyridin-2-yl]-4-oxobutanamide

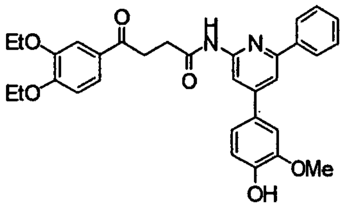

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and 2-(4-hydroxy-3-methoxyphenyl)-4,4,5,5-tetramethyl-1,3,2-dioxaborolan (31.3 mg) as starting materials and in the same manner as in Example 119, 4-(3,4-diethoxyphenyl)-N-[4-(4-hydroxy-3-methoxyphenyl)-6-phenylpyridin-2-yl]-4-oxobutanamide (6.5 mg) was obtained.

HPLC (220 nm) purity 99% (retention time 1.87 min)
MS (ESI+, m/e) 541 (M+H)

Example 150

4-(3,4-diethoxyphenyl)-4-oxo-N-[6-phenyl-4-(3,4,5-trimethoxyphenyl)pyridin-2-yl]butanamide

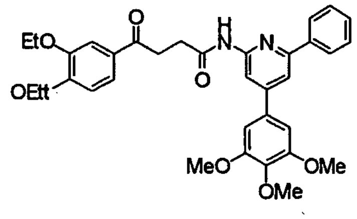

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3,4,5-trimethoxyphenyl)boronic acid (26.5 mg) as starting materials and in the same manner as in Example 119, 4-(3,4-diethoxyphenyl)-4-oxo-N-[6-phenyl-4-(3,4,5-trimethoxyphenyl)pyridin-2-yl] butanamide (16.9 mg) was obtained.
HPLC (220 nm) purity 100% (retention time 2.05 min)
MS (ESI+, m/e) 585 (M+H)

Example 151

4-(3,4-diethoxyphenyl)-N-[4-(4-hydroxy-3,5-dimethylphenyl)-6-phenylpyridin-2-yl]-4-oxobutanamide

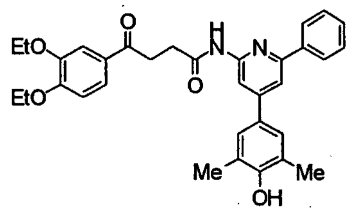

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and 2,6-dimethyl-4-(4,4,5,5-tetramethyl-1,3,2-dioxaborolan-2-yl)phenol (31.0 mg) as starting materials and in the same manner as in Example 119, 4-(3,4-diethoxyphenyl)-N-[4-(4-hydroxy-3,5-dimethylphenyl)-6-phenylpyridin-2-yl]-4-oxobutanamide (12.3 mg) was obtained.
HPLC (220 nm) purity 84% (retention time 1.93 min)
MS (ESI+, m/e) 539 (M+H)

Example 152

4-(3,4-diethoxyphenyl)-N-{4-[(1E)-octa-1-en-1-yl]-6-phenylpyridin-2-yl}-4-oxobutanamide

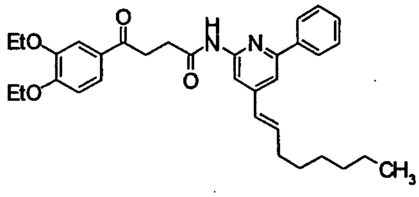

Using N-(4-chloro-6-phenylpyridin-2-yl)-4-(3,4-diethoxyphenyl)-4-oxobutanamide obtained in Example 43-(3) (22.6 mg), tris(dibenzylideneacetone)dipalladium (1.1 mg), biphenyl-2-yl(dicyclohexyl)phosphine (1.8 mg), 1N aqueous potassium carbonate solution (0.1 mL) and (1E)-octa-1-en-1-ylboronic acid (19.5 mg) as starting materials and in the same manner as in Example 119, 4-(2,3-dihydro-1-benzofuran-5-yl)-N-{4-[(IE)-octa-1-en-1-yl]-6-phenylpyridin-2-yl}-4-oxobutanamide (5.9 mg) was obtained.
HPLC (220 nm) purity 83% (retention time 2.26 min)
MS (ESI+, m/e) 529 (M+H)

Example 153

4-(2,3-dihydro-1-benzofuran-5-yl)-N-[4-(1-methyl-1H-pyrazol-4-yl)-6-phenylpyridin-2-yl]-4-oxobutanamide

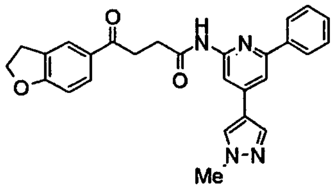

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and 1-methyl-4-(4,4,5,5-tetramethyl-1,3,2-dioxaborolan-2-yl)-1H-pyrazole (26.0 mg) as starting materials and in the same manner as in Example 119, 4-(2,3-dihydro-1-benzofuran-5-yl)-N-[4-(1-methyl-1H-pyrazol-4-yl)-6-phenylpyridin-2-yl]-4-oxobutanamide (2.2 mg) was obtained. HPLC (220 nm) purity 100% (retention time 1.87 min)
MS (ESI+, m/e) 453 (M+H)

Example 154

4-(2,3-dihydro-1-benzofuran-5-yl)-N-[4-(1H-indol-5-yl)-6-phenylpyridin-2-yl]-4-oxobutanamide

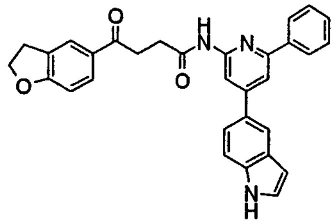

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and 5-(4,4,5,5-tetramethyl-1,3,2-dioxaborolan-2-yl)-1H-indole (30.3 mg) as starting materials and in the same manner as in Example 119, 4-(2,3-dihydro-1-benzofuran-5-yl)-N-[4-(1H-indol-5-yl)-6-phenylpyridin-2-yl]-4-oxobutanamide (5.1 mg) was obtained.

HPLC (220 nm) purity 80% (retention time 1.74 min)
MS (ESI+, m/e) 488 (M+H)

Example 155

4-(2,3-dihydro-1-benzofuran-5-yl)-N-[4-(4-methoxyphenyl)-6-phenylpyridin-2-yl]-4-oxobutanamide

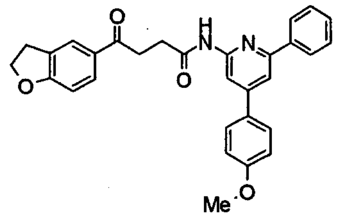

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (4-methoxyphenyl)boronic acid (19.0 mg) as starting materials and in the same manner as in Example 119, 4-(2,3-dihydro-1-benzofuran-5-yl)-N-[4-(4-methoxyphenyl)-6-phenylpyridin-2-yl]-4-oxobutanamide (12.0 mg) was obtained.
HPLC (220 nm) purity 92% (retention time 1.98 min)
MS (ESI+, m/e) 479.(M+H)

Example 156

4-(2,3-dihydro-1-benzofuran-5-yl)-N-{4-[4-(hydroxymethyl)phenyl]-6-phenylpyridin-2-yl}-4-oxobutanamide

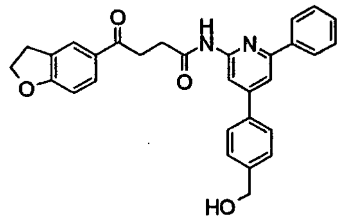

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (4-hydroxymethylphenyl)boronic acid (19.0 mg) as starting materials and in the same manner as in Example 119, 4-(2,3-dihydro-1-benzofuran-5-yl)-N-{4-[4-(hydroxymethyl)phenyl]-6-phenylpyridin-2-yl}-4-oxobutanamide (12.9 mg) was obtained.
HPLC (220 nm) purity 92% (retention time 1.82 min)
MS (ESI+, m/e) 479 (M+H)

Example 157

3-[4-(2-{[4-(2,3-dihydro-1-benzofuran-5-yl)-4-oxobutanoyl]amino}-6-phenylpyridin-4-yl)phenyl]propanoic acid

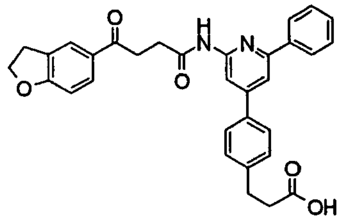

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and [4-(2-carboxyethyl)phenyl]boronic acid (24.2 mg) as starting materials and in the same manner as in Example 119, 3-[4-(2-{[4-(2,3-dihydro-1-benzofuran-5-yl)-4-oxobutanoyl]amino}-6-phenylpyridin-4-yl)phenyl]propanoic acid (5.2 mg) was obtained.
HPLC (220 nm) purity 94% (retention time 1.86 min)
MS (ESI+, m/e) 521 (M+H)

Example 158

methyl 3-[4-(2-{[4-(2,3-dihydro-1-benzofuran-5-yl)-4-oxobutanoyl]amino}-6-phenylpyridin-4-yl)phenyl]propanoate

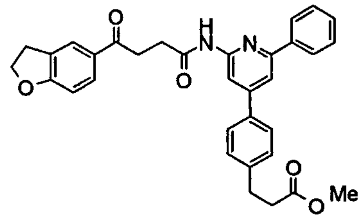

Using N-(4-chloro-6-phenylpyridin-2-yl)=4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and [4-(2-methoxycarboxyethyl)phenyl]boronic acid (26.0 mg) as starting materials and in the same manner as in Example 119, methyl 3-[4-(2-{[4-(2,3-dihydro-1-benzofuran-5-yl)-4-oxobutanoyl]amino}-6-phenylpyridin-4-yl)phenyl]propanoate (14.7 mg) was obtained. HPLC (220 nm) purity 98% (retention time 2.03 min)
MS (ESI+, m/e) 535 (M+H)

Example 159

methyl (2E)-3-[4-(2-{[4-(2,3-dihydro-1-benzofuran-5-yl)-4-oxobutanoyl]amino}-6-phenylpyridin-4-yl)phenyl]acrylate

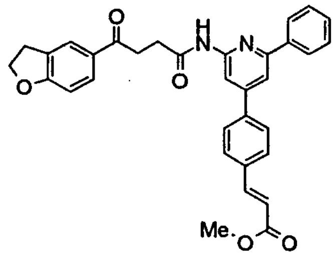

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and [4-(E-3-methoxy-3-oxo-1-propen-1-yl)phenyl]boronic acid (25.8 mg) as starting materials and in the same manner as in Example 119, methyl (2E)-3-[4-(2-{[4-(2,3-dihydro-1-benzofuran-5-yl)-4-oxobutanoyl]amino}-6-phenylpyridin-4-yl)phenyl]acrylate (11.0 mg) was obtained.
HPLC (220 nm) purity 98% (retention time 2.09 min)
MS (ESI+, m/e) 533 (M+H)

Example 160

4-(2,3-dihydro-1-benzofuran-5-yl)-N-[4-(4-(morpholin-4-yl)phenyl)-6-phenylpyridin-2-yl]-4-oxobutanamide

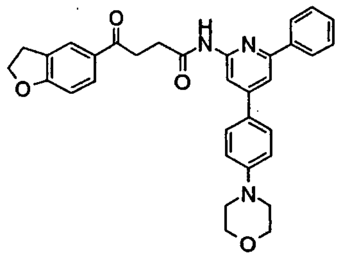

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and 4-[4-(4,4,5,5-tetramethyl-1,3,2-dioxaborolan-2-yl)phenyl] morpholine (36.1 mg) as starting materials and in the same manner as in Example 119, 4-(2,3-dihydro-1-benzofuran-5-yl)-N-[4-(4-(morpholin-4-yl)phenyl)-6-phenylpyridin-2-yl]-4-oxobutanamide (8.6 mg) was obtained.
HPLC (220 nm) purity 89% (retention time 1.86 min)
MS (ESI+, m/e) 534 (M+H)

Example 161

4-(2,3-dihydro-1-benzofuran-5-yl)-N-{4-[4-(morpholin-4-ylmethyl)phenyl]-6-phenylpyridin-2-yl}-4-oxobutanamide

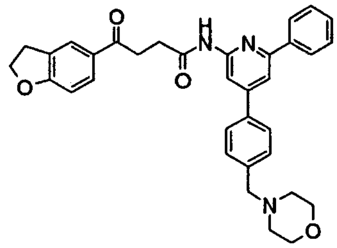

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and 4-[4-(4,4,5,5-tetramethyl-1,3,2-dioxaborolan-2-yl)benzyl] morpholine (37.9 mg) as starting materials and in the same manner as in Example 119,
4-(2,3-dihydro-1-benzofuran-5-yl)-N-{4-[4-(morpholin-4-ylmethyl)phenyl]-6-phenylpyridin-2-yl}-4-oxobutanamide (14.4 mg) was obtained.
HPLC (220 nm) purity 99% (retention time 1.60 min)
MS (ESI+, m/e) 548 (M+H)

Example 162

4-(2,3-dihydro-1-benzofuran-5-yl)-N-{4-[4-(dimethylamino)phenyl]-6-phenylpyridin-2-yl}-4-oxobutanamide

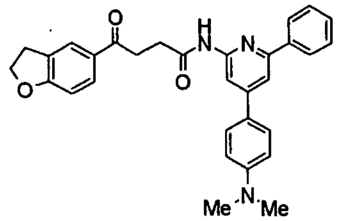

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (4-dimethylaminophenyl)boronic acid (20.6 mg) as starting materials and in the same manner as in Example 119, 4-(2,3-dihydro-1-benzofuran-5-yl)-N-{4-[4-(dimethylamino)phenyl]-6-phenylpyridin-2-yl}-4-oxobutanamide (2.4 mg) was obtained.
HPLC (220 nm) purity 85% (retention time 1.83 min)
MS (ESI+, m/e) 492 (M+H)

Example 163

N-[4-(3-cyanophenyl)-6-phenylpyridin-2-yl]-4-(2,3-dihydro-1-benzofuran-5-yl)-4-oxobutanamide

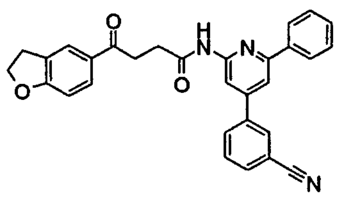

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3-cyanophenyl)boronic acid (18.4 mg) as starting materials and in the same manner as in Example 119, N-[4-(3-cyanophenyl)-6-phenylpyridin-2-yl]-4-(2,3-dihydro-1-benzofuran-5-yl)-4-oxobutanamide (10.5 mg) was obtained.
HPLC (220 nm) purity 98% (retention time 2.04 min)
MS (ESI+, m/e) 474 (M+H)

Example 164

N-{4-[3-(cyanomethyl)phenyl]-6-phenylpyridin-2-yl}-4-(2,3-dihydro-1-benzofuran-5-yl)-4-oxobutanamide

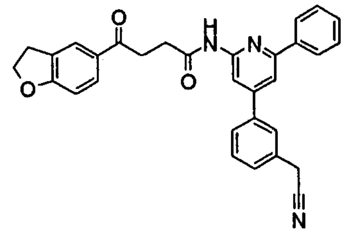

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(4, 4, 5, 5-tetramethyl-1,3,2-dioxaborolan-2-yl)phenyl] acetonitrile (30.4 mg) as starting materials and in the same manner as in Example 119, N-{4-[3-(cyanomethyl)phenyl]-6-phenylpyridin-2-yl}-4-(2,3-dihydro-1-benzofuran-5-yl)-4-oxobutanamide (10.7 mg) was obtained.
HPLC (220 nm) purity 99% (retention time 1.99 min)
MS (ESI+, m/e) 488 (M+H)

Example 165

4-(2,3-dihydro-1-benzofuran-5-yl)-N-[4-(3-hydroxyphenyl)-6-phenylpyridin-2-yl]-4-oxobutanamide

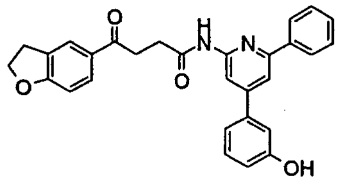

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3-hydroxyphenyl)boronic acid (17.2 mg) as starting materials and in the same manner as in Example 119, 4-(2,3-dihydro-1-benzofuran-5-yl)-N-[4-(3-hydroxyphenyl)-6-phenylpyridin-2-yl]-4-oxobutanamide (11.5 mg) was obtained.
HPLC (220 nm) purity 91% (retention time 1.88 min)
MS (ESI+, m/e) 465 (M+H)

Example 166

N-[4-(3-butoxyphenyl)-6-phenylpyridin-2-yl]-4-(2,3-dihydro-1-benzofuran-5-yl)-4-oxobutanamide

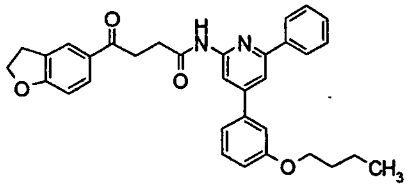

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3-butoxyphenyl)boronic acid (24.3 mg) as starting materials and in the same manner as in Example 119, N-[4-(3-butoxyphenyl)-6-phenylpyridin-2-yl]-4-(2,3-dihydro-1-benzofuran-5-yl)-4-oxobutanamide (11.5 mg) was obtained.
HPLC (220 nm) purity 91% (retention time 2.26 min)
MS (ESI+, m/e) 521 (M+H)

Example 167

4-(2,3-dihydro-1-benzofuran-5-yl)-N-{4-[3-(hydroxymethyl)phenyl]-6-phenylpyridin-2-yl}-4-oxobutanamide

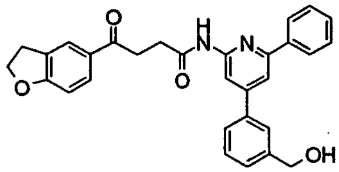

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3-hydroxymethylphenyl)boronic acid (19.0 mg) as starting materials and in the same manner as in Example 119, 4-(2,3-dihydro-1-benzofuran-5-yl)-N-{4-[3-(hydroxymethyl)phenyl]-6-phenylpyridin-2-yl}-4-oxobutanamide (6.8 mg) was obtained.
HPLC (220 nm) purity 99% (retention time 1.84 min)
MS (ESI+, m/e) 479 (M+H)

Example 168

4-(2,3-dihydro-1-benzofuran-5-yl)-N-{4-[3-(3-hydroxypropyl)phenyl]-6-phenylpyridin-2-yl}-4-oxobutanamide

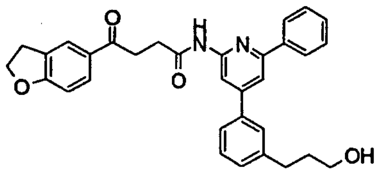

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(3-hydroxypropyl)phenyl]boronic acid (22.5 mg) as starting materials and in the same manner as in Example 119, 4-(2,3-dihydro-1-benzofuran-5-yl)-N-{4-[3-(3-hydroxypropyl)phenyl]-6-phenylpyridin-2-yl}-4-oxobutanamide (5.0 mg) was obtained.
HPLC (220 nm) purity 89% (retention time 1.90 min)
MS (ESI+, m/e) 507 (M+H)

Example 169

3-(2-{[4-(2,3-dihydro-1-benzofuran-5-yl)-4-oxobutanoyl]amino}-6-phenylpyridin-4-yl)benzoic acid

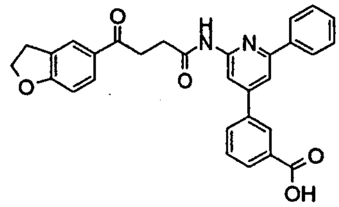

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and 3-(dihydroxyboryl)benzoic acid (20.7 mg) as starting materials and in the same manner as in Example 119, 3-(2-{[4-(2,3-dihydro-1-benzofuran-5-yl)-4-oxobutanoyl]amino}-6-phenylpyridin-4-yl)benzoic acid (1.9 mg) was obtained.
HPLC (220 nm) purity 93% (retention time 2.10 min)
MS (ESI+, m/e) 493 (M+H)

Example 170

methyl 3-(2-{[4-(2,3-dihydro-1-benzofuran-5-yl)-4-oxobutanoyl]amino}-6-phenylpyridin-4-yl)benzoate

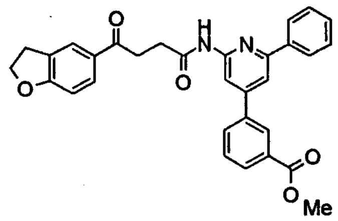

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(methoxycarbonyl)phenyl]boronic acid (22.5 mg) as starting materials and in the same manner as in Example 119, methyl 3-(2-{[4-(2,3-dihydro-1-benzofuran-5-yl)-4-oxobutanoyl]amino}-6-phenylpyridin-4-yl)benzoate (13.6 mg) was obtained.
HPLC (220 nm) purity 99% (retention time 2.09 min)
MS (ESI+, m/e) 507 (M+H)

Example 171

N-[4-(3-acetylphenyl)-6-phenylpyridin-2-yl]-4-(2,3-dihydro-1-benzofuran-5-yl)-4-oxobutanamide

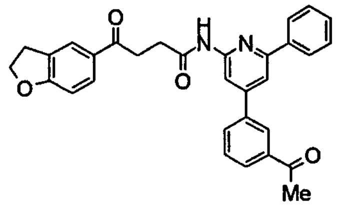

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3-acetylphenyl)boronic acid (20.5 mg) as starting materials and in the same manner as in Example 119, N-[4-(3-acetylphenyl)-6-phenylpyridin-2-yl]-4-(2,3-dihydro-1-benzofuran-5-yl)-4-oxobutanamide (12.4 mg) was obtained.
HPLC (220 nm) purity 99% (retention time 2.02 min)
MS (ESI+, m/e) 491 (M+H)

Example 172

3-[3-(2-{[4-(2,3-dihydro-1-benzofuran-5-yl)-4-oxobutanoyl]amino}-6-phenylpyridin-4-yl)phenyl]propanoic acid

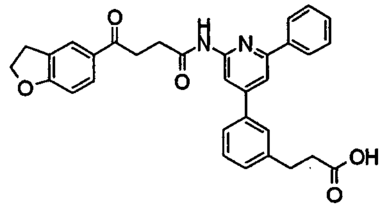

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(2-carboxyethyl)phenyl]boronic acid (24.2 mg) as starting materials and in the same manner as in Example 119, 3-[3-(2-{[4-(2,3-dihydro-1-benzofuran-5-yl)-4-oxobutanoyl]amino}-6-phenylpyridin-4-yl)phenyl]propanoic acid (9.7 mg) was obtained.
HPLC (220 nm) purity 90% (retention time 1.89 min)
MS (ESI+, m/e) 521 (M+H)

Example 173

methyl 3-[3-(2-{[4-(2,3-dihydro-1-benzofuran-5-yl)-4-oxobutanoyl]amino}-6-phenylpyridin-4-yl)phenyl]propanoate

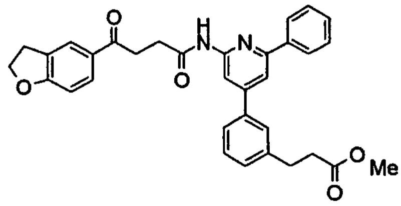

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and [3-(2-methoxycarboxyethyl)phenyl]boronic acid (26.0 mg) as starting materials and in the same manner as in Example 119, methyl 3-[3-(2-{[4-(2,3-dihydro-1-benzofuran-5-yl)-4-oxobutanoyl]amino}-6-phenylpyridin-4-yl)phenyl]propanoate (15.6 mg) was obtained. HPLC (220 nm) purity 96% (retention time 2.05 min)
MS (ESI+, m/e) 535 (M+H)

Example 174

(2E)-3-[3-(2-{[4-(2,3-dihydro-1-benzofuran-5-yl)-4-oxobutanoyl]amino}-6-phenylpyridin-4-yl)phenyl]acrylic acid

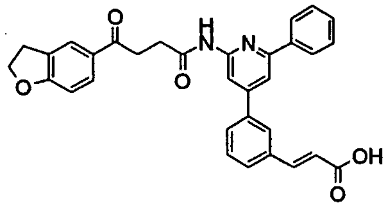

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (2E)-3-[3-(dihydroxyboryl)phenyl]acrylic acid (24.0 mg) as starting materials and in the same manner as in Example 119, (2E)-3-[3-(2-{[4-(2,3-dihydro-1-benzofuran-5-yl)-4-oxobutanoyl]amino}-6-phenylpyridin-4-yl)phenyl]acrylic acid (4.4 mg) was obtained. HPLC (220 nm) purity 96% (retention time 1.91 min)
MS (ESI+, m/e) 519 (M+H)

Example 175

4-(2,3-dihydro-1-benzofuran-5-yl)-4-oxo-N-{6-phenyl-4-[3-(trifluoromethoxy)phenyl]pyridin-2-yl}butanamide

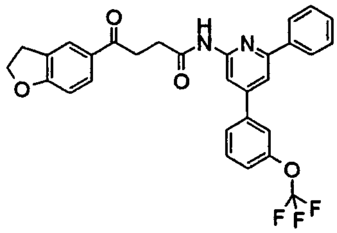

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8

mg), 1N aqueous potassium carbonate solution (0.1 mL) and [3-(trifluoromethoxy)phenyl]boronic acid (25.7 mg) as starting materials and in the same manner as in Example 119, 4-(2,3-dihydro-1-benzofuran-5-yl)-4-oxo-N-{6-phenyl-4-[3-(trifluoromethoxy)phenyl]pyridin-2-yl}butanamide (14.1 mg) was obtained.
HPLC (220 nm) purity 97% (retention time 2.23 min)
MS (ESI+, m/e) 533 (M+H)

Example 176

N-[4-(2-cyanophenyl)-6-phenylpyridin-2-yl]-4-(2,3-dihydro-1-benzofuran-5-yl)-4-oxobutanamide

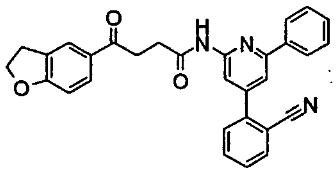

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (2-cyanophenyl)boronic acid (18.4 mg) as starting materials and in the same manner as in Example 119, N-[4-(2-cyanophenyl)-6-phenylpyridin-2-yl]-4-(2,3-dihydro-1-benzofuran-5-yl)-4-oxobutanamide (7.8 mg) was obtained.
HPLC (220 nm) purity 88% (retention time 2.04 min)
MS (ESI+, m/e) 474 (M+H)

Example 177

4-(2,3-dihydro-1-benzofuran-5-yl)-N-[4-(2-hydroxyphenyl)-6-phenylpyridin-2-yl]-4-oxobutanamide

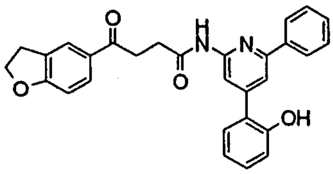

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (2-hydroxyphenyl)boronic acid (17.2 mg) as starting materials and in the same manner as in Example 119, 4-(2,3-dihydro-1-benzofuran-5-yl)-N-[4-(2-hydroxyphenyl)-6-phenylpyridin-2-yl]-4-oxobutanamide (10.6 mg) was obtained.
HPLC (220 nm) purity 81% (retention time 1.86 min)
MS (ESI+, m/e) 465 (M+H)

Example 178

4-(2,3-dihydro-1-benzofuran-5-yl)-N-[4-(2-methoxyphenyl)-6-phenylpyridin-2-yl]-4-oxobutanamide

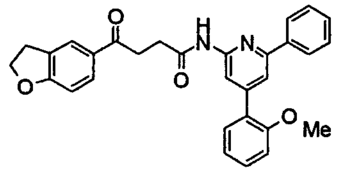

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (2-methoxyphenyl)boronic acid (19.0 mg) as starting materials and in the same manner as in Example 119, 4-(2,3-dihydro-1-benzofuran-5-yl)-N-[4-(2-methoxyphenyl)-6-phenylpyridin-2-yl]-4-oxobutanamide (14.0 mg) was obtained.
HPLC (220 nm) purity 96% (retention time 1.99 min)
MS (ESI+, m/e) 479 (M+H)

Example 179

4-(2,3-dihydro-1-benzofuran-5-yl)-N-{4-[2-(hydroxymethyl)phenyl]-6-phenylpyridin-2-yl}-4-oxobutanamide

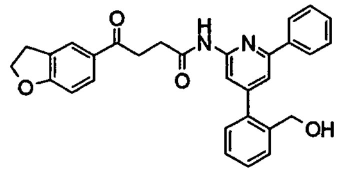

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and [2-(hydroxymethyl)phenyl]boronic acid (19.0 mg) as starting materials and in the same manner as in Example 119, 4-(2,3-dihydro-1-benzofuran-5-yl)-N-{4-[2-(hydroxymethyl)phenyl]-6-phenylpyridin-2-yl}-4-oxobutanamide (6.5 mg) was obtained.
HPLC (220 nm) purity 99% (retention time 1.88 min)
MS (ESI+, m/e) 479 (M+H)

Example 180

2-(2-{[4-(2,3-dihydro-1-benzofuran-5-yl)-4-oxobutanoyl]amino}-6-phenylpyridin-4-yl)benzoic acid

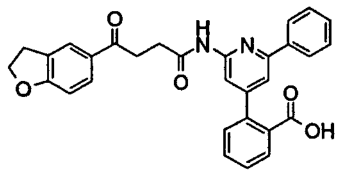

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and 2-(dihydroxyboryl)benzoic acid (20.7 mg) as starting materials and in the same manner as in Example 119, 2-(2-{[4-(2,3-dihydro-1-benzofuran-5-yl)-4-oxobutanoyl]amino}-6-phenylpyridin-4-yl)benzoic acid (2.6 mg) was obtained.

HPLC (220 nm) purity 92% (retention time 1.86 min)
MS (ESI+, m/e) 493 (M+H)

Example 181

4-(2,3-dihydro-1-benzofuran-5-yl)-N-[4-(4-hydroxy-3-methoxyphenyl)-6-phenylpyridin-2-yl]-4-oxobutanamide

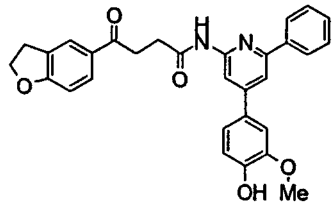

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and 2-(4-hydroxy-3-methoxyphenyl)-4,4,5,5-tetramethyl-1,3,2-dioxaborolan (31.3 mg) as starting materials and in the same manner as in Example 119,
4-(2,3-dihydro-1-benzofuran-5-yl)-N-[4-(4-hydroxy-3-methoxyphenyl)-6-phenylpyridin-2-yl]-4-oxobutanamide (13.3 mg) was obtained.
HPLC (220 nm) purity 91% (retention time 1.79 min)
MS (ESI+, m/e) 495 (M+H)

Example 182

4-(2,3-dihydro-1-benzofuran-5-yl)-4-oxo-N-[6-phenyl-4-(3,4,5-trimethoxyphenyl)pyridin-2-yl]butanamide

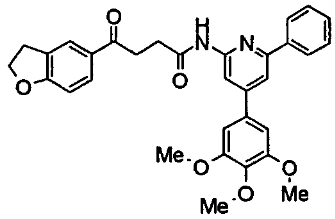

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (3,4,5-trimethoxyphenyl)boronic acid (26.5 mg) as starting materials and in the same manner as in Example 119, 4-(2,3-dihydro-1-benzofuran-5-yl)-4-oxo-N-[6-phenyl-4-(3,4,5-trimethoxyphenyl)pyridin-2-yl]butanamide (17.7 mg) was obtained. HPLC (220 nm) purity 93% (retention time 1.98 min)
MS (ESI+, m/e) 539 (M+H)

Example 183

4-(2,3-dihydro-1-benzofuran-5-yl)-N-[4-(4-hydroxy-3,5-dimethylphenyl)-6-phenylpyridin-2-yl]-4-oxobutanamide

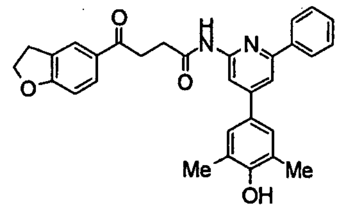

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and 2,6-dimethyl-4-(4,4,5,5-tetramethyl-1,3,2-dioxaborolan-2-yl)phenol (31.0 mg) as starting materials and in the same manner as in Example 119, 4-(2,3-dihydro-1-benzofuran-5-yl)-N-[4-(4-hydroxy-3,5-dimethylphenyl)-6-phenylpyridin-2-yl]-4-oxobutanamide (7.3 mg) was obtained.
HPLC (220 nm) purity 94% (retention time 1.86 min)
MS (ESI+, m/e) 493 (M+H)

Example 184

4-(2,3-dihydro-1-benzofuran-5-yl)-N-{4-[(1E)-octa-1-en-1-yl]-6-phenylpyridin-2-yl}-4-oxobutanamide

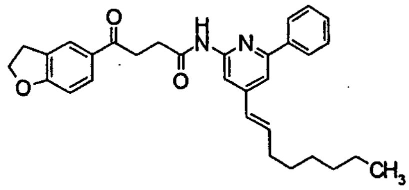

Using N-(4-chloro-6-phenylpyridin-2-yl)-4-(2,3-dihydro-1-benzofuran-5-yl)-4-oxobutanamide obtained in Example 114-(1) (20.3 mg), tris(dibenzylideneacetone)dipalladium (1.1 mg), biphenyl-2-yl(dicyclohexyl)phosphine (1.8 mg), 1N aqueous potassium carbonate solution (0.1 mL) and (1E)-octa-1-en-1-ylboronic acid (19.5 mg) as starting materials and in the same manner as in Example 119, 4-(2,3-dihydro-1-benzofuran-5-yl)-N-{4-[(1E)-octa-1-en-1-yl]-6-phenylpyridin-2-yl}-4-oxobutanamide (5.4 mg) was obtained.
HPLC (220 nm) purity 90% (retention time 2.21 min)
MS (ESI+, m/e) 483 (M+H)

**Formulation Example 1** (production of capsules)

| | | |
|---|---|---|
| 1) | compound of Example 4 | 30 mg |
| 2) | fine cellulose powder | 10 mg |
| 3) | lactose | 19 mg |
| 4) | magnesium stearate | 1 mg |
| | total | 60 mg |

1), 2), 3) and 4) are mixed and filled in gelatin capsules.

**Formulation Example 2** (production of tablets)

| | | |
|---|---|---|
| 1) | compound of Example 4 | 30 g |
| 2) | lactose | 50 g |
| 3) | corn starch | 15 g |
| 4) | carboxymethylcellulose calcium' | 44 g |
| 5) | magnesium stearate | 1 g |

(continued)

| **Formulation Example 2** (production of tablets) | |
|---|---|
| total of 1000 tablets | 140 g |
| The entire amounts of 1), 2) and 3), and 30 g of 4) are kneaded with water, dried in vacuo and granulated. | |

The granules are mixed with 14 g of 4) and 1 g of 5) and the mixture is compressed with a tableting machine, whereby 1000 tablets containing 30 mg of compound of Example 4 per tablet are obtained.

## Experimental Example 1

The genetic engineering described below followed the method described in a book (Maniatis et al., Molecular Cloning, Cold Spring Harbor Laboratory (1989)) or a method described in the protocol attached to the reagents.

(1) Cloning of human DGAT1 gene and preparation of recombinant baculovirus
Human DGAT1 gene was cloned by PCR using human adipocyte cDNA (Clontech, QUICK-Clone cDNA, human fat cell, cat# 637220) as a template and, based on the DGAT1 gene information reported by Case, S. et al. (Proc. Natl. Acad. Sci. U.S.A. 95 (22), 13018-13023 (1998)), a base sequence (245-1711 of Genbank Accession No. NM_ 012079) encoding DGAT1 was amplified with the following PCR primer. The primer base sequence is shown below.
DGAT1-U:

5' AATTAAGAATTCATGGGCGACTACAAAGACGATGACGACGGCGACCGCGGCAGCTCCCGGCGCC GG 3' (SEQ ID NO:1), and

DGAT2-L:

5' AATTAAACTAGTTCAGGCCTCTGCCGCTGGGGCCTCATAGTTGAG 3' (SEQ ID NO:2)

The PCR reaction was conducted using a KOD-plus kit (TOYOBO). The obtained PCR product was electrophoresed on agarose gel (1%), the DNA fragment amplified by PCR was recovered from the gel, and then digested with restriction enzymes EcoRI and SpeI. The DNA treated with the restriction enzymes was electrophoresed on agarose gel (1%), and the obtained DNA fragment was recovered and ligated with plasmid pFASTBAC1 (Invitrogen) digested with restriction enzymes EcoRI and SpeI to give expression plasmid pFB-DGAT1. The nucleotide sequence of the inserted fragment was confirmed and found to be identical with the gene sequence encoding the base sequence of DGAT1 (245-1711 of Genbank Accession No. NM 012079). Furthermore, using BAC-TO-BAC Baculovirus Expression System (Invitrogen), recombinant baculovirus BAC-DGAT1 was prepared.
(2) Preparation of microsome of Sf9 insect cells highly expressing DGAT1 enzyme
SF9 cells were sown at $1\times10^6$ cells/ml on Sf-900II SFM medium (1 L, Invitrogen) containing 10% fetal calf serum (Trace), 50 mg/L gentamicin (Invitrogen) and 0.1% Pluronic F-68 (Invitrogen), and shaking culture was performed using a 2 L volume Erlenmeyer at 27°C, 100 rpm. After culturing for 24 hr, recombinant baculovirus BAC-DGAT1 (6.7 mL) was added, and the mixture was further cultured for 3 days. The culture medium was centrifuged at 2,000 rpm for 5 min to give virus-infected cells. The infected cells were washed with a phosphate buffered saline (Invitrogen), centrifuged under the same conditions, and the cells were preserved at -80°C. The cryopreserved cells were thawed in ice, suspended in buffer A (50 mM Tris buffer (30 mL, pH 7.4) containing 20% glycerol, 0.15 M NaCl) supplemented with Complete Protease Inhibitor (Boehringer), and ruptured 3 times with a Polytron homogenizer (Kinematica) at 20,000 rpm for 30 sec. The Sf9 microsome fractions were obtained by a conventional method and cryopreserved at -80°C as a DGAT1 high expression Sf9 microsome.
(3) Determination of DGAT inhibitory activity

As a DGAT1 reaction buffer, a solution having a composition of 100 mM Tris-HCl (pH 7.5), 250 mM sucrose, 150 mM $MgCl_2$, 0.01% bovine serum albumin (BSA) was used. Using this buffer, a given concentration of the test compound and a composition (100 μl) of 25 μM dioleoylglycerol, 25 μM [14C]-Oleoyl-CoA, 5 μg protein/ml DGAT1 high expression Sf9 microsome, and 1% acetone were subjected to a triglyceride synthesis reaction at 32°C for 15 min. A mixture of 300 μL of chloroform:methanol (=1:2) was added to the reaction mixture to quench the reaction. The reaction mixture was sufficiently mixed and saline (200 μL) was added to partition the mixture between a chloroform layer (lower

layer) and an aqueous layer (upper layer). The chloroform layer (50 μL) was spotted on a thin layer chromatography silica gel plate (TLC plate, Merck) and developed with a solvent (n-hexane:diethyl ether:ethyl acetate:acetic acid =74: 15:15:1). The developed TLC plate was dried, contacted with a BAS imaging plate (manufactured by FUJIFILM) and measured with BAS2500 (manufactured by FUJIFILM) 16 hr later to numerically show the amount of [$^{14}$C] -triglyceride (TG amount) produced during the reaction. The inhibitory rate was calculated by the following formula:

Inhibitory rate (%) = (1-(TG amount in the case with addition of test compound - blank TG amount)/(control TG amount - blank TG amount))×100

The count of the triglyceride produced in the solution reacted without addition of the test compound was used as a "control TG amount", and the count of the triglyceride produced in the solution reacted without addition of the test compound and DGAT1 high expression Sf9 microsome was used as a "blank TG amount". In addition, the concentration ($IC_{50}$) of the test compound necessary for inhibiting the triglyceride synthesis by 50% was calculated by PRISM 3.02 (manufactured by GraphPad Software). The inhibitory activity is shown in Table 1 and Table2.

The inhibitory activity is shown by A ≤ 10nM < B ≤ 50nM < C ≤ 500nM < D ≤ 10000nM according to $IC_{50}$.

Table 1 DGAT inhibitory activity

| Ex. No | inhibitory activity | | Ex. No | inhibitory activity |
|---|---|---|---|---|
| 1 | A | | 34 | C |
| 2 | C | | 35 | D |
| 3 | A | | 36 | A |
| 4 | A | | 37 | C |
| 5 | A | | 38 | D |
| 6 | B | | 39 | D |
| 7 | A | | 40 | C |
| 8 | C | | 41 | C |
| 9 | C | | 42 | C |
| 10 | D | | 43 | C |
| 11 | B | | 44 | C |
| 12 | D | | 45 | C |
| 13 | C | | 46 | C |
| 14 | C | | 47 | B |
| 15 | C | | 48 | B |
| 16 | C | | 49 | B |
| 17 | B | | 50 | B |
| 18 | C | | 51 | C |
| 19 | B | | 52 | C |
| 20 | C | | 53 | C |
| 21 | D | | 54 | C |
| 22 | A | | 55 | C |
| 23 | D | | 56 | C |
| 24 | B | | 57 | A |

(continued)

| Ex. No | inhibitory activity | | Ex. No | inhibitory activity |
|---|---|---|---|---|
| 25 | A | | 58 | C |
| 26 | C | | 59 | C |
| 27 | A | | 60 | D |
| 28 | C | | 61 | B |
| 29 | B | | 62 | C |
| 30 | B | | 63 | C |
| 31 | C | | 64 | C |
| 32 | B | | 65 | B |
| 33 | C | | | |

Table 2 DGAT inhibitory activity

| Ex. No | inhibitory activity | | Ex. No | inhibitory activity | | Ex. No | inhibitory activity |
|---|---|---|---|---|---|---|---|
| 66 | D | | 106 | C | | 146 | A |
| 67 | C | | 107 | C | | 147 | A |
| 68 | B | | 108 | C | | 148 | D |
| 69 | C | | 109 | D | | 149 | B |
| 70 | C | | 110 | D | | 150 | D |
| 71 | A | | 111 | D | | 151 | B |
| 72 | B | | 112 | D | | 152 | D |
| 73 | C | | 113 | D | | 153 | D |
| 74 | A | | 114 | C | | 154 | B |
| 75 . | C | | 115 | C | | 155 | C |
| 76 | B | | 116 | D | | 156 | C |
| 77 | B | | 117 | C | | 157 | D |
| 78 | C | | 118 | D | | 158 | C |
| 79 | B | | 119 | C | | 159 | D |
| 80 | B | | 120 | C | | 160 | D |
| 81 | C | | 121 | A | | 161 | D |
| 82 | C | | 122 | C | | 162 | C |
| 83 | C | | 123 | A | | 163 | C |
| 84 | D | | 124 | B | | 164 | B |
| 85 | D | | 125 | D | | 165 | B |
| 86 | B | | 126 | C | | 166 | D |
| 87 | B | | 127 | D | | 167 | C |
| 88 | C | | 128 | B | | 168 | C |
| 89 | C | | 129 | D | | 169 | D |
| 90 | C | | 130 | D | | 170 | D |
| 91 | C | | 131 | C | | 171 | C |

(continued)

| Ex. No | inhibitory activity | | Ex. No | inhibitory activity | | Ex. No | inhibitory activity |
|---|---|---|---|---|---|---|---|
| 92 | C | | 132 | C | | 172 | D |
| 93 | C | | 133 | B | | 173 | B |
| 94 | C | | 134 | B | | 174 | D |
| 95 | C | | 135 | D | | 175 | C |
| 96 | C | | 136 | C | | 176 | A |
| 97 | C | | 137 | C | | 177 | B |
| 98 | C | | 138 | D | | 178 | B |
| 99 | C | | 139 | D | | 179 | C |
| 100 | D | | 140 | C | | 180 | D |
| 101 | D | | 141 | D | | 181 | B |
| 102 | C | | 142 | C | | 182 | D |
| 103 | D | | 143 | C | | 183 | C |
| 104 | C | | 144 | A | | 184 | D |
| 105 | C | | 145 | A | | | |

**Experimental Example 2**

The compound of Example 4 (Laborasol solution) was orally administered (4 mL/kg) to 13-week-old female KKAy mouse at 10, 30, 100 mg/kg. One hr later, [1-$^{14}$C]-oleic acid/BSA complex (12% BSA/PBS solution) was administered as a tracer at 18.5 MBq/kg/5 mL from the tail vein under ether anesthesia. One hr after the administration, the mouse was sacrificed by exsanguination, and an adipose tissue (fat surrounding ovary) and the skeletal muscle (gastrocnemial muscle) were isolated. Each tissue (0.30 g) was weighed and shredded, [9,10(n)-$^{3}$H]-triolein (about 100 kBq) was added as the external standard, and a 20% homogenate was prepared using a homogenizer (homogenate buffer: 0.25M Sucrose, 50 mM KC1, 5 mM $MgCl_2$, 1 mM EDTA, 50 mM Tris/HCl, pH 7.6). The homogenate (1.5 mL) was taken, chloroform:methanol (1:2) (4.5 mL) was added and the mixture was shaken at room temperature for 30 min. Chloroform (1.5 mL) and distilled water (1.8 mL) were further added and the mixture was shaken at room temperature for 30 min. The resulting shaken solution was centrifuged at 500 x g for 5 min. The lower layer (chloroform layer) was recovered, dried under a nitrogen gas, and redissolved in chloroform:methanol (2:1, 2.0 mL). The obtained solution (5 $\mu$L) was spotted on a TLC plate, developed with hexane:diethyl ether:ethyl acetate:acetic acid (80:10:10:0.13), and exposed to a BAS imaging for 5 days. The radioactivity of TG spot was measured by BAS2500 and the ratio relative to the radioactivity of the external standard ([$^{3}$H]-triolein) of the resultant product ([$^{14}$C]-TG) was calculated.

As a result, the compound of Example 4 reduced the [1-$^{14}$C]-oleic acid intake into the TG fraction in the adipose tissue and skeletal muscle in a dose-dependent manner. (shown in Fig. 1 and Fig. 2)

The compound of Example 4 inhibited DGAT1 in the mouse and reduced TG production in the adipose tissue and skeletal muscle. From these results, the compound of the present invention can be said to be useful as an agent for the prophylaxis or treatment of obesity, hyperlipidemia, diabetes or metabolic syndrome.

**Industrial Applicability**

The compounds of the present invention have a DGAT inhibitory action and are useful for the treatment or amelioration of DGAT-related diseases.

This application is based on patent application No. 25713/2005 filed in Japan, the contents of which are hereby incorporated by reference.

## Claims

1. A compound represented by the formula (I):

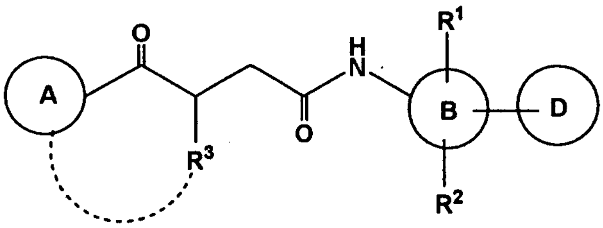

(I)

wherein
ring A is an optionally substituted ring (the ring should not be pyrrolidine, piperidine and piperazine),
ring B is an optionally substituted aromatic ring,
ring D is an optionally substituted ring (ring D should not be a benzene ring substituted by acylalkylcarbonylamino group(s)),
$R^1$ and $R^2$ are each independently a hydrogen atom or a substituent, and
$R^3$ is a hydrogen atom or a $C_{1-6}$ alkyl group, or $R^3$ is bonded to ring A to form a non-aromatic ring,

or a salt thereof,
provided that
N-[4-(4-fluorophenyl)-5-methyl-1,3-thiazol-2-yl]-4-(4-methoxyphenyl)-4-oxobutanamide,
N-[4-(4-methoxyphenyl)-5-methyl-1,3-thiazol-2-yl]-4-oxo-4-phenylbutanamide,
4-(4-tert-butylphenyl)-N-[4-(4-methylphenyl)-5-propyl-1,3-thiazol-2-yl]-4-oxobutanamide,
N-[4-(2,5-dimethoxyphenyl)-5-methyl-1,3-thiazol-2-yl]-4-(6-methyl-2-naphthyl)-4-oxobutanamide,
N-(biphenyl-2-yl)-4-oxo-4-phenylbutanamide,
N-(biphenyl-2-yl)-4-(4-methylphenyl)-4-oxobutanamide,
N-(biphenyl-2-yl)-4-(4-fluorophenyl)-4-oxobutanamide,
N-[4-(4-methylphenyl)-1,3-thiazol-2-yl]-4-oxo-4-(2-thienyl)butanamide,
4-(4-chlorophenyl)-N-[4-(4-fluorophenyl)-1,3-thiazol-2-yl]-4-oxobutanamide,
4-(2,5-dimethylphenyl)-N-[4-(4-fluorophenyl)-5-methyl-1,3-thiazol-2-yl]-4-oxobutanamide,
N-[4-(4-fluorophenyl)-5-methyl-1,3-thiazol-2-yl]-4-oxo-4-phenylbutanamide,
4-(4-fluorophenyl)-N-[4-(4-methoxyphenyl)-5-methyl-1,3-thiazol-2-yl]-4-oxobutanamide, and
N-[4-(4-methoxyphenyl)-5-phenyl-1,3-thiazol-2-yl]-4-oxo-4-phenylbutanamide
are excluded.

**2.** The compound of claim 1, wherein the ring for ring A is a benzene ring, tetrahydronaphthalene or dihydrobenzofuran.

**3.** The compound of claim 1, wherein the aromatic ring for ring B is a benzene ring, pyridine, pyrimidine, quinoline, isoquinoline, pyrrole, pyrazole, thiophene or thiazole.

**4.** The compound of claim 1, wherein the ring for ring D is a benzene ring.

**5.** The compound of claim 1, wherein ring D is a benzene ring optionally substituted by 1 to 3 substituents selected from

(1) a halogen atom;
(2) a $C_{1-6}$ alkyl group optionally substituted by 1 to 3 substituents selected from a hydroxy group, a $C_{1-6}$ alkoxy-carbonyl group, a carboxy group, a cyano group and a non-aromatic heterocyclic group;
(3) a $C_{2-6}$ alkenyl group optionally substituted by 1 to 3 substituents selected from a $C_{1-6}$ alkoxy-carbonyl group and a carboxy group;
(4) a $C_{1-6}$ alkoxy group optionally substituted by 1 to 3 substituents selected from

(i) a halogen atom,
(ii) a carboxy group,
(iii) a $C_{1-6}$ alkoxy group,
(iv) a $C_{1-6}$ alkoxy-carbonyl group,
(v) an amino group optionally mono- or di-substituted by substituent(s) selected from a $C_{1-6}$ alkyl group and a $C_{1-6}$ alkoxy-carbonyl group, and
(iv) a non-aromatic heterocyclic group;

(5) a $C_{3-10}$ cycloalkyl group;
(6) a $C_{1-3}$ alkylenedioxy group;
(7) a hydroxy group;
(8) a $C_{1-6}$ alkyl-carbonyl group;
(9) a $C_{1-6}$ alkoxy-carbonyl group;
(10) a carboxy group;
(11) a cyano group;
(12) an amino group optionally mono- or di-substituted by $C_{1-6}$ alkyl group(s);
(13) a carbamoyl group; and
(14) a non-aromatic heterocyclic group.

**6.** The compound of claim 1, wherein the substituent for $R^1$ or $R^2$ is an optionally substituted hydrocarbon group, an optionally substituted heterocyclic group, an optionally substituted hydroxy group, an acyl group or a halogen atom.

**7.** The compound of claim 1, which is
N-(5-benzyl-4-phenyl-1,3-thiazol-2-yl)-4-(4-ethoxyphenyl)-4-oxobutanamide;
N-(5-benzyl-4-phenyl-1,3-thiazol-2-yl)-4-oxo-4-phenylbutanamide;

N-(5-benzyl-4-phenyl-1,3-thiazol-2-yl)-4-(3,4-diethoxyphenyl)-4-oxobutanamide;
N-(5-benzyl-4-phenyl-1,3-thiazol-2-yl)-4-(4,5-diethoxy-2-methylphenyl)-4-oxobutanamide;
N-(5-benzyl-4-phenyl-1,3-thiazol-2-yl)-4-(3-ethoxyphenyl)-4-oxobutanamide;
4-(3,4-diethoxyphenyl)-N-(4,6-diphenylpyridin-2-yl)-4-oxobutanamide;
N-(5-benzyl-4-phenyl-1,3-thiazol-2-yl)-4-(3-bromo-4-ethoxyphenyl)-4-oxobutanamide;
N-(5-benzyl-4-phenyl-1,3-thiazol-2-yl)-4-(3-chloro-4-ethoxyphenyl)-4-oxobutanamide;
N-(5-benzyl-4-phenyl-1,3-thiazol-2-yl)-4-(3,4-dimethoxyphenyl)-4-oxobutanamide;
4-(3,4-diethoxyphenyl)-N-[4-(4-hydroxyphenyl)-6-phenylpyridin-2-yl]-4-oxobutanamide;
N-[4-(2-cyanophenyl)-6-phenylpyridin-2-yl]-4-(3,4-diethoxyphenyl)-4-oxobutanamide;
4-(3,4-diethoxyphenyl)-N-[4-(2-hydroxyphenyl)-6-phenylpyridin-2-yl]-4-oxobutanamide;
4-(3,4-diethoxyphenyl)-N-[4-(2-methoxyphenyl)-6-phenylpyridin-2-yl]-4-oxobutanamide; or
4-(3,4-diethoxyphenyl)-N-{4-[2-(hydroxymethyl)phenyl]-6-phenylpyridin-2-yl}-4-oxobutanamide.

**8.** A prodrug of the compound of claim 1.

**9.** A pharmaceutical agent comprising the compound of claim 1 or a prodrug thereof.

**10.** The pharmaceutical agent of claim 9, which is an agent for the prophylaxis or treatment of obesity, hyperlipidemia or diabetes.

**11.** A DGAT inhibitor comprising the compound of claim 1 or a prodrug thereof.

**12.** Use of the compound of claim 1 or a prodrug thereof for the production of an agent for the prophylaxis or treatment of obesity, hyperlipidemia or diabetes.

**13.** Use of the compound of claim 1 or a prodrug thereof for the production of a DGAT inhibitor.

**14.** A method for the prophylaxis or treatment of obesity, hyperlipidemia or diabetes in a mammal, which comprises administering the compound of claim 1 or a prodrug thereof to the mammal.

**15.** A method of inhibiting DGAT in a mammal, which comprises administering the compound of claim 1 or a prodrug thereof to the mammal.

**16.** A compound represented by the formula (II):

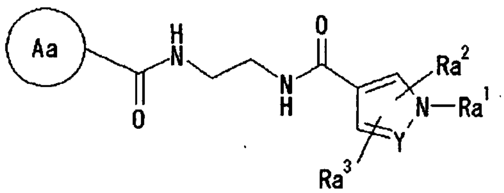

(II)

wherein
ring Aa is an optionally substituted aromatic hydrocarbon,
Y is CH or N,
$Ra^1$ is an optionally substituted hydrocarbon group, and
$Ra^2$ and $Ra^3$ are each independently a hydrogen atom or a substituent,
or a salt thereof.

**17.** The compound of claim 16, wherein the substituent for $Ra^2$ or $Ra^3$ is an optionally substituted hydrocarbon group.

**18.** The compound of claim 16, which is
N-{2-[(4-ethoxybenzoyl)amino]ethyl}-1-phenyl-3-(trifluoromethyl)-1H-pyrazole-4-carboxamide;
N-{2-[(4-ethoxybenzoyl)amino]ethyl}-1-(4-methoxyphenyl)-2-methyl-5-phenyl-1H-pyrrole-3-carboxamide; or
N-{2-[(4-ethoxybenzoyl)amino]ethyl}-2-methyl-1-[4-(methylthio)phenyl]-5-phenyl-1H-pyrrole-3-carboxamide.

**19.** A prodrug of the compound of claim 16.

**20.** A pharmaceutical agent comprising the compound of claim 16 or a prodrug thereof.

**21.** The pharmaceutical agent of claim 20, which is an agent for the prophylaxis or treatment of obesity, hyperlipidemia or diabetes;

**22.** A DGAT inhibitor comprising the compound of claim 16 or a prodrug thereof.

**23.** Use of the compound of claim 16 or a prodrug thereof for the production of an agent for the prophylaxis or treatment of obesity, hyperlipidemia or diabetes.

**24.** Use of the compound of claim 16 or a prodrug thereof for the production of a DGAT inhibitor.

**25.** A method for the prophylaxis or treatment of obesity, hyperlipidemia or diabetes in a mammal, which comprises administering the compound of claim 16 or a prodrug thereof to the mammal.

**26.** A method of inhibiting DGAT in a mammal, which comprises administering the compound of claim 16 or a prodrug thereof to the mammal.

FIG. 1

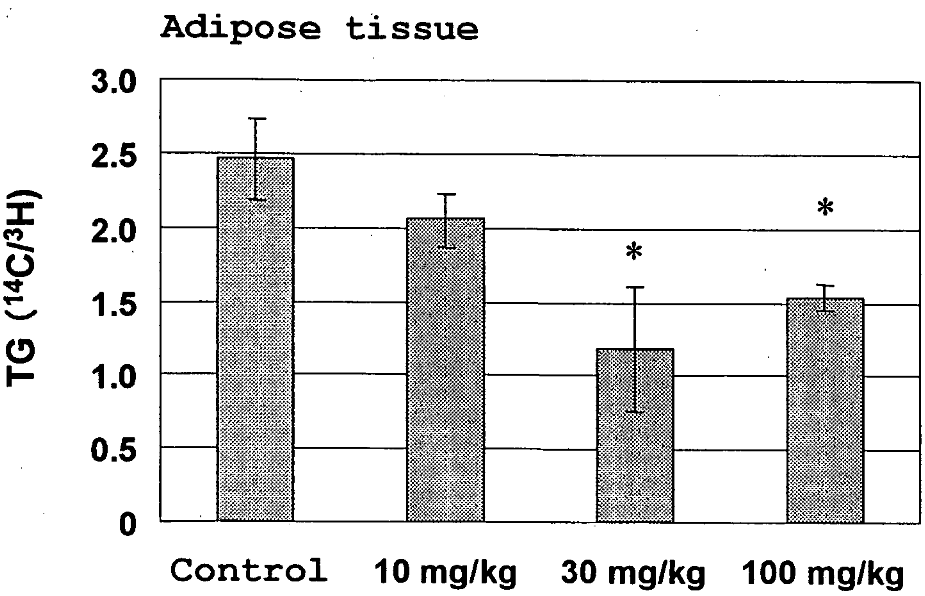
Adipose tissue
TG ($^{14}C/^{3}H$)
3.0
2.5
2.0
1.5
1.0
0.5
0
*
*
Control
10 mg/kg
30 mg/kg
100 mg/kg

FIG. 2

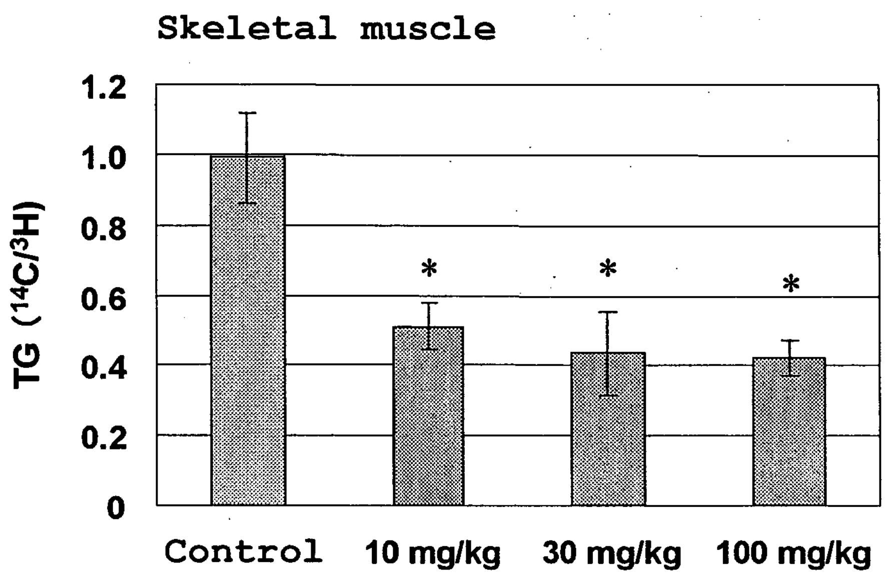
Skeletal muscle
1.2
1.0
0.8
0.6
0.4
0.2
0
TG ($^{14}C/^{3}H$)
*
*
*
Control
10 mg/kg
30 mg/kg
100 mg/kg

**INTERNATIONAL SEARCH REPORT**

International application No.
PCT/JP2006/301942

A. CLASSIFICATION OF SUBJECT MATTER
***C07C235/78***(2006.01), ***A61K31/167***(2006.01), ***A61K31/196***(2006.01), ***A61K31/216***(2006.01), ***A61K31/381***(2006.01), ***A61K31/40***(2006.01), ***A61K31/415***(2006.01), ***A61K31/426***(2006.01),

According to International Patent Classification (IPC) or to both national classification and IPC

B. FIELDS SEARCHED

Minimum documentation searched (classification system followed by classification symbols)
***C07C235/78***(2006.01), ***A61K31/167***(2006.01), ***A61K31/196***(2006.01), ***A61K31/216***(2006.01), ***A61K31/381***(2006.01), ***A61K31/40***(2006.01), ***A61K31/415***(2006.01), ***A61K31/426***(2006.01),

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | US 2005/0154020 A1 (Synaptic Pharmaceutical Corp.), 14 July, 2005 (14.07.05), pages 1 to 7 & WO 2005/69834 A2 | 1-13,16-24 |
| P,X | US 2005/0154022 A1 (H. Lundbeck A/S), 14 July, 2005 (14.07.05), pages 1 to 8 (Family: none) | 1-13,16-24 |
| A | WO 2004/100881 A2 (Bayer Pharmaceuticals Corp.), 25 November, 2004 (25.11.04), pages 3 to 20 & US 2004/0224997 A1 | 1-13,16-24 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 March, 2006 (15.03.06) | 28 March, 2006 (28.03.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/301942

**Box No. II Observations where certain claims were found unsearchable (Continuation of item 2 of first sheet)**

This international search report has not been established in respect of certain claims under Article 17(2)(a) for the following reasons:

1. [x] Claims Nos.: 14-15, 25-26
because they relate to subject matter not required to be searched by this Authority, namely:
It pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
- [ ] The additional search fees were accompanied by the applicant's protest and, where applicable, payment of a protest fee..
- [ ] The additional search fees were accompanied by the applicant's protest but the applicable protest fee was not paid within the time limit specified in the invitation.
- [ ] No protest accompanied the payment of additional search fees.

Form PCT/ISA/210 (continuation of first sheet (2)) (April 2005)

INTERNATIONAL SEARCH REPORT

International application No.
PCT/JP2006/301942

Continuation of A. CLASSIFICATION OF SUBJECT MATTER
(International Patent Classification (IPC))

***A61K31/427***(2006.01), ***A61K31/44***(2006.01), ***A61K31/47***(2006.01), ***A61K31/505***(2006.01), ***A61K31/535***(2006.01), ***A61P1/00***(2006.01), ***A61P3/04***(2006.01), ***A61P3/06***(2006.01), ***A61P3/10***(2006.01), ***A61P9/00***(2006.01), ***A61P9/10***(2006.01), ***A61P13/12***(2006.01), ***A61P15/00***(2006.01), ***A61P19/10***(2006.01), ***A61P21/00***(2006.01), ***A61P25/00***(2006.01), ***A61P25/16***(2006.01), ***A61P25/28***(2006.01), ***A61P29/00***(2006.01), ***A61P35/00***(2006.01), ***A61P43/00***(2006.01), ***C07D207/34***(2006.01), ***C07D213/75***(2006.01), ***C07D215/38***(2006.01), ***C07D231/14***(2006.01), ***C07D231/38***(2006.01), ***C07D239/42***(2006.01), ***C07D277/28***(2006.01), ***C07D295/12***(2006.01), ***C07D333/36***(2006.01), ***C07D409/12***(2006.01), ***C07D417/12***(2006.01)

(According to International Patent Classification (IPC) or to both national classification and IPC)

Continuation of B. FIELDS SEARCHED
Minimum documentation searched (International Patent Classification (IPC))

***A61K31/427***(2006.01), ***A61K31/44***(2006.01), ***A61K31/47***(2006.01), ***A61K31/505***(2006.01), ***A61K31/535***(2006.01), ***A61P1/00***(2006.01), ***A61P3/04***(2006.01), ***A61P3/06***(2006.01), ***A61P3/10***(2006.01), ***A61P9/00***(2006.01), ***A61P9/10***(2006.01), ***A61P13/12***(2006.01), ***A61P15/00***(2006.01), ***A61P19/10***(2006.01), ***A61P21/00***(2006.01), ***A61P25/00***(2006.01), ***A61P25/16***(2006.01), ***A61P25/28***(2006.01), ***A61P29/00***(2006.01), ***A61P35/00***(2006.01), ***A61P43/00***(2006.01), ***C07D207/34***(2006.01), ***C07D213/75***(2006.01), ***C07D215/38***(2006.01), ***C07D231/14***(2006.01), ***C07D231/38***(2006.01), ***C07D239/42***(2006.01), ***C07D277/28***(2006.01), ***C07D295/12***(2006.01), ***C07D333/36***(2006.01), ***C07D409/12***(2006.01), ***C07D417/12***(2006.01)

Minimum documentation searched (classification system followed by classification symbols)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004067635 A
- WO 2004047755 A
- US 20040224997 A
- WO 9958510 A
- WO 0138325 A
- WO 0125228 A
- WO 0342204 A
- WO 9844921 A
- WO 9845285 A
- WO 9922735 A
- WO 0114372 A
- WO 9710224 A
- WO 0182925 A
- WO 0187834 A
- US 3239564 A
- JP 2005025713 A


### Non-patent literature cited in the description


- *Proc. Natl. Acad. Sci. USA.,* 1998, vol. 95, 13018-13023
- *The Journal of Biochemistry,* 2001, vol. 276 (42), 38862-38869
- *The Journal of Biochemistry,* 2001, vol. 276 (42), 38870-38876
- *Biochem. Journal,* 2001, vol. 359, 707-714
- *Nature Genetics,* 2000, vol. 25, 87-90
- *The Journal of Clinical Investigation,* 2002, vol. 109, 175-181
- *The Journal of Clinical Investigation,* 2002, vol. 109, 1049-1055
- *The Journal of Biochemistry,* December 2003, vol. 10, 1074
- *The Journal of Biochemistry,* 1999, vol. 274, 35577-35582
- Development of Pharmaceutical Products. Molecule Design. Hirokawa Shoten, 1990, vol. 7, 163-198
- Protective Groups in Organic Synthesis. John Wiley and Sons, 1980
- *Chem Ber,* 1961, vol. 94, 698-704
- *Tetrahedron,* 2002, vol. 58, 8581-8590
- *J. Org. Chem.,* 1984, vol. 49, 3314-3322
- *J. Med. Chem.,* 1983, vol. 26, 1499-1504
- *Chem. Pharm. Bull.,* 1988, vol. 36, 4389-4402
- *J. Org. Chem.,* 1980, vol. 45, 4767
- *J. Synth. Org. Chem. Jpn,* 1973, vol. 31, 328
- *J. Chem. Soc. Perkin Trans.,* 1972, vol. 1, 2524
- *Heterocycles,* 2000, vol. 53, 291
- **MANIATIS et al.** Molecular Cloning. Cold Spring Harbor Laboratory, 1989
- **CASE, S. et al.** *Proc. Natl. Acad. Sci. U.S.A.,* 1998, vol. 95 (22), 13018-13023